# EUROPEAN PATENT APPLICATION

(11) **EP 4 635 956 A1**
(43) Date of publication of application: **22.10.2025**
(21) Application number: 23902824.4
(22) Date of filing: 15.12.2023
(51) Int. Cl.: C07D 487/04, C07D 417/14, C07D 401/14, C07D 405/14, A61K 31/45, A61K 31/44, A61P 35/00

(54) **PROTAC CHIMERIC COMPOUND, PREPARATION METHOD THEREFOR AND USE THEREOF**

(30) Priority: 16.12.2022 CN 202211626637; 28.06.2023 CN 202310775874
(71) Applicant: Hangzhou Zhongmeihuadong Pharmaceutical Co., Ltd., GongShu District HangZhou, Zhejiang 310011 (CN)
(72) Inventor: ZHANG, Zhimin, Hangzhou, Zhejiang 310011 (CN); ZHAI, Wenqiang, Hangzhou, Zhejiang 310011 (CN); WU, Mengqiang, Hangzhou, Zhejiang 310011 (CN); WANG, Feng, Hangzhou, Zhejiang 310011 (CN); CHANG, Yujie, Hangzhou, Zhejiang 310011 (CN); WANG, Ling, Hangzhou, Zhejiang 310011 (CN); WANG, Linli, Hangzhou, Zhejiang 310011 (CN); LIU, Dongzhou, Hangzhou, Zhejiang 310011 (CN)
(74) Representative: Nederlandsch Octrooibureau
(86) International application number: PCT/CN2023/139101
(87) International publication number: WO 2024/125631

(57) **Abstract**

A compound represented by general formula (I) or a pharmaceutically acceptable salt or a stereoisomer thereof, and a use thereof in treating diseases such as tumors, immunological diseases, and inflammatory diseases, as well as a pharmaceutical preparation and a pharmaceutical composition of the compound, and a use thereof.

## Description

### Technical Field

The present invention belongs to the pharmaceutical field and in particular, relates to a PROTAC chimeric compound and a use thereof in the manufacture of a medicament for the treatment or prophylaxis of diseases such as tumors.

### Background

The proteolysis-targeting chimera (PROTAC) technology stems from scientists' discovery of the ubiquitin (Ub) regulated protein degradation process. Eukaryotic cells are constantly striving to maintain appropriate protein levels, and are producing and degrading thousands of proteins at every moment. A key factor in maintaining protein balance is a small protein molecule called ubiquitin. When it is linked to proteins, it causes these proteins to be transported to the proteasome for degradation.

Targeted protein degradation is an emerging direction in the field of drug research and development. Targeted protein degradation drugs aim to design small molecules into a new type of drugs. The role of traditional small molecules is to block the function of proteins, while the role of targeted protein degraders is to degrade these proteins by delivering them to the proteasome.

Dr. Craig Crews and Dr. Raymond Deshaies designed a series of bifunctional chimeric molecules based on polypeptide compounds to induce the degradation of methionyl aminopeptidase 2 (MetAP-2) and formally proposed the concept of PROTAC and filed the relevant patent WO2002020740A3. However, since these compounds that play a role of linkage based on large and bulky peptides have difficulty in entering cells, the first generation of PROTACs was declared a failure.

In 2008, the Crews team designed the second generation of PROTACs useful in the degradation of the androgen receptor (AR) based on the E3 ubiquitin-protein ligase MDM2.

In 2015, the Crews team designed a new generation of PROTACs based on the novel E3 ubiquitin ligase VHL and CRBN ligands.

HPK1 (Hematopoietic progenitor kinase 1), also known as MAP 4K1 (mitogen-activated protein kinase kinase kinase kinase 1), is a serine/threonine kinase that is a member of the MAP4K family. In addition, there are five members in this family, including MAP4K2, MAP4K3, MAP4K4, MAP4K5, and MAP4K6.

The main process involved in the regulation of TCR by HPK1 includes: (1) the TCR binds to extracellular antigens via MHC, thereby activating the TCR pathway to transmit signals to downstream adaptor protein molecules; (2) the adaptor protein tyrosine kinases Lck and Zap70 activate SLP76, which then phosphorylates HPK1; (3) the activated HPK1 then phosphorylates the receptor protein SLP-76; (4) the phosphorylation reaction of SLP-76 provides multiple protein binding sites for 14-3-3 (a TCR pathway inhibitory protein) receptor protein to form a complex; (5) the phosphorylated complex of SLP-76 participates in the down-regulation of the Erk signaling pathway and is connected to the ubiquitylation degradation process of SLP76, resulting in a decrease in the TCR signaling pathway and T cell proliferation. In conclusion, HPK1 can negatively regulate the TCR signaling pathway. Therefore, HPK1 can be used as a new regulatory mechanism of T-cell mediated immune responses and become a new target for immunological anti-tumor therapy. HPK1 can bind to many adaptor proteins, such as interacting with the SLP-76 family, CARD11, HIS, HIP-55, the GRB2 family, LAT, and the CRK family, so as to activate the JNK/SAPK signaling pathway of hematopoietic stem cells, thereby negatively regulating the TCR pathway. The blockade of the Erk MAPK pathway is an inhibitory mechanism that negatively regulates the TCR-induced IL-2 gene transcription.

HPK1 can bind to many adaptor proteins, such as interacting with the SLP-76 family, CARD11, HIS, HIP-55, the GRB2 family, LAT, and the CRK family, so as to activate the JNK/SAPK signaling pathway of hematopoietic stem cells, thereby negatively regulating the TCR pathway. However, MAP4K3, also known as GLK kinase, has a biological function that is exactly opposite to that of HPK1. GLK can promote the activation of the TCR pathway by binding to downstream adaptor proteins.

In order to better meet the great clinical needs, the present invention aims to provide a PROTAC compound with a novel structure and HPK1 inhibitory/degradative activity, which has good physicochemical properties and drugable properties. The compound of the present invention or a pharmaceutically acceptable salt thereof has good safety, excellent efficacy and high bioavailability. Therefore, the compound of the present invention has good application potential in the treatment of diseases mediated by HPK1.

### Summary of the Invention

One objective of the present invention is to provide a targeted PROTAC compound having good HPK1 inhibitory/degradative activity, or a pharmaceutically acceptable salt or a stereoisomer thereof. The compound of the present invention also demonstrates good performance in terms of HPK1 selectivity, and has the effect of inducing the production of IL2, and further a good HPK1 protein degradation ability. Furthermore, the compound of the present invention has good physicochemical properties (e.g., solubility, physical and/or chemical stability), good pharmacokinetic properties (e.g., good bioavailability, good metabolic stability, a suitable half-life and duration of action), good safety (low toxicity (e.g. reduced cardiotoxicity) and/or fewer side effects), less susceptibility to drug resistance, etc.

Another objective of the present invention is to provide a use of the compound, or a pharmaceutically acceptable salt or a stereoisomer thereof in the treatment of diseases such as tumors, immunological diseases or inflammatory diseases.

The compound of the present invention has a structure represented by general formula (I):

[B-L]ₙ-HPK1 ligand (I),

or a pharmaceutically acceptable salt, or a stereoisomer thereof,
wherein:
the HPK1 ligand is, for example, an HPK1 inhibitor; B is a degradation tag, such as an E3 ligase ligand; L is a linking group between the B and the HPK1 ligand; n is the number of the degradation tags attached to the HPK1 ligand and is selected from 1, 2, or 3.

The attachment site of the group that binds to the E3 ligase, the number of the attachments, and the choice of the attachment site on the HPK1 inhibitor will all affect the activity of the compound.

### S1. HPK1 Ligand

In a first aspect, the HPK1 ligand according to the present invention is a compound of formula (H-I) as described below. The first aspect includes the following embodiments:
Embodiment 1: a compound of general formula (H-I) : 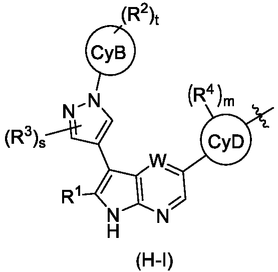 or a pharmaceutically acceptable salt, or a stereoisomer thereof,
   wherein:
   W is selected from CR¹ or N;
   ring CyB is selected from 4-10 membered cycloalkyl, 4-10 membered heterocyclyl, 5-8 membered aryl, or 5-8 membered heteroaryl;
   R¹ is independently selected from hydrogen, halogen, -C₁₋₈ alkyl, -C₂₋₈ alkenyl, -C₂₋₈ alkynyl, cycloalkyl, heterocyclyl, aryl, heteroaryl, -CN, -NO₂, or -OR^{1a}, wherein the -C₁₋₈ alkyl, -C₂₋₈ alkenyl, -C₂₋₈ alkynyl, cycloalkyl, heterocyclyl, aryl, or heteroaryl is each optionally substituted with halogen, hydroxyl, -C₁₋₈ alkoxy, cycloalkyl, heterocyclyl, aryl, or heteroaryl;
   R² is selected from hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{2a}, -SO₂R^{2a}, -COR^{2a}, -CO₂R^{2a}, -CONR^{2a}R^{2b}, -C(=NR^{2a})NR^{2b}R^{2c}, -NR^{2a}R^{2b}, -NR^{2a}COR^{2b}, -NR^{2a}CONR^{2b}R^{2c}, -NR^{2a}CO₂R²⁶, - NR^{2a}SONR^{2b}R^{2c}, -NR^{2a}SO₂NR^{2b}R^{2c}, or -NR^{2a}SO₂R^{2b}, wherein the -C₁₋₈ alkyl, -C₂₋₈ alkenyl, - C₂₋₈ alkynyl, cycloalkyl, heterocyclyl, aryl, or heteroaryl is each optionally substituted with halogen, hydroxyl, -C₁₋₈ alkoxy, cycloalkyl, heterocyclyl, aryl, or heteroaryl;
   R^{2a}, R^{2b} and R^{2c} are each independently hydrogen, -C₁₋₈ alkyl, -C₂₋₈ alkenyl, -C₂₋₈ alkynyl, cycloalkyl, heterocyclyl, aryl, or heteroaryl, wherein the -C₁₋₈ alkyl, -C₂₋₈ alkenyl, -C₂₋₈ alkynyl, cycloalkyl, heterocyclyl, aryl, or heteroaryl is each optionally substituted with at least one substituent R^{2d}; or
   (R^{2a} and R^{2b}), (R^{2b} and R^{2c}), or (R^{2c} and R^{2a}), together with the one or more atoms to which they are attached, form a 3- to 9-membered ring comprising 0, 1, or 2 heteroatoms independently selected from nitrogen, oxygen, or optionally oxidized sulfur as one or more ring members, wherein the ring is optionally substituted with at least one substituent R^{2e};
   wherein R^{2d} and R^{2e} are each independently hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{2f}, -SO₂R^{2f}, - COR^{2f}, -CO₂R^{2f}, -CONR^{2f}R^{2g}, -C(=NR^{2f})NR^{2g}R^{2h}, -NR^{2f}R^{2g}, -NR^{2f}COR^{2g}, -NR^{2f}CONR^{2g}R^{2h}, - NR^{2f}CO₂R^{2g}, -NR^{2f}SONR^{2g}R^{2h}, -NR^{2f}SO₂NR^{2g}R^{2h}, or -NR^{2f}SO₂R^{2g}, wherein the -C₁₋₈ alkyl, - C₂₋₈ alkenyl, -C₂₋₈ alkynyl, cycloalkyl, heterocyclyl, aryl, or heteroaryl is each optionally substituted with at least one substituent selected from halogen, -C₁₋₈ alkyl, -OR²ⁱ, -NR²ⁱR^{2j}, cycloalkyl, heterocyclyl, aryl, or heteroaryl;
   R^{2f}, R^{2g}, R^{2h}, R²ⁱ, and R^{2j} are each independently hydrogen, -C₁₋₈ alkyl, C₁₋₈ alkoxy-C₁₋₈ alkyl-, -C₂₋₈ alkenyl, -C₂₋₈ alkynyl, cycloalkyl, heterocyclyl, aryl, or heteroaryl;
   R³ is selected from hydrogen, halogen, -C₁₋₈ alkyl, -C₂₋₈ alkenyl, -C₂₋₈ alkynyl, cycloalkyl, heterocyclyl, aryl, heteroaryl, -CN, or -NO₂;
   R⁴ is independently hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{4a}, -SO₂R^{4a}, -SO₂NR^{4a}R^{4b}, -COR^{4a}, -CO₂R^{4a}, -CONR^{4a}R^{4b}, -C(=NR^{4a})NR^{4b}R^{4c}, -NR^{4a}R^{4b}, -NR^{4a}COR^{4b}, -NR^{4a}CONR^{4b}R^{4c}, -NR^{4a}CO₂R^{4b}, - NR^{4a}SONR^{4b}R^{4c}, -NR^{4a}SO₂NR^{4b}R^{4c}, or -NR^{4a}SO₂R^{4b}, wherein the -C₁₋₈ alkyl, -C₂₋₈ alkenyl, - C₂₋₈ alkynyl, cycloalkyl, heterocyclyl, aryl, or heteroaryl is each optionally substituted with at least one substituent R^{4d};
   R^{4a}, R^{4b}, and R^{4c} are each independently hydrogen, -C₁₋₈ alkyl, -C₂₋₈ alkenyl, -C₂₋₈ alkynyl, cycloalkyl, heterocyclyl, aryl, or heteroaryl, wherein the -C₁₋₈ alkyl, -C₂₋₈ alkenyl, -C₂₋₈ alkynyl, cycloalkyl, heterocyclyl, aryl, or heteroaryl is each optionally substituted with at least one substituent R^{4e};
   R^{4d} and R^{4e} are each independently hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{4f}, -SO₂R^{4f}, - SO₂NR^{4f}R^{4g}, -COR^{4f}, -CO₂R^{4f}, -CONR^{4f}R^{4g}, -C(=NR^{4f})NR^{4g}R^{4h}, -NR^{4f}R^{4g}, -NR^{4f}COR^{4g}, - NR^{4f}CONR^{4g}R^{4h}, -NR^{4f}CO₂R^{4f}, -NR^{4f}SONR^{4f}R^{4g}, -NR^{4f}SO₂NR^{4g}R^{4h}, or -NR^{4f}SO₂R^{4g}, wherein the -C₁₋₈ alkyl, -C₂₋₈ alkenyl, -C₂₋₈ alkynyl, cycloalkyl, heterocyclyl, aryl, or heteroaryl is each optionally substituted with at least one substituent selected from halogen, -C₁₋₈ alkyl, -OR⁴ⁱ, - NR⁴ⁱR^{4j}, cycloalkyl, heterocyclyl, aryl, or heteroaryl;
   R^{4f}, R^{4g}, R^{4h}, R⁴ⁱ, and R^{4j} are each independently hydrogen, -C₁₋₈ alkyl, C₁₋₈ alkoxy-C₁₋₈ alkyl-, -C₂₋₈ alkenyl, -C₂₋₈ alkynyl, cycloalkyl, heterocyclyl, aryl, or heteroaryl;
   s is 0, 1, or 2, provided that the valence theory is satisfied;
   t is 0, 1, 2, 3, or 4, provided that the valence theory is satisfied;
   m is 0, 1, 2, 3, or 4, provided that the valence theory is satisfied;
   CyD is selected from cycloalkyl, heterocyclyl, aryl, or heteroaryl.
Embodiment 2: The compound of Embodiment 1, wherein R¹ is selected from hydrogen, halogen, -C₁₋₈ alkyl, preferably hydrogen, halogen, -C₁₋₆ alkyl, more preferably hydrogen.
Embodiment 3: The compound of any one of Embodiments 1 and 2, wherein CyB is selected from 4-10 membered heterocyclyl, 5-8 membered aryl, or 5-8 membered heteroaryl;
Embodiment 4: The compound of Embodiment 3, wherein the ring CyB is selected from 5-6 membered nitrogen-containing heterocyclyl, 5-6 membered oxygen-containing heterocyclyl, or 5-6 membered nitrogen-containing heteroaryl.
Embodiment 5: The compound of Embodiment 4, wherein the ring CyB is selected from cyclopropyl, cyclobutyl, cyclopentyl, cyclohexyl, oxetanyl, tetrahydrofuranyl, tetrahydropyranyl, azetidinyl, tetrahydropyrrolyl, piperidinyl, or pyridinyl.
Embodiment 6: The compound of Embodiment 5, wherein the ring CyB is selected from 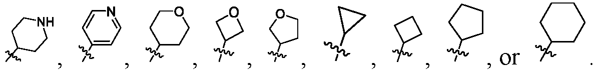
Embodiment 7: The compound of Embodiment 4, wherein the ring CyB is selected from 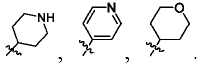
Embodiment 8: The compound of any one of Embodiments 1-7, wherein said R² is selected from hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{2a}, -SO₂R^{2a}, -COR^{2a}, -CO₂R^{2a}, -CONR^{2a}R^{2b}, - C(=NR^{2a})NR^{2b}R^{2c}, -NR^{2a}R^{2b}, -NR^{2a}COR^{2b}, -NR^{2a}CONR^{2b}R^{2c}, -NR^{2a}CO₂R²⁶, -NR^{2a}SONR^{2b}R^{2c}, -NR^{2a}SO₂NR^{2b}R^{2c}, or -NR^{2a}SO₂R^{2b}, wherein the -C₁₋₈ alkyl, -C₂₋₈ alkenyl, -C₂₋₈ alkynyl, cycloalkyl, heterocyclyl, aryl, or heteroaryl is each optionally substituted with halogen, hydroxyl, -C₁₋₈ alkoxy, cycloalkyl, heterocyclyl, aryl, or heteroaryl;
   R^{2a}, R^{2b} and R^{2c} are each independently hydrogen, -C₁₋₈ alkyl, -C₂₋₈ alkenyl, -C₂₋₈ alkynyl, cycloalkyl, heterocyclyl, aryl, or heteroaryl, wherein the -C₁₋₈ alkyl, -C₂₋₈ alkenyl, -C₂₋₈ alkynyl, cycloalkyl, heterocyclyl, aryl, or heteroaryl is each optionally substituted with at least one halogen, -C₁₋₈ alkyl, -CN, hydroxyl, or -NO₂;
   t is selected from 0, 1, 2, 3, or 4;
   further,
   R² is selected from -C₁₋₈ alkyl, preferably -C₁₋₃ alkyl, more preferably methyl or ethyl; t is 0 or 1.
Embodiment 9: The compound of any one of Embodiments 3-8, wherein the 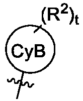 is selected from 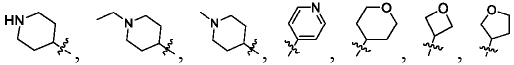 (including 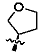 and 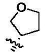 ), 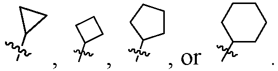
Embodiment 10: The compound of any one of Embodiments 3-8, wherein the 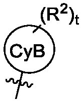 is selected from 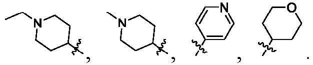
Embodiment 11: The compound of any one of Embodiments 1-10, wherein said R³ is selected from hydrogen, halogen, -C₁₋₈ alkyl, preferably halogen, more preferably fluorine; s is selected from 0 or 1.
Embodiment 12: The compound of any one of Embodiments 1-11, wherein R⁴ is independently hydrogen, halogen, -CN, -C₁₋₈ alkyl, wherein the -C₁₋₈ alkyl is optionally substituted with at least one substituent R^{4d};
   R^{4d} is each independently hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{4f}, -SO₂R^{4f}, -SO₂NR^{4f}R^{4g}, - COR^{4f}, -CO₂R^{4f}, -CONR^{4f}R^{4g}, -C(=NR^{4f})NR^{4g}R^{4h}, -NR^{4f}R^{4g}, -NR^{4f}COR^{4g}, -NR^{4f}CONR^{4g}R^{4h}, - NR^{4f}CO₂R^{4f}, -NR^{4f}SONR^{4f}R^{4g}, -NR^{4f}SO₂NR^{4g}R^{4h}, or -NR^{4f}SO₂R^{4g}, wherein the -C₁₋₈ alkyl, - C₂₋₈ alkenyl, -C₂₋₈ alkynyl, cycloalkyl, heterocyclyl, aryl, or heteroaryl is each optionally substituted with at least one substituent selected from halogen, -C₁₋₈ alkyl, -OR⁴ⁱ, -NR⁴ⁱR^{4j}, cycloalkyl, heterocyclyl, aryl, or heteroaryl;
   R^{4f}, R^{4g}, R^{4h}, R⁴ⁱ, and R^{4j} are each independently hydrogen, -C₁₋₈ alkyl, C₁₋₈ alkoxy-C₁₋₈ alkyl-, -C₂₋₈ alkenyl, -C₂₋₈ alkynyl, cycloalkyl, heterocyclyl, aryl, or heteroaryl;
   m is selected from 0, 1, 2, or 3.
Embodiment 13: The compound of Embodiment 12, wherein said R⁴ is -CN, halogen, preferably fluorine, chlorine, or bromine, more preferably fluorine, or chlorine.
Embodiment 14: The compound of Embodiment 12, wherein said R⁴ is -C₁₋₈ alkyl, preferably -C₁₋₃ alkyl, more preferably methyl or ethyl.
Embodiment 15: The compound of Embodiment 12, wherein said R⁴ is -C₁₋₈ alkyl substituted with -NR^{4f}R^{4g}, wherein R^{4f} and R^{4g} are independently -C₁₋₈ alkyl, preferably methyl.
Embodiment 16: The compound of Embodiment 15, wherein said R⁴ is 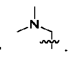
Embodiment 17: The compound of Embodiment 12, wherein said R⁴ is -OR^{4f}, wherein R^{4f} is selected from hydrogen, -C₁₋₈ alkyl, preferably -C₁₋₃ alkyl, more preferably methyl or ethyl.
Embodiment 18: The compound of Embodiment 17, wherein said R⁴ is 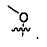
Embodiment 19: The compound of any one of Embodiments 12-17, wherein said R⁴ is methyl, ethyl, fluorine, chlorine, -CN, 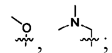 m is selected from 0, 1, or 2.
Embodiment 20: The compound of Embodiment 12, wherein R⁴ is -C₁₋₈ alkyl, preferably -C₁₋₃ alkyl, more preferably methyl, or ethyl, which is substituted with at least one halogen.
Embodiment 21: The compound of Embodiment 20, wherein the halogen is fluorine, chlorine, or bromine, preferably fluorine, or chlorine.
Embodiment 22: The compound of Embodiment 20 or 21, wherein said R⁴ is -CHF₂, - CH₂F, -CF₃, - CH₂CHF₂, - CH₂CH₂F, or -CH₂CF₃, preferably -CF₃.
Embodiment 23: The compound of Embodiments 1 to 22, wherein said CyD is selected from 5-membered heteroaryl ring, phenyl ring, 6-membered heteroaryl ring, C₈₋₁₂ heterocyclyl, wherein the heteroaryl ring and the heterocyclyl comprise 0, 1 or 2 heteroatoms independently selected from nitrogen, oxygen or optionally oxidized sulfur as one or more ring members.
Embodiment 24: The compound of Embodiment 23, wherein said CyD is selected from 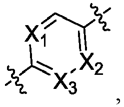 and X₁, X₂, and X₃ are each independently selected from CH, C or N.
Embodiment 25: The compound of Embodiment 23, wherein said CyD is selected from 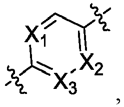 and X₁, X₂, and X₃ are each independently selected from C or N.
Embodiment 26: The compound of Embodiment 25, wherein said CyD is selected from phenyl ring.
Embodiment 27: The compound of Embodiment 24, wherein said CyD is selected from 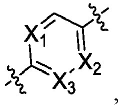 and X₁, X₂, and X₃ are each independently selected from CH, C, or N, provided that at least one of X₁, X₂, and X₃ is N.
Embodiment 28: The compound of Embodiment 25, wherein said CyD is selected from 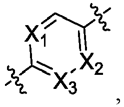 and X₁, X₂, and X₃ are each independently selected from C or N, provided that X₁, X₂, and X₃ cannot be C at the same time.
Embodiment 29: The compound of Embodiment 27, wherein said CyD is selected from pyridyl, pyrimidinyl, pyridazinyl, pyrazinyl or triazinyl, preferably 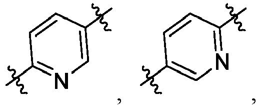 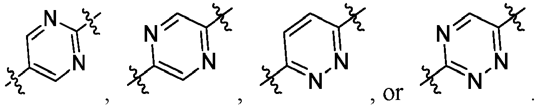
Embodiment 30: The compound of Embodiment 28, wherein said CyD is selected from 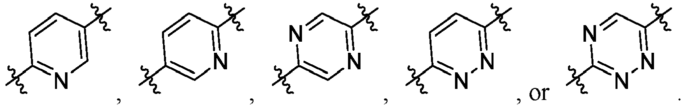
Embodiment 31: The compound of Embodiment 23, wherein said CyD is selected from 5-membered heteroaryl ring comprising 0, 1, or 2 heteroatoms independently selected from nitrogen, oxygen, or optionally oxidized sulfur as one or more ring members.
Embodiment 32: The compound of Embodiment 31, wherein said CyD is selected from 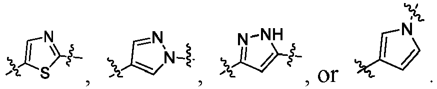
Embodiment 33: The compound of any one of Embodiments 1-32, wherein W is selected from CH or N.

In a second aspect, the HPK1 ligand according to the present invention is a compound of formula (H-II) as described below. The second aspect includes the following embodiments.

Embodiment 1: A compound represented by general formula (H-II): 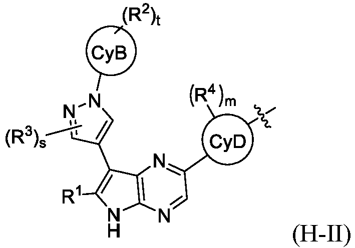 or a pharmaceutically acceptable salt, or a stereoisomer thereof,
wherein R¹, CyB, R², t, R³, s, R⁴, m, and CyD are each as defined in the first aspect above.

Embodiment 2: The compound of Embodiment 1 wherein the 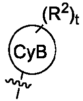 is 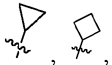 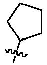 or 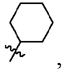 preferably

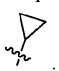

Embodiment 3: The compound of Embodiment 1 or 2, wherein said R¹ and R³ are each hydrogen.

Embodiment 4: The compound of any one of Embodiments 1-3, wherein said CyD is pyridyl, preferably 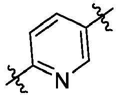 or

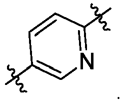

Embodiment 5: The compound of any one of Embodiment 1-4, wherein said R⁴ is hydrogen.

In a third aspect, the HPK1 ligand according to the present invention is a compound of formula (H-III) as described below. The third aspect includes the following embodiments:
Embodiment 1: A compound represented by general formula (H-III): 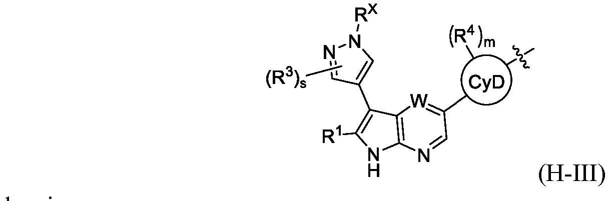 wherein:
   W, R¹, R³, s, R⁴, m, and CyD are each as defined in the first aspect above;
   R^{X} is selected from H and C₁₋₈ alkyl, wherein the C₁₋₈ alkyl is optionally substituted with one or more substituents independently selected from deuterium, tritium, halogen, -OH, -CN, - NR^{Xa}R^{Xb}, -OR^{Xa}, -CO-NHR^{Xb}, -CO-NR^{Xa}R^{Xb}, and 3-10-membered cycloalkyl; and
   R^{Xa} and R^{Xb} are each independently selected from C₁₋₈ alkyl.
Embodiment 2: The compound of Embodiment 1, wherein R^{X} is selected from H and C₁₋₄ alkyl, wherein the C₁₋₄ alkyl is optionally substituted with 1, 2, or 3 substituents independently selected from deuterium, F, Cl, Br, -OH, -CN, -NR^{Xa}R^{Xb}, -OR^{Xa}, -CO-NHR^{Xb}, -CO-NR^{Xa}R^{Xb}, and 3-7 membered cycloalkyl; and
   R^{Xa} and R^{Xb} are each independently selected from C₁₋₄ alkyl.
Embodiment 3: The compound of Embodiment 1 or 2, wherein R^{X} is selected from H and methyl, ethyl, isopropyl, isobutyl, sec-butyl, and tert-butyl, wherein the methyl, ethyl, isopropyl, isobutyl, sec-butyl, and tert-butyl are optionally substituted with 1, 2, or 3 substituents independently selected from deuterium, F, Cl, -OH, -CN, -NR^{Xa}R^{Xb}, -OR^{Xa}, -CO-NHR^{Xb}, -CO-NR^{Xa}R^{Xb}, and 3-6 membered cycloalkyl; and
   R^{Xa} and R^{Xb} are each independently methyl.
Embodiment 4: The compound of any one of Embodiments 1-3, wherein R^{X} is selected from H, methyl, -CD₃, ethyl, isopropyl, -CH₂F, -CHF₂, -CF₃, -CH₂CH₂F, -CH₂CHF₂, -CH₂CF₃, 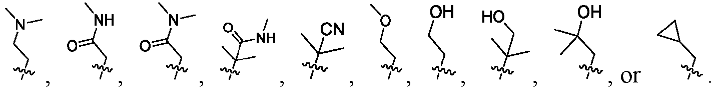
Embodiment 5: A compound represented by general formula (H-III): 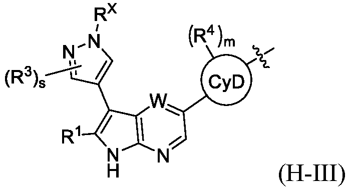 wherein:
   W, R¹, R³, s, R⁴, m, and CyD are each as defined in the first aspect above;
   R^{X} is selected from C₁₋₈ alkyl, or C₁₋₈ alkyl substituted with -NR^{Xa}R;
   R^{Xa} and R^{Xb} are each independently selected from C₁₋₈ alkyl, preferably methyl.
Embodiment 6: The compound of Embodiment 5, wherein said R^{X} is selected from methyl,

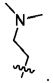

In a fourth aspect, the HPK1 ligand according to the present invention is a compound of formula (H-IV) as described below. The fourth aspect includes the following embodiments:
Embodiment 1: A compound represented by general formula (H-IV): 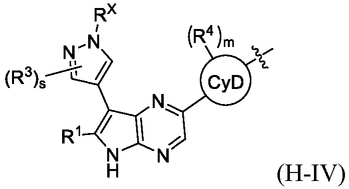 wherein R¹, R³, s, R⁴, m, CyD, and R^{X} are each as defined in the third aspect above.

In a fifth aspect, the HPK1 ligand according to the present invention is a compound of formula (H-V) as described below. The fifth aspect includes the following embodiments:
Embodiment 1: A compound represented by general formula (H-V): 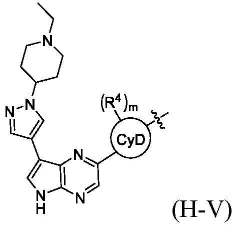 or a pharmaceutically acceptable salt, or a stereoisomer thereof,
   wherein:
   R⁴ is independently hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{4a}, -SO₂R^{4a}, -SO₂NR^{4a}R^{4b}, -COR^{4a}, -CO₂R^{4a}, -CONR^{4a}R^{4b}, -C(=NR^{4a})NR^{4b}R^{4c}, -NR^{4a}R^{4b}, -NR^{4a}COR^{4b}, -NR^{4a}CONR^{4b}R^{4c}, -NR^{4a}CO₂R^{4b}, - NR^{4a}SONR^{4b}R^{4c}, -NR^{4a}SO₂NR^{4b}R^{4c}, or -NR^{4a}SO₂R^{4b}, wherein the -C₁₋₈ alkyl, -C₂₋₈ alkenyl, - C₂₋₈ alkynyl, cycloalkyl, heterocyclyl, aryl, or heteroaryl is each optionally substituted with at least one substituent R^{4d};
   R^{4a}, R^{4b}, and R^{4c} are each independently hydrogen, -C₁₋₈ alkyl, -C₂₋₈ alkenyl, -C₂₋₈ alkynyl, cycloalkyl, heterocyclyl, aryl, or heteroaryl, wherein the -C₁₋₈ alkyl, -C₂₋₈ alkenyl, -C₂₋₈ alkynyl, cycloalkyl, heterocyclyl, aryl, or heteroaryl is each optionally substituted with at least one substituent R^{4e};
   R^{4d} and R^{4e} are each independently hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{4f}, -SO₂R^{4f}, - SO₂NR^{4f}R^{4g}, -COR^{4f}, -CO₂R^{4f}, -CONR^{4f}R⁴⁸, -C(=NR^{4f})NR^{4g}R ^{4h}, -NR^{4f}R^{4g}, -NR^{4f}COR^{4g}, - NR^{4f}CONR^{4g}R^{4h}, -NR^{4f}CO₂R^{4f}, -NR^{4f}SONR^{4f}R^{4g}, -NR^{4f}SO₂NR^{4g}R^{4h}, or -NR^{4f}SO₂R^{4g}, wherein the -C₁₋₈ alkyl, -C₂₋₈ alkenyl, -C₂₋₈ alkynyl, cycloalkyl, heterocyclyl, aryl, or heteroaryl is each optionally substituted with at least one substituent selected from halogen, -C₁₋₈ alkyl, -OR⁴ⁱ, - NR⁴ⁱR^{4j}, cycloalkyl, heterocyclyl, aryl, or heteroaryl;
   R^{4f}, R^{4g}, R^{4h}, R⁴ⁱ, and R^{4j} are each independently hydrogen, -C₁₋₈ alkyl, C₁₋₈ alkoxy-C₁₋₈ alkyl-, -C₂₋₈ alkenyl, -C₂₋₈ alkynyl, cycloalkyl, heterocyclyl, aryl, or heteroaryl;
   m is 0, 1, 2, 3, or 4, provided that the valence theory is satisfied;
   CyD is selected from cycloalkyl, heterocyclyl, aryl, or heteroaryl.
Embodiment 2: The compounds of Embodiment 1, said CyD is selected from 5-membered heteroaryl ring, phenyl ring, 6-membered heteroaryl ring, or C₈₋₁₂ heterocyclyl, wherein the heteroaryl ring and the heterocyclyl comprise 0, 1 or 2 heteroatoms independently selected from nitrogen, oxygen or optionally oxidized sulfur as one or more ring members.
Embodiment 3: The compound of Embodiment 2, wherein said CyD is selected from 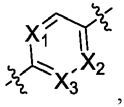 and X₁, X₂, and X₃ are each independently selected from CH, C, or N.
Embodiment 4: The compound of Embodiment 2, wherein said CyD is selected from 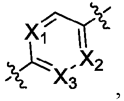 and X₁, X₂, and X₃ are each independently selected from C or N.
Embodiment 5: The compound of Embodiment 4, wherein said CyD is selected from phenyl ring.
Embodiment 6: The compound of Embodiment 3, wherein said CyD is selected from 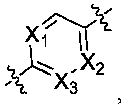 and X₁, X₂, and X₃ are each independently selected from CH, C, or N, provided that at least one of X₁, X₂, and X₃ is N.
Embodiment 7: The compound of Embodiment 4, wherein said CyD is selected from 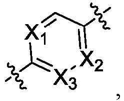 and X₁, X₂, and X₃ are each independently selected from C or N, provided that X₁, X₂, and X₃ cannot be C at the same time.
Embodiment 8: The compound of Embodiment 6, wherein said CyD is selected from pyridyl, pyrimidinyl, pyridazinyl, pyrazinyl or triazinyl, preferably 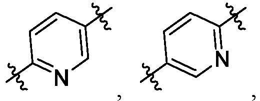 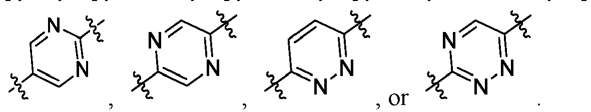
Embodiment 9: The compound of Embodiment 7, wherein said CyD is selected from 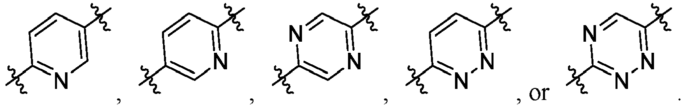
Embodiment 10: The compound of Embodiment 2, wherein said CyD is selected from 5-membered heteroaryl ring comprising 0, 1, or 2 heteroatoms independently selected from nitrogen, oxygen, or optionally oxidized sulfur as one or more ring members.
Embodiment 11: The compound of Embodiment 10, wherein said CyD is selected from 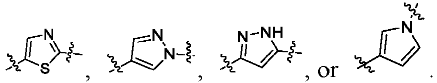
Embodiment 12: The compound of any one of Embodiments 1-11, wherein R⁴ is independently hydrogen, halogen, -CN, or -C₁₋₈ alkyl, wherein the -C₁₋₈ alkyl is optionally substituted with at least one substituent R^{4d};
   R^{4d} is each independently hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{4f}, -SO₂R^{4f}, -SO₂NR^{4f}R^{4g}, - COR^{4f}, -CO₂R^{4f}, -CONR^{4f}R^{4g}, -C(=NR^{4f})NR^{4g}R^{4h}, -NR^{4f}R^{4g}, -NR^{4f}COR^{4g}, -NR^{4f}CONR^{4g}R^{4h}, - NR^{4f}CO₂R^{4f}, -NR^{4f}SONR^{4f}R^{4g}, -NR^{4f}SO₂NR^{4g}R^{4h}, or -NR^{4f}SO₂R^{4g}, wherein the -C₁₋₈ alkyl, - C₂₋₈ alkenyl, -C₂₋₈ alkynyl, cycloalkyl, heterocyclyl, aryl, or heteroaryl is each optionally substituted with at least one substituent selected from halogen, -C₁₋₈ alkyl, -OR⁴ⁱ, -NR⁴ⁱR^{4j}, cycloalkyl, heterocyclyl, aryl, or heteroaryl;
   R^{4f}, R^{4g}, R^{4h}, R⁴ⁱ, and R^{4j} are each independently hydrogen, -C₁₋₈ alkyl, C₁₋₈ alkoxy-C₁₋₈ alkyl-, -C₂₋₈ alkenyl, -C₂₋₈ alkynyl, cycloalkyl, heterocyclyl, aryl, or heteroaryl;
   m is selected from 0, 1, 2, or 3.
Embodiment 13: The compound of Embodiment 12, wherein said R⁴ is -CN, halogen, preferably fluorine, chlorine, or bromine, more preferably fluorine, or chlorine.
Embodiment 14: The compound of Embodiment 12, wherein said R⁴ is -C₁₋₈ alkyl, preferably -C₁₋₃ alkyl, more preferably methyl or ethyl.
Embodiment 15: The compound of any one of Embodiments 12-14, wherein said R⁴ is methyl, ethyl, or fluorine; m is selected from 0 or 1.

In a sixth aspect, the HPK1 ligand according to the present invention is a compound of formula (H-VI) as described below. The sixth aspect includes the following embodiments:
Embodiment 1: A compound represented by general formula (H-VI): 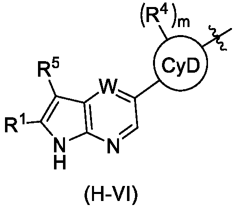 or a pharmaceutically acceptable salt, or a stereoisomer thereof,
   wherein:
   R⁵ is selected from hydrogen, halogen, -C₁₋₈ alkyl, -C₂₋₈ alkenyl, -C₂₋₈ alkynyl, -CN, -NO₂, -OR^{2a}, -SO₂R^{2a}, -COR^{2a}, -CO₂R^{2a}, -CONR^{2a}R^{2b}, -C(=NR^{2a})NR^{2b}R^{2c}, -NR^{2a}R^{2b}, -NR^{2a}COR^{2b}, - NR^{2a}CONR^{2b}R^{2c}, -NR^{2a}CO₂R²⁶, -NR^{2a}SONR^{2b}R^{2c}, -NR^{2a}SO₂NR^{2b}R^{2c}, or -NR^{2a}SO₂R^{2b}, wherein the -C₁₋₈ alkyl, -C₂₋₈ alkenyl, or -C₂₋₈ alkynyl is each optionally substituted with halogen, hydroxyl, -C₁₋₈ alkoxy, cycloalkyl, heterocyclyl, aryl, or heteroaryl;
   R¹, R^{2a}, R^{2b}, R^{2c}, R⁴, m, and CyD are each as defined in the first aspect above.
Embodiment 2: The compound of Embodiment 1, wherein R⁵ is -C₁₋₈ alkyl, preferably - C₁₋₃ alkyl, more preferably methyl or ethyl.

In a seventh aspect, the HPK1 ligand according to the present invention is a compound of formula (H-VII) as described below. The seventh aspect includes the following embodiments:
Embodiment 1: A compound represented by general formula (H-VII): 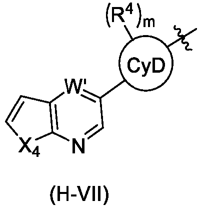 or a pharmaceutically acceptable salt, or a stereoisomer thereof,
   wherein:
   X₄ is selected from O or an optionally oxidized S heteroatom;
   W' is CR⁶ or N;
   R⁶ is selected from halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{2a}, -SO₂R^{2a}, -COR^{2a}, -CO₂R^{2a}, -CONR^{2a}R^{2b}, -C(=NR^{2a})NR^{2b}R^{2c}, -NR^{2a}R^{2b}, -NR^{2a}COR^{2b}, -NR^{2a}CONR^{2b}R^{2c}, -NR^{2a}CO₂R²⁶, - NR^{2a}SONR^{2b}R^{2c}, -NR^{2a}SO₂NR^{2b}R^{2c}, or -NR^{2a}SO₂R^{2b}, wherein the -C₁₋₈ alkyl, -C₂₋₈ alkenyl, - C₂₋₈ alkynyl, cycloalkyl, heterocyclyl, aryl, or heteroaryl is each optionally substituted with halogen, hydroxyl, -C₁₋₈ alkoxy, cycloalkyl, heterocyclyl, aryl, or heteroaryl;
   R^{2a}, R^{2b}, R^{2c}, R⁴, m, and CyD are each as defined in the first aspect above.
Embodiment 2: The compound of Embodiment 1, wherein X₄ is O or S, preferably S.
Embodiment 3: The compound of Embodiment 1 or 2, wherein W' is CR⁶.
Embodiment 4: The compound of any of Embodiments 1-3, wherein R⁶ is selected from halogen, -CN, -NO₂, or -NR^{2a}R^{2b}.
Embodiment 5: The compound of Embodiment 4, wherein R⁶ is -NR^{2a}R^{2b}.
Embodiment 6: The compound of any of Embodiments 1-5, wherein R^{2a} and R^{2b} are each independently hydrogen or -C₁₋₈ alkyl, preferably -C₁₋₃ alkyl, more preferably methyl or ethyl.
Embodiment 7: The compound of Embodiment 5, wherein R⁶ is -NH₂.
Embodiment 8: The compound of any of Embodiments 1-7, wherein said CyD is selected from C₃₋₁₀ cycloalkyl, phenyl ring, 5-6 membered monocyclic heteroaryl ring, 9-10 membered heteroaryl ring or 5-10 membered heterocyclyl, wherein the heteroaryl ring and the heterocyclyl comprise 1 or 2 heteroatoms independently selected from nitrogen, oxygen or optionally oxidized sulfur as ring members.
Embodiment 9: The compound of Embodiment 8, wherein said CyD is 9-10-membered bicyclic heteroaryl ring comprising 1 or 2 nitrogen heteroatoms, preferably

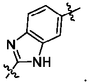

In some embodiments, the present invention provides a compound of formula (I) as described in the first aspect to the seventh aspect, or a pharmaceutically acceptable salt thereof, or a stereoisomer thereof, wherein the HPK1 ligand compound is selected from those shown in Table 1-1 below:

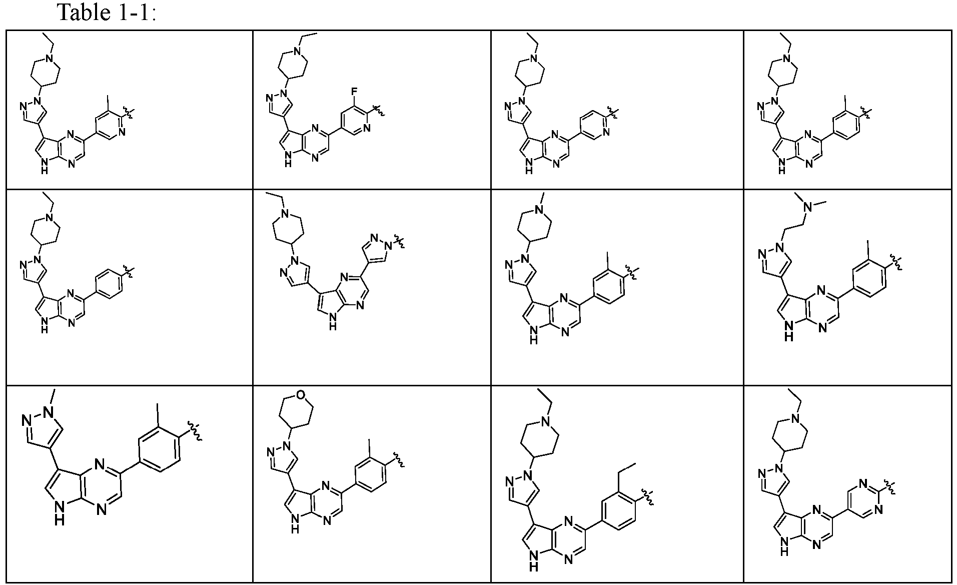

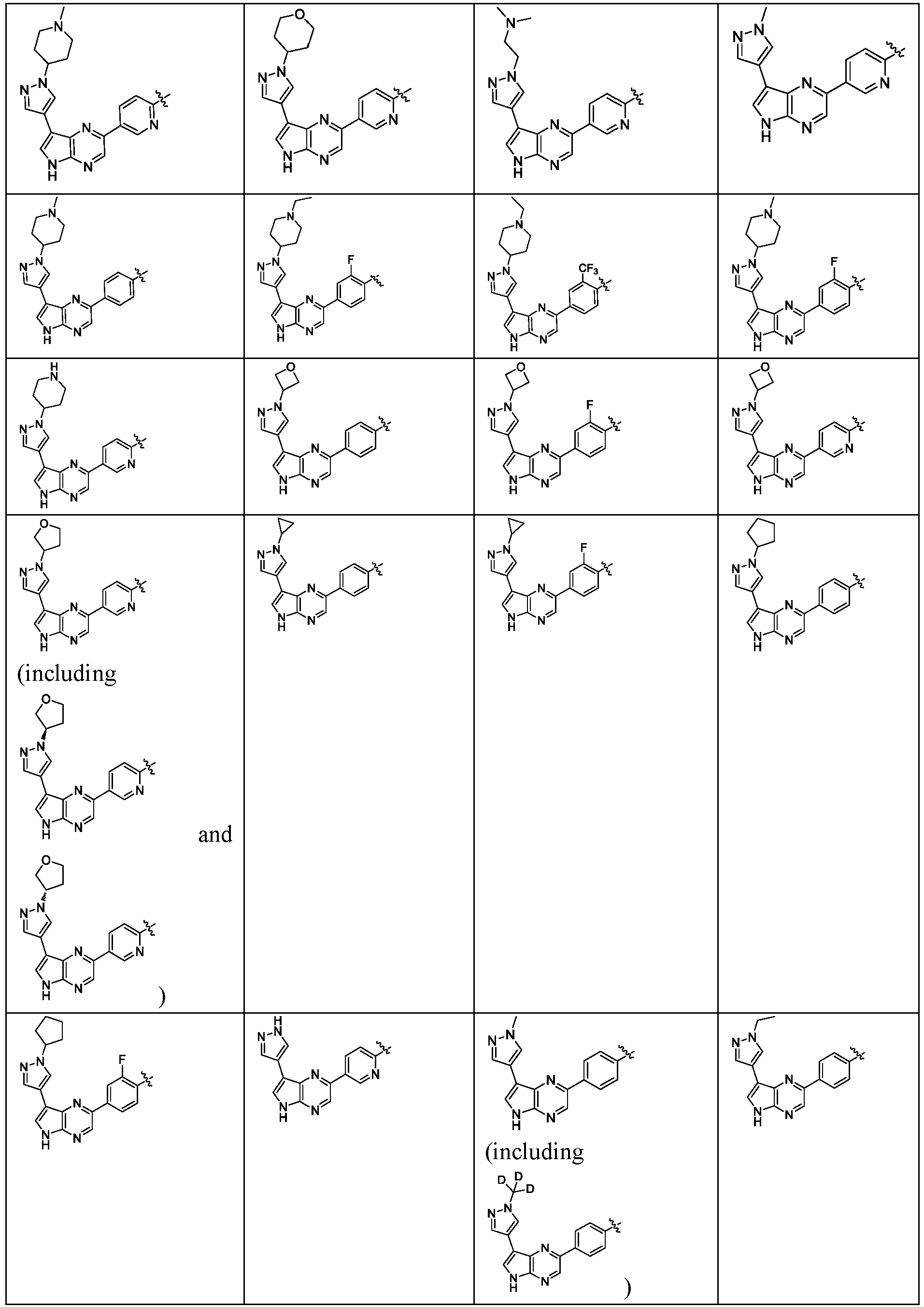

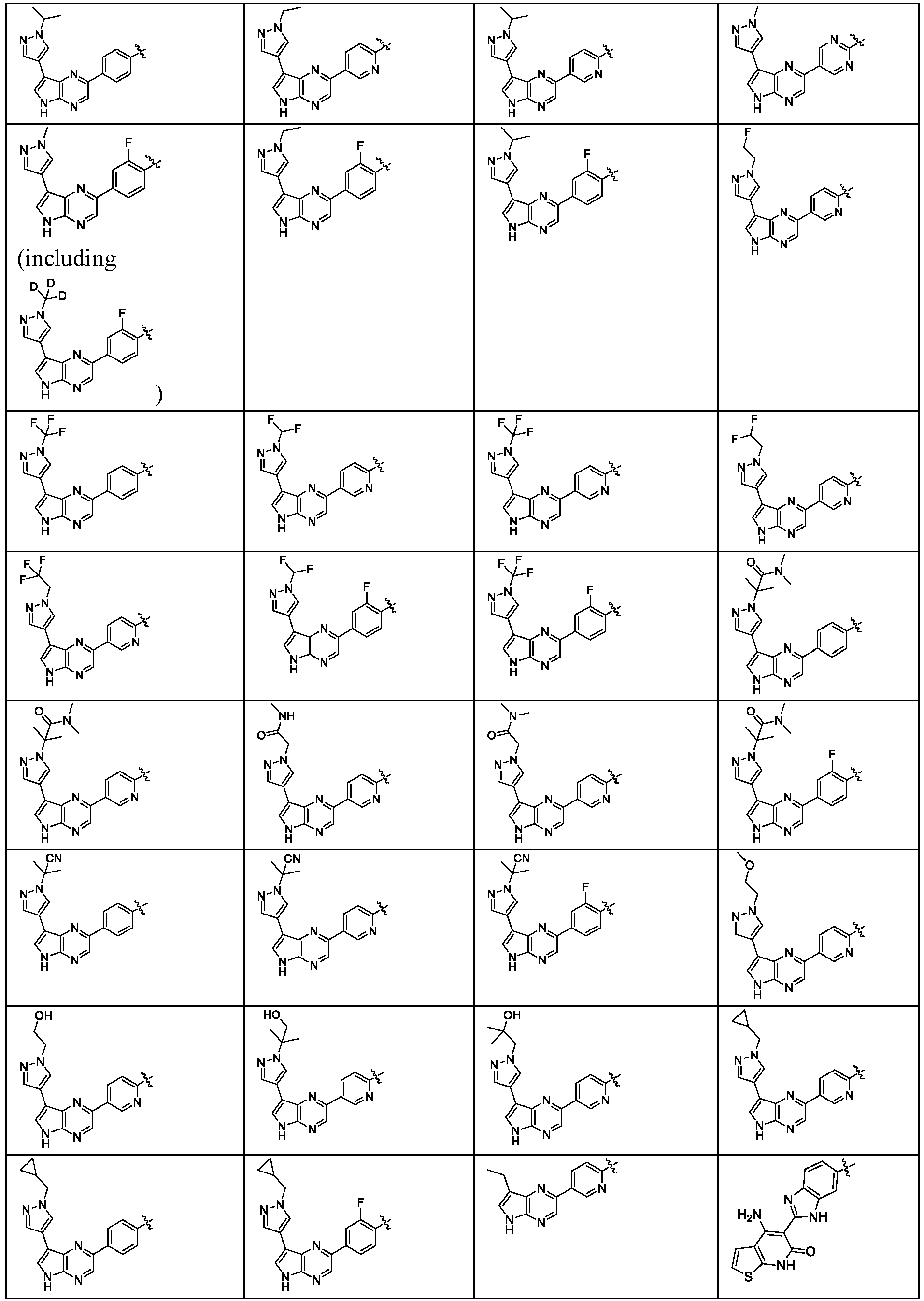

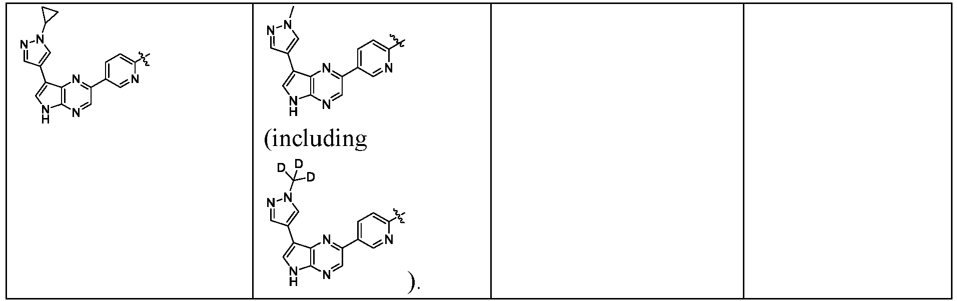

The present invention provides the HPK1 ligand compound as described above, or a pharmaceutically acceptable salt, or a stereoisomer thereof, wherein the HPK1 ligand compounds is preferably selected from the exemplified compounds of the HPK1 ligand disclosed herein, as shown in Tables 1-2 below:

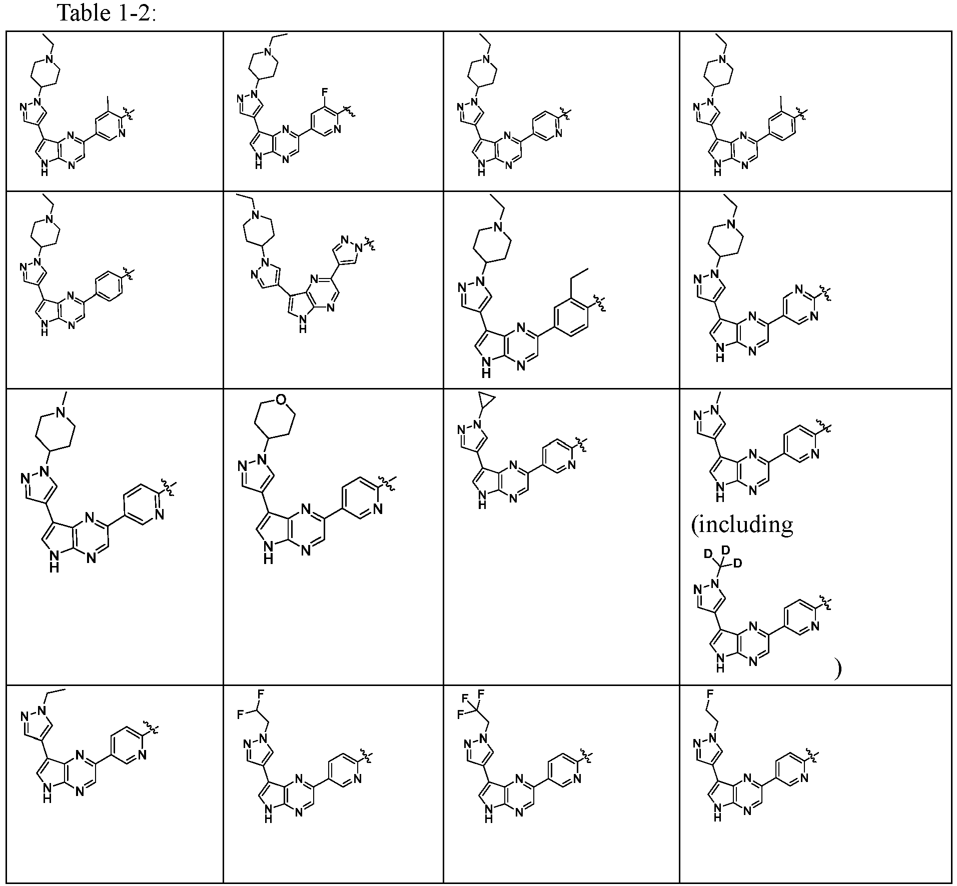

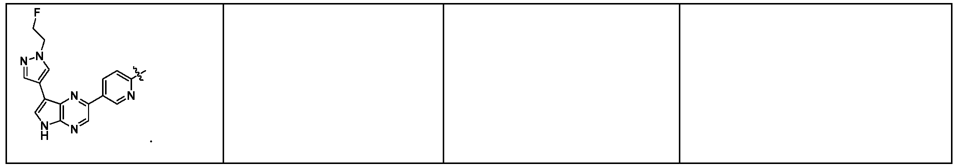

### S2. Degradation Tag B

In some embodiments, B is a group that binds to an E3 ligase, wherein the E3 ligase is selected from vonHippel-Lindau(VHL), Cereblon, XIAP, E3A, MDM2, Anaphase-promoting complex (APC), UBR5(EDD1), SOCS/BC-box/eloBC/CUL5/RING, LNXp80, CBX4, CBLL1, HACE1, HECTD1, HECTD2, HECTD3, HECW1, HECW2, HERC1, HERC2, HERC3, HERC4, HUWE1, ITCH, NEDD4, NEDD4L, PPIL2, PRPF19, PIAS1, PIAS2, PIAS3, PIAS4, RANBP2, RNF4, RBX1, SMURF1, SMURF2, STUB1, TOPORS, TRIP12, UBE3A, UBE3B, UBE3C, UBE4A, UBE4B, UBOX5, UBR5, WWP1, WWP2, Parkin, A20/TNFAIP3, AMFR/gp78, ARA54, β-TrCP1/BTRC, BRCA1, CBL, CHIP/STUB1, E6, E6AP/UBE3A, F-box protein 15/FBXO15, FBXW7/Cdc4, GRAIL/RNF128, HOIP/RNF31, cIAP-1/HIAP-2, cIAP-2/HIAP-1, cIAP(pan), ITCH/AIP4, KAP1, MARCH8, MindBomb1/MIB1, MindBomb2/MIB2, MuRF1/TRIM63, NDFIP1, NEDD4, NleL, Parkin, RNF2, RNF4, RNF8, RNF168, RNF43, SART1, Skp2, SMURF2, TRAF-1, TRAF-2, TRAF-3, TRAF-4, TRAF-5, TRAF-6, TRIM5, TRIM21, TRIM32, UBR5, or ZNRF3.

Further, said B is a group that binds to an E3 ligase selected from VHL, Cereblon, MDM2, or cIAP.

In some embodiments, said B is of a formula as shown below: 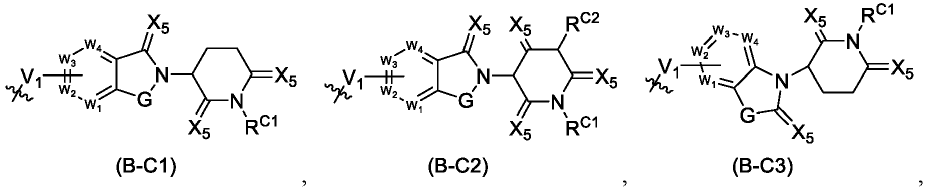 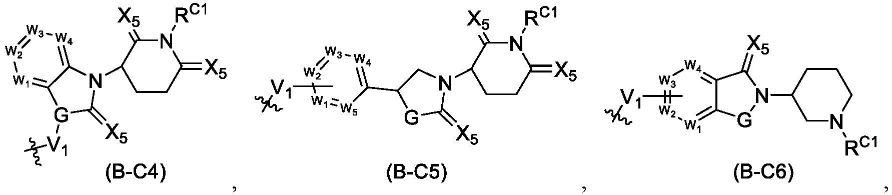 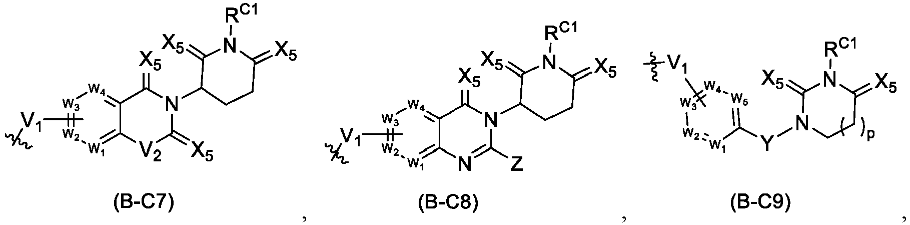 wherein:
G is each independently selected from CR^{C2}R^{C3}, NR^{C2}, CO, or SO₂;
Y is selected from a bond or NH;
p is selected from 0, 1, or 2;
W₁, W₂, W₃, W₄, and W₅ are each independently selected from N or CR^{C4};
X₅ is each independently selected from O or S;
Each V₁ is independently absent, or is selected from NH, O, S, SO, SO₂, SO₂NR^{C2}, CO, CO₂, C(O)NR^{C2}, C(S)NR^{C2}, NR^{C2}, NR^{C2}CO, NR^{C2}CONR^{C3}, -C₁₋₈ alkyl, -C₂₋₈ alkenyl, -C₂₋₈ alkynyl, cycloalkyl, heterocyclyl, aryl, or heteroaryl, wherein the -C₁₋₈ alkyl, -C₂₋₈ alkenyl, -C₂₋₈ alkynyl, cycloalkyl, heterocyclyl, aryl, or heteroaryl is each optionally substituted with R^{C4}, preferably each V₁ is independently absent, -O-, -CH₂-, -CH=CH-, or -NH-;
V₂ is each independently selected from CR^{C2}R^{C3}, NR^{C2}, O, or S;
Z is each independently selected from hydrogen, halogen, hydroxyl, amino, -C₁₋₈ alkyl, - C₂₋₈ alkenyl, -C₂₋₈ alkynyl, cycloalkyl, heterocyclyl, aryl, or heteroaryl, wherein the -C₁₋₈ alkyl, -C₂₋₈ alkenyl, -C₂₋₈ alkynyl, cycloalkyl, heterocyclyl, aryl, or heteroaryl is each optionally substituted with R^{C5};
R^{C1}, R^{C2}, R^{C3}, R^{C4}, and R^{C5} are selected from hydrogen, carboxy, cyano, nitro, a halogen atom, -C₁₋₈ alkyl, -C₂₋₈ alkenyl, -C₂₋₈ alkynyl, cycloalkyl, heterocyclyl, aryl, heteroaryl, -OR^{C6}, -SO₂R^{C6}, -SO₂NR^{C6}R^{C7}, -COR^{C6}, -CO₂R^{C6}, -CONR^{C6}R^{C7}, -POR^{C6}R^{C7}, -NR^{C6}R^{C7}, -NR^{C6}COR^{C7}, -NR^{C6}CONR^{C7}R^{C8}, -NR^{C6}CO₂R^{C7}, -NR^{C6}SO₂NR^{C7}R^{C8}, or -NR^{C6}SO₂R^{C7}, wherein the -C₁₋₈ alkyl, -C₂₋₈ alkenyl, -C₂₋₈ alkynyl, cycloalkyl, heterocyclyl, aryl, or heteroaryl is each optionally substituted with at least one substituent R^{C9};
R^{C6}, R^{C7}, R^{C8} and R^{C9} are selected from hydrogen, halogen, hydroxyl, amino, -C₁₋₈ alkyl, -C₂₋₈ alkenyl, -C₂₋₈ alkynyl, cycloalkyl, heterocyclyl, aryl, or heteroaryl.

Further, G is selected from CH₂, CO, SO₂, NH, or N-C₁₋₆ alkyl;
X₅ is selected from O or S;
V₁ is absent, NH, O, -C₁₋₈ alkyl, or -C₂₋₈ alkenyl, preferably is absent, -O-, -CH₂-, - CH=CH-, or -NH-.
V₂ is selected from NH, N-C₁₋₆ alkyl, N-C₆₋₁₀ aryl, N-3-10 membered heterocyclyl, N-5-10 membered heteroaryl, N-C₃₋₁₀ cycloalkyl, O, or S;
Z is selected from C₁₋₆ alkyl, C₃₋₁₀ alkyl, halogen, or hydrogen;
R^{C1} is selected from hydrogen, C₁₋₃ alkyl, hydroxyl, or -CH₂-3-10 membered heterocyclyl;
W₁, W₂, W₃, W₄, and W₅ are independently optionally selected from N or -CR^{C4}, wherein said R^{C4} is each independently selected from hydrogen, halogen, C₁₋₃ alkyl, halo C₁₋₃ alkyl, hydroxy, deuterated C₁₋₃ alkyl, or 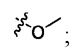 preferably, W₁, W₂, W₃, W₄, and W₅ are selected from -CR^{C4}, and R^{C4} is selected from hydrogen, halogen (fluorine, chlorine), methyl, or

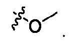

In some embodiments, said B is selected from: 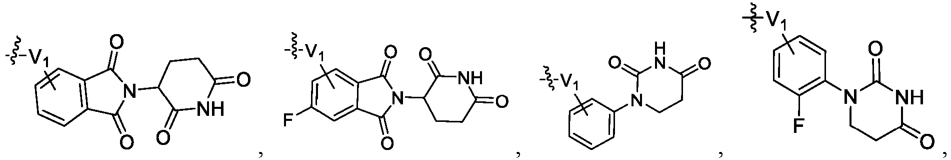 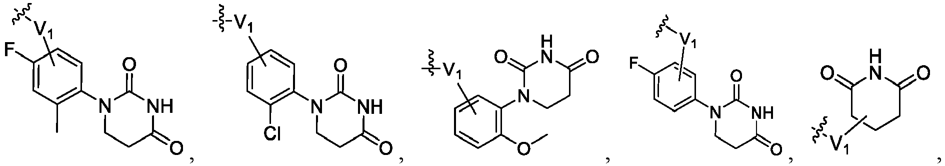 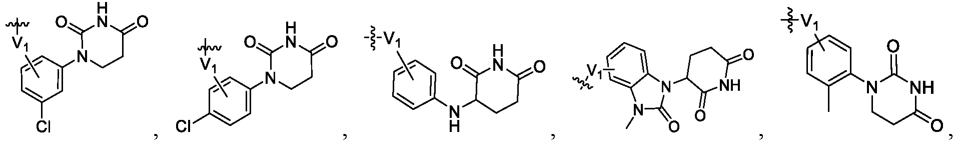 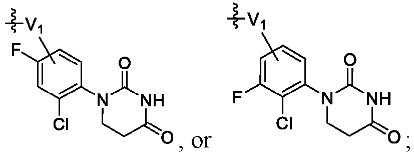 V₁ is absent, -O-, -CH₂-, -CH=CH-, or -NH-.

In some embodiments, said B is selected from: 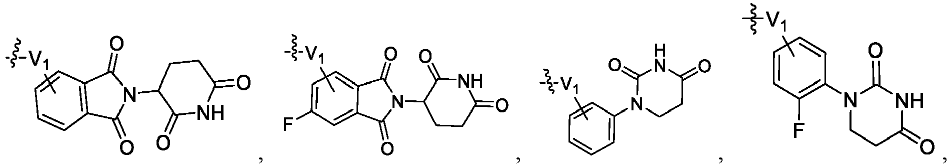 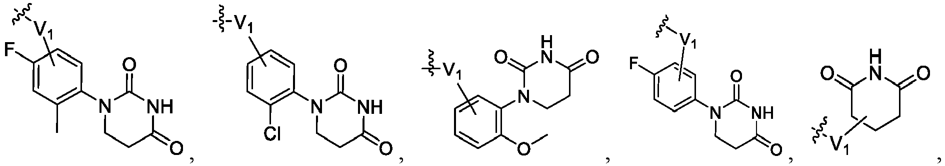 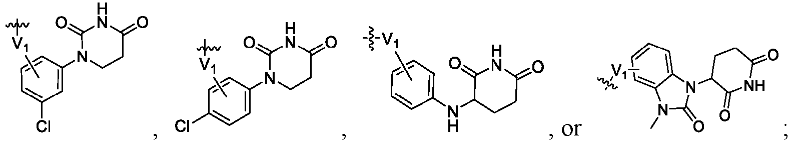 and
V₁ is absent or is selected from -O-, -CH₂-, -CH=CH- or -NH-.

In some embodiments, said B is selected from: 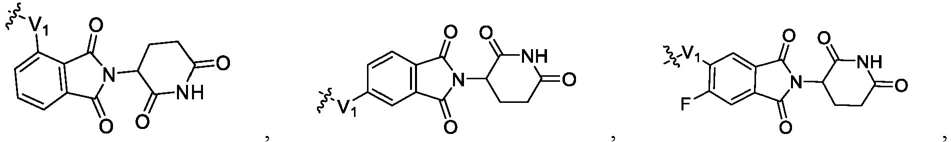 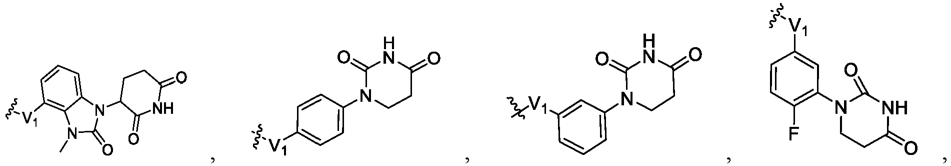 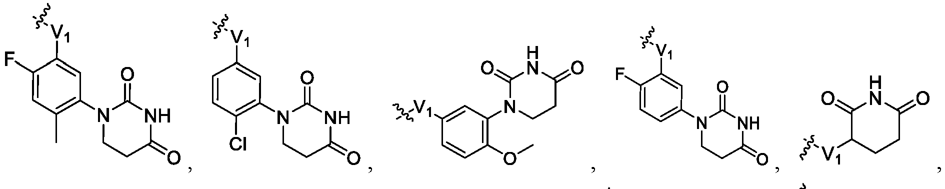 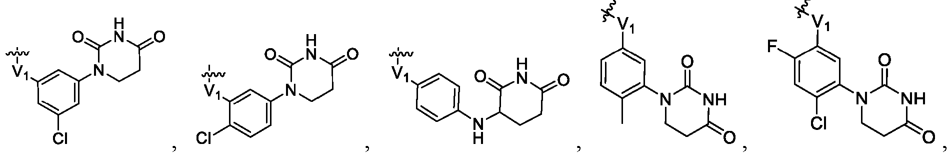 or 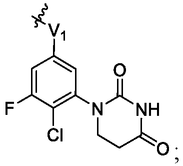 and
V₁ is absent, -O-, -CH₂-, or -NH-, preferably is absent.

Still further, said B is selected from: 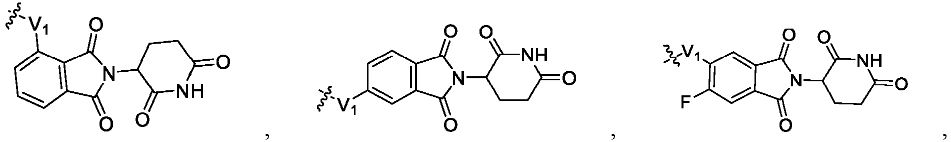 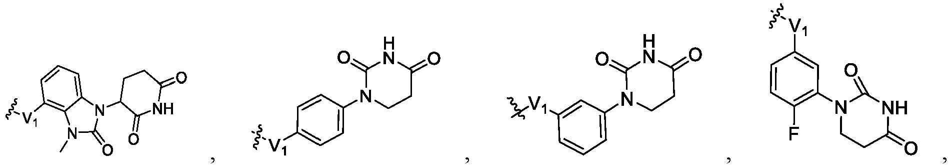 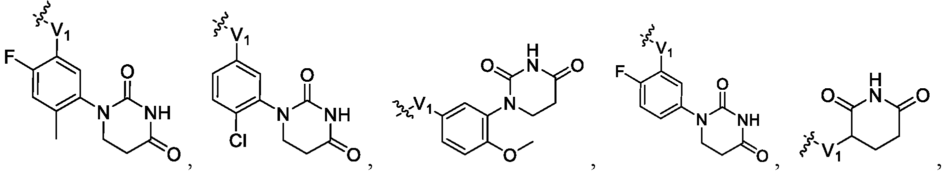 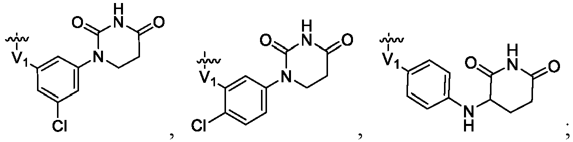 and
V₁ is absent or selected from -O-, -CH₂-, or -NH-, preferably is absent.

In some embodiments, said B is as shown below: 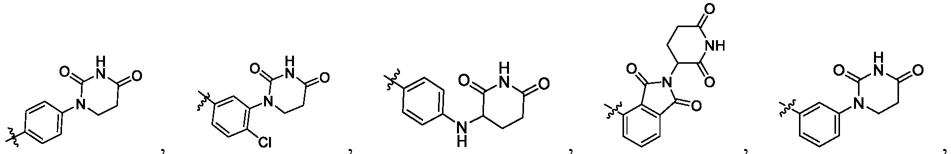 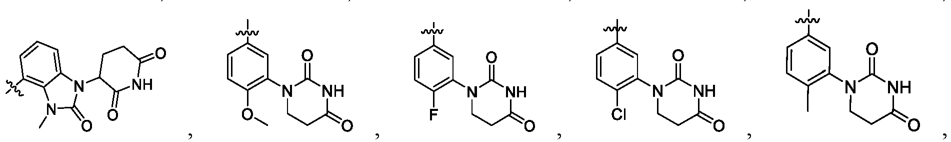 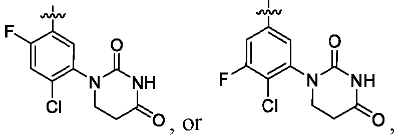 preferably

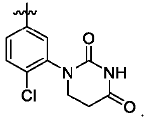

In some embodiments, said B is as shown below:

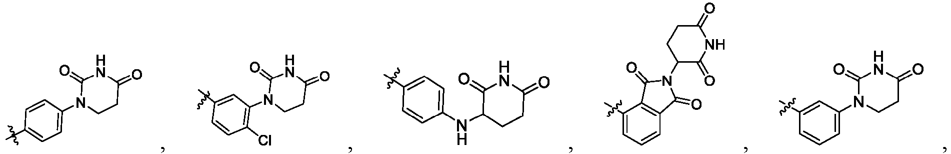

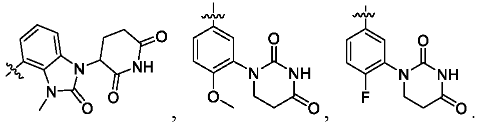

In other some embodiments, said B is of a formula as shown below: 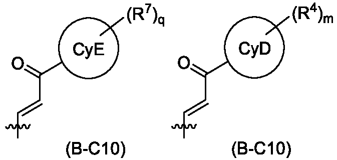 wherein:
CyD, R⁴ and m are as defined in the first aspect of the Section "***S1. HPK1 Ligand***" above.

In some of such embodiments, said CyD is phenyl; R⁴ is -OR^{4f}, wherein R^{4f} is selected from hydrogen, -C₁₋₈ alkyl, preferably -C₁₋₃ alkyl, more preferably methyl or ethyl; and m is 1.

In some of such embodiments, said B is as shown below:

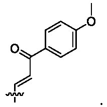

### S3. Linking group L

L is a linking group between the group B and the HPK1 ligand.

In some embodiments, said L is (LNK)ᵤ; wherein:
each LNK is independently absent, or is selected from C₁₋₈ alkylene, C₂₋₈ alkynylene, 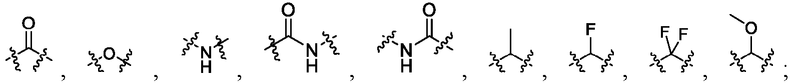 or cycloalkyl, heterocyclyl, heteroaryl, or aryl, which is optionally substituted with one or more halogen or C₁₋₈ alkyl, wherein the cycloalkyl is preferably selected from 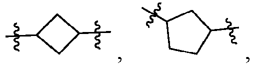 or 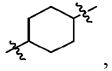 wherein the heterocyclyl is preferably selected from 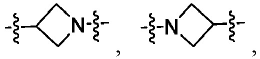 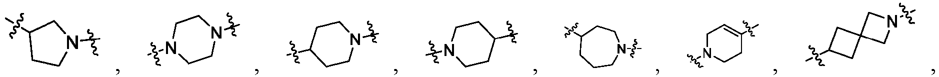 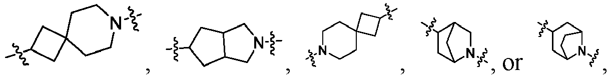 and wherein the aryl is preferably selected from a benzene ring;
m is selected from an integer between 1 and 8; and
u is an integer between 1 and 20.

In some embodiments, said L is (LNK)ᵤ; wherein:
each LNK is independently absent, or is selected from C₁₋₈ alkylene, C₂₋₈ alkynylene, 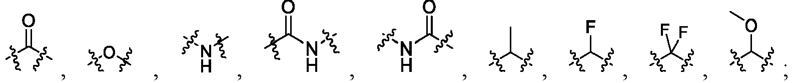 or cycloalkyl, heterocycloalkyl, a heteroaryl ring or an aryl ring, which is optionally substituted with one or more halogen or C₁₋₈ alkyl, wherein the cycloalkyl is selected from 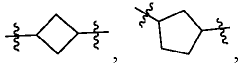 or 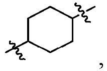 wherein the heterocycloalkyl is selected from 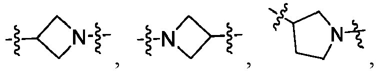 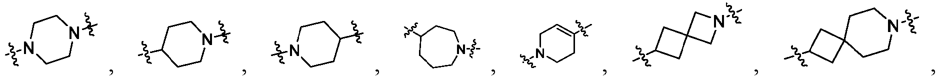 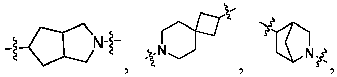 or , and wherein the aryl ring is selected from a benzene ring;
m is selected from an integer between 1 and 8;
u is an integer between 1 and 20.

In some embodiments of the present invention, said L is selected from:

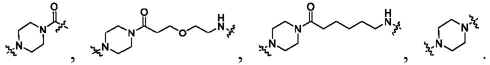

In some embodiments of the present invention, said L is selected from:

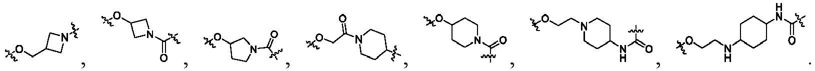

In some embodiments of the present invention, said L is selected from:

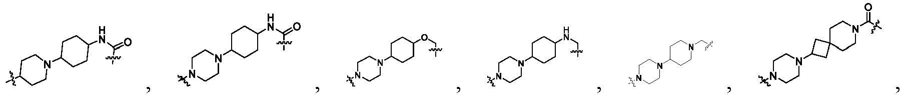

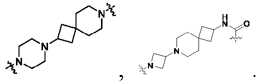

In some embodiments of the present invention, said L is:

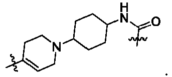

In some embodiments of the present invention, said L is selected from: 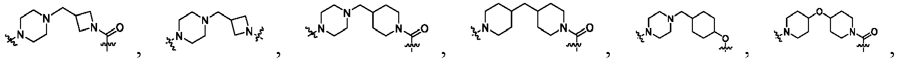 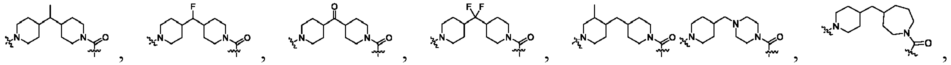 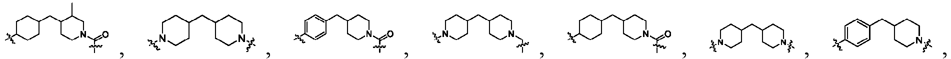 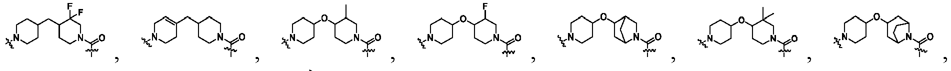 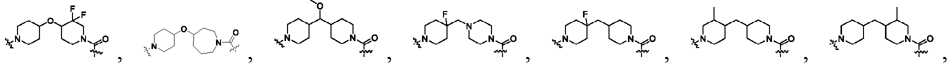 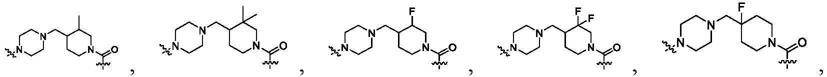 preferably

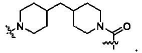

In some embodiments of the present invention, said L is selected from: 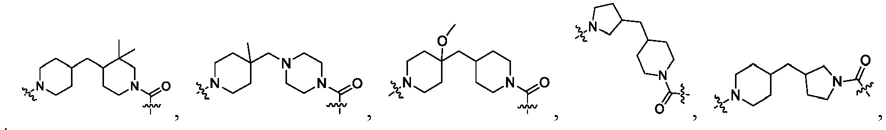 or

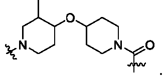

In some embodiments of the present invention, said L is selected from:

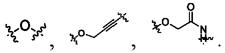

In some embodiments of the present invention, said L is selected from:

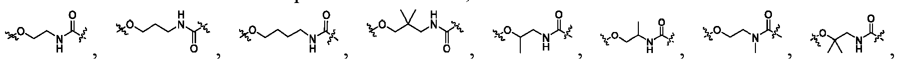

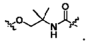

In some embodiments of the present invention, said L is selected from:

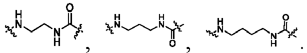

In some embodiments of the present invention, said L is selected from:

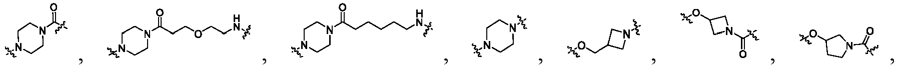

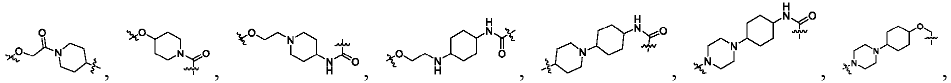

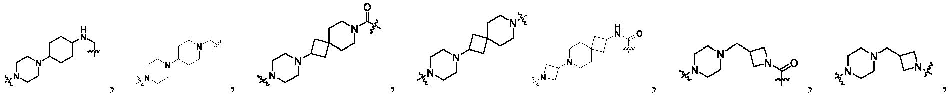

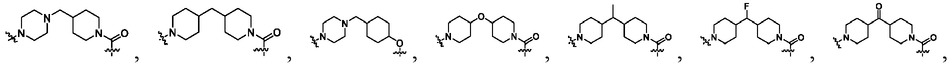

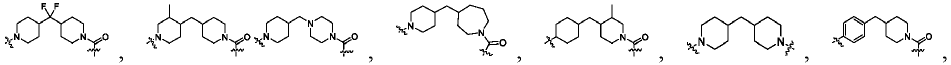

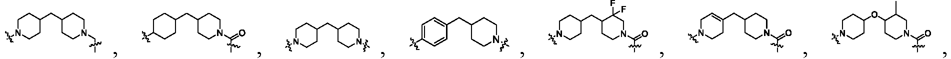

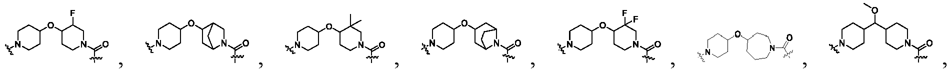

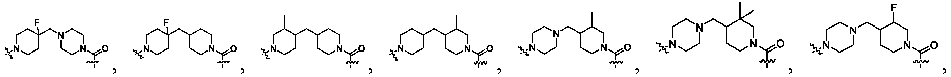

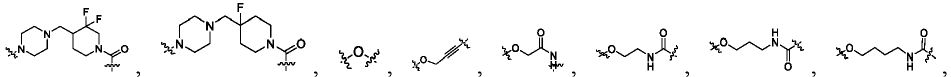

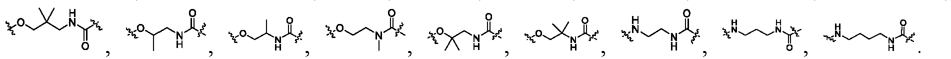

In some embodiments of the present invention, the compound of general formula (I) is a compound of formula (II): 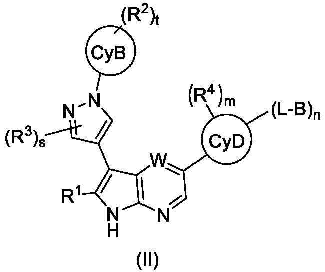 wherein:
said W, R¹, R², t, R³, s, CyB, R⁴, m, and CyD are each as defined in the first aspect of the Section *"**S1. HPK1 Ligand"*** above; and
said L, B and n are as defined herein.

In particular, said B is as defined in the Section ***"S2. Degradation Tag B".*** In particular, said L is as defined in the Section ***"S3. Linker L".***

In some of such embodiments, said CyB is selected from

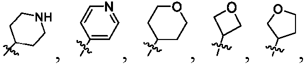

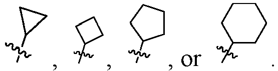

In some embodiments, said CyB is selected from

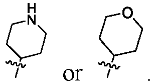

In some embodiments, said CyB is 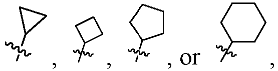 preferably

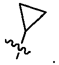

In some embodiments, the 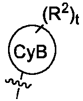 is selected from 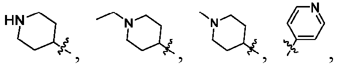 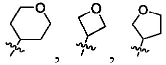 (including 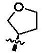 and

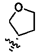

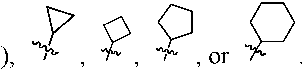

In some embodiments, the 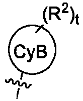 is selected from

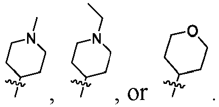

In some embodiments, the 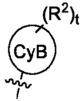 is 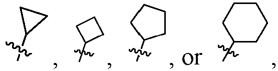 preferably

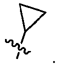

In some embodiments, the W is N.

In some embodiments, said R¹ and R³ are each hydrogen.

In some embodiments, said CyD is pyridyl, preferably

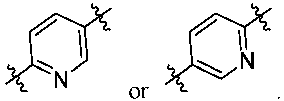

In exemplary embodiments, said R⁴ is hydrogen.

In some embodiments, said L is

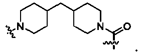

In some embodiments, said B is: 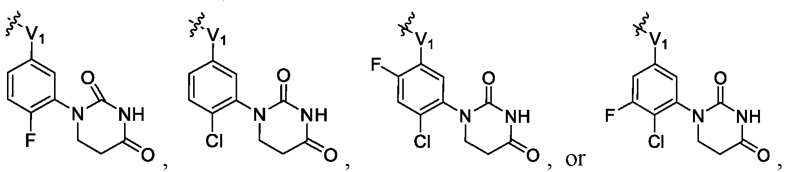 preferably 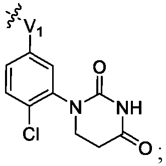 and
V₁ is absent, -O-, -CH₂-, or -NH-, preferably absent.

Preferably, said B is 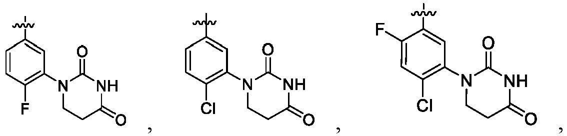 or 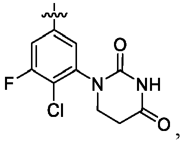 more preferably

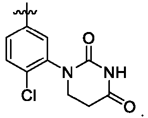

In some embodiments, n is 1.

In some embodiments, the compound of general formula (I) is a compound of formula (III): 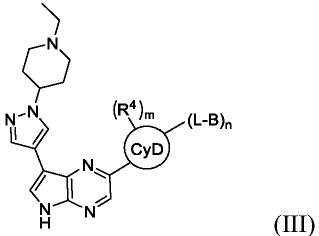 wherein:
said R⁴, m, and CyD are each as defined in the second aspect of the Section "***S1. HPK1 Ligand***" above; and
said L, B and n are as defined herein.

In particular, said B is as defined in the Section ***"S2. Degradation Tag B".*** In particular, said L is as defined in the Section ***"S3. Linker L".***

In some embodiments, the compound of general formula (I) is a compound of formula (IV): 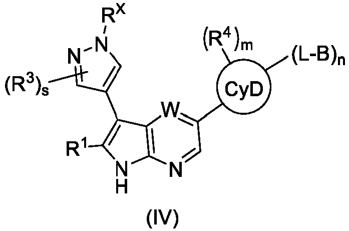 wherein:
said W, R¹, R³, s, R⁴ and CyD are each as defined in the first aspect and the second aspect of the Section ***"S1. HPK1 Ligand"*** above; and
said R^{X} is as defined in the second aspect of the Section "***S1. HPK1 Ligand***" above;
said L, B and n are as defined herein.

In particular, said B is as defined in the Section ***"S2. Degradation Tag B".*** In particular, said L is as defined in the Section ***"S3. Linking group L".***

In some such embodiments, said W is N.

In some embodiments, said R¹ is hydrogen or -C₁₋₃ alkyl, preferably hydrogen.

In some embodiments, said R³ is selected from hydrogen or -C₁₋₃ alkyl, preferably hydrogen.

In some embodiments, said s is 0 or 1.

In some embodiments, R^{X} is selected from a C₁₋₄ alkyl, wherein the C₁₋₄ alkyl is optionally substituted with 1, 2, or 3 substituents independently selected from deuterium, F, Cl, Br, -OH, -CN, -NR^{Xa}R^{Xb}, or -OR^{Xa}. In some of such embodiments, the C₁₋₄ alkyl is selected from methyl, ethyl, isopropyl, isobutyl, sec-butyl, and tert-butyl, preferably methyl or ethyl.

In some embodiments, R^{Xa} and R^{Xb} are each independently selected from C₁₋₄ alkyl, preferably methyl.

In some embodiments, R^{X} is selected from methyl or ethyl, wherein the methyl or ethyl is optionally substituted with 1, 2, or 3 substituents independently selected from deuterium, F, Cl, -OH, -CN, and -OCH₃. In some embodiments, R^{X} is selected from methyl or ethyl, wherein the methyl or ethyl is optionally substituted with 1, 2, or 3 substituents independently selected from deuterium, F, and -OCH₃. In some embodiments, R^{X} is selected from methyl, -CD₃, ethyl, - CH₂F, -CHF₂, -CF₃, -CH₂CH₂F, -CH₂CHF₂, -CH₂CF₃, or 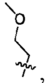 preferably, methyl, -CD₃, ethyl, -CH₂CH₂F, -CH₂CHF₂, -CH₂CF₃, or

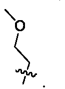

In some embodiments, the CyD is selected from 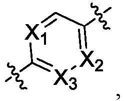 wherein X₁, X₂, and X₃ are each independently selected from CH, C, or N, provided that at least one of X₁, X₂, and X₃ is N.

In some embodiments, the CyD is selected from 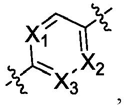 and X₁, X₂, and X₃ are each independently selected from C or N, provided that X₁, X₂, and X₃ cannot be C at the same time.

In some embodiments, CyD is selected from pyridyl, preferably 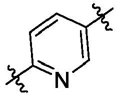 or

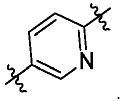

In some embodiments, R⁴ is independently selected from hydrogen or -C₁₋₃ alkyl, preferably hydrogen.

In some embodiments, m is 0 or 1.

In some embodiments, said L is

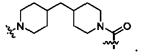

In some embodiments, said B is: 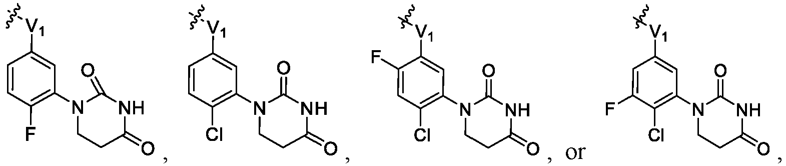 preferably 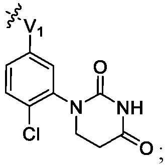 and
V₁ is absent, -O-, -CH₂-, or -NH-, preferably absent.

Preferably, said B is 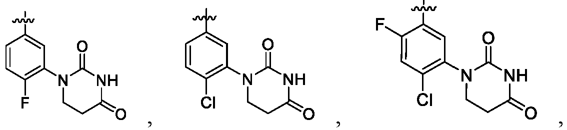 or 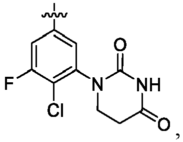 more preferably

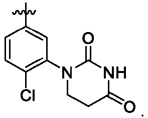

In some embodiments, n is 1.

The present invention provides the compound as described above, or a pharmaceutically acceptable salt or a stereoisomer thereof, selected from the exemplified compounds disclosed herein, as shown in Table 2 below.

**Table 2:**

| | |
|---|---|
| 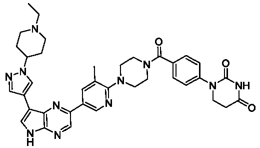 C001 | 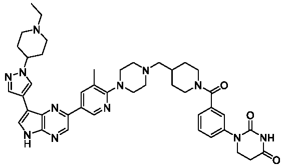 C002 |
| 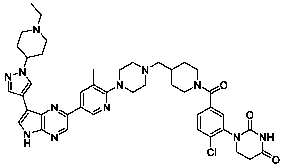 C003 | 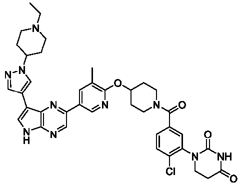 C004 |
| 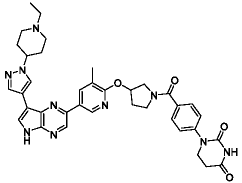 C005 | 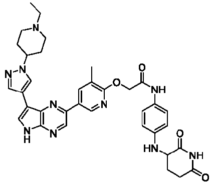 C006 |
| 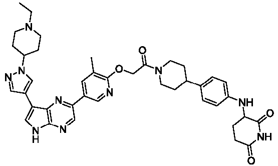 C007 | 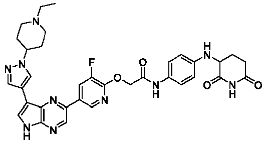 C008 |
| 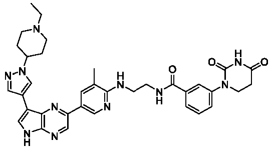 C009 | |
| 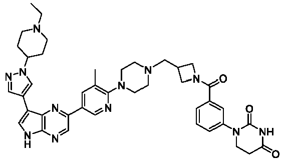 C011 | 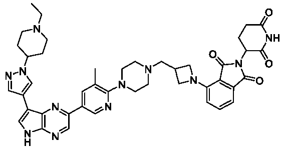 C012 |
| 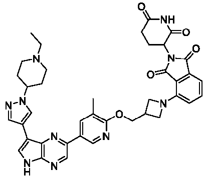 C013 | 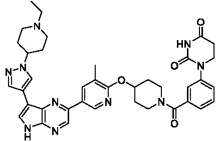 C014 |
| 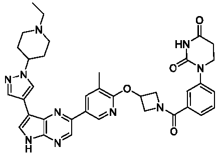 C015 | 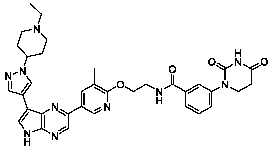 C016 |
| 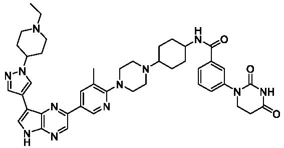 C017 | 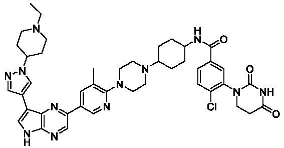 C018 |
| 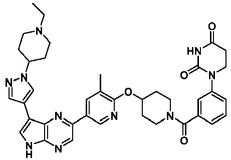 C019 | 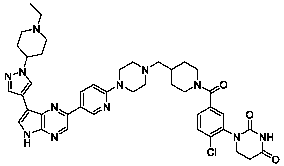 C020 |
| 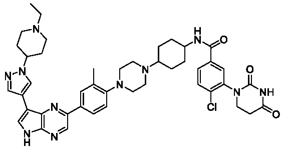 C021 | 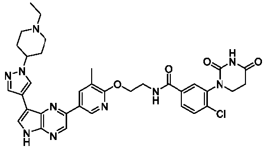 C022 |
| 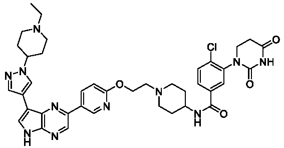 C023 | 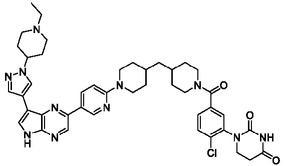 C024 |
| 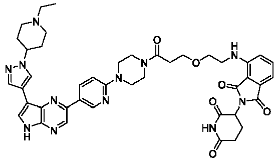 C025 | 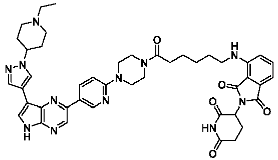 C026 |
| 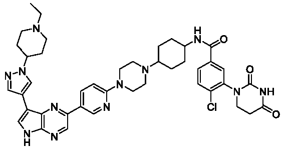 C027 | 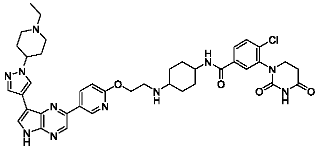 C028 |
| 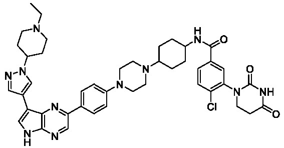 C029 | 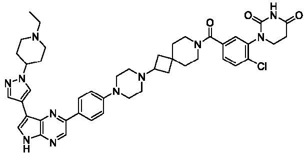 C030 |
| 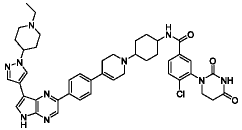 C031 | 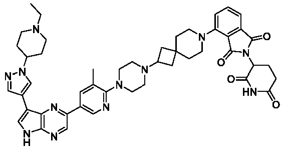 C032 |
| 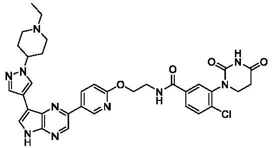 C033 | 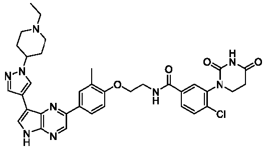 C034 |
| 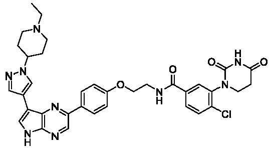 C035 | 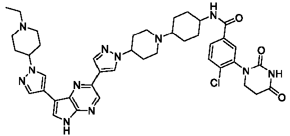 C036 |
| 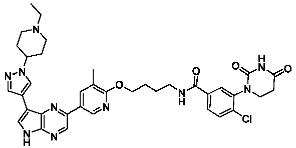 C037 | 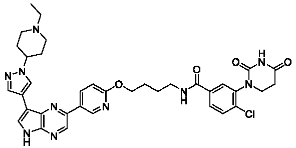 C038 |
| 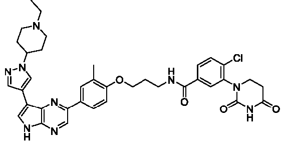 C039 | 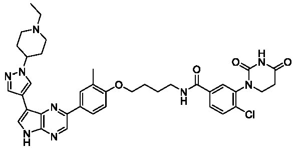 C040 |
| 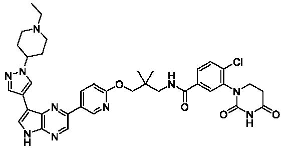 C041 | 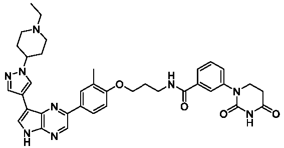 C042 |
| 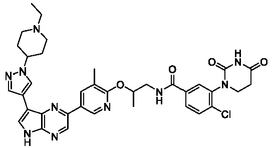 C043 | 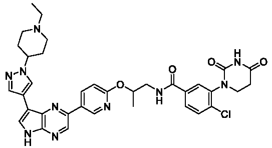 C044 |
| 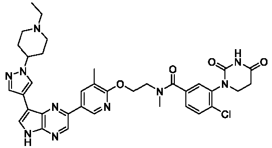 C045 | 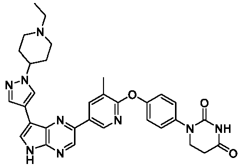 C046 |
| 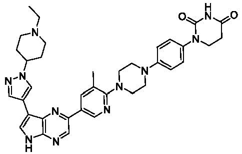 C047 | 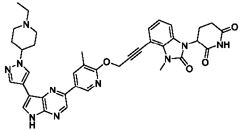 C048 |
| 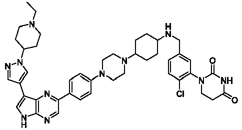 C049 | 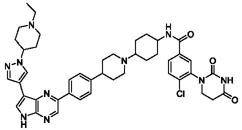 C050 |
| 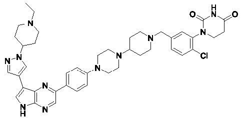 C051 | 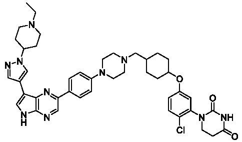 C052 |
| 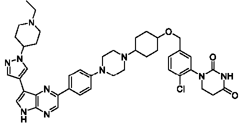 C053 | 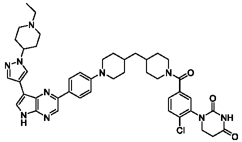 C054 |
| 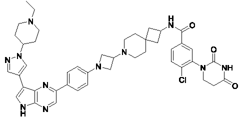 C055 | 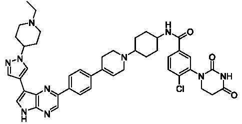 C056 |
| 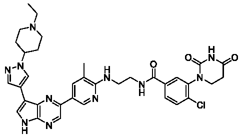 C057 | 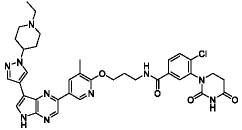 C058 |
| 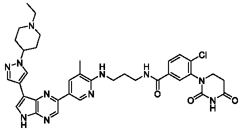 C059 | 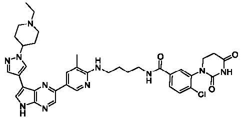 C060 |
| 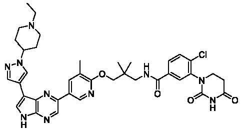 C061 | 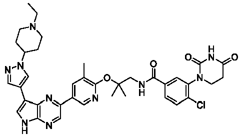 C062 |
| 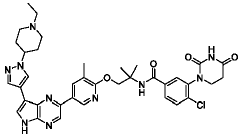 C063 | 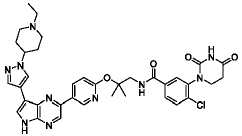 C064 |
| 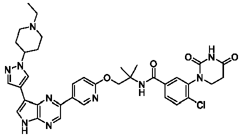 C065 | 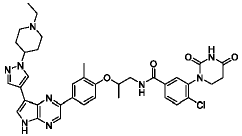 C066 |
| 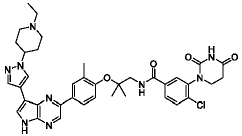 C067 | 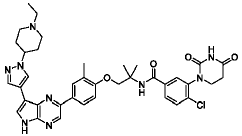 C068 |
| 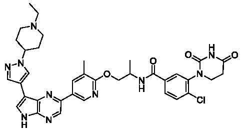 C069 | 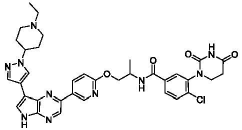 C070 |
| 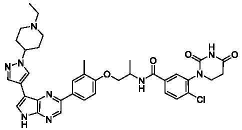 C071 | 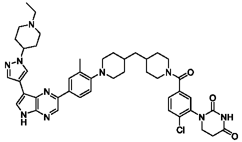 C072 |
| 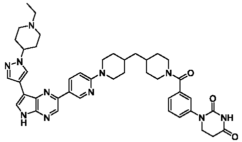 C073 | 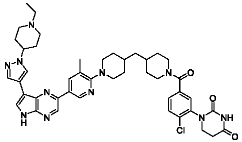 C074 |
| 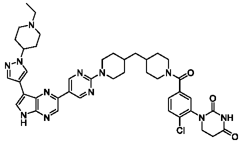 C075 | 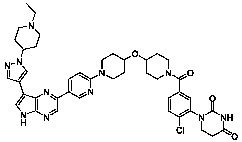 C076 |
| 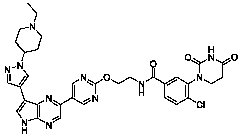 C077 | 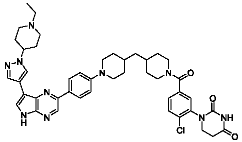 C078 |
| 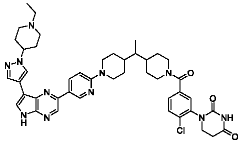 C079 | 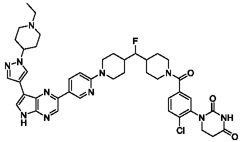 C080 |
| 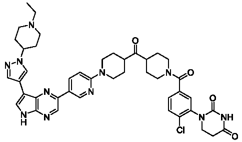 C081 | 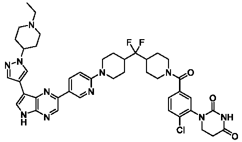 C082 |
| 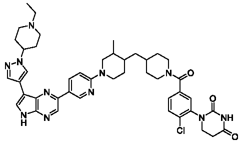 C083 | 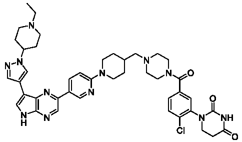 C084 |
| 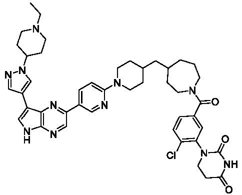 C085 | 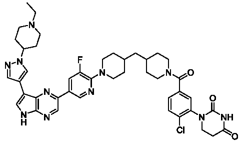 C086 |
| 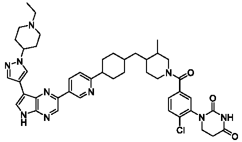 C087 | 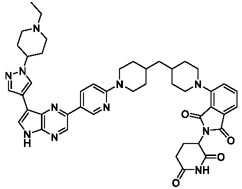 C088 |
| 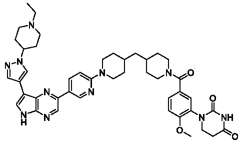 C089 | 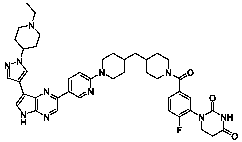 C090 |
| 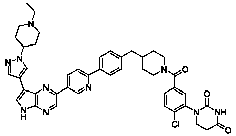 C091 | 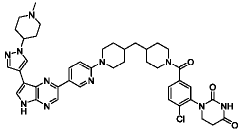 C092 |
| 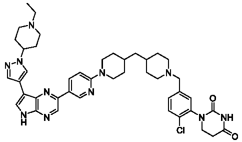 C093 | 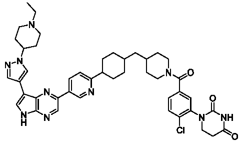 C094 |
| 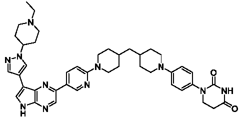 C095 | 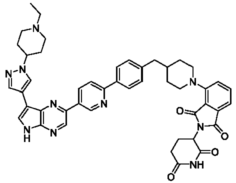 C096 |
| 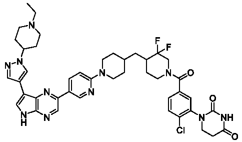 C097 | 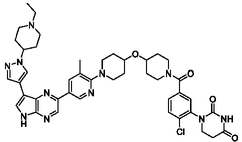 C098 |
| 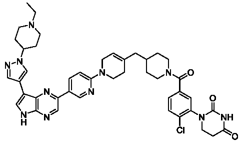 C099 | 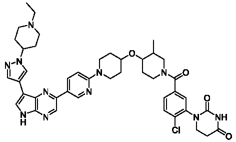 C100 |
| 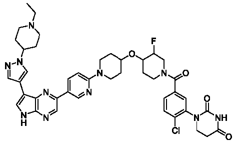 C101 | 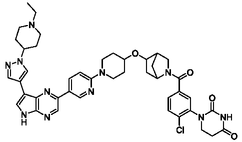 C102 |
| 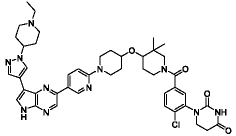 C103 | 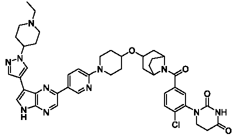 C104 |
| 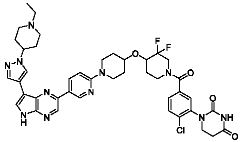 C105 | 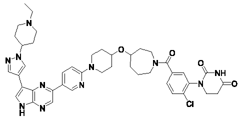 C106 |
| 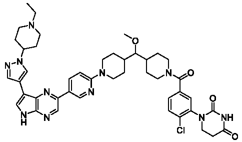 C107 | 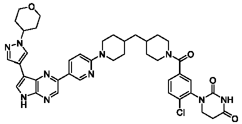 C108 |
| 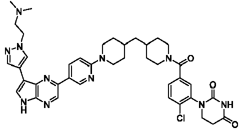 C109 | 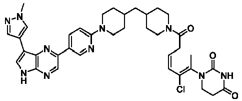 C110 |
| 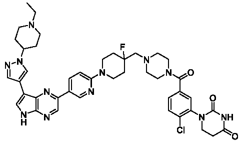 C111 | 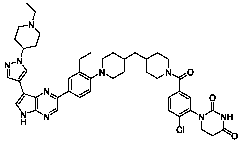 C112 |
| 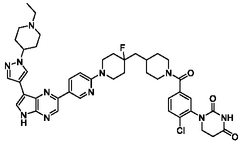 C113 | 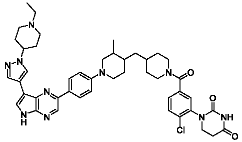 C114 |
| 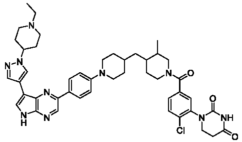 C115 | 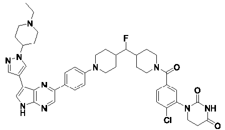 C116 |
| 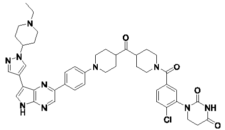 C117 | 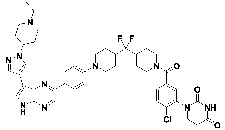 C118 |
| 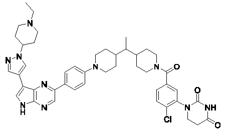 C119 | 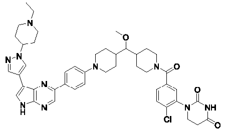 C120 |
| 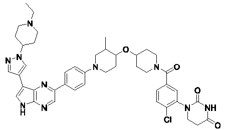 C121 | 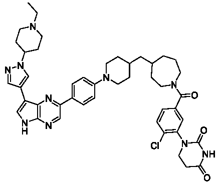 C122 |
| 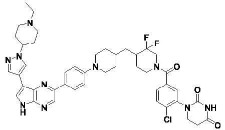 C123 | 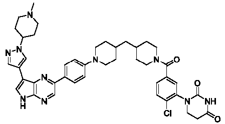 C124 |
| 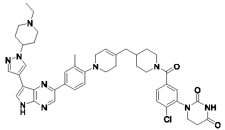 C125 | 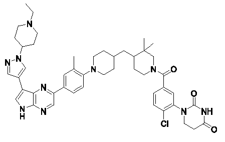 C126 |
| 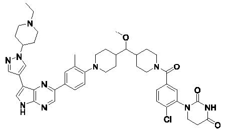 C127 | 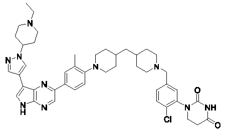 C128 |
| 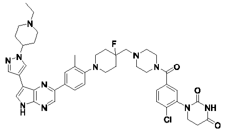 C129 | 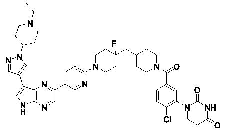 C130 |
| 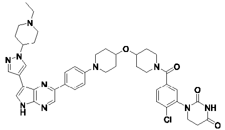 C131 | 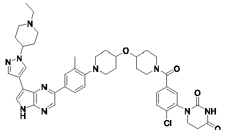 C132 |
| 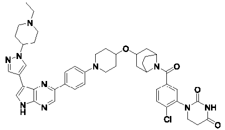 C133 | 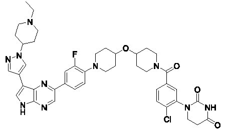 C134 |
| 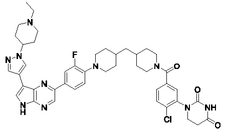 C135 | 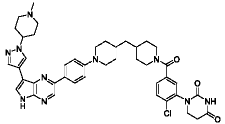 C136 |
| 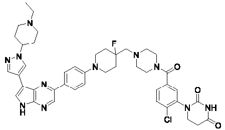 C137 | 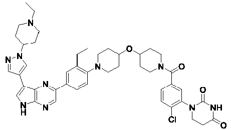 C138 |
| 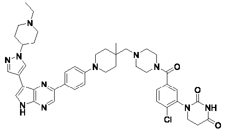 C139 | 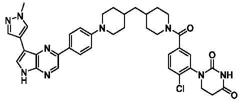 C140 |
| 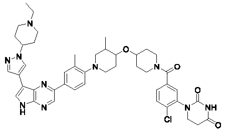 C141 | 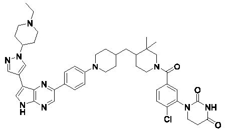 C142 |
| 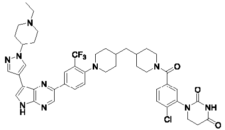 C143 | 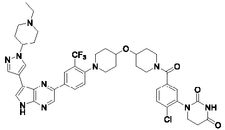 C144 |
| 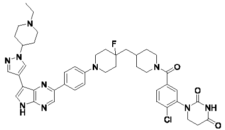 C145 | 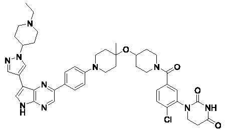 C146 |
| 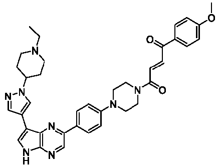 C147 | 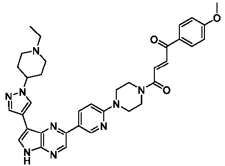 C148 |
| 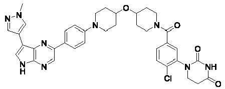 C149 | 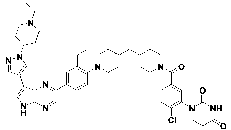 C150 |
| 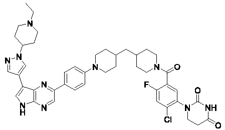 C151 | 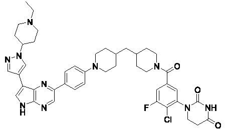 C152 |
| 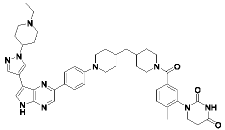 C153 | 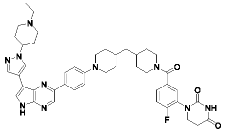 C154 |
| 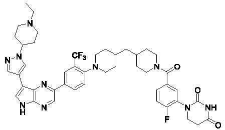 C155 | 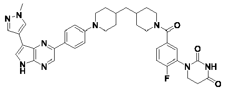 C156 |
| 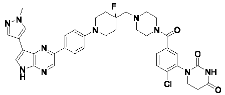 C157 | 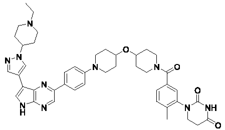 C158 |
| 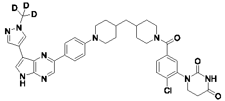 C159 | 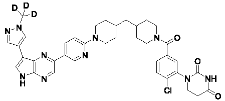 C160 |
| 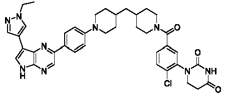 C161 | 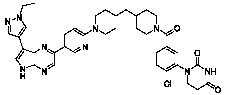 C162 |
| 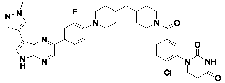 C163 | 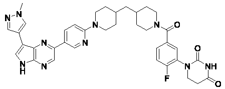 C164 |
| 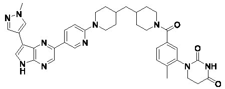 C165 | 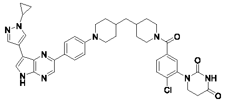 C166 |
| 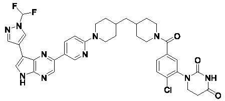 C167 | 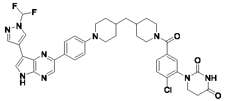 C168 |
| 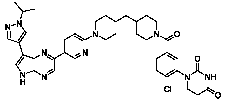 C169 | 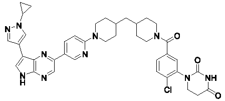 C170 |
| 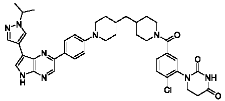 C171 | 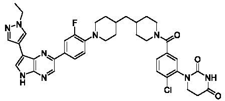 C172 |
| 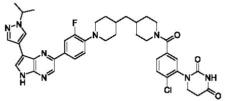 C173 | 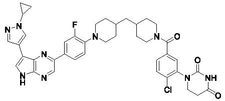 C174 |
| 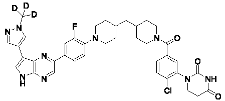 C175 | 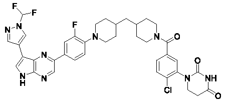 C176 |
| 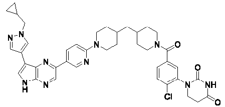 C177 | 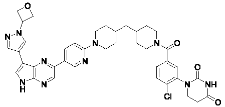 C178 |
| 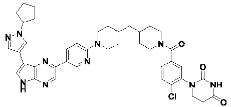 C179 | 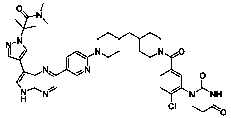 C180 |
| 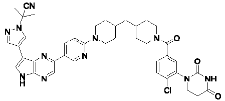 C181 | 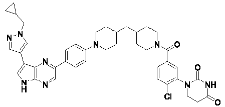 C182 |
| 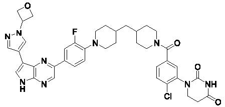 C183 | 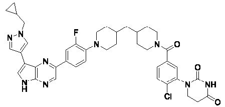 C184 |
| 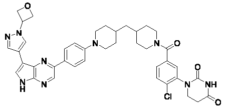 C185 | 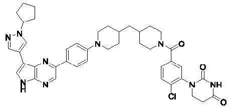 C186 |
| 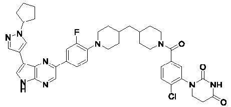 C187 | 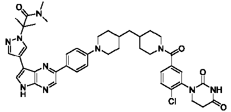 C188 |
| 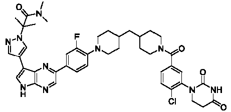 C189 | 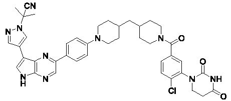 C190 |
| 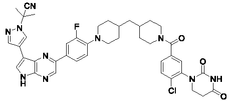 C191 | 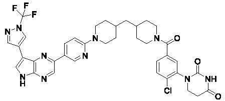 C192 |
| 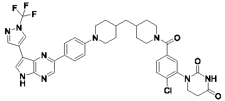 C193 | 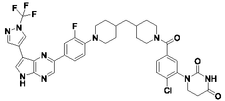 C194 |
| 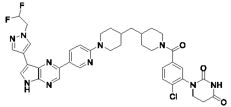 C195 | 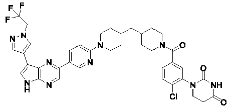 C196 |
| 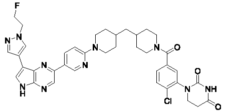 C197 | 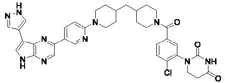 C198 |
| 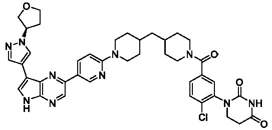 C199 | 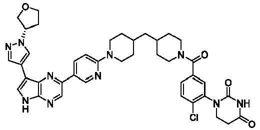 C200 |
| 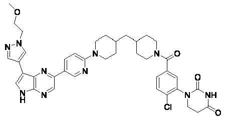 C201 | 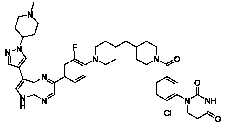 C202 |
| 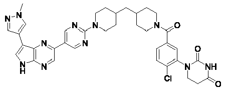 C203 | 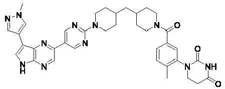 C204 |
| 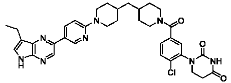 C205 | 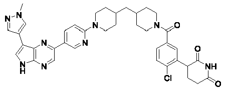 C206 |
| 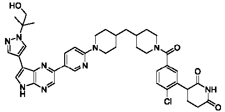 C207 | 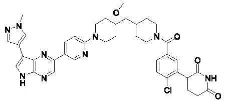 C208 |
| 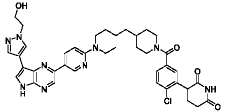 C209 | 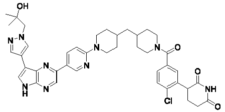 C210 |
| 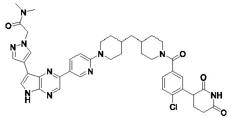 C211 | 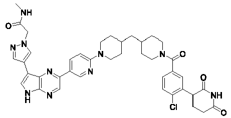 C212 |
| 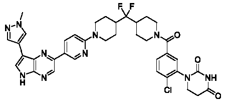 C213 | 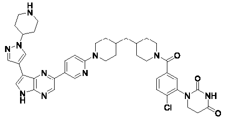 C214 |
| 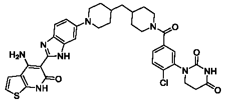 C215 | 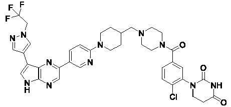 C216 |
| 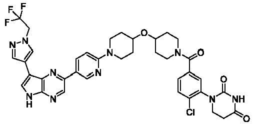 C217 | 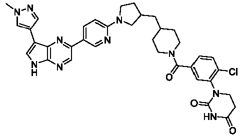 C218 |
| 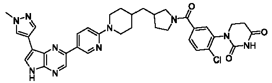 C219. | |

### Pharmaceutical composition and use

The present invention also provides a pharmaceutical composition comprising any of the compounds according to the present invention, or a pharmaceutically acceptable salt thereof, or a stereoisomer thereof. The pharmaceutical composition optionally comprises one or more pharmaceutically acceptable carriers, and is prepared into any pharmaceutically acceptable pharmaceutical formulation.

The present invention also provides a pharmaceutical formulation of any of the compounds according to the present invention, or a pharmaceutically acceptable salt thereof, or a stereoisomer thereof, which may optionally comprise one or more pharmaceutically acceptable carriers.

The compound of the general formula (I) according to the present invention, or a pharmaceutically acceptable salt thereof, or a stereoisomer thereof has excellent, highly selective inhibitory activity against HPK1 and can treat and/or prevent diseases such as tumors, immunological diseases, inflammatory diseases.

The present invention provides a method of degrading/inhibiting HPK1 activity comprising administering to a subject the compound according to the present invention, or a pharmaceutically acceptable salt thereof, or a stereoisomer thereof, including the compound of general formula (I) or the specific compound exemplified herein.

The compound provided by the present invention has a good HPK1 inhibitory/degradative activity and has good physicochemical properties and drugable properties. The compound of the present invention has good potential in the treatment of diseases mediated by HPK1.

WO2021057872 A1 discloses a PROTAC compound formed by an inhibitor of the MAP4K family and a Cereblon protein ligand. The present inventor has surprisingly found that the compound of the present invention shows improved HPK1 selectivity.

The present invention also provides a method for treating a patient suffering from a disease that can be modulated by HPK1, comprising administering to the patient an effective amount of the compound according to the present invention (such as the compound in accordance with the general formula (I), or the specific compound exemplified herein), or a pharmaceutically acceptable salt thereof, or a stereoisomer thereof.

The present invention also provides a method for inhibiting/degrading HPK1 activity in a patient in need of inhibition of the HPK1 activity, comprising administering to the patient an effective amount of the compound according to the present invention (such as the compound in accordance with general formula (I) or the specific compound exemplified herein), or a pharmaceutically acceptable salt thereof, or a stereoisomer thereof.

The present invention also provides a use of any one of the compounds according to the present invention, or a pharmaceutically acceptable salt thereof, or a stereoisomer thereof, in the manufacture of a medicament for the treatment or prophylaxis of a related HPK1-mediated disease. HPK1 has a negative feedback regulatory effect in the T cell-mediated signaling pathway. Therefore, the compound according to the present invention can be applied as an antitumor agent in the treatment of cancers or non-cancerous proliferative diseases. Further, the HPK1-mediated disease includes, but is not limited to, lung cancer, squamous epithelial cell carcinoma, bladder cancer, stomach cancer, ovarian cancer, peritoneal cancer, breast cancer, breast ductal carcinoma, head and neck cancer, endometrial carcinoma, carcinoma of corpus uteri, rectal cancer, liver cancer, kidney cancer, renal pelvis carcinoma, esophageal carcinoma, esophageal adenocarcinoma, glioma, prostate cancer, thyroid cancer, cancers of the female reproductive system, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin's lymphoma, villous adenoma of the large intestine, melanoma, cytoma and sarcoma, and myelodysplastic syndrome.

The present invention also provides a method for the prophylaxis and/or treatment of an HPK1-mediated diseases and a related disease, comprising administering to a subject a therapeutically effective amount of the compound according to the present invention, or a pharmaceutically acceptable salt thereof, or a stereoisomer thereof, or a composition comprising the compound according to the present invention. Further, the HPK1-mediated disease and related disease are selected from lung cancer, squamous epithelial cell carcinoma, bladder cancer, stomach cancer, ovarian cancer, peritoneal cancer, breast cancer, breast ductal carcinoma, head and neck cancer, endometrial carcinoma, carcinoma of corpus uteri, rectal cancer, liver cancer, kidney cancer, renal pelvis carcinoma, esophageal carcinoma, esophageal adenocarcinoma, glioma, prostate cancer, thyroid cancer, cancers of the female reproductive system, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin's lymphoma, villous adenoma of the large intestine, melanoma, cytoma and sarcoma, and myelodysplastic syndrome.

The present invention also provides a method for treating a patient with a cancer, comprising administering to the patient an effective amount of the compound according to the invention (e.g., the compound of general formula (I)), or a pharmaceutically acceptable salt thereof, or a stereoisomer thereof.

### Definitions

The compounds as described in the present invention are named according to the chemical structural formulas. If the naming of the compounds does not conform to the chemical structural formulas when representing the same compound, the chemical structural formulas shall prevail.

In the present invention, unless otherwise specified, the scientific and technical terms used herein have the meanings as commonly understood by those skilled in the art. However, definitions of some terms are provided below for a better understanding of the present invention. Where the definitions and interpretations of the terms provided herein differ from those commonly understood by those skilled in the art, the definitions and explanations of the terms provided herein shall prevail.

As used herein (including the aspects as set forth), the singular forms such as "a", "an" and "the" include plural corresponding referents unless the context clearly dictates otherwise.

The term "or" is used to mean, and is used interchangeably with, the term "and/or", unless the context clearly dictates otherwise.

The term "optional", "optionally" or "optional" means that a subsequently described event or condition may, but not necessarily, occur and that the description includes cases in which the event or condition occurs and cases in which the event or condition does not occur.

The term "substituted" means that any one or more hydrogen atoms on a particular atom are substituted by a substituent which may include heavy hydrogen and variants of hydrogen, as long as the valence state of the particular atom is normal and the substituted compound is stable. When the substituent is oxo (i.e., = O), it means that two hydrogen atoms are substituted. Oxo substitution does not occur on aromatic groups. The term "optionally substituted" refers to being substituted or unsubstituted. Unless otherwise specified, the kind and number of the substituent may be arbitrary on a chemically achievable basis.

The term "alkyl" refers to a hydrocarbon group selected from straight and branched saturated hydrocarbon groups comprising 1 to 18 (such as 1 to 12, further, such as 1 to 10, still further, such as 1 to 8, or 1 to 6, or 1 to 4, or 1 to 3, or 1 to 2) carbon atoms.

The term "halogen" means fluorine (F), chlorine (Cl), bromine (Br), and iodine (I).

The term "haloalkyl" means an alkyl group in which one or more hydrogens have been replaced by one or more halogen atoms (such as fluorine, chlorine, bromine, and iodine). Examples of the haloalkyl include C₁₋₈ haloalkyl, C₁₋₆ haloalkyl, or C₁₋₄ haloalkyl, but are not limited to -CF₃, -CH₂Cl, -CH₂CF₃, -CHCl₂, etc.

The term "alkenyl" refers to a hydrocarbon group selected from straight and branched hydrocarbon groups comprising at least one C=C double bond and 2 to 18 (such as 2 to 8, further, such as 2 to 6) carbon atoms.

The term "alkynyl" refers to a hydrocarbon radical selected from straight and branched hydrocarbon groups comprising at least one C≡C triple bond and 2 to 18 (such as 2 to 8, further, such as 2 to 6) carbon atoms.

The term "alkyloxy" or "alkoxy" refers to an alkyl group as defined above that is attached to the parent molecule moiety via an oxygen atom. Examples of the alkyloxy (e.g., C₁₋₆ alkyloxy or C₁₋₄ alkyloxy) include, but are not limited to, methoxy, ethoxy, isopropoxy, propoxy, n-butoxy, tert-butoxy, pentyloxy, hexyloxy, and the like.

The term "alkoxy-alkyl-" refers to an alkyl group as defined above that is substituted with an alkoxy group as defined above. Examples of the alkoxy-alkyl- (e.g. C₁₋₈ alkoxy-C₁₋₈ alkyl-) include, but are not limited to, methoxymethyl, ethoxymethyl, isopropoxymethyl, or propoxymethyl, and the like.

The term "cycloalkyl" refers to a hydrocarbon group selected from saturated cyclic hydrocarbon groups, including monocyclic and polycyclic (e.g., bicyclic and tricyclic) groups. It includes fused cycloalkyls, bridged cycloalkyls, or spiro-cycloalkyls.

For example, the cycloalkyl may contain 3 to 12 (such as 3 to 10, further, such as 3 to 8, further, such as 3 to 6, 3 to 5, or 3 to 4) carbon atoms. Even further, for example, the cycloalkyl may be selected from monocyclic groups comprising 3 to 12 (such as 3 to 10, further, such as 3 to 8, 3 to 6) carbon atoms. Examples of the monocyclic cycloalkyl groups include cyclopropyl, cyclobutyl, cyclopentyl, cyclohexyl, cyclohexadienyl, cycloheptyl, cyclooctyl, cyclononyl, cyclodecyl, cycloundecyl, and cyclododecyl. Particularly, examples of the saturated monocyclic cycloalkyl (such as C₃₋₈ cycloalkyl) include, but are not limited to, cyclopropyl, cyclobutyl, cyclopentyl, cyclohexyl, cycloheptyl, and cyclooctyl.

The term "spiro-cycloalkyl" refers to a cyclic structure containing carbon atoms and formed from at least two rings that share one atom. For example, a 7- to 12-membered spiro-cycloalkyl refers to a cyclic structure containing from 7 to 12 carbon atoms and formed from at least two rings that share one atom.

The term "fused cycloalkyl" refers to a fused ring containing carbon atoms and formed from two or more rings that share two adjacent atoms. For example, a 4- to 10-membered fused cycloalkyl refers to a fused ring containing 4 to 10 ring carbon atoms and formed from two or more rings that share two adjacent atoms.

The term "bridged cycloalkyl" refers to a cyclic structure containing carbon atoms and formed from two rings which share two atoms that are not adjacent to each other. For example, a 7- to 10-membered bridged cycloalkyl refers to a cyclic structure containing from 7 to 12 carbon atoms and formed from two rings which share two atoms that are not adjacent to each other.

The term "cycloalkenyl" refers to a non-aromatic cyclic alkyl of 3 to 10 carbon atoms having a single ring or multiple rings and having at least one double bond and preferably 1 to 2 double bonds. In one embodiment, the cycloalkenyl is cyclopentenyl (1-cyclopent-1-enyl, 1-cyclopent-2-enyl, 1-cyclopent-3-enyl) or cyclohexenyl (1-cyclohex-1-enyl, 1-cyclohex-2-enyl, 1-cyclohex-3-enyl), preferably cyclohexenyl.

The term "cycloalkynyl" refers to a non-aromatic cycloalkyl group of 5 to 10 carbon atoms having a single ring or multiple rings and having at least one triple bond.

The term "aryl", either used alone or in combination with other terms, refers to a group selected from:
- 5- and 6-membered carbocyclic aromatic rings such as phenyl;
- a bicyclic ring system such as a 7- to 12-membered bicyclic ring system, wherein at least one ring is carbocyclic and aromatic, such as naphthyl and indanyl; and
- a tricyclic ring system such as 10- to 15-membered tricyclic ring system, wherein at least one ring is carbocyclic and aromatic, such as fluorenyl.

The terms "aromatic hydrocarbon ring" and "aryl" can be used interchangeably herein. In some embodiments, a monocyclic or bicyclic aromatic hydrocarbon ring has 5 to 10 ring-forming carbon atoms (i.e., C₅₋₁₀ aryl). Examples of the monocyclic or bicyclic aromatic hydrocarbon ring include, but are not limited to, phenyl, naphthalen-1-yl, naphthalen-2-yl, anthracenyl, phenanthrenyl, etc. In some embodiments, the aromatic hydrocarbon ring is a naphthalene ring (naphthalen-1-yl or naphthalen-2-yl), or a phenyl ring. In some embodiments, the aromatic hydrocarbon ring is a phenyl ring.

The term "heteroaryl" refers to a group selected from:
- a 5-, 6-, or 7-membered aromatic monocyclic ring containing at least one heteroatom, for example, 1 to 4 heteroatoms, or, in some embodiments, 1 to 3 heteroatoms, and in some embodiments, 1 to 2 heteroatoms, wherein the heteroatoms are selected from nitrogen (N), sulfur (S), and oxygen (O), and the remaining ring atoms are carbon;
- a 7- to 12-membered bicyclic ring containing at least one heteroatom, for example, 1 to 4 heteroatoms, or in some embodiments 1 to 3 heteroatoms, or in other embodiments 1 or 2 heteroatoms, wherein the heteroatoms are selected from N, O, and S, and the remaining ring atoms are carbon, and wherein at least one ring is aromatic and at least one heteroatom is present in the aromatic ring; and
- an 11- to 14-membered tricyclic ring containing at least one heteroatom, for example, 1 to 4 heteroatoms, or in some embodiments 1 to 3 heteroatoms, or in other embodiments 1 or 2 heteroatoms, wherein the heteroatoms are selected from N, O, and S, and the remaining ring atoms are carbon, and wherein at least one ring is aromatic and at least one heteroatom is present in the aromatic ring.

When the total number of S and O atoms in the heteroaryl exceeds 1, those heteroatoms are not adjacent to each other. In some embodiments, the total number of S and O atoms in the heteroaryl is not more than 2. In some embodiments, the total number of S and O atoms in the aromatic heterocycle is not more than 1. When the heteroaryl contains more than one heteroatom as ring members, the heteroatoms may be the same or different. A nitrogen atom in one or more rings of the heteroaryl can be oxidized to form an N-oxide. The term "C-linked heteroaryl" as used herein means that the heteroaryl is attached to the core molecule through a bond from a C atom in the heteroaryl ring.

The term "heteroaryl", in some embodiments, refers to a monocyclic or bicyclic aromatic heterocycle having 5, 6, 7, 8, 9, or 10 ring-forming members, wherein 1, 2, 3, or 4 heteroatoms as ring members are independently selected from nitrogen (N), sulfur (S), and oxygen (O), and the remaining ring members are carbon. In some embodiments, the monocyclic or bicyclic aromatic heterocycle is a monocyclic or bicyclic ring comprising 1 or 2 heteroatoms as ring members independently selected from nitrogen (N), sulfur (S), and oxygen (O). In some embodiments, the monocyclic or bicyclic aromatic heterocycle is a 5- to 6-membered heteroaryl ring that is monocyclic and has 1 or 2 heteroatoms as ring members independently selected from nitrogen (N), sulfur (S), and oxygen (O). In some embodiments, the monocyclic or bicyclic aromatic heterocyclic ring is an 8- to 10-membered heteroaryl ring that is bicyclic and has 1 or 2 heteroatoms as ring members independently selected from nitrogen, sulfur, and oxygen.

The term "heterocyclyl", "heterocycle", or "heterocyclic" are interchangeable and refer to a non-aromatic heterocyclyl that includes one or more heteroatoms selected from nitrogen, oxygen, or optionally oxidized sulfur as ring members and the remaining ring members being carbon, including a monocyclic ring, a fused ring, a bridged ring, and a spiro ring, i.e. containing a monocyclic heterocyclyl, a bridged heterocyclyl, a spiro-heterocyclyl, and a fused heterocyclyl. As used herein, the term "optionally oxidized sulfur" refers to S, SO or SO₂.

The term "monocyclic heterocyclyl" refers to a monocyclic group in which at least one ring member is a heteroatom selected from nitrogen, oxygen, or optionally oxidized sulfur. The heterocycle can be saturated or partially saturated.

Representative examples of exemplary monocyclic 4- to 10-membered heterocyclyl include, but are not limited to, the following groups: 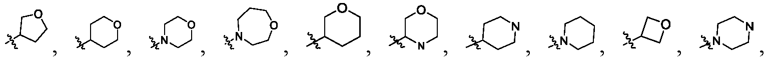 wherein the wavy line indicates the point of attachment.

The term "spiro-heterocyclyl" refers to a 5- to 20-membered polycyclic heterocyclyl having rings connected by one common carbon atom (referred to as a spiro-atom), which contains one or more heteroatoms selected from nitrogen, oxygen, or optionally oxidized sulfur as ring members and the remaining ring members being carbon. One or more rings of a spiro-heterocyclyl may contain one or more double bonds, but none of the rings have a fully conjugated π-electron system. Preferably, the spiro-heterocyclyl group is 6- to 14-membered, and more preferably 7- to 12-membered. The spiro-heterocyclyl is classified as monospiro-heterocyclyl, dispiro-heterocyclyl, or polyspiro-heterocyclyl groups, depending on the number of the shared spiro-atoms, and preferably refers to monospiro-heterocyclyl or dispiro-heterocyclyl groups, and more preferably 4-membered/4-membered, 3-membered/5-membered, 4-membered/5-membered, 4-membered/6-membered, 5-membered/5-membered, or 5-membered/6-membered monospiro-heterocyclyl groups.

The term "fused heterocyclyl" refers to a 5- to 20-membered polycyclic heterocyclyl in which each ring in the system shares a pair of adjacent atoms (carbon and carbon atoms, or carbon and nitrogen atoms) with another ring, which contains one or more heteroatoms selected from nitrogen, oxygen, or optionally oxidized sulfur as ring members and the remaining ring members being carbon. One or more rings of the fused heterocyclyl may contain one or more double bonds, but none of the rings have a fully conjugated π-electron system. Preferably, the fused heterocyclyl is 6- to 14-membered, preferably 7- to 12-membered and more preferably 7-to 10-membered. The fused heterocyclyl is classified as bicyclic, tricyclic, tetracyclic, or polycyclic fused heterocyclyl groups, depending on the number of member rings, preferably refers to bicyclic or tricyclic fused heterocyclyl, and more preferably a 5-membered/5-membered, or 5-membered/6-membered bicyclic fused heterocyclyl groups.

The term "bridge-connected heterocyclyl" or "bridged heterocyclyl" refers to a 5- to 14-membered polycyclic heterocyclyl in which every two rings in the system share two non-adjacent atoms, which contains one or more heteroatoms selected from nitrogen, oxygen or optionally oxidized sulfur as ring members and the remaining ring members being carbon. One or more rings of the bridged heterocyclyl may contain one or more double bonds, but none of the rings have a fully conjugated π-electron system. Preferably, the bridged heterocyclyl is 6-to 14-membered, and more preferably 7- to 10-membered. The bridged heterocyclyl is divided into bicyclic, tricyclic, tetracyclic or polycyclic bridged heterocyclyl groups, depending on the number of member rings, and preferably refer to bicyclic, tricyclic or tetracyclic bridged heterocyclyl groups, and more preferably bicyclic or tricyclic bridged heterocyclyl groups.

The term "alkylene" refers to a divalent alkyl as defined above, and and means a saturated straight or branched divalent hydrocarbon group with a length of 1 to 18 carbon atoms (C₁₋₁₈), wherein the alkylene may be optionally substituted independently with one or more substituents as described below. In another embodiment, the alkylene has one to eight carbon atoms (C₁₋₈) or one to six carbon atoms (C₁₋₆). Examples of the alkylene include but are not limited to methylene (-CH₂-), ethylene (-CH₂CH₂-), propylene (-CH₂CH₂CH₂-), etc.

The term "alkenylene" refers to a divalent alkenyl as defined above, and means a straight or branched divalent hydrocarbon group with a length of two to eight carbon atoms (C₂₋₈) having at least one unsaturated site, i.e. a carbon-carbon sp² double bond, wherein the alkenylene may be optionally substituted independently with one or more substituents as described herein, and includes groups with *"cis"* and *"trans"* orientations, or alternatively, "*E*" and "*Z*" orientations. Examples include, but are not limited to, vinylidene (-CH=CH-), allylidene (-CH₂CH=CH-), and the like.

The term "alkynylene" refers to a divalent alkynyl as defined above, and means a straight or branched divalent hydrocarbon group with a length of three to eight carbon atoms (C₃₋₈) having at least one unsaturated site, i.e. a carbon-carbon sp triple bond, wherein the alkynylene may be optionally substituted independently with one or more substituents as described herein. Examples include, but are not limited to, propynylene (propargylidene, -CH₂C≡C-) and the like.

The term "cycloalkylene" refers to a divalent cycloalkyl as defined above. The term "heterocyclylene" refers to a divalent heterocyclyl as defined above. The term "arylene" refers to a divalent aryl as defined above. The term "heteroarylene" refers to a divalent heteroarylene as defined above.

The compounds disclosed herein may contain asymmetric centers and therefore can exist as enantiomers. "Enantiomers" refer to two stereoisomers of a compound, which are non-superimposable mirror images of each other. In cases where the compounds disclosed herein have two or more asymmetric centers, they can also exist as diastereomers. Enantiomers and diastereomers fall within the broader category of stereoisomers. All such possible stereoisomers, including substantially pure resolved enantiomers, racemic mixtures thereof, and mixtures of diastereomers, are intended to be included. All stereoisomers of the compounds and/or their pharmaceutically acceptable salts disclosed herein are intended to be included. Unless specifically mentioned otherwise, a reference to one isomer applies to any possible isomer. Whenever an isomeric composition is not specified, all possible isomers are included.

The term "substantially pure" as used herein means that the target stereoisomer contains not more than 35% (such as not more than 30%, further such as not more than 25%, even further such as not more than 20%) by weight of any one or more of other stereoisomers. In some embodiments, the term "substantially pure" means that the target stereoisomer contains not more than 10% (e.g., not more than 5%, such as not more than 1%) by weight of any one or more of other stereoisomers.

When the compounds disclosed herein contain olefinic double bonds, unless otherwise specified, such double bonds are intended to include both *E* and *Z* geometric isomers.

When the compounds disclosed herein contain a disubstituted cyclohexyl or cyclobutyl, the substituents found on the cyclohexyl or cyclobutyl ring can be in both *cis* and *trans* forms. The *cis* form means that both of the two substituents are found on the upper side of the two substituted positions on the carbon atoms, while the *trans* form means that they are on opposite sides.

A "pharmaceutically acceptable salt" refers to those salts that are suitable for use in contact with tissues of human beings and lower animals within reasonable medical judgment without excessive toxicity, irritation, allergic reactions, etc., and are commensurate with a reasonable benefit/risk ratio. Pharmaceutically acceptable salts can be prepared *in situ* during the final isolation and purification of the compounds disclosed herein, or separately by reacting a free base functional group with a suitable organic acid, or by reacting an acidic group with a suitable base.

In addition, if a compound disclosed herein is obtained as an acid addition salt, the free base can be obtained by basifying a solution of the acid salt. Conversely, if the product is a free base, an addition salt, such as a pharmaceutically acceptable addition salt, may be produced by dissolving the free base in a suitable organic solvent and/or water and treating the solution with an acid, in accordance with conventional procedures for preparing acid addition salts from base compounds. One skilled in the art will recognize various synthetic methods that may be used to prepare non-toxic pharmaceutically acceptable addition salts without undue experimentation.

As defined herein, "a pharmaceutically acceptable salt thereof" includes the salts of at least one compound of formula (I) and the salts of the stereoisomers of the compound of formula (I), such as the salts of enantiomers and/or the salts of diastereomers.

Unless otherwise specified, when a certain group has one or more connectable sites, any one or more of the sites of the group may be connected to another group by a chemical bond. When the connection mode of the chemical bond is undetermined and there are H atoms at a connectable site, then when the chemical bond is attached, the number of the H atoms at this site will correspondingly decrease as a function of the number of the connected chemical bonds to become a group with the corresponding valency. The chemical bond linking the said site to another group can be represented by a straight solid line bond 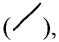 or a wavy line 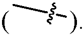 For example, the straight solid line bond in -OCH₃ indicates that it is connected to another group through the oxygen atom in this group; the wavy lines in 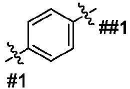 indicate that it is connected to other groups through the carbon atoms at position #1 and position ##1 of the phenyl group.

When applied to an animals, human beings, experimental subjects, cells, tissues, organs, or biological fluids, the terms "administration", "administering", "treating" and "treatment" mean the contact of an exogenous pharmaceutical agent, therapeutic agent, diagnostic agent or composition with the animals, human beings, subjects, cells, tissues, organs, or biological fluids. The treatment of cells encompasses contacting a reagent with the cells, and contacting a reagent with a fluid, wherein the fluid is contacted with the cells. The terms "administering" and "treating" also mean *in vitro* and *ex vivo* treatment of, for example, a cell by a reagent, a diagnostic agent, a binding compound, or by another cell. The term "subject" herein includes any organisms, preferably animals, more preferably mammals (such as rats, mice, dogs, cats, and rabbits), most preferably a human being.

The term "effective amount" or "therapeutically effective amount" refers to an amount of an active ingredient (such as a compound) that, when administered to a subject for treating a disease or at least one clinical symptom of a disease or disorder, is sufficient to affect such treatment of the disease, disorder or symptom.

The term "disease" refers to any disease, illness, condition, symptom, or indication, and is interchangeable with the term "condition" or "disorder".

Throughout the specification and the aspects that follow, unless the context requires otherwise, the term "comprise" and variations such as "comprises" and "comprising" are intended to indicate the presence of the features following the same, but not to preclude the presence or addition of one or more other functions. As used herein, the term "comprising" may be replaced with the terms "containing", "including", or sometimes with "having".

Throughout the specification and the aspects that follow, the term "Cₙ₋ₘ" indicates a range that includes the endpoints, where n and m are integers and indicate the number of carbons. Examples include C₁₋₈, C₁₋₆, etc.

Unless clearly defined elsewhere in this document, all other technical and scientific terms used herein have the meanings as commonly understood by a person of ordinary skill in the field to which the present invention pertains.

### Identification and characterization of compounds.

The Examples provide the preparation of representative compounds of formula (I) and the corresponding data for structural identification. ¹HNMR spectra were obtained on a Bruker instrument (400 MHz) and chemical shifts were reported in ppm. Tetramethylsilane was used as the internal standard (0.00 ppm). The representation method of ¹H NMR: s = singlet, d = doublet, t = triplet, m = multiplet, br = broadened, dd = doublet of doublets, dt = doublet of triplets. Coupling constants were reported in Hz if given.

Mass spectra were obtained using an LC/MS instrument and the ionization mode was either ESI or APCI. Unless otherwise indicated, the test methods are as follows:

| | Time (min) | Flow Rate (mL/min) | %A (0.1%FA H₂O) | %B (Acetonitrile) |
|---|---|---|---|---|
| 1 | 0.00 | 1 | 85 | 15 |
| 2 | 2.50 | 1 | 5 | 95 |
| 3 | 4.00 | 1 | 5 | 95 |
| 4 | 4.01 | 1 | 85 | 15 |
| 5 | 5.00 | 1 | 85 | 15 |

Silica gel plates used in Thin Layer Chromatography were silica gel plates for thin layer chromatography (TLC) from Yantai HUANGHAI HSGF254 or Qingdao GF254.

The specification of the silica gel plates used was 0.15 mm to 0.2 mm, and the specification for the separation and purification of products by thin-layer chromatography was 0.4 mm to 0.5 mm. For column chromatography, the silica gel with a mesh size of 200-300 mesh from Yantai Huanghai Silica Gel was generally used as the carrier.

In the following examples, unless otherwise indicated, all temperatures were in degrees Celsius. Unless otherwise indicated, various starting materials and reagents were either commercially available or were synthesized according to known methods, and were used without further purification. Unless otherwise indicated, commercial suppliers include, but are not limited to, Aldrich Chemical Company, ABCR GmbH & Co.KG, Acros Organics, Shanghai Bide Pharmaceutical Technology Co., Ltd., and Shanghai Shaoyuan Reagent Co., Ltd, from which the materials and reagents can be purchased.
DMSO-d₆: deuterated dimethylsulfoxide.
DIEA: N,N-diisopropylethylamine
Rt: retention time.

Unless specifically stated in the Examples, the solutions in the reactions were aqueous solutions.

For the purification of compounds, eluent systems for column chromatography and thin-layer chromatography were used, which were selected from: A: petroleum ether and ethyl acetate system; B: dichloromethane and methanol system; C: dichloromethane and ethyl acetate system, D: dichloromethane and ethanol system, in which the volume ratio of the solvents may vary, depending on the polarity of the compound. Additionally, a small amount of acidic or basic reagents could be added for adjustment, such as acetic acid or triethylamine.

### Biological Assays

The pharmacological properties of the compounds of the invention may be confirmed by numerous biological assays. The following exemplary biological assays were performed with the compounds of the present invention.

### Experimental Example 1. Assay of in vitro HPK1 enzymatic activity

Compounds were evaluated for their ability to inhibit human HPK1 by IC₅₀ determination using the ADP-Glo method to measure the kinase activity.

Buffer conditions of the enzyme: 50 mM Hepes (pH 7.5), 10 mM MgCl₂, 1 mM EGTA, 0.01% Brij35, 2 mM DTT.

Preparation of the Kinase and substrate mixture: the working concentration of the kinase in the HPK1 reaction solution was 7 nM and the working concentration of ATP was 20 uM.

### Test procedures

The compound was diluted with DMSO in a dilution plate, with a maximum starting concentration of 1 uM and 10 concentration gradient points (4-fold dilution).

Each of the above 10 concentration gradient points of the compound was then diluted 50-fold into the kinase reaction buffer and shaken on a shaker for 20 min. The kinase was prepared in an enzyme reaction buffer and 2 µl of the HPK1 kinase was added per well of the reaction plate at a HPK1 kinase concentration of 7 nM.

1 µl of diluted compound in the buffer was added per well, and the plate was sealed with a plate sealing film, centrifuged at 1000 g for 30 seconds, and left at room temperature for 10 minutes. An ATP solution was prepared with an enzyme reaction buffer and 2 µl of the ATP solution was added to the reaction plate (the working concentration of ATP was 20 uM). The plate was sealed with a plate sealing film, centrifuged at 1000 g for 30 seconds, and reacted at room temperature for 60 minutes. 4 µL of ADP-Glu was transferred to a 384 reaction plate, centrifuged at 1000 rpm/min for 1 min, and incubated at 25°C for 40 min. 8 µL of the Detection solution was transferred to a 384 reaction plate, centrifuged at 1000 rpm/min for 1 min, and incubated at 25°C for 40 min. The RLU (Relative luminescence unit) signal was read using a BMG microplate reader, and the signal intensity was used to characterize the activity level of the kinase.

The kinase activity data was expressed by comparing the kinase activity of the test compound and the blank group (containing only DMSO), and the IC₅₀ value was obtained by curve fitting using the Prism software (GraphPad7.0).

The following table shows the HPK1 IC₅₀ values of the compounds of the present invention.

| Compound | HPK 1 IC₅₀ (nM) | Compound | HPK1 IC₅₀ (nM) | Compound | HPK1 IC₅₀ (nM) |
|---|---|---|---|---|---|
| C001 | 5.2 | C018 | 1.8 | C034 | 1.2 |
| C002 | 2.6 | C019 | 2.0 | C035 | 2.5 |
| C003 | 3.6 | C020 | 5.5 | C036 | 3.7 |
| C004 | 1.6 | C021 | 0.5 | C037 | 14.2 |
| C006 | 3.2 | C022 | 1.4 | C038 | 21.7 |
| C007 | 5.2 | C023 | 6.4 | C039 | 1.4 |
| C008 | 3.5 | C024 | 2.3 | C040 | 2.6 |
| C009 | 8.8 | C025 | 8.8 | C041 | 14.6 |
| C011 | 0.7 | C026 | 1.6 | C042 | 2.1 |
| C012 | 1.9 | C027 | 0.9 | C043 | 27.8 |
| C014 | 1.5 | C028 | 2.4 | C044 | 50.4 |
| C015 | 6.2 | C029 | 1.6 | C045 | 5.1 |
| C016 | 15.0 | C030 | 0.2 | | |
| C017 | 0.9 | C033 | 7.5 | | |

The HPK1 IC₅₀ values of all the compounds of the present invention are not more than 60 nM, and the HPK 1 IC₅₀ values of most of the compounds of the present invention are not more than 20 nM, indicating that all the compounds of the present invention have good inhibitory activity against HPK1, and have good application potential in treating HPK1-mediated diseases.

### Experimental Example 2. IL2 Activity Assay

The EC50 value for IL2 activation in human PBMC was determined by ELISA to evaluate the ability of the compound to activate T cells.
(1) Pre-treatment of a 96-well plate in an IL2 kit: The CD3 antibody was plated at 5 µg/ml, 100 µl per well, and coated overnight at 4°C.
(2) PBMC cells (10⁵/well) were plated on the 96-well plate. The compound was diluted with DMSO in a dilution plate, with a maximum starting concentration of 10 µM, 8 concentration gradient points (3-fold dilution), and then added to the 96-well plate along with the addition of 5 µg/ml CD28 antibody. Incubation was performed for 24 h.
(3) The ELISA plate was washed twice with PBS, and an ELISA dilution was added at 100 µl/well. Then 100 µl of the standard and the incubated samples obtained in step 2 were added separately, and incubated for 2 h at room temperature.
(4) Each well was washed 3 times by adding with 400 µl washing buffer per well, and was finally blotted to dryness with a clean absorbent paper.
(5) 200 µl of the IL2 conjugate was added per well, covered with a fresh tape, and incubated for 2 h at room temperature. Step (4) was repeated.
(6) 200 µl of the substrate was added per well under the protection from light. The plate was incubated at room temperature for 20 min.
(7) 50 µL of a stop solution was added per well. The color of the wells should be from blue to yellow. Signals at 450 nM were read by a microplate reader, and the signal intensity was used to characterize the activity level of IL2. EC50 values were obtained by fitting using the Prism software.

The following table shows that the compounds of the present invention have an effect of inducing IL2 production.

| Compound | IL2 EC₅₀ (nM) | Compound | IL2 EC₅₀ (nM) |
|---|---|---|---|
| C003 | 110.9 | C132 | 34.58 |
| C006 | 178.8 | C134 | 41.68 |
| C017 | 323.5 | C135 | 44.1 |
| C018 | 102.2 | C139 | 103.86 |
| C021 | 37.9 | C153 | 37.07 |
| C022 | 95.6 | C154 | 103.71 |
| C024 | 196 | C160 | 45.47 |
| C110 | 55.59 | C162 | 27.33 |
| C122 | 59.85 | C170 | 23.81 |
| C131 | 41.68 | C196 | 34.68 |

### Experimental Example 3. HPK1 Protein Degradation Assay

Degradation of HPK1 proteins and DC50/Dmax were determined by the WB method in order to evaluate the *in vitro* degradative ability of the compound.

The RAMOS cell line was selected as the degradation cell line. Incubation Conditions: RMPI 1640 culture medium supplemented with 10% FBS, 37°C, 5% CO₂.

### Experimental procedures:

(1) Plating cells: in a 6 cm dish with 4 ml of the culture medium, cells plated at 2 x 10⁶ cells/dish.
(2) Drug addition: the compound (stock solution concentration: 10 mM) was diluted with DMSO in a dilution plate, with a maximum starting concentration of 10 µM and 7 concentration gradient points (3-fold dilution). 4 µl of each diluted solution was taken, added to a dish, and incubated for 16 h.
(3) Cells were harvested, added with a lysis buffer, and centrifuged at 12000 rpm/min for 20 min. The supernatant was collected. After quantification using a BCA quantification kit, 5 x loading buffer was added to denature for 10 min.
(4) 10 µg of denatured protein was added to the SDS-PAGE gel. After electrophoresis and membrane transfer, the plate was blocked with 5% BSA for 1 h, incubate with the primary antibody at 4°C overnight. After washing off the primary antibody, the secondary antibody was added, incubated at room temperature for 1 h, and washed off.

Imaging: the membranes were scanned with a two-color fluorescence imaging system. The results of Western blot were quantified using Image J software, and DC₅₀ and Dmax were calculated by comparison with the control.

The table below shows the degradative activity of the compounds of the present invention against HPK1.

| Compound | DC₅₀ (nM) | Dmax (%) | Compound | DC₅₀ (nM) | Dmax (%) |
|---|---|---|---|---|---|
| C024 | 2.1 | 77 | C162 | 29.16 | 86.50 |
| C022 | 55 | 86 | C167 | 23.88 | 66.78 |
| C084 | 1.21 | 93.00 | C170 | 52.25 | 87.09 |
| C085 | 385 | 56.4 | C177 | 632.3 | 51.57 |
| C092 | 21.36 | -- | C181 | 214 | 64.38 |
| C110 | 9.349 | 93.00 | C195 | 18.7 | 80.69 |
| C115 | 23.54 | 66.4 | C196 | 8.8 | 93.78 |
| C131 | 385.6 | 52 | C197 | 5.28 | 77.54 |
| C132 | 29.34 | 73 | C198 | 32.92 | 91.84 |
| C134 | 7.219 | 78 | C199 | 83.01 | 83.77 |
| C135 | 175.8 | 63 | C201 | 30.63 | 76.10 |
| C137 | 229.6 | 58.44 | C203 | 5.99 | 91.74 |
| C139 | 52.36 | 80.90 | C204 | 58.02 | 85.42 |
| C140 | 197.6 | 50.68 | C205 | 89.93 | 83.00 |
| C153 | 616.2 | 52.47 | C206 | 190.3 | 73.00 |
| C160 | 12.65 | 88.85 | C209 | 29.03 | 84.00 |

The compounds of the present invention have good ability to degrade HPK1 proteins.

### Experimental Example 4. Determination of pharmacokinetic properties

Female Balb/c mice (weighing 18-22 g) were fasted overnight prior to the experiment. The test compound was dissolved in the vehicle, DMSO: PEG400: 30% captisol: HCl=5: 20: 70: 5 (v/v/v/v), and administered by single oral gavage at a dose of 10 mg/kg or by intravenous injection at a dose of 2 mg/kg. Blood samples were collected from the orbital venous plexus at 15 minutes, 30 minutes, and 1, 2, 4, 6, 8, and 24 h after administration. Approximately 0.08 mL of blood was collected at each time point and placed in a 1.5 mL centrifuge tube containing EDTA-2K anticoagulant. Blood samples were centrifuged (3200 g, 10 min, 4°C) within 2 h to obtain plasma samples. Plasma samples were frozen at -70°C to -80°C in an ultra-low temperature freezer prior to processing. Before sample processing, the plasma samples were taken out of the freezer. After thawing at room temperature, 20 µL of of each plasma sample was added into a 96-well plate, and then 120 µL of acetonitrile containing an internal standard was added to precipitate proteins. After vortex mixing, the samples were centrifuged at 4950 g for 15 min at 4°C. The supernatant was taken and mixed with an equal volume of 0.1% aqueous formic acid solution for LC-MS/MS analysis.

SD rats (half male and half female) weighing about 220 g were fasted overnight before the experiment. The compounds of the invention were prepared in the vehicle, DMSO: PEG400: 30% captisol: HCL=5: 20: 70: 5 (v/v/v/v). The rats were administered a single intravenous injection (2 mg/mL) at a volume of 2 mL/kg, and a single oral gavage of the compounds of the present invention at doses of 5 mg/kg, 2 mg/kg, and 1 mg/kg at a volume of 10 mL/kg.

Blood samples were collected from the orbital venous plexus before administration and 0.083 (collected only for the intravenous administration), 0.25, 0.5, 1, 2, 4, 6, 8, and 24 h after administration. Approximately 0.150 mL of blood was collected at each time point and placed in a 1.5 mL centrifuge tube containing EDTA-2K anticoagulant. The blood samples were centrifuged (3200 g, 10 min, 4°C) within 1 h to obtain plasma samples. The plasma samples were frozen at -70°C to -80°C in an ultra-low temperature freezer prior to processing. Before sample processing, the plasma samples were taken out of the freezer. After thawing at room temperature, 20 µL of each plasma sample was taken and added to a 96-well plate, and then 120 µL of acetonitrile containing the internal standard was added to precipitate proteins. After vortex mixing, the samples were centrifuged at 4700 g for 15 min at 4°C. The supernatant was taken and mixed with an equal volume of 0.1% aqueous formic acid solution for LC-MS/MS analysis.

The pharmacokinetic results of single administration in mice are shown in the table below.

| Compound No. | iv (2 mg/kg) | | | | po (10 mg/kg) | | | |
|---|---|---|---|---|---|---|---|---|
| | CL (mL/hr/kg) | AUC (ng·hr/mL) | Vz (mL/hr/kg) | t_{1/2} (hr) | Cₘₐₓ (ng/mL) | AUC (ng·hr/mL) | F% | t_{1/2} (hr) |
| C110 | 496.1 | 4031.5 | 701.3 | 0.98 | 840 | 2209 | 17.69 | 3.35 |
| C160 | 640 | 5333 | 602 | 1.25 | 2459 | 7965 | 29.6 | 3.19 |
| C170 | 414 | 4826 | 1329 | 0.7 | 1390 | 6669 | 27.64 | 4.22 |
| C162 | 757 | 2639 | 763 | 0.7 | 1370 | 3693 | 28.0 | 1.66 |
| C195 | 47.1 | 42500 | 164 | 2.41 | 14300 | 43964 | 20.7 | 2.8 |
| C196 | 35.9 | 55748 | 121.2 | 2.34 | 14700 | 57823 | 20.7 | 2.55 |
| C197 | 270 | 7397 | 917 | 2.35 | 4680 | 9202 | 25.1 | 3.18 |
| C201 | 395 | 5052 | 1230 | 2.16 | 4497 | 8657 | 34.2 | 2.63 |

The results show that the compounds of the present invention exhibited good exposure (AUC) when administered orally and intravenously, and had a good oral half-life and oral bioavailability.

### Preparation examples

The present invention will be further described in detail combination with with specific examples, but these examples do not limit the scope of the present invention. The following examples are used to understand the method and core idea of the present invention. For those skilled in the art, any possible changes or substitutions without departing from the concept of the present invention shall fall within the protection scope of the present invention. For the experimental methods in the examples of the present invention for which specific conditions are not specified, they are usually conventional conditions or in accordance with the conditions recommended by the raw material or commodity manufacturers. For the reagents for which the sources are not specified, they are usually conventional reagents that can be purchased through commercial channels.

### Synthesis of intermediates

### Preparation of intermediate warhead 3

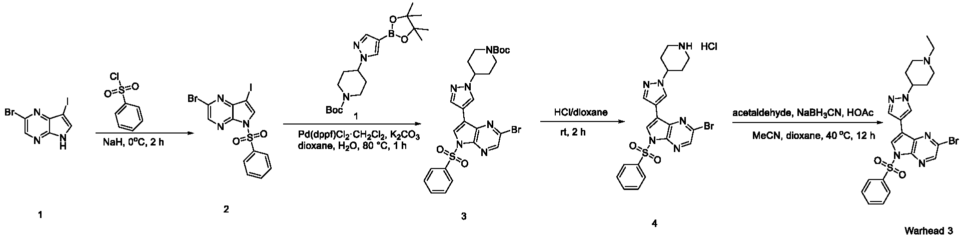

### Step 1: Preparation of intermediate 2

Compound 1 (10 g, 30.8 mmol) was dissolved in tetrahydrofuran (100 mL). NaH (1.8 g, 46.2 mmol) was added at 0°C, and stirred under nitrogen protection at 0°C for 0.5 h. Then, benzenesulfonyl chloride (8 g, 46.2 mmol) was added in an ice bath, raised to room temperature, and stirred for 3 h. After completion of the reaction, the reaction solution was poured into ice water and filtered to give the compound 2. LCMS (ESI) m/z: 464.2[M+H]⁺.

### Step 2: Preparation of intermediate 3

Compound 2 (1.0 g, 2.15 mmol) and compound 1 (0.975 g, 2.585 mmol) were dissolved in dioxane (22 mL). Potassium carbonate (0.595 g, 4.305 mmol) was dissolved in water (7.5 mL) and mixed with the above-mentioned solution. Pd(dppf)Cl₂·CH₂Cl₂ (315 mg, 0.43 mmol) was added. The mixed solution was stirred at 80°C under nitrogen protection for 1 h. LCMS showed no starting material remained. The reaction solution was cooled to room temperature and extracted three times with dichloromethane. The organic phases were combined and washed twice with saturated brine. The organic phases were dried over sodium sulfate and filtered. The filtrate was concentrated. The crude product was purified by column chromatography to give intermediate 3. LCMS (ESI) m/z: 589.2[M+H]⁺.

### Step 3: Preparation of intermediate 4

Compound 3 (7 g, 11.9 mmol) was dissolved in a 4M hydrochloric acid solution in dioxane (70 mL) and the reaction solution was stirred at room temperature for 2 h. After the reaction was complete, the solvent was directly dried by rotary evaporation to give a crude product. The crude product was washed with ether to obtain intermediate 4. LCMS (ESI) m/z: 488.9[M+H]⁺.

### Step 4: preparation of intermediate warhead 3

Compound 4 (3.5 g, 6.68 mmol) was dissolved in a mixed solvent of acetonitrile (140 mL) and dioxane (36 mL), then acetaldehyde (2.64 mL, 13.36 mmol) and acetic acid (0.38 mL, 6.68mmol) were added successively. The reaction solution was stirred at room temperature for 1 h. Then, sodium cyanoborohydride (1470 mg, 23.38 mmol) was added at room temperature. The mixture was stirred at 40°C for another 4 h, and the previous procedures were repeated with stirring being continued at 40°C for 12 h. After completion of the reaction, the mixture was adjusted to ph = 14 with aqueous ammonia. After extraction by adding dichloromethane, the organic layer was washed with brine, dried over anhydrous sodium sulfate and concentrated in vacuum to give a crude product. The crude product was purified by column chromatography to give intermediate warhead 3, and dissolved by adding DCM, followed by addition of aqueous Ammonia. The organic phase was separated and concentrated. LCMS (ESI) m/z:515.0[M+H]⁺.

### Example 1: Preparation of compound C001

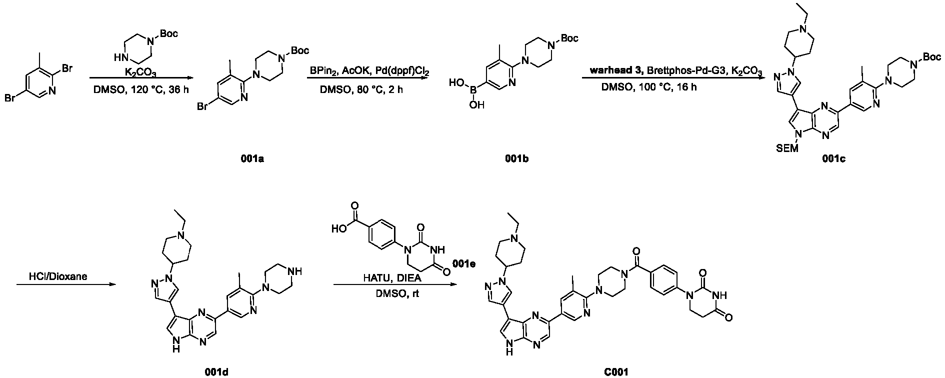

### Step 1:

2,5-Dibromo-3-methylpyridine (5 g, 20.1 mmol), 1-tert-butoxycarbonyl piperazine (5.6 g, 30.1 mmol) and K₂CO₃ (8.3 g, 60.2 mmol) were dissolved in dimethyl sulfoxide (50 mL) and the mixture was stirred at 120°C for 36 h. The reactants were partially converted as demonstrated by LCMS, and the product was detected. The reaction solution was cooled to room temperature, quenched by the addition of water (100 mL), and extracted with ethyl acetate (30 mL*2). The organic layer was dried with anhydrous sodium sulfate, filtered, and concentrated in vacuum to give a crude product. The crude product was purified by silica gel chromatography to give the compound 001a. LCMS: [M+H]⁺=356.2.

### Step 2:

Compound 001a (2.8 g, 7.89 mmol), bis(pinacolato)diboron (3 g, 11.83 mmol), potassium acetate (2.32 g, 23.66 mmol), and Pd(dppf)Cl₂ (576 mg, 0.789 mmol) were dissolved in dimethyl sulfoxide (30 mL) and the mixture was stirred at 80°C for 2 h under a nitrogen atmosphere. LCMS showed the complete conversion of the reactants, and the product was detected. The reaction solution was cooled to room temperature, quenched by the addition of water (100 mL), and extracted with ethyl acetate (30 mL*2). The organic layer was dried with anhydrous sodium sulfate, filtered, and concentrated in vacuum to give a residue. The residue was purified by silica gel chromatography to give the compound 001b. LCMS: [M+H]⁺=322.4.

### Step 3:

Warhead 3 (200 mg, 0.623 mmol), Compound 001b (287 mg, 0.623 mmol), potassium carbonate (258 mg, 1.87 mmol), and Brettphos-Pd-G3 (56 mg, 0.0623 mmol) were dissolved in dimethyl sulfoxide (2 mL). The mixture was stirred at 100°C overnight under a nitrogen atmosphere. LCMS showed the complete conversion of the reactants, and the product was detected. The reaction solution was cooled to room temperature, quenched by the addition of water (50 mL), and extracted with ethyl acetate (30 mL*2). The organic layer was dried with anhydrous sodium sulfate, filtered, and concentrated in vacuum to give a residue. The residue was purified by silica gel chromatography to give the compound 001c. LCMS: [M+H]⁺=702.5.

### Step 4:

Compound 001c (250 mg, 0.357 mmol) was dissolved in a hydrochloric acid solution in dioxane (5 mL, 4M) and stirred at room temperature overnight. LCMS showed the complete conversion of the reactants, and the product was detected. The reaction solution was distilled under a reduced pressure to obtain a crude product 001d. LCMS: [M+H]⁺=472.2.

### Step 5:

Compounds 001d (16 mg, 0.028 mmol), 001e (9 mg, 0.036 mmol), HATU (16 mg, 0.042 mmol) and N, N-diisopropylethyl amine (15 mg, 0.112 mmol) were dissolved in dimethyl sulfoxide (2 mL) at room temperature and the reaction solution was stirred at room temperature overnight. LCMS showed the complete conversion of the reactants, and the product was detected. The reaction solution was quenched by the addition of water (50 mL) and extracted with dichloromethane (30 mL*2). The organic layer was dried with anhydrous sodium sulfate, filtered, and concentrated in vacuum to give a residue. The residue was purified by Pre-TLC to give the compound C001. LCMS: Rt=2.190min; **LCMS:** [M+H]⁺=688.4.

### Example 2: Preparation of compound C002

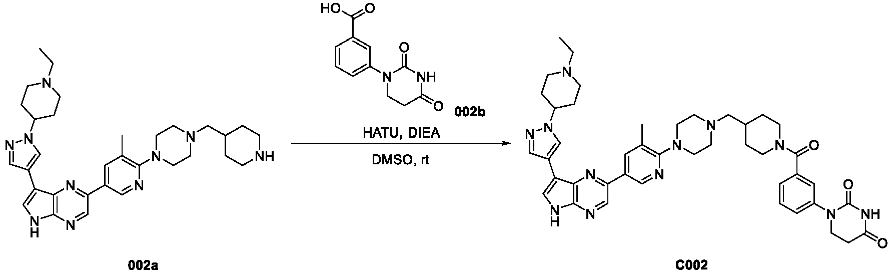

Compounds 002a (35 mg, 0.062 mmol), 002b (19 mg, 0.081 mmol), DIEA (32 mg, 0.248 mmol), and HATU (30.3 mg, 0.08 mmol) were dissolved in dimethyl sulfoxide (2 mL) and the mixture was stirred at room temperature for 16 h. LCMS showed the complete conversion of the reactants, and the product was detected. The reaction solution was cooled to room temperature, quenched by the addition of water (50 mL), and extracted with dichloromethane (30 mL*2). The organic layer was dried with anhydrous sodium sulfate, filtered, and concentrated in vacuum to give a crude product. The crude product was purified by Pre-TLC to give the compound C002. LCMS: Rt=2.099 min; [M+H]⁺=785.3.

### Example 3: Preparation of compound C003

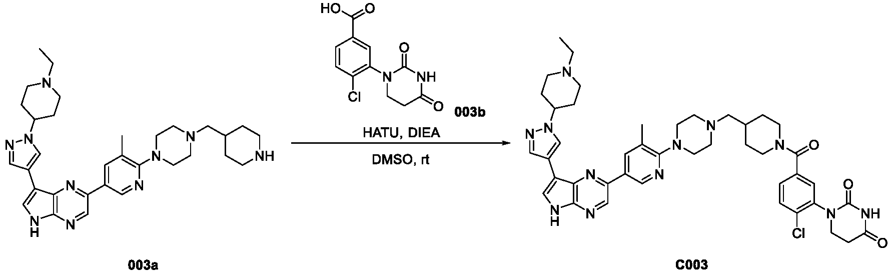

Compounds 003a (33 mg, 0.058 mmol), 003b (21 mg, 0.075 mmol), DIEA (30 mg, 0.232 mmol), and HATU (29 mg, 0.075 mmol) were dissolved in dimethyl sulfoxide (2 mL) and the mixture was stirred at room temperature for 16 h. LCMS showed the complete conversion of the reactants, and the product was detected. The reaction solution was cooled to room temperature, quenched by the addition of water (50 mL), and extracted with dichloromethane (30 mL*2). The organic layer was dried with anhydrous sodium sulfate, filtered, and concentrated in vacuum to give a crude product. The crude product was purified by Pre-TLC to give the compound C003. LCMS: [M+H]⁺=819.3.

### Example 4: Preparation of compound C004

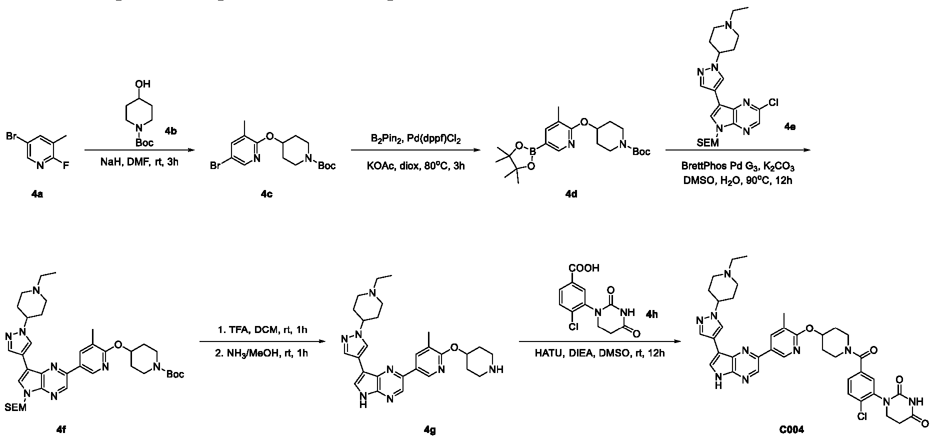

### Step 1:

Compounds 4a (500 mg, 2.63 mmol) and 4b (795 mg, 3.95 mmol) were dissolved in DMF (10 mL). NaH (210 mg, 5.26 mmol) was added in an ice bath and stirred at room temperature for 12 h. TLC showed the complete conversion of the raw materials, and the product was detected. The reaction solution was added with 30 mL of water and extracted with ethyl acetate (20 mL*3). The organic phase was dried over anhydrous sodium sulfate and filtered. The filtrate was concentrated in vacuum to give a residue. The residue was purified by column chromatography to give the compound 4c.

### Step 2:

Compound 4c (970 mg, 2.61 mmol), B₂Pin₂ (1.33 g, 5.23 mmol), KOAc (770 mg, 7.84 mmol), and Pd(dppf)Cl₂ (212 mg, 0.26 mmol) were dissolved in dioxane (20 mL) at room temperature, replaced with nitrogen three times, raised to 80°C, and stirred at this temperature for 3 h. TLC showed the complete conversion of the raw materials, and the product was detected. After completion of the reaction, the reaction solution was cooled to room temperature and filtered. The filtrate was concentrated in vacuum to give a residue. The residue was purified by column chromatography to give the compound 4d.

### Step 3:

Compounds 4d (136 mg, 0.33 mmol), 4e (100 mg, 0.22 µmol), BrettPhos Pd G3 (20 mg, 22 µmol), and K₂CO₃ (90 mg, 0.65 mmol) were dissolved in DMSO/H₂O (4 mL/0.8 mL) at room temperature, replaced with nitrogen three times, raised to 100°C, and stirred at this temperature for 12 h. LCMS showed the complete conversion of the reactants, and the product was detected. The reaction solution was added with 30 mL of water and extracted with dichloromethane (20 mL*3). The organic phase was dried over anhydrous sodium sulfate and filtered. The filtrate was concentrated in vacuum to give a residue. The residue was purified by Prep-TLC to give the compound 4f.

### Step 4:

Compound 4f (123 mg, 75.8 µmol) was dissolved in DCM (3 mL). TFA (1 mL) was added with stirring, and stirring was continued at room temperature for 1 h. LCMS showed the complete conversion of the reactants, and the product was detected. The reaction solution was distilled under reduced pressure. The crude product was dissolved in MeOH (3 mL), added with methanolic ammonia solution (1 mL) with stirring, and stirring was continued at room temperature for 1 h. LCMS showed the complete conversion of the reactants, and the product was detected. The reaction solution was distilled under reduced pressure to give a crude compound 4g.

### Step 5:

Compounds 4g (83 mg, 0.17 mmol), 4h (69 mg, 0.26 mmol), DIEA (67 mg, 0.51 mmol), and HATU (130 mg, 0.34 mmol) were dissolved in DMSO (2 mL) and stirred at room temperature for 12 h. LCMS showed the complete conversion of the reactants, and the product was detected. The reaction solution was added with 30 mL of water and extracted with dichloromethane (20 mL*3). The organic phase was dried over anhydrous sodium sulfate and filtered. The filtrate was concentrated in vacuum to give a residue. The residue was purified by Prep-TLC to give the final product C004. LCMS: Rt =1.918 min, [M+H]⁺= 737.4.

¹H NMR (500 MHz, DMSO-d6) δ 12.05 (d, J = 2.8 Hz, 1H), 10.52 (s, 1H), 8.85 (d, J = 2.1 Hz, 2H), 8.38 (d, J = 2.2 Hz, 2H), 8.12 (d, J = 2.7 Hz, 1H), 8.10 (s, 1H), 7.69 - 7.63 (m, 2H), 7.47 (dd, J = 8.2, 2.1 Hz, 1H), 5.43 (dt, J = 7.5, 3.7 Hz, 1H), 3.95 (d, J = 19.7 Hz, 1H), 3.78 (q, J = 9.4, 6.3 Hz, 1H), 3.64 (dt, J = 12.7, 6.4 Hz, 3H), 3.06 - 2.96 (m, 3H), 2.75 (q, J = 7.5, 6.7 Hz, 2H), 2.41 (d, J = 10.0 Hz, 2H), 2.30 (s, 3H), 2.04 (q, J = 14.0, 10.0 Hz, 8H), 1.80 (s, 2H), 1.23 (d, J = 3.6 Hz, 1H), 1.04 (t, J = 7.2 Hz, 3H).

### Example 5: Preparation of compound C005

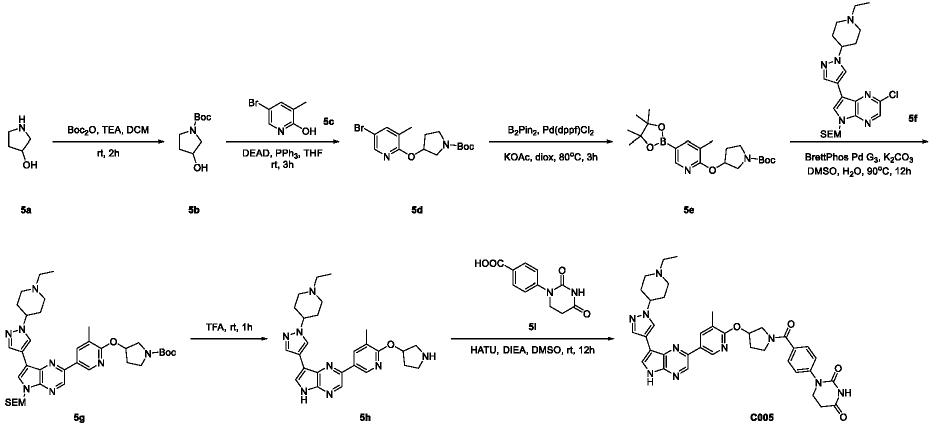

### Step 1:

Compound 5a (1 g, 11.48 mmol), and TEA (1.74 g, 17.22 mmol) were dissolved in DCM (20 mL). Boc₂O (3.26 g, 14.92 mmol) was added in an ice bath and stirred at room temperature for 2 h. TLC showed the complete conversion of the raw materials, and the product was detected. The reaction solution was distilled under a reduced pressure to obtain a residue. The residue was purified by column chromatography to give the compound 5b.

### Steps 2-6:

Steps 2-6 were carried out in accord with the procedures described in steps 1-5 of Example 4, except that compounds 4b and 4a in step 1 of Example 4 were replaced with compounds 5b and 5c, respectively, and compound 4h in step 5 of Example 4 was replaced with compound 5i, to give the compound C005. LCMS: Rt = 1.773 min, [M+H]⁺= 689.4, purity = 98.77%.

¹H NMR (500 MHz, DMSO-d6) δ 12.16 - 11.95 (m, 1H), 10.44 (d, J = 12.9 Hz, 1H), 8.98 - 8.72 (m, 2H), 8.48 - 8.33 (m, 2H), 8.17 - 8.04 (m, 2H), 7.59 (dd, J = 23.1, 8.2 Hz, 2H), 7.41 (dd, J = 20.2, 8.2 Hz, 2H), 5.65 (d, J = 49.4 Hz, 1H), 4.26 (s, 1H), 3.97 (ddd, J = 31.1, 12.8, 4.7 Hz, 1H), 3.83 (dt, J = 19.8, 6.6 Hz, 2H), 3.73 (dd, J = 9.7, 6.5 Hz, 2H), 3.65 - 3.53 (m, 1H), 3.01 (s, 2H), 2.71 (dt, J = 24.4, 6.6 Hz, 2H), 2.42 (s, 1H), 2.27 (d, J = 35.2 Hz, 3H), 2.05 (s, 5H), 1.05 (t, J = 7.2 Hz, 3H).

### Example 6: Preparation of compound C006

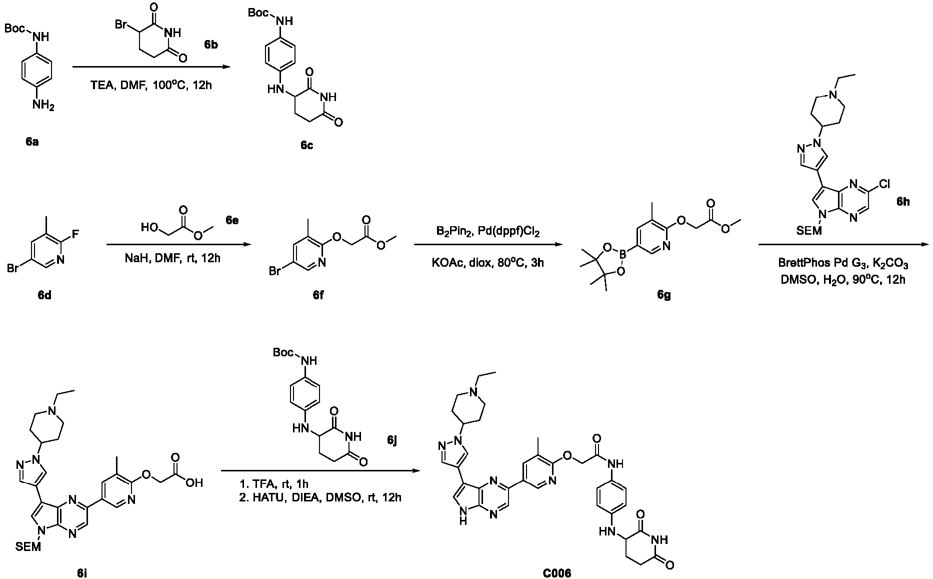

### Step 1:

Compounds 6a (500 mg, 24.00 mmol), 6b (922 mg, 48.00 mmol), and TEA (486 mg, 48.00 mmol) were dissolved in DMF (20 mL), raised to 100°C and stirred at this temperature for 12 h. TLC showed the complete conversion of the raw materials, and the product was detected. The reaction solution was added with 30 mL of water and extracted with ethyl acetate (100 mL*3). The organic phase was dried over anhydrous sodium sulfate and filtered. The filtrate was concentrated in vacuum to give a residue. The reaction solution was distilled under reduced pressure to give a residue. The residue was purified by column chromatography to give the compound 6c.

### Step 2:

Compounds 6d (500 mg, 2.63 mmol) and 6e (380 mg, 4.21 mmol) were dissolved in DMF (40 mL). NaH (632 mg, 15.80 mmol) was added in an ice bath and stirred at room temperature for 12 h. TLC showed the complete conversion of the raw materials, and the product was detected. The reaction solution was added with 100 mL of water and extracted with ethyl acetate (100 mL*3). The organic phase was dried over anhydrous sodium sulfate and filtered. The filtrate was concentrated in vacuum to give a residue. The reaction solution was distilled under reduced pressure to give a residue. The residue was purified by column chromatography to give the compound 6f.

### Step 3:

Compound 6f (430 mg, 1.65 mmol), B₂Pin₂ (840 mg, 3.31 mmol), KOAc (485 mg, 4.96 mmol), and Pd(dppf)Cl₂ (134 mg, 0.16 mmol) were dissolved in dioxane (15 mL) at room temperature, replaced with nitrogen three times, raised to 80°C, and stirred at this temperature for 3 h. TLC showed the complete conversion of the raw materials, and the product was detected. After completion of the reaction, the reaction solution was cooled to room temperature and filtered. The filtrate was concentrated in vacuum to give a residue. The residue was purified by column chromatography to give the compound 6g.

### Step 4:

Compounds 6g (300 mg, 0.98 mmol), 6h (300 mg, 0.65 mmol), BrettPhos Pd G3 (59 mg, 65 µmol), and K₂CO₃ (270 mg, 1.95 mmol) were dissolved in DMSO/H₂O (12 mL/3 mL) at room temperature, replaced with nitrogen three times, raised to 100°C, and stirred at this temperature for 12 h. LCMS showed the complete conversion of the reactants, and the product was detected. The reaction solution was added with 30 mL of water and 3 mL of formic acid and extracted with dichloromethane (50 mL*3). The organic phase was dried over anhydrous sodium sulfate and filtered. The filtrate was concentrated in vacuum to give a residue. The residue was purified by column chromatography to give the compound 6i.

### Step 5:

Compounds 6i (80 mg, 0.14 mmol), and 6j (65 mg, 0.20 mmol) were dissolved in TFA (5 mL) and stirred at room temperature for 1 h. LCMS showed the complete conversion of the reactants, and the product was detected. The reaction solution was distilled under reduced pressure to obtain a crude product, which was dissolved with DMSO (2 mL). DIEA (53 mg, 0.41 mmol) and HATU (103 mg, 0.27 mmol) were added and stirred at room temperature for 2h. LCMS showed the complete conversion of the reactants, and the product was detected. The product was purified by preparative chromatography to give the final product C006. LCMS: Rt = 1.795 min, [M+H]⁺= 663.4, purity = 94.00%.

¹H NMR (500 MHz, DMSO-d6) δ 12.06 (d, J = 2.7 Hz, 1H), 10.77 (s, 1H), 9.72 (s, 1H), 8.86 (s, 1H), 8.81 (d, J = 2.3 Hz, 1H), 8.41 (dd, J = 2.5, 1.0 Hz, 1H), 8.39 (s, 1H), 8.16 (s, 1H), 8.13 (d, J = 2.7 Hz, 1H), 8.11 (d, J = 0.7 Hz, 1H), 7.36 - 7.28 (m, 2H), 6.64 (d, J = 9.0 Hz, 2H), 5.70 (d, J = 7.6 Hz, 1H), 4.99 (s, 2H), 4.44 - 4.22 (m, 2H), 3.16 (d, J = 11.6 Hz, 2H), 2.79 - 2.68 (m, 1H), 2.64 - 2.58 (m, 2H), 2.38 (s, 3H), 2.17 - 2.04 (m, 6H), 1.86 (qd, J = 12.2, 4.6 Hz, 1H), 1.10 (t, J = 7.2 Hz, 3H).

### Example 7: Preparation of compound C008

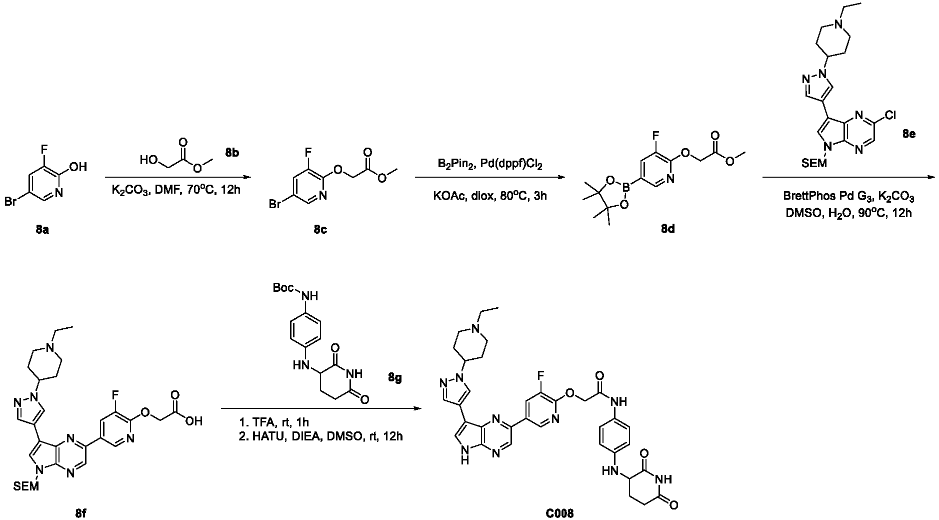

Following the method as described in Steps 2-5 of Example 6, except that compound 6d and NaH in Step 2 of Example 6 were replaced with compound 8a and K₂CO₃, respectively, and that the reaction temperature was set to 70°C, compound C008 was obtained. LCMS: Rt = 1.741 min, [M+H]⁺= 667.4.

### Example 8: Preparation of compound C009

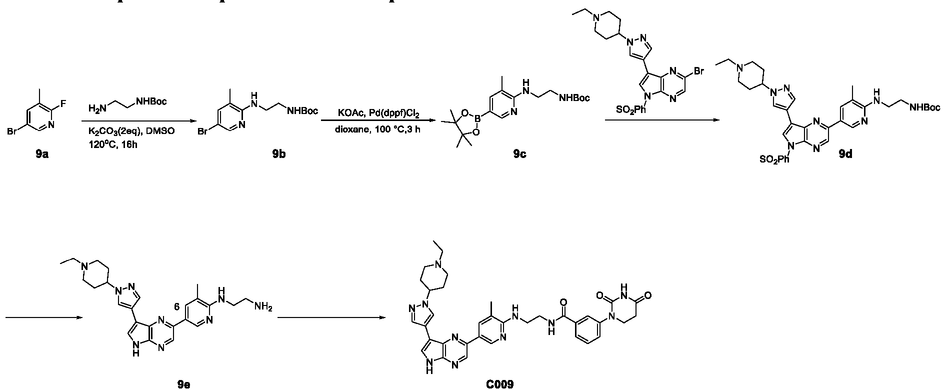

### Step 1:

Compound 9a (1.0 g, 5.3mmol), N-Boc-1,2-ethylenediamine (1.02 g, 6.38 mmol), K₂CO₃ (1.47 g, 10.6 mmol) were dissolved in DMSO (20ML) and reacted at 120°C for 6 h. TLC showed the complete conversion of the raw materials, and the product was detected. The reaction solution was added with 100 mL of water and extracted with ethyl acetate (100 mL*3). The organic phase was dried over anhydrous sodium sulfate and filtered. The filtrate was concentrated in vacuum to give a residue. The reaction solution was distilled under reduced pressure to give a residue. The residue was purified by column chromatography (PE:EA=5:1) to give the compound 9b. [M+H]⁺=230.0.

### Step 2:

Compound 9b (0.5 g, 1.52 mmol), B₂Pin₂ (578.9 mg, 2.28 mmol), KOAc ((447 mg, 4.56 mmol), and Pd(dppf)Cl₂ (111 mg, 0.152 mmol) were dissolved in dioxane (5 mL) at room temperature, replaced with nitrogen three times, raised to 80°C, and stirred at this temperature for 3 h. TLC showed the complete conversion of the raw materials, and the product was detected. After completion of the reaction, the reaction solution was cooled to room temperature and filtered. The filtrate was concentrated in vacuum to give a residue. The residue was purified by column chromatography to give the compound 9c. [M+H]⁺=378.0.

### Step 3:

Compound 9c (0.4 g, 1.06 mmol), warhead3 (300 mg, 0.58 mmol), BrettPhos Pd G3 (60 mg, 66 µmol), and K₂CO₃ (270 mg, 1.98 mmol) were dissolved in DMSO/H₂O (4 mL/1 mL) at room temperature, replaced with nitrogen three times, raised to 100°C, and stirred at this temperature for 12 h. LCMS showed the complete conversion of the reactants, and the product was detected. The reaction solution was added with 30 mL of water and 3 mL of formic acid and extracted with dichloromethane (50 mL*3). The organic phase was dried over anhydrous sodium sulfate and filtered. The filtrate was concentrated in vacuum to give a residue. The residue was purified by column chromatography to give the compound 9d.

### Step 4:

Compound 9d was dissolved in 10 ml of HCl/dioxane and stirred at room temperature for 30 minutes to give the crude compound 9e. [M+H]⁺=446.

### Step 5:

Compound 9e (130 mg, 0.29 mmol), 3-(2,4-dioxotetrahydropyrimidin-1(2H)-yl)benzoic acid (68 mg, 0.29 mmol), HATU (165 mg, 0.435 mmol), and DIEA (75 mg, 0.58 mmol) were dissolved in 10 ml of DMSO and reacted at room temperature for 1 h. LCMS showed the complete conversion of the reactants, and the product was detected. The product was purified by preparative chromatography to give the final product C009. [M+H]⁺=662.4, purity: 95.3%.

¹H NMR (400 MHz, MeOD) δ 8.75 (s, 1H), 8.59 (d, J = 12.9 Hz, 2H), 8.36 (s, 1H), 8.21 (s, 1H), 8.04 (s, 1H), 7.86 (s, 1H), 7.80 - 7.74 (m, 1H), 7.57 - 7.50 (m, 2H), 4.71 - 4.60 (m, 1H), 3.89 (t, J = 6.7 Hz, 2H), 3.77 (s, 6H), 3.30 - 3.14 (m, 4H), 2.80 (t, J = 6.7 Hz, 2H), 2.41 (d, J = 13.5 Hz, 7H), 1.40 (t, J = 7.3 Hz, 3H).

### Example 9: Preparation of compound C010

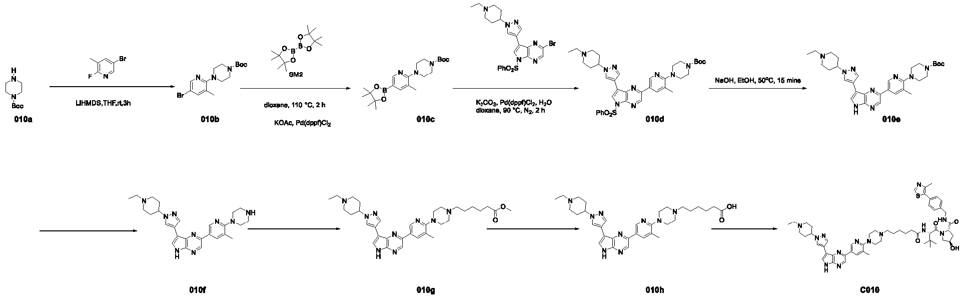

### Step 1:

Compound 010a (10 g, 53 mmol) was dissolved in anhydrous THF (200 ml). LiHMDS (80.6 mL, 80.65 mmol) was added at 0°C and reacted for 0.5 h. Then, 2-fluoro-3-methyl-5-bromopyridine (10.07 g, 53 mmol) was added to the system above and reacted at room temperature for 3 h. LCMS showed the complete conversion of the reactants, and the product was detected. The reaction solution was added with 100 mL of water and extracted with ethyl acetate (100 mL*3). The organic phase was dried over anhydrous sodium sulfate and filtered. The filtrate was concentrated in vacuum to give a residue. The reaction solution was distilled under reduced pressure to give a residue. The residue was purified by column chromatography to give the compound 010b. [M+H]⁺=356.1.

### Step 2:

Compound 010b (3.0 g, 8.45 mmol), B₂Pin₂ (3.2 g, 12.67 mmol), KOAc (2.485 g, 25.3 mmol), and Pd(dppf)Cl₂ (618 mg, 0.845 mmol) were dissolved in dioxane (30 mL) at room temperature, replaced with nitrogen three times, raised to 110°C, and stirred at this temperature for 3 h. TLC showed the complete conversion of the raw materials, and the product was detected. After completion of the reaction, the reaction solution was cooled to room temperature and filtered. The filtrate was concentrated in vacuum to give a residue. The residue was purified by column chromatography to give the compound 010c. [M+H]⁺=404.0.

### Step 3:

Compound 010c (2.14 g, 5.3 mmol), Warhead3 (2.72 g, 5.3 mmol), Pd(dppf)Cl₂ (387 mg, 5.3 mmol) and K₂CO₃ (1.46 g, 10.6 mmol) were dissolved in DMSO/H₂O (20 mL/5 mL) at room temperature, replaced with nitrogen three times, raised to 100°C, and stirred at this temperature for 12 h. LCMS showed the complete conversion of the reactants, and the product was detected. The reaction solution was added with 30 mL of water and 3 mL of formic acid and extracted with dichloromethane (50 mL*3). The organic phase was dried over anhydrous sodium sulfate and filtered. The filtrate was concentrated in vacuum to give a residue. The residue was purified by column chromatography to give the compound 010d. [M+H]⁺=712.

### Step 4:

Compound 010d was dissolved in 20 ml of ethanol, then sodium hydroxide (60 mg, 1.4 mmol) was added and stirred at 50°C for 15 minutes. LCMS showed the complete conversion of the reactants, and the product was detected. The reaction solution was concentrated in vacuum to give the crude compound 010e. [M+H]⁺=572.

### Step 5:

Compound 010e was dissolved in 10 ml of HCl/dioxane and stirred at room temperature for 30 minutes to give the crude compound 010f. [M+H]⁺=472.

### Step 6:

Compound 010f (400 mg, 0.85 mmol), methyl 6-bromohexanoate (195 mg, 0.93 mmol), K₂CO₃ (586.5 mg, 4.25 mmol), and KI (705.5 mg, 4.25 mmol) were dissolved in ACN and reacted at 90°C for 6 h. LCMS showed the complete conversion of the reactants, and the product was detected. The reaction solution was added with 30 mL of water and extracted with dichloromethane (50 mL*3). The organic phase was dried over anhydrous sodium sulfate and filtered. The filtrate was concentrated in vacuum to give a residue. Compound 010g was obtained by column chromatography.

### Step 7:

Compound 010g (200 mg, 0.33 mmol) and LiOH (79 mg, 3.3 mmol) were dissolved in a 10 ml system of THF/H₂O = 1: 1, and stirred at room temperature for 20 h. LCMS showed the complete conversion of the reactants, and the product was detected. The product was directly dried by rotary evaporation to give the compound 010h. [M+H]⁺=586.

### Step 8:

Compound 010h (100 mg, 0.17 mmol), (2S,4R)-1-((S)-2-amino-3,3-dimethylbutanoyl)-4-hydroxy-N-(4-(4-methylthiazol-5-yl)benzyl)pyrrolidine-2-carboxamide (90 mg, 0.34 mmol), HATU (130 mg, 0.75 mmol), and DIEA (122 mg, 0.044 mmol) were dissolved in DMF (2ml) and reacted at room temperature for 2 h. LCMS showed the complete conversion of the reactants, and the product was detected. The product was purified by preparative chromatography to give the final product C010. LCMS: Rt=2.180min; [M+H]⁺=998.7.

### Example 10: Preparation of compound C011

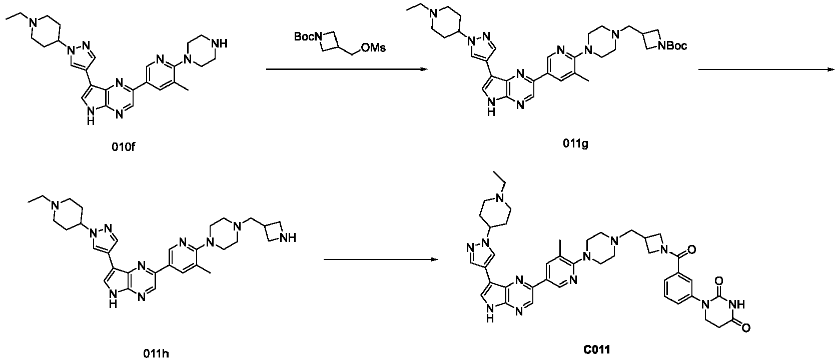

### Step 1:

Compound 010f (400 mg, 0.85 mmol, prepared according to Example 9, steps 1-5) was dissolved in CAN (10 ml). Tert-butyl 3-(methanesulfonyloxymethyl)azetidine-1-carboxylate (226 mg, 0.85 mmol), DIEA (548 mg, 4.25 mmol), and KI (706 mg, 4.25 mmol) were added and reacted at 100°C for 16 h. LCMS showed the complete conversion of the reactants, and the product was detected. The reaction solution was added with 30 mL of water and extracted with dichloromethane (50 mL*3). The organic phase was dried over anhydrous sodium sulfate and filtered. The filtrate was concentrated in vacuum to give a residue. Compound 011g was obtained by column chromatography.

### Step 2:

Compound 011g was dissolved in 10 ml of HCl/dioxane and stirred at room temperature for 30 minutes to give the crude compound 011h.

### Step 3:

Compound 011h (100 mg, 0.185 mmol) was dissolved in DMF (2ml). 3-(2,4-Dioxotetrahydropyrimidin-1(2H)-yl)benzoic acid (48 mg, 0.185 mmol), HATU (105 mg, 0.27 mmol), DIEA (95 mg, 0.74 mmol) were added and reacted at room temperature for 1 h. LCMS showed the complete conversion of the reactants, and the product was detected. The product was purified by preparative chromatography to give C011.

### Example 11: Preparation of compound C017

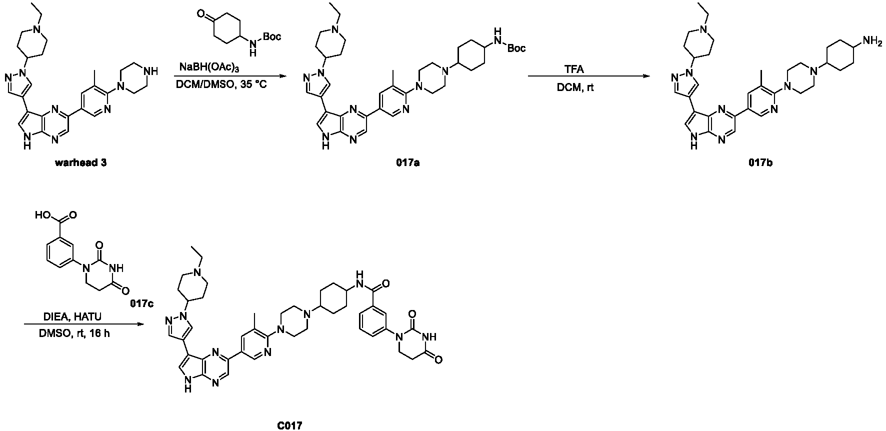

### Step 1:

Warhead 3 (167 mg, 0.142 mmol), 4-N-Boc-aminocyclohexanone (113 mg, 0.532 mmol), and acetic acid (21 mg, 0.142 mmol) were dissolved in dichloromethane/dimethyl sulfoxide (4 mL/4 mL). The mixture was stirred at 35°C for 16 h. Sodium triacetylborohydride (376 mg, 1.77 mmol) was then added to the mixture and the reaction was then stirred at 35°C for 2 h. LCMS showed the complete conversion of the reactants, and the product was detected. The reaction solution was cooled to room temperature, quenched by the addition of saturated ammonium chloride solution (50 mL), and extracted with dichloromethane (30 mL*2). The organic layer was dried with anhydrous sodium sulfate, filtered, and concentrated in vacuum to give a residue. The residue was purified by silica gel chromatography to give the compound 017a. LCMS: [M+H]⁺=669.4.

### Step 2:

Compound 017a (105 mg, 0.157 mmol) was dissolved in dichloromethane/dimethylsulfoxide (1 mL/5 mL) and the reaction solution was stirred at room temperature for 16 h. LCMS showed the complete conversion of the reactants, and the product was detected. The reaction solution was distilled under reduced pressure to give the compound 017b. LCMS: [M+H]⁺=569.4.

### Step 3:

Compounds 017b (45 mg, 0.079 mmol), 017c (24 mg, 0.103 mmol), HATU (45 mg, 0.118 mmol), and N,N-diisopropylethyl amine (41 mg, 0.316 mmol) were dissolved in dimethyl sulfoxide (2 mL) and the mixture was stirred at room temperature overnight. LCMS showed the complete conversion of the reactants, and the product was detected. The reaction solution was cooled to room temperature, quenched by the addition of water (50 mL), and extracted with dichloromethane (30 mL*2). The organic layer was dried with anhydrous sodium sulfate, filtered, and concentrated in vacuum to give a residue. The residue was purified by Pre-TLC to give the compound C017. LCMS: [M+H]⁺=785.6. ¹H NMR (500 MHz, DMSO-d₆) δ 12.06 (s, 1H), 10.42 (s, 1H), 8.96 (s, 1H), 8.85 (s, 1H), 8.39 (s, 1H), 8.30 (s, 1H), 8.24 (d, J = 6.7 Hz, 1H), 8.17 - 8.06 (m, 2H), 7.80 (d, J = 10.4 Hz, 1H), 7.74 (d, J = 6.8 Hz, 1H), 7.47 (q, J = 8.0 Hz, 2H), 4.47 - 4.39 (m, 1H), 4.07 - 3.90 (m, 2H), 3.87 - 3.79 (m, 2H), 3.27 - 3.10 (m, 5H), 2.82 - 2.56 (m, 6H), 2.38 (s, 3H), 2.33 - 2.06 (m, 6H), 2.03 - 1.73 (m, 7H), 1.66 - 1.48 (m, 4H), 1.18 - 1.04 (m, 3H).

### Example 12: Preparation of compound C023

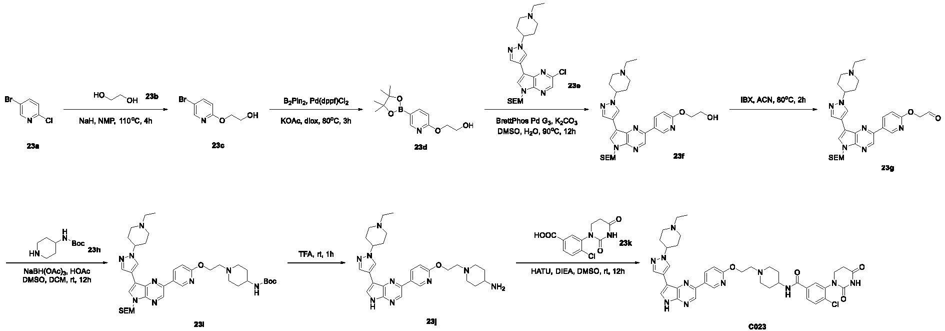

### Step 1:

Compounds 23a (1 g, 5.20 mmol), and 23b (645 mg, 10.39 mmol) were dissolved in NMP (20 mL). NaH (416 mg, 10.39 mmol) was added, raised to 110°C and stirred at this temperature for 12 h. TLC showed the complete conversion of the raw materials, and the product was detected. The reaction solution was distilled under reduced pressure to obtain a residue. The residue was purified by column chromatography to give the compound 23c.

### Step 2:

Compound 23c (1.25 g, 5.73 mmol), B₂Pin₂ (2.91 g, 10.47 mmol), KOAc (1.69 g, 17.20 mmol), and Pd(dppf)Cl₂ (465 mg, 0.57 mmol) were dissolved in dioxane (25 mL) at room temperature, replaced with nitrogen three times, raised to 80°C, and stirred at this temperature for 3 h. TLC showed the complete conversion of the raw materials, and the product was detected. After completion of the reaction, the reaction solution was cooled to room temperature and filtered. The filtrate was concentrated in vacuum to give a residue. The residue was purified by column chromatography to give the compound 8d.

### Step 3:

Compounds 23d (345 mg, 1.30 mmol), 23e (400 mg, 0.87 mmol), BrettPhos Pd G3 (79 mg, 87 µmol), and K₂CO₃ (360 mg, 2.60 mmol) were dissolved in DMSO/H₂O (10 mL/2 mL) at room temperature, replaced with nitrogen three times, raised to 100°C, and stirred at this temperature for 12 h. LCMS showed the complete conversion of the reactants, and the product was detected. The reaction solution was added with 30 mL of water and extracted with dichloromethane (50 mL*3). The organic phase was dried over anhydrous sodium sulfate and filtered. The filtrate was concentrated in vacuum to give a residue. The residue was purified by column chromatography to give the compound 23f.

### Step 4:

Compound 23f (382 mg, 0.68 mmol) was dissolved in ACN (10 mL). IBX (379 mg, 1.36 mmol) was added, raised to 80°C, and stirred at this temperature for 2 h. Most of the starting material was converted as demonstrated by LCMS, and the product was detected. After completion of the reaction, the reaction solution was cooled to room temperature and filtered. The filtrate was concentrated in vacuum to give a residue. The residue was purified by column chromatography to give the compound 23g.

### Step 5:

Compounds 23g (50 mg, 89 µmol) and 23h (36 mg, 0.18 mmol) were dissolved in DMSO/DCM (2 mL/2 mL) at room temperature. 0.1 mL of acetic acid was added and stirred for 1 h at room temperature. Sodium borohydride acetate (95 mg, 0.45 mmol) was added and stirred for 1 h at room temperature. Most of the starting material was converted as demonstrated by LCMS, and the product was detected. The reaction solution was added with 30 mL of water and extracted with dichloromethane (20 mL*3). The organic phase was dried over anhydrous sodium sulfate and filtered. The filtrate was concentrated in vacuum to give a residue. The residue was purified by Prep-TLC to give the compound 23i.

### Step 6:

Compound 23i (15 mg, 20.1 µmol) was dissolved in TFA (3 mL) and stirred at room temperature for 1 h. LCMS showed the complete conversion of the reactants, and the product was detected. The reaction solution was distilled under reduced pressure to give the crude compound 23j.

### Step 7:

Compounds 23j (10 mg, 20.2 µmol), 23k (8 mg, 30.3 µmol), DIEA (8 mg, 60.5 µmol), and HATU (15 mg, 40.3 mmol) were dissolved in DMSO (2 mL) and stirred at room temperature for 12 h. LCMS showed the complete conversion of the reactants, and the product was detected. The reaction solution was added with 30 mL of water and extracted with dichloromethane (20 mL*3). The organic phase was dried over anhydrous sodium sulfate and filtered. The filtrate was concentrated in vacuum to give a residue. The product was purified by preparative chromatography to give the final product C023. LCMS: Rt = 1.489 min, [M+H]⁺ = 766.4.

### Example 13: Preparation of compound C025

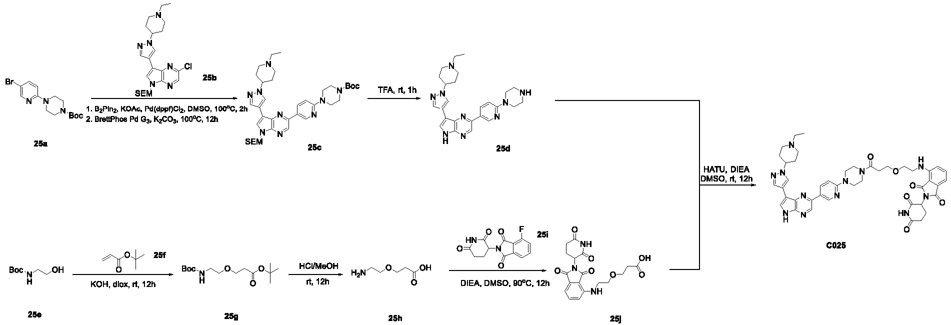

### Step 1:

Compounds 25a (445 mg, 1.30 mmol), 8b (300 mg, 0.65 mmol), KOAc (320 mg, 3.25 mmol), and Pd(dppf)Cl₂ (53 mg, 65.1 µmol) were dissolved in DMSO (10 mL), replaced with nitrogen three times, raised to 100°C, and stirred at this temperature for 3 h. LCMS showed the complete conversion of the starting materials. The reaction solution cooled to room temperature. BrettPhos G3 (59 mg, 65.1 µmol) and K₂CO₃ (270 mg, 1.95 mmol) were added, replaced with nitrogen three times, raised to 100°C and stirred at this temperature for 12 h. LCMS showed the complete conversion of the reactants, and the product was detected. The reaction solution was added with 100 mL of water and extracted with ethyl acetate (100 mL*3). The organic phase was dried over anhydrous sodium sulfate and filtered. The filtrate was concentrated in vacuum to give a residue. The reaction solution was distilled under reduced pressure to give a residue. The residue was purified by column chromatography to give the compound 25c.

### Step 2:

Compound 25c (85 mg, 0.12 mmol) was dissolved in TFA (3 mL) and stirred at room temperature for 1 h. LCMS showed the complete conversion of the reactants, and the product was detected. The reaction solution was distilled under reduced pressure to give the crude compound 25d.

### Step 3:

Compounds 25e (1 g, 6.20 mmol), 25f (1.59 g, 12.41 mmol) were dissolved in dioxane/60% KOH aq. (3 mL/0.2 mL) and stirred at room temperature for 12 h. TLC showed the complete conversion of the reactants, and the product was detected. The reaction solution was added with 30 mL of water and extracted with ethyl acetate (50 mL*3). The organic phase was dried over anhydrous sodium sulfate and filtered. The filtrate was concentrated in vacuum to give a residue. The residue was purified by column chromatography to give the compound 25g.

### Step 4:

Compound 25g (1.09 g, 3.77 mmol) was dissolved in MeOH (5 mL), added with methanolic hydrochloric acid (10 mL) and stirred at room temperature for 12 h. The reaction solution was distilled under reduced pressure to give the crude compound 25 h.

### Step 5:

Compounds 25h (425 mg, 3.62 mmol), and 25i (500 mg, 1.81 mmol) were dissolved in MeOH/DIEA (5 mL/1 mL), raised to 90°C, and stirred at this temperature for 12 h. LCMS showed the complete conversion of the starting materials. The reaction solution was cooled to room temperature. The reaction solution was added with 30 mL of water, adjusted with 1N HCl to pH =7, and extracted with ethyl acetate (50 mL*3). The organic phase was dried over anhydrous sodium sulfate and filtered. The filtrate was concentrated in vacuum to give a residue. The reaction solution was distilled under reduced pressure to give a residue. The residue was purified by column chromatography to give the compound 25j.

### Step 6:

Compounds 25j (32 mg, 82.0 µmol), 25d (25 mg, 54.6 µmol), DIEA (22 mg, 163.9 µmol), and HATU (42 mg, 109.3 µmol) were dissolved in DMSO (2 mL) and stirred at room temperature for 12 h. LCMS showed the complete conversion of the reactants, and the product was detected. The product was purified by preparative chromatography to give the final product C025. LCMS: Rt = 1.737 min, [M+H]⁺ = 829.6.

### Example 14: Preparation of compound C026

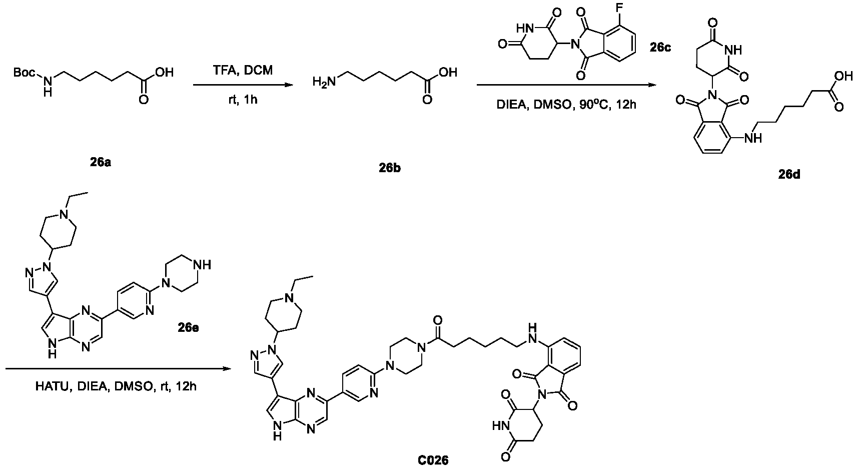

### Step 1:

Compound 26a (837 mg, 3.62 mmol) was dissolved in DCM (10 mL). TFA (1 mL) was added and stirred at room temperature for 1 h. The reaction solution was distilled under reduced pressure to give the crude compound 26b.

### Step 2:

Compounds 26b (474 mg, 3.62 mmol) and 26c (500 mg, 1.81 mmol) were dissolved in MeOH/DIEA (5 mL/1 mL), raised to 90°C, and stirred at this temperature for 12 h. LCMS showed the complete conversion of the starting materials. The reaction solution was cooled to room temperature. The reaction solution was added with 30 mL of water, adjusted with 1N HCl to pH = 7, and extracted with ethyl acetate (50 mL*3). The organic phase was dried over anhydrous sodium sulfate and filtered. The filtrate was concentrated in vacuum to give a residue. The reaction solution was distilled under reduced pressure to give a residue. The residue was purified by column chromatography to give the compound 26d.

### Step 3:

Compounds 26d (32 mg, 82.0 µmol), 25d (25 mg, 54.6 µmol), DIEA (22 mg, 163.9 µmol), and HATU (42 mg, 109.3 µmol) were dissolved in DMSO (2 mL) and stirred at room temperature for 12 h. LCMS showed the complete conversion of the reactants, and the product was detected. The product was purified by preparative chromatography to give the final product C026. LCMS: Rt = 1.902 min, [M+H]⁺= 827.1.

### Example 15: Preparation of compound C027

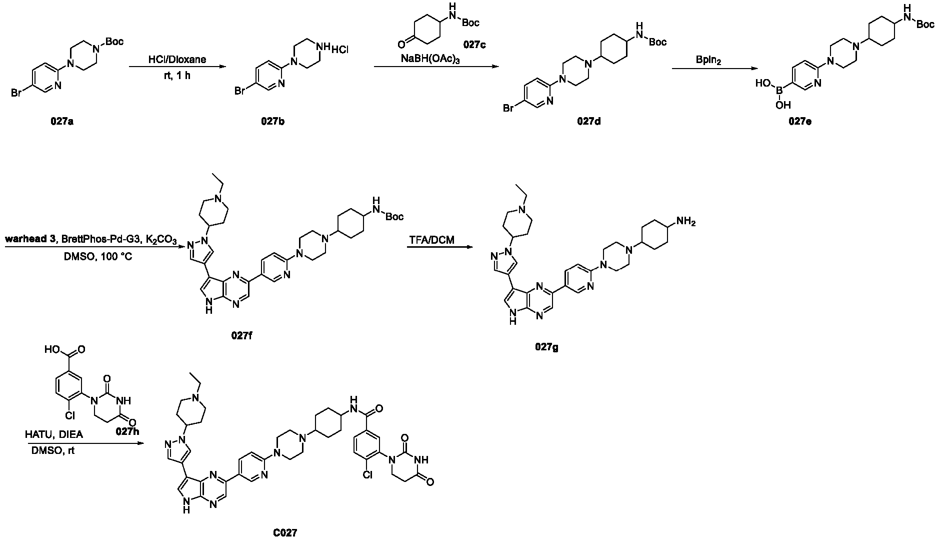

### Step 1:

Compound 027a (900 mg, 2.64 mmol) was dissolved in HCl/dioxane (10 mL, 4M) at room temperature and stirred for 1 h. LCMS showed the complete conversion of the reactants, and the product was detected. The reaction solution was distilled under reduced pressure to give the compound 027b. LCMS: [M+H]⁺=242.1.

### Step 2:

Compound 027b (731 mg, 2.62 mmol) and DIEA (339 mg, 2.62 mmol) were dissolved in dichloromethane/dimethyl sulfoxide (5 mL/5 mL) and stirred for 10 min. Compound 027c (672 mg, 3.15 mmol) and acetic acid (157 mg, 2.62 mmol) were then added to the reaction solution and stirred at 35°C for 16 h. NaBH(OAc)₃ (1.67 g, 7.87 mmol) was then added to the reaction solution and stirred at 35°C for 1 h. LCMS showed the complete conversion of the reactants, and the product was detected. The reaction solution was poured into a saturated sodium bicarbonate solution (60 mL) and extracted with dichloromethane (30 mL*2). The organic layer was dried with anhydrous sodium sulfate, filtered, and concentrated in vacuum to give a crude product. The crude product was purified by flash silica gel column to give the compound 027d. LCMS: [M+H]⁺=439.2.

### Step 3:

Compound 027d (510 mg, 1.16 mmol), potassium acetate (228 mg, 2.33 mmol), bis(pinacolato)diboron (384 mg, 1.51 mmol), and Pd(dppf)Cl₂ (85 mg, 0.116 mmol) were dissolved in dimethyl sulfoxide (5 mL). The reaction solution was stirred at 100°C for 2 h under a nitrogen atmosphere. LCMS showed the complete conversion of the reactants, and the product was detected. The reaction solution was cooled to room temperature, quenched by the addition of water (60 mL), and extracted with dichloromethane (20 mL*2). The organic layer was dried with anhydrous sodium sulfate, filtered, and concentrated in vacuum to give a crude product. The crude product was purified by flash silica gel column to give the compound 027e. LCMS: [M+H]⁺=405.2.

### Step 4:

Compound 027e (54 mg, 0.159 mmol), warhead 3 (50 mg, 0.097 mmol), potassium carbonate (44 mg, 0.318 mmol), and BrettPhos-Pd-G3 (49 mg, 0.005 mmol) were dissolved in dimethyl sulfoxide (2 mL). The reaction solution was stirred at 100°C for 3 h under a nitrogen atmosphere. LCMS showed the complete conversion of the reactants, and the product was detected. The reaction solution was cooled to room temperature, quenched by the addition of water (50 mL), and extracted with dichloromethane (20 mL*3). The organic layer was dried with anhydrous sodium sulfate, filtered, and concentrated in vacuum to give a crude product. The crude product was purified by flash silica gel column to give the compound 027f. LCMS: [M+H]⁺=655.4.

### Step 5:

Compound 027f (25 mg, 0.0382 mmol) was dissolved in trifluoroacetic acid/dichloromethane (1 mL/5 mL) at room temperature and stirred for 2 h. LCMS showed the complete conversion of the reactants, and the product was detected. The reaction solution was distilled under reduced pressure to give the compound 027g. LCMS: [M+H]⁺=555.2.

### Step 6:

Compounds 027g (21 mg, 0.038 mmol), 027h (12 mg, 0.045 mmol), DIEA (24 mg, 0.189 mmol) and HATU (19 mg, 0.049 mmol) were dissolved in dimethyl sulfoxide (2 mL). The mixture was stirred at room temperature for 16 h. LCMS showed the complete conversion of the reactants, and the product was detected. The reaction solution was cooled to room temperature, quenched by the addition of water (50 mL), and extracted with dichloromethane (30 mL*2). The organic layer was dried with anhydrous sodium sulfate, filtered, and concentrated in vacuum to give a crude product. The crude product was purified by Pre-TLC to give the compound C027. LCMS: [M+H]⁺=805.5.

### Example 16: Preparation of compound C028

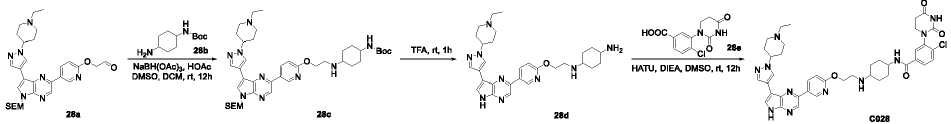

### Step 1:

Compounds 28a (50 mg, 89 µmol) and 28b (38 mg, 0.18 mmol) were dissolved in DMSO/DCM (2 mL/2 mL) at room temperature. 0.1 mL of acetic acid was added and stirred at room temperature for 1 h. Sodium borohydride acetate (95 mg, 0.45 mmol) was added and stirred at room temperature for 1 h. Most of the starting material was converted as demonstrated by LCMS, and the product was detected. The reaction solution was added with 30 mL of water and extracted with dichloromethane (20 mL*3). The organic phase was dried over anhydrous sodium sulfate and filtered. The filtrate was concentrated in vacuum to give a residue. The residue was purified by Prep-TLC to give the compound 28c.

### Step 2:

Compound 28c (15 mg, 19.7 µmol) was dissolved in TFA (3 mL) and stirred at room temperature for 1 h. LCMS showed the complete conversion of the reactants, and the product was detected. The reaction solution was distilled under reduced pressure to give the crude compound 28d.

### Step 3:

Compounds 28d (10 mg, 18.9 µmol), 28e (8 mg, 28.3 µmol), DIEA (8 mg, 56.6 µmol), and HATU (15 mg, 37.8 µmol) were dissolved in DMSO (2 mL) and stirred at room temperature for 12 h. LCMS showed the complete conversion of the reactants, and the product was detected. The reaction solution was added with 30 mL of water and extracted with dichloromethane (20 mL*3). The organic phase was dried over anhydrous sodium sulfate and filtered. The filtrate was concentrated in vacuum to give a residue. The product was purified by preparative chromatography to give the final product C028. LCMS: Rt = 1.522 min, [M+H]⁺= 780.5.

### Example 17: Preparation of compound C030

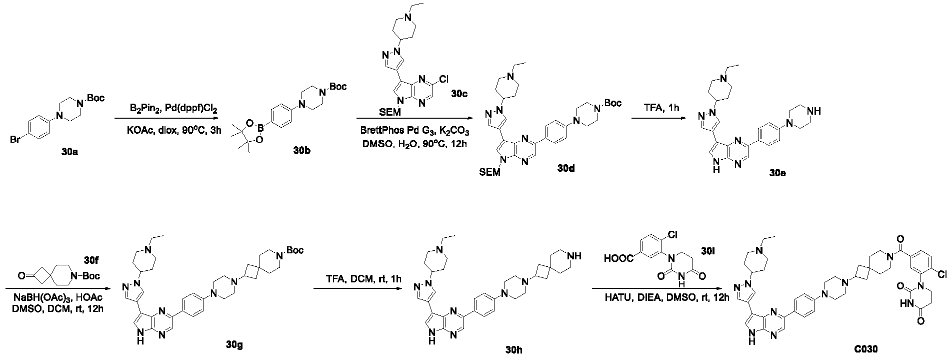

### Step 1:

Compound 30a (2 g, 5.86 mmol), B₂Pin₂ (2.98 mg, 11.72 mmol), KOAc (1.73 g, 17.58 mmol), and Pd(dppf)Cl₂ (475 mg, 0.59 mmol) were dissolved in dioxane (40 mL) at room temperature, replaced with nitrogen three times, raised to 90°C, and stirred at this temperature for 3 h. TLC showed the complete conversion of the raw materials, and the product was detected. After completion of the reaction, the reaction solution was cooled to room temperature and filtered. The filtrate was concentrated in vacuum to give a residue. The residue was purified by column chromatography to give the compound 30b.

### Step 2:

Compounds 30b (631 mg, 1.63 mmol), 30c (500 mg, 1.08 mmol), BrettPhos Pd G3 (98 mg, 0.11 mmol) and K₂CO₃ (450 mg, 3.25 mmol) were dissolved in DMSO/H₂O (10 mL/2 mL) at room temperature, replaced with nitrogen three times, raised to 90°C, and stirred at this temperature for 12 h. LCMS showed the complete conversion of the reactants, and the product was detected. The reaction solution was added with 30 mL of water and extracted with dichloromethane (50 mL*3). The organic phase was dried over anhydrous sodium sulfate and filtered. The filtrate was concentrated in vacuum to give a residue. The residue was purified by column chromatography to give the compound 30d.

### Step 3:

Compound 28c (354 mg, 0.52 mmol) was dissolved in TFA (3 mL) and stirred at room temperature for 1 h. LCMS showed the complete conversion of the reactants, and the product was detected. The reaction solution was distilled under reduced pressure to give the crude compound 30e.

### Step 4:

Compounds 30e (50 mg, 0.11 mmol) and 30f (53 mg, 0.22 mmol) were dissolved in DMSO/DCM (2 mL/2 mL) at room temperature. 0.1 mL of acetic acid was added and stirred at room temperature for 1 h. Sodium borohydride acetate (70 mg, 0.33 mmol) was added and stirred at room temperature for 1 h. Most of the starting material was converted as demonstrated by LCMS, and the product was detected. The reaction solution was added with 30 mL of water and extracted with dichloromethane (20 mL*3). The organic phase was dried over anhydrous sodium sulfate and filtered. The filtrate was concentrated in vacuum to give a residue. The residue was purified by Prep-TLC to give the compound 30g.

### Step 5:

Compound 30g (57 mg, 83.8 µmol) was dissolved in DCM (3 mL). TFA (1 mL) was added and stirred at room temperature for 1 h. LCMS showed the complete conversion of the reactants, and the product was detected. The reaction solution was distilled under reduced pressure to give the crude compound 30h.

### Step 6:

Compounds 30h (49 mg, 82.8 µmol), 30i (33 mg, 124.2 µmol), DIEA (32 mg, 248.4 µmol), and HATU (63 mg, 165.6 µmol) were dissolved in DMSO (2 mL) and stirred at room temperature for 1 h. LCMS showed the complete conversion of the reactants, and the product was detected. The reaction solution was added with 30 mL of water and extracted with dichloromethane (20 mL*3). The organic phase was dried over anhydrous sodium sulfate and filtered. The filtrate was concentrated in vacuum to give a residue. The residue was purified by Prep-TLC to give the final product C030. LCMS: Rt = 1.570 min, [M+H]⁺= 830.6.

### Example 18: Preparation of compound C032

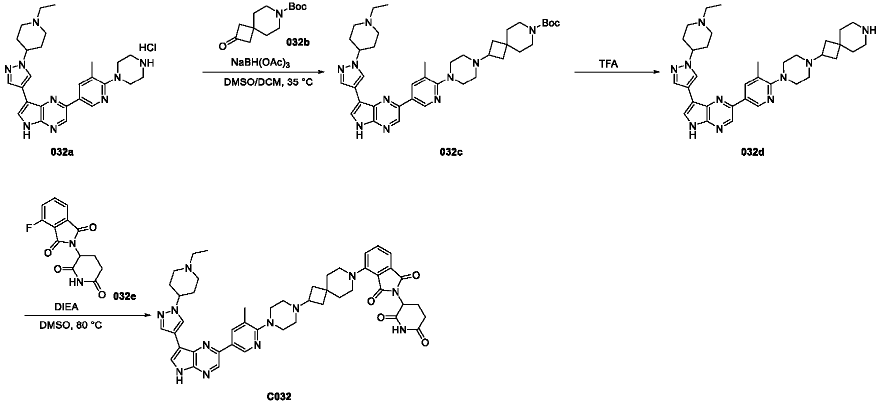

### Step 1:

Compounds 032a (100 mg, 0.212 mmol) and 032b (66 mg, 0.275 mmol) were dissolved in dichloromethane/dimethylsulfoxide (4 mL/4 mL). The mixture was stirred at 35°C for 16 h. Sodium triacetylborohydride (225 mg, 1.06 mmol) was then added to the mixture, and then stirred at 35°C for reaction for 2 h. LCMS showed the complete conversion of the reactants, and the product was detected. The reaction solution was cooled to room temperature, quenched by the addition of saturated ammonium chloride solution (50 mL), and extracted with dichloromethane (30 mL*2). The organic layer was dried with anhydrous sodium sulfate, filtered, and concentrated in vacuum to give a residue. The residue was purified by silica gel chromatography to give the compound 032c. LCMS: [M+H]⁺=695.5.

### Step 2:

Compound 032c (60 mg, 0.086 mmol) was dissolved in dichloromethane (5 mL). Trifluoroacetic acid (1 mL) was added at room temperature and stirred for reaction for 2 h. LCMS showed the complete conversion of the reactants, and the product was detected. The reaction solution was distilled under reduced pressure to give the compound 032d. LCMS: [M+H]⁺=595.4.

### Step 3:

Compounds 32d (51 mg, 0.086 mmol), 032e (24 mg, 0.087 mmol), DIEA (55 mg, 0.426 mmol) were dissolved in dimethyl sulfoxide (2 mL) at room temperature and the reaction solution was stirred at 80°C for 2 h. LCMS showed the complete conversion of the reactants, and the product was detected. The reaction solution was cooled to room temperature, quenched with water (50 mL) and extracted with methanol/dichloromethane (20 mL/20 mL*2). The organic layer was dried with anhydrous sodium sulfate, filtered, and concentrated in vacuum to give a residue. The residue was purified by Pre-TLC to give the compound C032. **LCMS:** [M+H]⁺=851.6. ¹H **NMR (500 MHz, DMSO-d₆)** *δ* 12.04 (s, 1H), 11.09 (s, 1H), 8.96 (d, J = 2.2 Hz, 1H), 8.84 (s, 1H), 8.38 (s, 1H), 8.30 - 8.28 (m, 1H), 8.11 (d, J = 2.5 Hz, 1H), 8.09 (s, 1H), 7.70 - 7.66 (m, 1H), 7.34 (dd, J = 11.7, 7.9 Hz, 2H), 5.09 (dd, J = 12.6, 5.5 Hz, 2H), 4.24 (s, 2H), 3.26 (d, J = 3.7 Hz, 2H), 3.23 - 3.10 (m, 6H), 3.01 - 2.96 (m, 2H), 2.92 - 2.74 (m, 4H), 2.66 - 2.56 (m, 2H), 2.46 (s, 2H), 2.39 - 2.33 (m, 4H), 2.11 - 1.96 (m, 8H), 1.80 - 1.72 (m, 2H), 1.71 - 1.60 (m, 4H), 1.04 (t, J = 7.2 Hz, 3H).

### Example 19: Preparation of compound C033

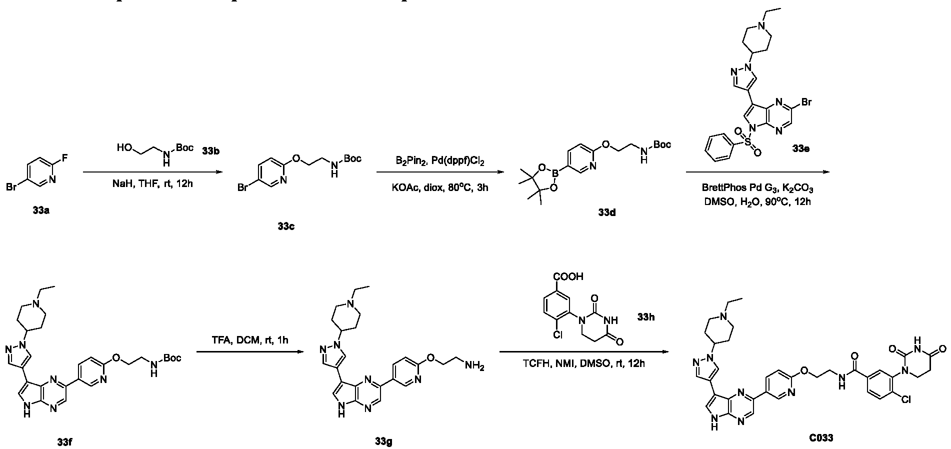

### Step 1:

Compounds 33a (1 g, 5.68 mmol) and 33b (1.37 g, 8.52 mmol) were dissolved in THF (20 mL). NaH (570 mg, 14.21 mmol) was added and stirred at room temperature for 12 h. TLC showed the complete conversion of the raw materials, and the product was detected. The reaction solution was added with 100 mL of water and extracted with ethyl acetate (100 mL*3). The organic phase was dried over anhydrous sodium sulfate and filtered. The filtrate was concentrated in vacuum to give a residue. The reaction solution was distilled under reduced pressure to give a residue. The residue was purified by column chromatography to give the compound 33c.

### Step 2:

Compound 33c (1.37 g, 4.32 mmol), B₂Pin₂ (2.19 g, 8.64 mmol), KOAc (1.27 g, 12.96 mmol), and Pd(dppf)Cl₂ (350 mg, 0.43 mmol) were dissolved in dioxane (30 mL) at room temperature, replaced with nitrogen three times, raised to 80°C, and stirred at this temperature for 3 h. TLC showed the complete conversion of the raw materials, and the product was detected. After completion of the reaction, the reaction solution was cooled to room temperature and filtered. The filtrate was concentrated in vacuum to give a residue. The residue was purified by column chromatography to give the compound 33d.

### Step 3:

Compounds 33d (80 mg, 0.22 mmol), 33e (75 mg, 0.15 mmol), BrettPhos Pd G3 (14 mg, 15 µmol), and K₂CO₃ (60 mg, 0.44 mmol) were dissolved in DMSO/H₂O (2 mL/0.5 mL) at room temperature, replaced with nitrogen three times, raised to 90°C, and stirred at this temperature for 12 h. LCMS showed the complete conversion of the reactants, and the product was detected. The reaction solution was added with 30 mL of water and extracted with dichloromethane (50 mL*3). The organic phase was dried over anhydrous sodium sulfate and filtered. The filtrate was concentrated in vacuum to give a residue. The residue was purified by column chromatography to give the compound 33f.

### Step 4:

Compound 33f (69 mg, 0.13 mmol) was dissolved in DCM (3 mL). TFA (1 mL) was added and stirred at room temperature for 1 h. LCMS showed the complete conversion of the reactants, and the product was detected. The reaction solution was distilled under reduced pressure to give the crude compound 33g.

### Step 5:

Compounds 33g (56 mg, 0.13 mmol), 33h (53 mg, 0.19 mmol), NMI (37 mg, 0.45 mmol), and TCFH (44 mg, 0.16 mmol) were dissolved in DMSO (2 mL) and stirred at room temperature for 1 h. LCMS showed the complete conversion of the reactants, and the product was detected. The reaction solution was added with 30 mL of water and extracted with dichloromethane (20 mL*3). The organic phase was dried over anhydrous sodium sulfate and filtered. The filtrate was concentrated in vacuum to give a residue. The residue was purified by Prep-TLC to give the final product C033. LCMS: Rt = 1.719 min, [M+H]⁺ = 683.4.

### Example 20: Preparation of compound C034

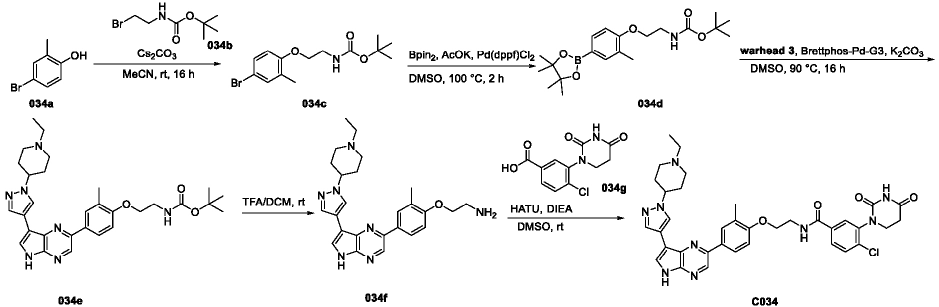

### Step 1:

Compounds 034a (1 g, 5.35 mmol), 034b (1.2 g, 5.35 mmol), and cesium carbonate (1.74 g, 5.35 mmol) were dissolved in acetonitrile (10 mL). The reaction solution was stirred at room temperature for 16 h. TLC showed the complete conversion of the reactants, and a new point was detected. The reaction solution was distilled under reduced pressure to obtain a crude product. The crude product was purified by silica gel chromatography to give the compound 034c.

### Step 2:

Compound 034c (1.212 g, 3.67 mmol) was dissolved in dimethyl sulfoxide (5 mL). Potassium acetate (1.08 g, 11.01 mmol), bis(pinacolato)diboron (1.21 g, 4.77 mmol) and Pd(dppf)Cl₂ (0.27 g, 0.367 mmol) were added at room temperature. The reaction solution was stirred for reaction at 100°C for 2 h under a nitrogen atmosphere. LCMS showed the complete conversion of the reactants, and the product was detected. The crude product was distilled under reduced pressure. The crude product was purified by silica gel chromatography to give the compound 034d. LCMS: [M-Bu+H]⁺=278.1.

### Step 3:

Compound 034d (64 mg, 0.176 mmol), warhead 3 (70 mg, 0.136 mmol), potassium carbonate (56 mg, 0.407 mmol), and BrettPhos-Pd-G3 (12 mg, 0.0136 mmol). The reaction solution was stirred for reaction at 90°C for 16 h under a nitrogen atmosphere. LCMS showed the complete conversion of the reactants, and the product was detected. The reaction solution was cooled to room temperature, quenched by the addition of water (50 mL), and extracted with dichloromethane (20 mL*3). The organic layer was dried with anhydrous sodium sulfate, filtered, and concentrated in vacuum to give a crude product. The crude product was purified by silica gel chromatography to give the compound C034e. LCMS: [M+H]⁺=546.4.

### Step 4:

Compound 034e (42 mg, 0.077 mmol) was dissolved in trifluoroacetic acid/dichloromethane (1 mL/5 mL) at room temperature and stirred for 2 h. LCMS showed the complete conversion of the reactants, and the product was detected. The reaction solution was distilled under reduced pressure to give the compound 034f. LCMS: [M+H]⁺=446.3.

### Step 5:

Compounds 034f (34 mg, 0.076 mmol), 034g (25 mg, 0.092 mmol), DIEA (50 mg, 0.381 mmol), and HATU (38 mg, 0.099 mmol) were dissolved in dimethyl sulfoxide (2 mL). The mixture was stirred at room temperature for 16 h. LCMS showed the complete conversion of the reactants, and the product was detected. The reaction solution was cooled to room temperature, quenched by the addition of water (50 mL), and extracted with dichloromethane (20 mL*2). The organic layer was dried with anhydrous sodium sulfate, filtered, and concentrated in vacuum to give a crude product. The crude product was purified by Pre-TLC to give the compound C034. LCMS: Rt=2.200 min; [M+H]⁺=696.4.

### Example 21: Preparation of compound C038

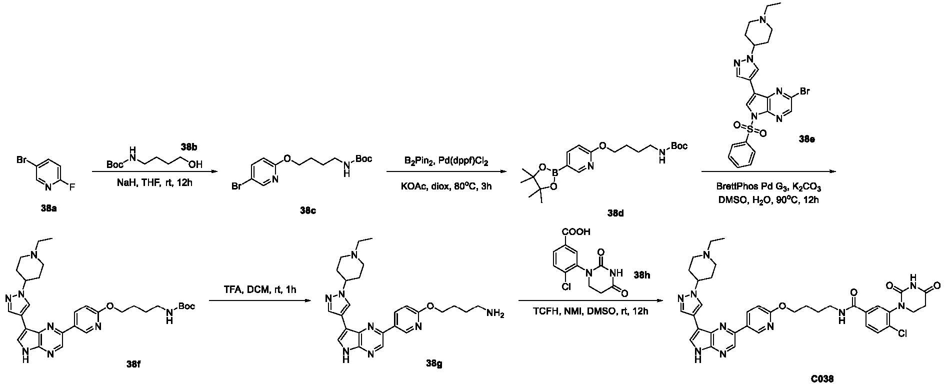

Following the method as described in Example 19, except that compound 33b in Step 1 was replaced with compound 38b, the final product C038 was obtained. LCMS: Rt = 1.796 min, [M+H]⁺ = 711.4.

### Example 22: Preparation of compound C039

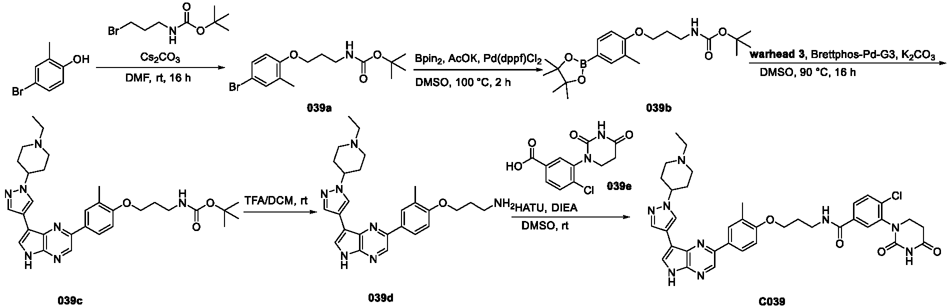

Following the method as described in Example 20, except that compound 034b was replaced with N-Boc-3-aminopropyl bromide and acetonitrile was replaced with DMF in Step 1, compound C039 was obtained. **LCMS:** [M+H]⁺=710.4. **¹H NMR (500 MHz, DMSO-d₆) *δ*** 11.98 (s, 1H), 10.53 (s, 1H), 8.81 (s, 1H), 8.76 (t, J = 5.6 Hz, 1H), 8.36 (s, 1H), 8.12 (s, 1H), 8.08 (d, J = 2.7 Hz, 1H), 8.03 (d, J = 8.2 Hz, 2H), 7.99 (d, J = 2.1 Hz, 1H), 7.87 (dd, J = 8.4, 2.1 Hz, 1H), 7.69 (d, J = 8.4 Hz, 1H), 7.09 (d, J = 8.2 Hz, 1H), 4.27 (s, 2H), 4.15 (d, J = 6.0 Hz, 2H), 3.76 (ddd, J = 13.6, 8.2, 5.8 Hz, 1H), 3.63 (dt, J = 11.8, 6.0 Hz, 1H), 3.54 - 3.47 (m, 2H), 3.25 (s, 1H), 3.05 (s, 2H), 2.75 (q, J = 7.2, 6.6 Hz, 2H), 2.30 (s, 3H), 2.21 - 1.96 (m, 8H), 1.12 - 1.03 (m, 3H).

Compound 039b: LCMS: [M-Bu+H]⁺=292.2. Compound 039c: LCMS: [M+H]⁺=560.3. Compound 039d: LCMS: [M+H]⁺=460.3.

### Example 23: Preparation of compound C040

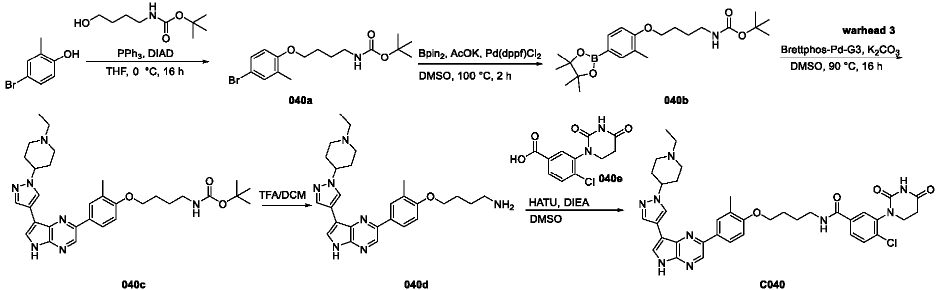

Following the method as described in Example 20, except that compound 034b was replaced with 4-(N-tert-butoxycarbonylamino)-1-butanol, THF was replaced with acetonitrile and the reaction occurred in an ice bath in Step 1, compound C040 was obtained. **LCMS:** Rt=1.998 min; [M+H]⁺=724.4.

Compound 039b: LCMS: [M-Bu+H]⁺=292.2. Compound 040c: LCMS: [M+H]⁺=560.3. Compound 040d: LCMS: [M+H]⁺=460.4.

### Example 24: Preparation of compound C041

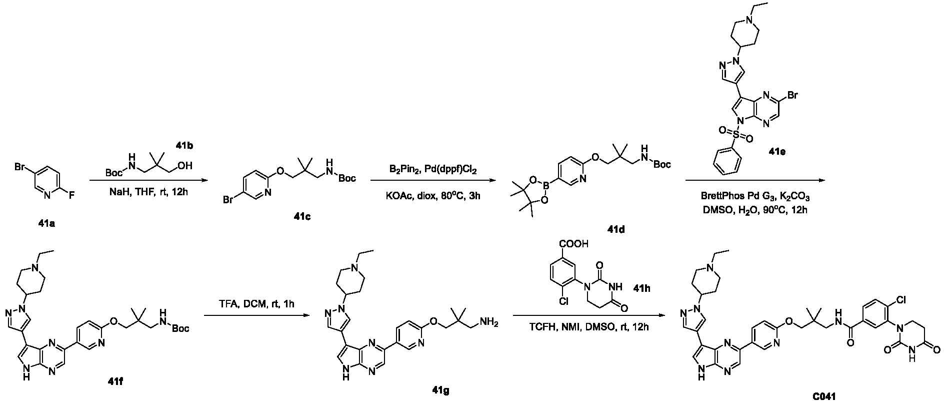

Following the method as described in Example 19, except that compound 33b was replaced with compound 41b in step 1, compound C041 was obtained. LCMS: Rt = 1.911 min, [M+H]⁺= 725.4.

### Example 25: Preparation of compound C042

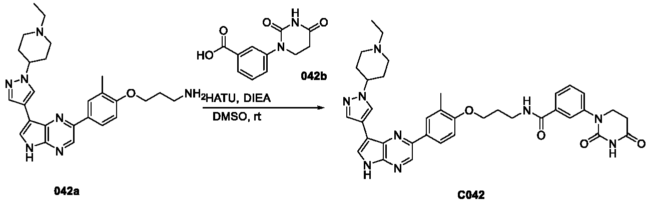

### Step 1:

Compounds 042a (21 mg, 0.046 mmol), 042b (14 mg, 0.059 mmol), DIEA (30 mg, 0.23 mmol) and HATU (23 mg, 0.059 mmol) were dissolved in dimethyl sulfoxide (2 mL). The mixture was stirred at room temperature for 16 h. LCMS showed the complete conversion of the reactants, and the product was detected. The reaction solution was cooled to room temperature, quenched by the addition of water (50 mL), and extracted with dichloromethane (20 mL*2). The organic layer was dried with anhydrous sodium sulfate, filtered, and concentrated in vacuum to give a crude product. The crude product was purified by Pre-TLC to give the compound C042.

**LCMS:** [M+H]⁺=676.4.

**¹H NMR (500 MHz, DMSO-d₆) *δ*** 12.02 (s, 1H), 10.43 (s, 1H), 8.82 (s, 1H), 8.68 (d, J = 5.6 Hz, 1H), 8.38 (s, 1H), 8.15 (s, 1H), 8.12 (d, J = 2.4 Hz, 1H), 8.04 (d, J = 7.5 Hz, 2H), 7.82 (d, J = 1.8 Hz, 1H), 7.76 - 7.72 (m, 1H), 7.53 - 7.44 (m, 2H), 7.09 (d, J = 8.7 Hz, 1H), 4.14 (t, J = 6.0 Hz, 2H), 3.83 (t, J = 6.7 Hz, 2H), 3.61 - 3.47 (m, 4H), 3.26 (s, 1H), 3.15 - 3.07 (m, 2H), 2.72 (t, J = 6.6 Hz, 2H), 2.31 (s, 3H), 2.07 (dd, J = 12.5, 6.1 Hz, 2H), 1.28 (dd, J = 16.9, 6.6 Hz, 6H), 1.25 - 1.21 (m, 3H).

### Example 26: Preparation of compound C044

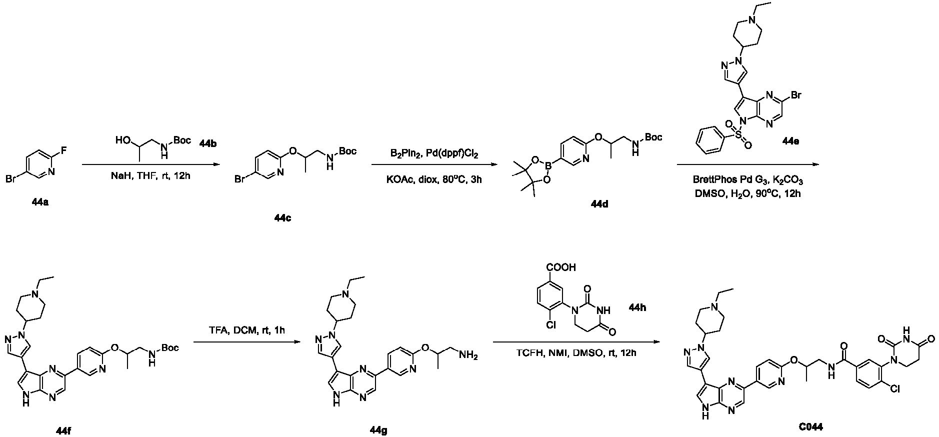

Following the method as described in Example 19, except that compound 33b in Step 1 was replaced with compound 44b, compound C044 was obtained. LCMS: Rt = 1.767 min, [M+H]⁺= 697.4.

### Example 27: Preparation of compound C091

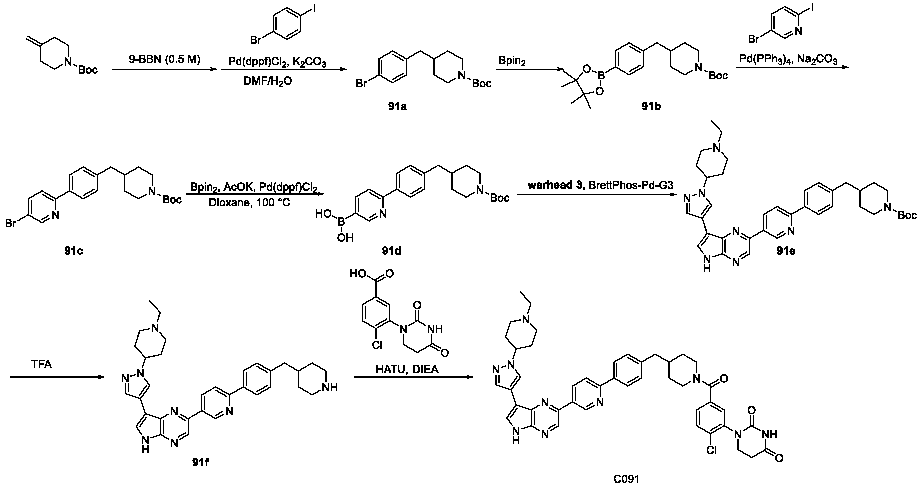

### Step 1:

The compound N-Boc-4-methylenepiperidine (2 g, 10.14 mmol) was dissolved in 9-BBN (20 mL) (0.5 M in THF) at room temperature. The mixture was stirred at 60°C for 3 h under a nitrogen atmosphere. The THF was then distilled under reduced pressure. N,N-dimethylformamide (DMF; S0, 20 mL) and water (S1, 2 mL) were added as solvents. Then 1-bromo-4-iodobenzene (2.6 g, 9.19 mmol), potassium carbonate (2.8 g, 20.28 mmol), and Pd(dppf)Cl₂ (0.74 g, 1.01 mmol) were added and the mixture was stirred at 60°C for 2 h under nitrogen protection. TLC showed the complete conversion of the reactants, and a new point was generated. The reaction solution was poured into water (100 mL) and then extracted with ethyl acetate (30 mL*2). The organic layer was dried over anhydrous sodium sulfate, filtered, and concentrated in vacuum to give a crude product. The crude product was purified by silica gel chromatography to give the compound 91a.

### Step 2:

Compound 91a (1.32 g, 3.73 mmol) was dissolved in dioxane (20 mL) at room temperature. Bis(pinacolato)diboron (1.42 g, 5.59 mmol), potassium acetate (1.10 g, 11.19 mmol), and Pd(dppf)Cl₂ (0.27 g, 0.37 mmol) were added and the mixture was stirred at 100°C for 2 h under a nitrogen atmosphere. TLC showed the complete conversion of the reactants, and a new point was detected. The reaction solution was poured into water (50 mL) and then extracted with ethyl acetate (30 mL *2). The organic layer was dried over anhydrous sodium sulfate, filtered, and concentrated in vacuum to give a crude product. The crude product was purified by silica gel chromatography to give the compound 91b.

### Step 3:

Compound 91b (1.5 g, 3.73 mmol) was dissolved in toluene (10 mL) and water (5 mL) at room temperature. 5-Bromo-2-iodopyridine (1.32 g, 4.65 mmol), sodium carbonate (1.35 g, 12.69 mmol) and tetrakis(triphenylphosphine)palladium (0.24 g, 0.21 mmol) were added. The mixture was stirred at 90°C for 16 h under a nitrogen atmosphere. TLC showed the complete conversion of the reactants, and a new point was detected. The reaction solution was poured into water (50 mL) and then extracted with ethyl acetate (30 mL*2). The organic layer was dried over anhydrous sodium sulfate, filtered, and concentrated in vacuum to give a crude product. The crude product was purified by silica gel chromatography to give the compound 91c.

### Step 4:

Compound 91c (100 mg, 0.23 mmol) was dissolved in dioxane (5 mL) at room temperature. Potassium acetate (68 mg, 0.69 mmol), bis(pinacolato)diboron (88 mg, 0.35 mmol), and Pd(dppf)Cl₂ (0.017 g, 0.023 mmol) were added. The mixture was stirred at 100°C for 2 h under a nitrogen atmosphere. LCMS showed the complete conversion of the reactants, and the product was detected. The reaction solution was filtered. The filtrate was concentrated in vacuum to give a crude product. The crude product was purified by silica gel chromatography to give the compound 91d. **LCMS:** [M+H]⁺=397.2.

### Step 5:

Compound warhead3 (95 mg, 0.18 mmol) was dissolved in DMSO (2 mL) and water (0.2 mL) at room temperature. Compound 91d (90 mg, 0.23 mmol), potassium carbonate (75 mg, 0.54 mmol) and BrettPhos-G3-Pd (16 mg, 0.018 mmol) were added. The mixture was stirred at 90°C for 16 h under a nitrogen atmosphere. LCMS showed the complete conversion of the reactants, and the product was detected. The reaction solution was poured into water (50 mL) and then extracted with DCM (30 mL*2). The organic layer was dried over anhydrous sodium sulfate, filtered, and concentrated in vacuum to give a crude product. The crude product was purified by silica gel chromatography to give the compound 91e. LCMS: [M+H]⁺=647.5.

### Step 6:

Compound 91e (118 mg, 0.18 mmol) was dissolved in dichloromethane (10 mL) at room temperature. Trifluoroacetic acid (1 mL) was added and the mixture was stirred for 2 h at room temperature. LCMS showed the complete conversion of the reactants, and the product was detected. The reaction was dried by rotary evaporation to give the crude compound 91f. LCMS: [M+H]⁺=547.3.

### Step 7:

Compound 91f (33 mg, 0.060 mmol) was dissolved in DMSO (2 mL) at room temperature. 4-Chloro-3-(2,4-dioxotrihydropyrimidin-1(2H)-yl)benzoic acid (21 mg, 0.078 mmol), HATU (30 mg, 0.079 mmol), and DIPEA (R3, 39 mg, 0.30 mmol) were added. The mixture was stirred at room temperature under a nitrogen atmosphere for 16 h. LCMS showed the complete conversion of the reactants, and the product was detected. The reaction solution was poured into water (50 mL) and then extracted with DCM (30 mL*2). The organic layer was dried over anhydrous sodium sulfate, filtered, and concentrated in vacuum to give a crude product. The crude product was purified by Pre-TLC to give the compound C091 (9.69 mg, yield: 18.72%).

**LCMS:** [M+H]⁺=797.4; **¹H NMR (500 MHz, DMSO-*d6*)** δ 12.15 (s, 1H), 10.51 (s, 1H), 9.52 (d, J = 1.7 Hz, 1H), 9.02 (s, 1H), 8.68 (dd, J = 8.4, 2.2 Hz, 1H), 8.42 (s, 1H), 8.18 (d, J = 2.6 Hz, 1H), 8.15 - 8.07 (m, 4H), 7.64 (d, J = 8.2 Hz, 1H), 7.56 (d, J = 1.9 Hz, 1H), 7.39 (dd, J = 8.2, 1.9 Hz, 1H), 7.35 (d, J = 8.2 Hz, 2H), 4.50 - 4.41 (m, 1H), 4.29 - 4.23 (m, 1H), 3.79 - 3.73 (m, 1H), 3.66 - 3.52 (m, 2H), 3.01 (d, J = 10.6 Hz, 3H), 2.75 (q, J = 7.5, 6.8 Hz, 3H), 2.62 (dt, J = 13.7, 6.3 Hz, 3H), 2.39 (q, J = 7.3 Hz, 3H), 2.11 - 1.99 (m, 6H), 1.88 (s, 1H), 1.72 (s, 1H), 1.64 - 1.57 (m, 1H), 1.04 (t, J = 7.2 Hz, 3H).

### Example 28: Preparation of compound C096

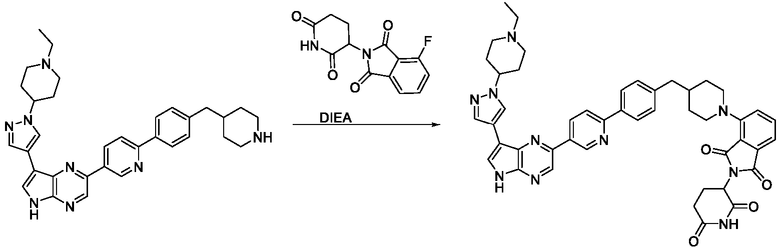

Compound 91f (33 mg, 0.060 mmol) was dissolved in DMSO (2 mL) at room temperature. 2-(2,6-dioxo-piperidin-3-yl)-4-fluoro-isoindole-1,3-dione (22 mg, 0.078 mmol) and DIPEA (39 mg, 0.30 mmol) were added. The mixture was stirred at 80°C for 4 h under a nitrogen atmosphere. LCMS showed the complete conversion of the reactants, and the product was detected. The reaction solution was poured into water (50 mL) and then extracted with DCM (30 mL*2). The organic layer was dried over anhydrous sodium sulfate, filtered, and concentrated in vacuum to give a crude product. The crude product was purified by Pre-TLC (MeOH/DCM=13%) to give the compound C096 (14.37 mg, yield: 28.06 %). **LCMS:** [M+H]⁺=803.5; **¹H NMR (500 MHz, DMSO-d6)** δ 12.16 (s, 1H), 11.09 (s, 1H), 9.53 (s, 1H), 9.03 (s, 1H), 8.68 (d, J = 8.1 Hz, 1H), 8.43 (s, 1H), 8.14 (dd, J = 19.3, 11.7 Hz, 5H), 7.67 (t, J = 7.8 Hz, 1H), 7.38 (d, J = 7.9 Hz, 2H), 7.35 - 7.25 (m, 2H), 5.09 (dd, J = 12.7, 5.4 Hz, 1H), 4.31 - 4.23 (m, 1H), 3.70 (d, J = 9.5 Hz, 2H), 3.02 (d, J = 9.9 Hz, 2H), 2.91 - 2.80 (m, 3H), 2.67 (d, J = 6.0 Hz, 2H), 2.59 (d, J = 18.4 Hz, 1H), 2.45 - 2.39 (m, 2H), 2.13 - 1.95 (m, 7H), 1.80 - 1.70 (m, 3H), 1.56 - 1.39 (m, 3H), 1.05 (t, J = 7.0 Hz, 3H).

### Example 29: Preparation of compound C084

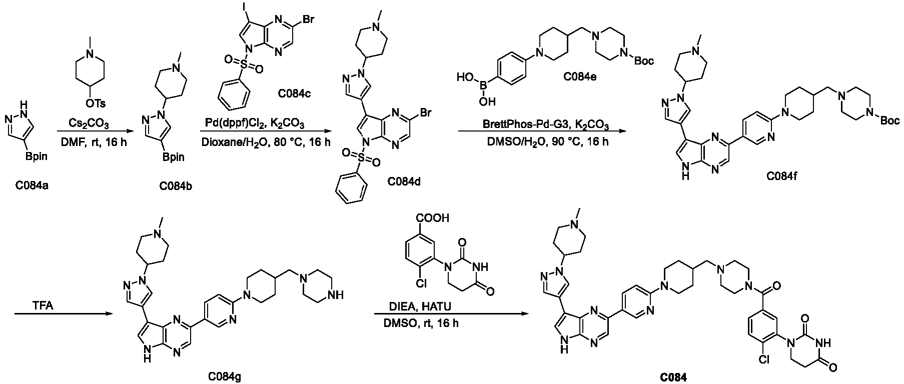

### Step 1:

Compound C084a (2 g, 10.31 mmol) was dissolved in DMF (10 mL) at room temperature. Cesium carbonate (4.03 g, 12.37 mmol) and 1-methylpiperidin-4-yl 4-methylbenzenesulfonate (1.61 g, 11.34 mmol) were added. The mixture was stirred at room temperature for 16 h. LCMS showed the complete conversion of the reactants, and the product was detected. The reaction solution was filtered. The filtrate was concentrated in vacuum to give the crude compound C084b (2.14 g, yield 99.79%).

### Step 2:

Compound C084c (530 mg, 1.21 mmol) was dissolved in dioxane (20 mL) at room temperature. Bis(pinacolato)diboron (368.72 mg, 1.45 mmol), potassium acetate (356 mg, 3.63 mmol), and Pd(dppf)Cl₂·DCM (99 mg, 0.12 mmol) were added. The mixture was stirred at 90°C for 6 h under a nitrogen atmosphere. LCMS showed the complete conversion of the reactants, and the product was detected. The reaction solution was cooled to room temperature, and filtered through Celite. The filter cake was washed with dioxane (20 mL). The filtrate was taken to give the crude compound C084d (480 mg, 1.188 mmol), which was used for the next step directly. LCMS: [M+H]⁺=404.3.

### Step 3:

Compound C084d (52 mg, 0.12 mmol) was dissolved in DMSO (2 mL) and water (0.2 mL) at room temperature. C084e (62 mg, 0.12 mmol), potassium carbonate (50 mg, 0.36 mmol), and BrettPhos-G3-Pd (10 mg, 0.011 mmol) were added. The mixture was stirred at 90°C for 16 h under a nitrogen atmosphere. LCMS showed the complete conversion of the reactants, and the product was detected. The reaction solution was poured into water (30 mL) and then extracted with dichloromethane (20 mL*2). The organic layer was dried over anhydrous sodium sulfate, filtered, and concentrated in vacuum to give a crude product. The crude product was purified by silica gel chromatography (methanol:dichloromethane = 0-14%) to give the compound C084f (37 mg, 0.055 mmol).

### Step 4:

Compound C084f (30 mg, 0.054 mmol) was dissolved in dichloromethane (5 mL) at room temperature. TFA (1 mL) was added and the mixture was stirred at room temperature for 2 h. LCMS showed the complete conversion of the reactants, and the product was detected. The reaction was poured into water (30 mL) and then extracted with dichloromethane (20 mL*2). The organic layer was dried over anhydrous sodium sulfate, filtered, and concentrated in vacuum to give the crude compound C084g (24 mg, 0.053 mmol).

### Step 5

Compound C084g (24 mg, 0.053 mmol) was dissolved in DMSO (2 mL) at room temperature. 4-Chloro-3-(2,4-dioxotrihydropyrimidin-1(2H)-yl)benzoic acid (19 mg, 0.071 mmol), HATU (27 mg, 0.071 mmol), and DIPEA (35 mg, 0.27 mmol) were added. The mixture was stirred at room temperature for 16 h. LCMS showed the complete conversion of the reactants, and the product was detected. The reaction solution was poured into water (50 mL) and then extracted with DCM (30 mL*2). The organic layer was dried over anhydrous sodium sulfate, filtered, and concentrated in vacuum to give a crude product. Purification by Pre-HPLC afforded compound C084 (7.06 mg, yield: 18.07%). LCMS: [M+H]⁺=792.4.

### Example 30: Preparation of compound C085

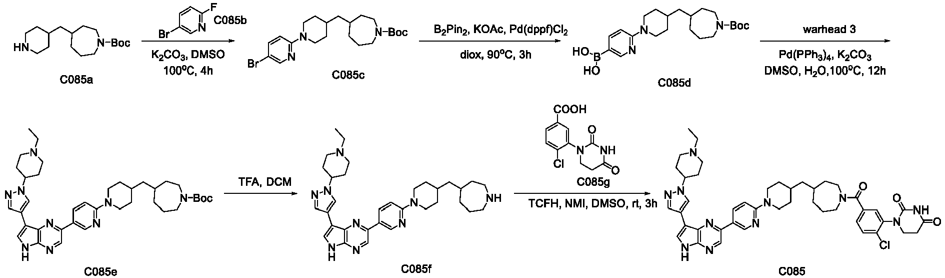

### Step 1:

Compounds C085a (1.8 g, 6.07 mmol), C085b (1.6 g, 9.11 mmol), K₂CO₃ (2.1 g, 15.18mmol) were dissolved in DMSO (40 mL), replaced with nitrogen three times, raised to 100°C, and stirred at this temperature for 4 h. TLC showed the complete conversion of the raw materials, and the product was detected. The reaction solution was added with 50 mL of water and extracted with EA (50 mL*3). The organic phase was dried over anhydrous sodium sulfate and filtered. The filtrate was concentrated in vacuum to give a residue. The residue was purified by column chromatography (PE: EA = 10: 1) to give the compound C085c (1.09 g, 39.7% yield).

### Step 2:

Compound C085c (1.09 g, 2.41 mmol), KOAc (0.71 g, 7.23 mmol), B₂pin₂ (1.22 g, 4.82 mmol), and Pd(dppf)Cl₂ (176 mg, 0.24 mmol) were dissolved in dioxane (20 mL), replaced with nitrogen three times, raised to 90°C, and stirred for 2 h. LCMS showed the complete conversion of the reactants, and the product was detected. Filtration was performed at the end of the reaction and the filtrate was concentrated in vacuum to give a residue. The residue was purified by column chromatography (DCM:MeOH=20:1) to give C085d (180 mg, 15.0 % yield).

### Step 3:

Compound C085d (119 mg, 0.29 mmol), warhead 3 (100 mg, 0.19 mmol), Pd(PPh₃)₄ (22 mg, 0.02 mmol), and K₂CO₃ (79 mg, 0.57 mmol) were dissolved in DMSO and H₂O (3 mL/0.5 mL), replaced with nitrogen three times, raised to 90°C, and stirred for 12 h. LCMS showed the complete conversion of the reactants, and the product was detected. Filtration was performed at the end of the reaction. The reaction solution was added with 50 mL of water, and extracted with DCM (30 mL*3). The organic phase was dried over anhydrous sodium sulfate and filtered. The filtrate was concentrated in vacuum. The residue was purified by column chromatography (DCM:MeOH=4:1) to give C085e (72 mg, 56.3 % yield).

### Step 4:

Compound C085e (73 mg, 0.11 mmol) was dissolved in DCM and TFA (3 mL/0.5 mL) and stirred at room temperature for 1 h. LCMS showed the complete conversion of the reactants, and the product was detected. The reaction solution was distilled under reduced pressure to give the crude compound C085f.

### Step 5:

Compounds C085f, C085g (44.3 mg, 0.17 mmol), and NMI (31.6 mg, 0.39 mmol) were dissolved in DMSO (2 mL). TCFH (337 mg, 0.13 mmol) was added with stirring and stirred at room temperature for 2 h. LCMS showed the complete conversion of the reactants, and the product was detected. The reaction solution was added with 50 mL of water and extracted with DCM (30 mL*3). The organic phase was dried over anhydrous sodium sulfate and filtered. The filtrate was concentrated in vacuum. The residue was purified by a preparation plate (DCM:NH₃/MeOH=12:1) to give the final product C085 (31.54 mg, 35.06% yield). LCMS: Rt = 1.817 min, [M+H]⁺= 818.6.

### Example 31: Preparation of compound C092

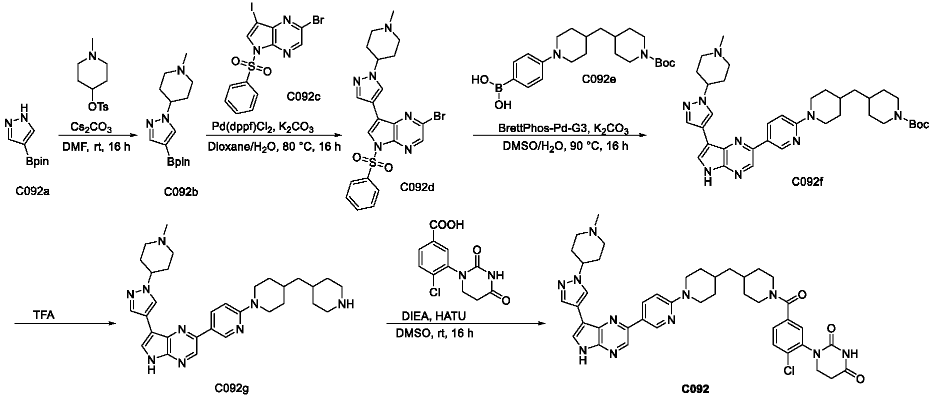

Following the method as described in Example 29, except that compound C084e in the third step was replaced with compound C092e, compound C092 was obtained (7.06 mg, yield: 18.07%). LCMS: [M+H]⁺=791.4.

### Example 32: Preparation of compound C098

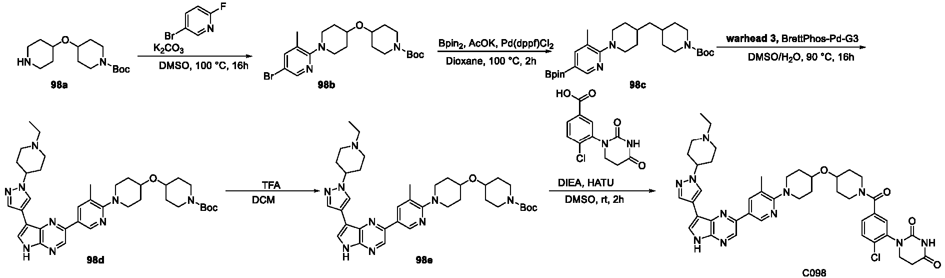

Following the method as described in Example 30, except that compound C085a in the first step was replaced with compound 98a, compound C098 was obtained. LCMS: Rt=2.120min; [M+H]⁺=820.5.

### Example 33: Preparation of compound C108

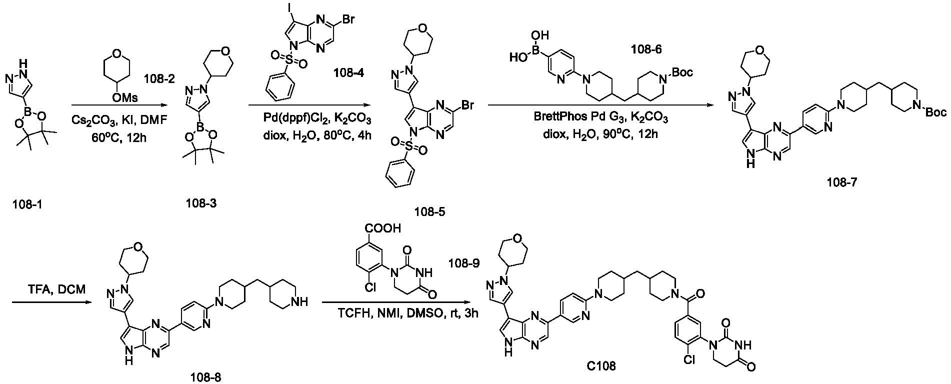

### Step 1:

Compound 108-1 (2 g, 10.31 mmol), compound 108-2 (3.72 g, 20.62 mmol), Cs₂CO₃ (10.08 g, 30.93 mmol), and KI (0.17 g, 1.03 mmol) were dissolved in DMF (40 mL), replaced with nitrogen three times, raised to 60°C, and stirred at this temperature for 12 h. TLC showed the complete conversion of the raw materials, and the product was detected. Filtration was performed at the end of the reaction and the filtrate was concentrated in vacuum to give a residue. The residue was purified by column chromatography (PE:EA=1:3) to give the compound 108-3 (3.03 g).

### Step 2:

Compound 108-3 (600 mg, 2.16 mmol), compound 108-4 (500 mg, 1.08 mmol), Pd(dppf)Cl₂ (176 mg, 0.22 mmol), and K₂CO₃ (299 mg, 2.16 mmol) were dissolved in dioxane and H₂O (10 mL/3 mL), was replaced with nitrogen three times, raised to 80°C and stirred for 3 h. LCMS showed the complete conversion of the reactants, and the product was detected. Filtration was performed at the end of the reaction and the filtrate was concentrated in vacuum to give a residue. The residue was purified by column chromatography (PE: EA = 1: 1) to give the compound 108-5 (280 mg, 53.2 % yield).

### Step 3:

Compound 108-5 (100 mg, 0.2 mmol), compound 108-6 (120 mg, 0.3 mmol), BrettPhos Pd G3 (18 mg, 0.02 mmol), and K₂CO₃ (83 mg, 0.6 mmol) were dissolved in dioxane and H₂O (3 mL/0.5 mL), replaced with nitrogen three times, raised to 90°C, and stirred for 12 h. LCMS showed the complete conversion of the reactants, and the product was detected. Filtration was performed at the end of the reaction and the filtrate was concentrated in vacuum to give a residue. The residue was purified by column chromatography (pure EA) to give the compound 108-7 (87 mg, 67.8 % yield).

### Step 4:

Compound 108-7 (87 mg, 0.14 mmol) was dissolved in DCM and TFA (3 mL/0.5 mL) and stirred at room temperature for 1 h. LCMS showed the complete conversion of the reactants, and the product was detected. The reaction solution was distilled under reduced pressure to give the crude compound 108-8.

### Step 5:

Compound 108-8, compound 108-9 (56.4 mg, 0.21 mmol), and NMI (40 mg, 0.49 mmol) were dissolved in DMSO (2 mL). TCFH (47 mg, 0.17 mmol) was added with stirring and stirred at room temperature for 2 h. LCMS showed the complete conversion of the reactants, and the product was detected. Purification by preparative chromatography afforded the final product C108 (37.73 mg, 34.85% yield). LCMS: Rt = 2.000 min, [M+H]⁺=777.5, purity = 99.51%.

### Example 34: Preparation of compound C109

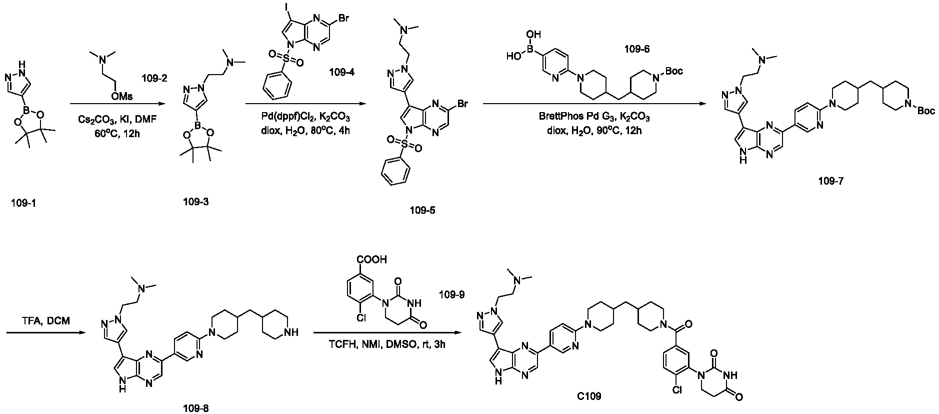

Following the method as described in Example 33, except that compound 108-1 in the first step was replaced with compound 109-2, compound C109 (19.01 mg, 34.85% yield, purity = 99.14%) was prepared. LCMS: Rt=2.145min; [M+H]⁺=765.3.

### Example 35: Preparation of compound C110

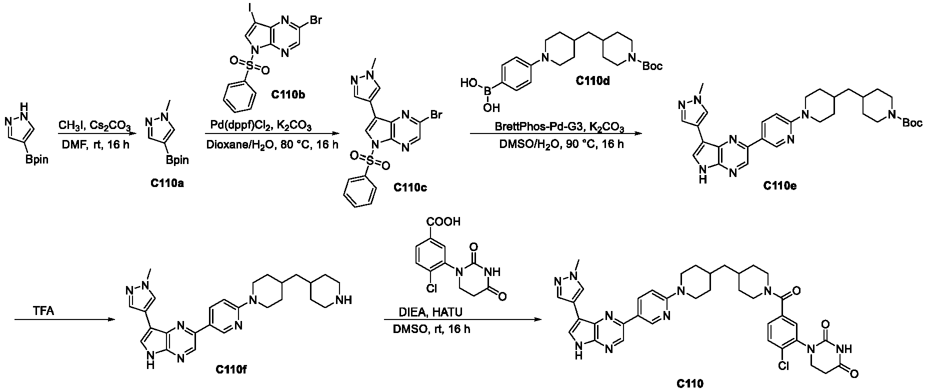

Following the method as described in Example 29, except that 1-methylpiperidin-4-yl 4-methylbenzenesulfonate in the first step was replaced with iodine methane, compound C110 (7.06 mg, yield: 18.07%) was prepared. LCMS: [M+H]⁺=707.4.

C110a: LCMS: [M+H]⁺=208.1. C110c: LCMS: [M+H]⁺=404.3. C110f: LCMS: [M+H]⁺=457.1.

### Example 36: Preparation of compound C122

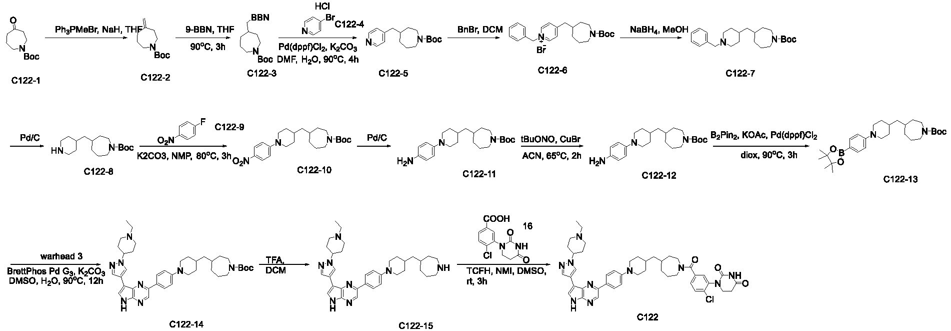

### Step 1:

Ph3PMeBr (12.56 g, 35.16 mmol) was dissolved in THF (40 mL). NaH (1.88 g, 46.88 mmol) was added with stirring in an ice bath, replaced with nitrogen three times, raised to 90°C, and stirred at this temperature for 3 h. Compound C122-1 (5 g, 23.44 mmol) was dissolved in THF (40 mL), cooled to room temperature, added dropwise with stirring, and stirred at room temperature overnight. TLC showed the complete conversion of the raw materials, and the product was detected. The reaction solution was added with 150 mL of water in an ice bath, and extracted with EA (100 mL*3). The organic phase was dried over anhydrous sodium sulfate and filtered. The filtrate was concentrated in vacuum to give a residue. The residue was purified by column chromatography (PE: EA= 3: 1) to give the compound C122-2 (3.83 g, 77.3% yield).

### Step 2:

Compound C122-2 (3.81 g, 18.04 mmol) was dissolved in THF (50 mL). 9-BBN (30 mL, 0.5M in THF) was added with stirring, replaced with nitrogen three times, raised to 90°C, and stirred at this temperature for 2 h. The reaction solution was cooled to room temperature, and the solvent was removed by rotary evaporation. Compound 4 (2.7 g, 13.88 mmol), Pd(dppf)Cl₂ (1.01 g, 1.39 mmol), K₂CO₃ (5.76 g, 41.64 mmol), DMF, and H₂O (50 mL/15 mL) were added, replaced with nitrogen three times, raised to 90°C and stirred at this temperature for 2 h. LCMS showed the complete conversion of the reactants, and the product was detected. Filtration was performed at the end of the reaction. The filtrate was added with 150 mL of water, and extracted with EA (100 mL*3). The organic phase was dried over anhydrous sodium sulfate and filtered. The filtrate was concentrated in vacuum to give a residue. The residue was purified by column chromatography (PE:EA=1:1) to give the compound C122-5 (4.58 g).

### Step 3:

C122-5 (4.58 g, 15.77 mmol) was dissolved in DCM (80 mL). Benzyl bromide (3.78 g, 22.08 mmol) was added dropwise with stirring and stirred at room temperature for 24 h. LCMS showed the complete conversion of the reactants, and the product was detected. The reaction solution was concentrated in vacuum to give the crude compound C122-6.

### Step 4:

Compound C122-6 was dissolved in MeOH (20 mL). NaBH4 (2.98 g, 78.9 mmol) was added with stirring in an ice bath, slowly raised to room temperature, and stirred for 24 h. LCMS showed the complete conversion of the reactants, and the product was detected. After completion of the reaction, the reaction solution was added with 150 mL of water in an ice bath, and extracted with EA(100 mL*3). The organic phase was dried over anhydrous sodium sulfate and filtered. The filtrate was concentrated in vacuum to give the crude compound C122-7 (4.58 g).

### Step 5:

Compound C122-7 (6 g, 15.52 mmol) was dissolved in MeOH (50 mL). Pd/C (500 mg) was added with stirring, replaced with hydrogen three times, and stirred at room temperature for 12 h. LCMS showed the complete conversion of the reactants, and the product was detected. Filtration was performed at the end of the reaction. The filtrate was distilled under reduced pressure to give the crude compound C122-8.

The synthetic procedures in subsequent steps were the same as those described in Example 40 for the synthesis of compound C135. The final product C122 (10.57 mg, 33.27 % yield, purity = 99.96 %) was obtained. LCMS: Rt = 1.951 min, [M+H]⁺=817.5.

### Example 37: Preparation of compound C131

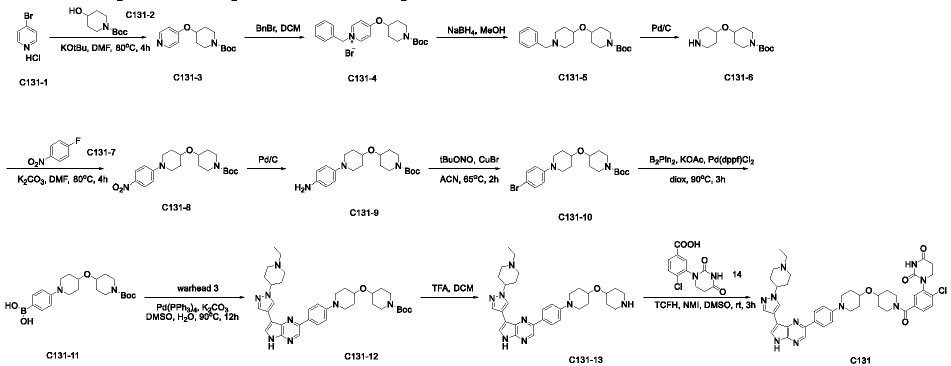

### Step 1:

Compound C131-1 (5 g, 25.71 mmol) and compound C131-2 (7.76 g, 38.56 mmol) were dissolved in DMF (70 mL). KOtBu (5.77 g, 51.42 mmol) was added with stirring, replaced with nitrogen three times, raised to 80°C, and stirred at this temperature for 4 h. TLC showed the complete conversion of the raw materials, and the product was detected. Filtration was performed at the end of the reaction. The filtrate was added with 150 mL of water, and extracted with EA (100 mL*3). The organic phase was dried over anhydrous sodium sulfate and filtered. The filtrate was concentrated in vacuum to give a residue. The residue was purified by column chromatography (pure EA) to give the compound C131-3 (6.92 g, 96.7% yield).

The synthetic procedures in subsequent steps were the same as those described for compound C122. The final product C131 (3.32 mg, 30.47 % yield, purity = 93.26 %) was obtained. LCMS: Rt = 1.831 min, [M+H]⁺= 805.5.

### Example 38: Preparation of compound C132

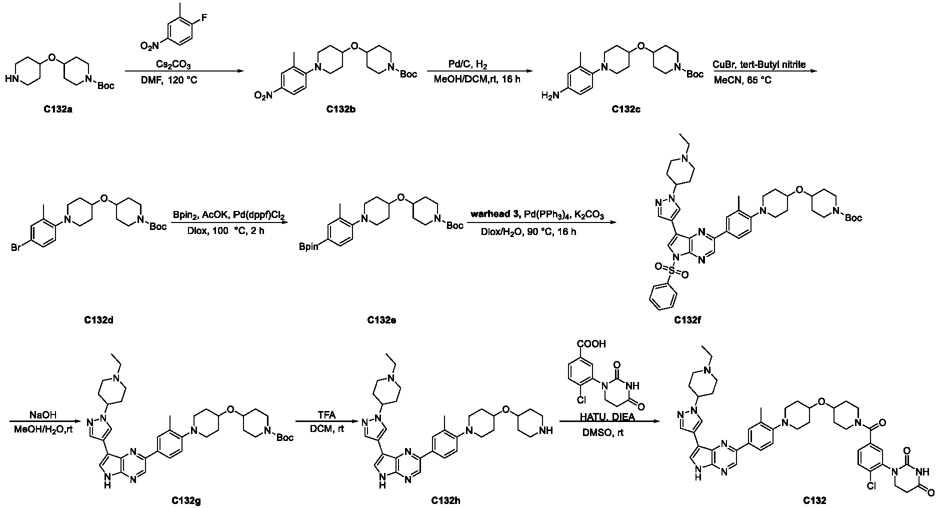

### Step 1:

Compound C132a (1.4 g, 4.92 mmol) was dissolved in DMF (15 mL) at room temperature. 2-Fluoro-5-nitrotoluene (0.84 g, 5.41 mmol) and cesium carbonate (4.8 g, 14.73 mmol) were added. The mixture was stirred at 120°C for 5 h. TLC showed the complete conversion of the reactants, and a new point was detected. The reaction solution was poured into water (100 mL) and then extracted with ethyl acetate (30 mL *2). The organic layer was dried over anhydrous sodium sulfate, filtered, and concentrated in vacuum to give a crude product. The crude product was purified by silica gel chromatography (petroleum ether: ethyl acetate = 0-4%) to give the compound C132b (689 mg, yield: 33.36%).

### Step 2:

Compound C132b (393 mg, 0.94 mmol) was dissolved in methanol (5 mL) and dichloromethane (1 mL) at room temperature. Pd/C (10.00 mg, 0.094 mmol) (5%) was added and the reaction solution was stirred at room temperature for 16 h. LCMS showed the complete conversion of the reactants, and the product was detected. The reaction solution was filtered and concentrated in vacuum to give the crude compound C132c (364 mg, yield: 99.75%). LCMS: [M+H]⁺=390.3.

### Step 3:

Compound tert-butyl nitrite (288 mg, 2.79 mmol, purity 100%) and cuprous bromide (334 mg, 2.33 mmol) were dissolved in acetonitrile (5 mL) at room temperature and raised to 65°C. Then, compound C132c (364 mg, 0.93 mmol) dissolved in acetonitrile (10 mL) was added. The reaction solution was stirred at 65°C for 2 h under a nitrogen atmosphere. TLC showed the complete conversion of the reactants, and a new point was detected. The reaction solution was filtered and concentrated in vacuum to give a crude product. The crude product was purified by silica gel chromatography (petroleum ether: ethyl acetate = 0-9%) to give the compound C132b (73 mg, yield: 17.23 %).

### Step 4:

Compound C132d (73 mg, 0.16 mmol) was dissolved in dioxane (5 mL) at room temperature. Bis(pinacolato)diboron (82 mg, 0.32 mmol, purity 100%), Pd(dppf)Cl₂·DCM (13 mg, 0.016 mmol), and potassium acetate (48 mg, 0.49 mmol) were added. The reaction solution was stirred at 100°C for 2 h under a nitrogen atmosphere. LCMS showed the complete conversion of the reactants, and the product was detected. The reaction solution was filtered and concentrated in vacuum to give a crude product. The crude product was purified by silica gel chromatography (petroleum ether: ethyl acetate = 0-6%) to give the compound C132e (56 mg, yield: 69.50 %). LCMS: [M+H]⁺=501.4.

### Step 5:

Compound warhead 3 (56 mg, 0.11 mmol) was dissolved in dioxane (5 mL) and water (1 mL) at room temperature. Compound C132e (55 mg, 0.11 mmol), potassium carbonate (46 mg, 0.33 mmol), and Pd(PPh₃)₄ (13 mg, 0.011 mmol) were added. The reaction solution was stirred at 80°C under a nitrogen atmosphere for 16 h. LCMS showed the complete conversion of the reactants, and the product was detected. The reaction solution was filtered and concentrated in vacuum to give a crude product. The crude product was purified by silica gel chromatography (methanol: dichloromethane = 0-6%) to give the compound C132f (86 mg, yield: 97.84 %). LCMS: [M+H]⁺=809.6.

### Step 6:

Compound C132f (86 mg, 0.11 mmol) was dissolved in methanol (5 mL) and water (1 mL) at room temperature. NaOH (44 mg, 1.1 mmol) was added and the reaction solution was stirred at room temperature for 2 h. LCMS showed the complete conversion of the reactants, and the product was detected. The reaction was poured into water (30 mL) and then extracted with dichloromethane (30 mL*2). The organic phase was dried over anhydrous sodium sulfate, filtered, and concentrated in vacuum to give the crude compound C132g (71 mg, yield: 99.86 %). LCMS: [M+H]⁺=669.5.

### Step 7:

Compound C132g (71 mg, 0.11 mmol) was dissolved in DCM (5 mL) at room temperature. Trifluoroacetic acid (1 mL) was added and the reaction solution was stirred at room temperature for 2 h. LCMS showed the complete conversion of the reactants, and the product was detected. The reaction was concentrated in vacuum to give the crude compound C132h (60 mg, yield: 99.38%). LCMS: [M+H]⁺=569.5.

### Step 8:

Compound C132h (60 mg, 0.11 mmol) was dissolved in DMSO (3 mL) at room temperature. 4-Chloro-3-(2,4-dioxotrihydropyrimidin-1(2H)-yl)benzoic acid (39 mg, 0.15 mmol), HATU (55 mg, 0.14 mmol), and DIPEA (71 mg, 0.55 mmol) were added. The reaction solution was stirred at room temperature for 16 h. LCMS showed the complete conversion of the reactants, and the product was detected. The reaction solution was poured into water (50 mL) and then extracted with dichloromethane (20 mL*2). The organic layer was dried over anhydrous sodium sulfate, filtered, and concentrated in vacuum to give a crude product. Purification by Pre-HPLC gave compound C132 (12.7 mg, yield: 14.60%). LCMS: [M+H]⁺=819.5.

1H NMR (500 MHz, DMSO-d6) δ 11.97 (s, 1H), 10.52 (s, 1H), 8.80 (s, 1H), 8.35 (s, 1H), 8.12 (s, 1H), 8.08 (d, J = 2.3 Hz, 1H), 7.99 (d, J = 8.1 Hz, 2H), 7.65 (d, J = 8.2 Hz, 1H), 7.59 (s, 1H), 7.42 (d, J = 9.3 Hz, 1H), 7.15 (d, J = 8.0 Hz, 1H), 4.22 (m, 1H), 4.03 - 3.96 (m, 1H), 3.81 - 3.75 (m, 2H), 3.64 (m, 2H), 3.12 - 3.05 (m, 3H), 3.03 - 2.97 (m, 2H), 2.79 - 2.69 (m, 5H), 2.44 - 2.38 (m, 2H), 2.37 (s, 3H), 2.17 - 1.93 (m, 10H), 1.70 - 1.57 (m, 3H), 1.55 - 1.41 (m, 3H), 1.04 (t, J = 7.1 Hz, 3H).

### Example 39: Preparation of compound C134

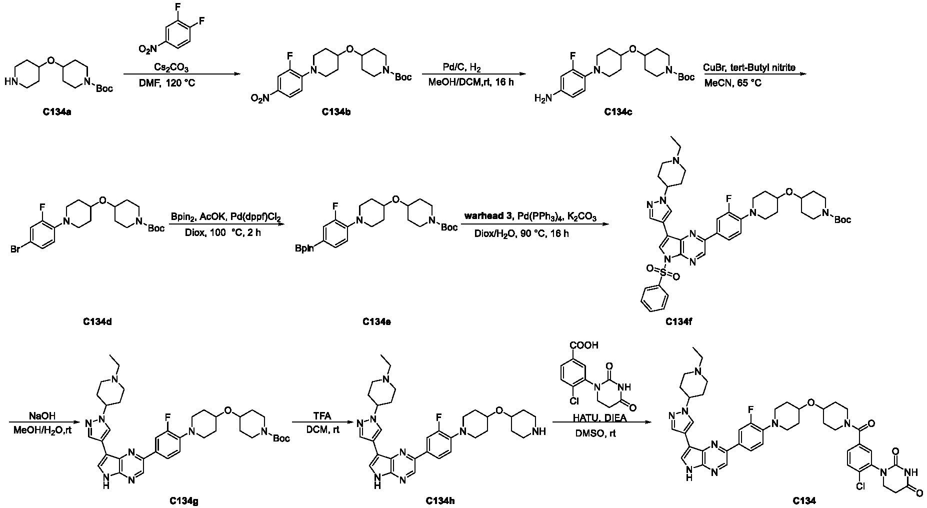

Following the method as described in Example 38, except that 2-fluoro-5-nitrotoluene in the first step was replaced with 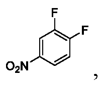 compound C134 was obtained (12.7 mg, yield: 14.60%). LCMS: [M+H]⁺=824.4.

C134c: LCMS: [M+H]⁺=390.3. C134e: LCMS: [M+H]⁺=501.4. C134f: LCMS: [M+H]⁺=809.6. C134g: LCMS: [M+H]⁺=669.5. C134h: LCMS: [M+H]⁺=569.5.

### Example 40: Preparation of compound C135

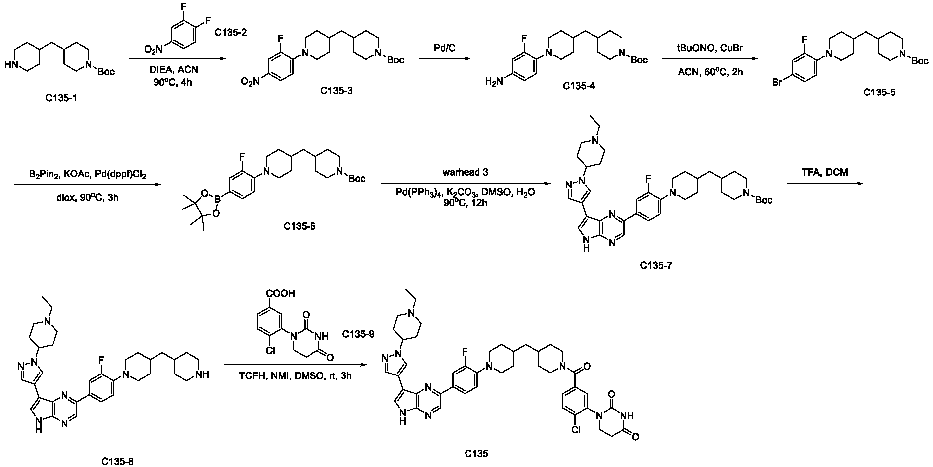

### Step 1:

Compound C135-1 (2 g, 7.08 mmol), compound C135-2 (1.69 g, 10.62 mmol), DIEA(1.83 g, 14.16 mmol) were dissolved in ACN (30 mL), raised to 90°C, and stirred at this temperature for 3 h. TLC showed the complete conversion of the raw materials, and the product was detected. The reaction solution was added with 50 mL of water and extracted with EA (50 mL*3). The organic phase was dried over anhydrous sodium sulfate and filtered. The filtrate was concentrated in vacuum to give a residue. The residue was purified by column chromatography (PE: EA = 4: 1) to give the compound C135-3 (2.34 g, 78.4 % yield).

### Step 2:

Compound C135-3 (2.34g, 5.55 mmol) was dissolved in MeOH (30 mL). Pd/C (230 mg) was added with stirring, replaced with hydrogen three times, and stirred at room temperature for 1 h. LCMS showed the complete conversion of the reactants, and the product was detected. Filtration was performed at the end of the reaction. The filtrate was distilled under reduced pressure to give the crude compound C135-4.

### Step 3:

CuBr (0.95 g, 6.65 mmol) and tBuONO (0.86 g, 8.31 mmol) were dissolved in ACN (15 mL), replaced with nitrogen three times, raised to 65°C, and stirred for 10 min. The crude product C135-4 was dissolved in ACN (15 mL), dropped with stirring, and maintained the temperature and stirred for 2 h. LCMS showed the complete conversion of the reactants, and the product was detected. Filtration was performed at the end of the reaction and the filtrate was concentrated in vacuum to give a residue. The residue was purified by column chromatography (PE: EA = 9: 1) to give the compound C135-5 (335 mg, 13.3 % yield).

### Step 4:

Compound C135-5 (0.34 g, 0.74 mmol), KOAc (0.22 g, 2.22 mmol), B₂pin₂ (0.38 g, 1.48 mmol), pd(dppf)Cl₂ (55 mg, 0.07 mmol) were dissolved in dioxane (5 mL), replaced with nitrogen three times, raised to 90°C, and stirred for 2 h. LCMS showed the complete conversion of the reactants, and the product was detected. Filtration was performed at the end of the reaction and the filtrate was concentrated in vacuum to give a residue. The residue was purified by column chromatography (PE: EA = 4: 1) to give the compound C135-6 (270 mg, 73.1 % yield).

### Step 5:

Compound C135-6 (248 mg, 0.49 mmol), warhead 3 (170 mg, 0.33 mmol), Pd(PPh₃)₄ (38 mg, 0.07 mmol), and K₂CO₃ (127 mg, 0.99 mmol) were dissolved in DMSO and H₂O (3mL/0.5 mL), replaced with nitrogen three times, raised to 90°C, and stirred for 12 h. LCMS showed the complete conversion of the reactants, and the product was detected. After the completion of the reaction, the reaction solution was filtered. After the completion of the reaction, the reaction solution was added with 50 mL of water and extracted with DCM (30 mL*3). The organic phase was dried over anhydrous sodium sulfate and filtered. The filtrate was concentrated in vacuum to give a residue. The residue was purified by a preparation plate (DCM:MeOH=4:1) to give the compound C135-7 (96 mg, 43.3% yield).

### Step 6:

Compound C135-7 (116 mg, 0.17 mmol) was dissolved in DCM and TFA (3 mL/0.5 mL) and stirred at room temperature for 1 h. LCMS showed the complete conversion of the reactants, and the product was detected. The reaction solution was distilled under reduced pressure to give a crude compound C135-8.

### Step 7:

Compound C135-8, compound C135-9 (68.5 mg, 0.26 mmol), NMI (49 mg, 0.6 mmol) were dissolved in DMSO (2 mL). TCFH (57 mg, 0.2 mmol) was added with stirring and stirred at room temperature for 2 h. LCMS showed the complete conversion of the reactants, and the product was detected. The product was purified by preparative chromatography to give the final product (45.22 mg, 32.00% yield, purity = 99.81%). LCMS: Rt = 2.153 min, [M+H]⁺=821.6.

### Example 41: Preparation of compound C136

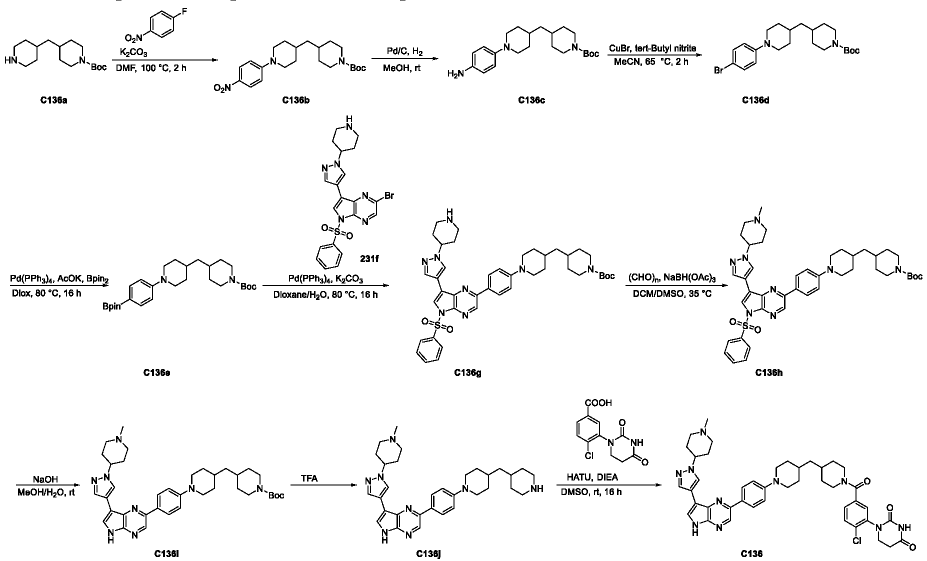

### Step 1:

Compound C136a (211 mg, 0.75 mmol) was dissolved in DMF (5 mL) at room temperature, p-fluoronitrobenzene (127 mg, 0.90 mmol) and potassium carbonate (311 mg, 2.25 mmol) were added. The reaction solution was stirred at 100°C for 3 h. TLC showed the complete conversion of the reactants, and a new point was detected. The reaction solution was poured into water (50 mL) and then extracted with ethyl acetate (30 mL*2). The organic layer was dried over anhydrous sodium sulfate, filtered, and concentrated in vacuum to give a crude product. The crude product was purified by silica gel chromatography (petroleum ether: ethyl acetate = 0-14%) to give the compound C136b (193 mg, yield: 64.02 %).

### Step 2:

Compound C136b (193 mg, 0.48 mmol) was dissolved in methanol (5 mL) at room temperature, Pd/C (20 mg, 0.19 mmol) (5%) was added and the reaction solution was stirred at room temperature under hydrogen atmosphere for 16 h. LCMS showed the complete conversion of the reactants, and the product was detected. The reaction solution was filtered. The filtrate was concentrated in vacuum to give the compound C136c (178 mg, yield: 99.63%). LCMS: [M+H]⁺=374.3.

### Step 3:

Tert-butyl nitrite (149 mg, 1.44 mmol) and CuBr (172 mg, 1.20 mmol) were dissolved in acetonitrile (5 mL) at room temperature and raised to 65°C. Then, compound C136c (178 mg, 0.48 mmol) dissolved in acetonitrile (5 mL) was added and the reaction solution was stirred at 65°C for 2 h under a nitrogen atmosphere. TLC showed the complete conversion of the reactants, and a new point was detected. The reaction solution was filtered and concentrated in vacuum to give a crude product. The crude product was purified by silica gel chromatography (petroleum ether: ethyl acetate = 0-7%) to give the compound C136d (43 mg, yield: 20.63 %).

### Step 4:

Compound C136d (43 mg, 0.098 mmol) was dissolved in 1,4-dioxane (5 mL) at room temperature. Bis(pinacolato)diboron (50 mg, 0.20 mmol), potassium acetate (29 mg, 0.30 mmol), and Pd(dppf)Cl₂·DCM (8 mg, 0.0098 mmol) were added. The reaction solution was stirred at 80°C for 16 h under a nitrogen atmosphere. TLC showed the complete conversion of the reactants, and a new point was detected. The reaction solution was filtered and concentrated in vacuum to give a crude product. The crude product was purified by silica gel chromatography (petroleum ether: ethyl acetate = 0-10%) to give the compound C136e (50 mg, yield: 100%).

### Step 5:

Compound C136e (50 mg, 0.10 mmol) was dissolved in 1,4-dioxane (5 mL) and water (1 mL) at room temperature. C136f (50 mg, 0.10 mmol), tetrakis(triphenylphosphine)palladium (23.11 mg, 0.020 mmol), and potassium carbonate (55.28 mg, 0.40 mmol) were added. The mixture was stirred at 80°C under a nitrogen atmosphere. LCMS showed the complete conversion of the reactants, and the product was detected. The reaction solution was filtered and concentrated in vacuum to give a crude product. The crude product was purified by silica gel chromatography (methanol: dichloromethane = 0-7%) to give the compound C136g (70 mg, yield: 44.33 %). LCMS: [M+H]⁺=765.4.

### Step 6:

Compound C136g (70 mg, 0.092 mmol) was dissolved in dichloromethane (2 mL) and DMSO (2 mL) at room temperature. Paraformaldehyde (8.3 mg, 0.28 mmol) and acetic acid (10 mg, 0.17 mmol) were added. The mixture was stirred at 35°C for 2 h under a nitrogen atmosphere. Sodium triacetylborohydride (98 mg, 0.46 mmol) was then added to the mixture and the reaction was then stirred at 35°C to react for 16 h. LCMS showed the complete conversion of the reactants, and the product was detected. The reaction solution was poured into water (50 mL) and then extracted with dichloromethane (30 mL*2). The organic layer was dried over anhydrous sodium sulfate, filtered, and concentrated in vacuum to give a crude product. The crude product was purified by silica gel chromatography (methanol: dichloromethane = 0-6%) to give the compound C136h (22 mg, yield: 30.82 %). LCMS: [M+H]⁺=779.5.

### Step 7:

Compound C136h (22 mg, 0.028 mmol) was dissolved in methanol (5 mL) and water (1 mL) at room temperature. NaOH (11 mg, 0.28 mmol) was added and the reaction solution was stirred at room temperature for 2 h. LCMS showed the complete conversion of the reactants, and the product was detected. The reaction was poured into water (20 mL) and then extracted with dichloromethane (20 mL*2). The organic layer was dried over anhydrous sodium sulfate, filtered, and concentrated in vacuum to give the crude compound C136i (18 mg, yield: 99.74 %). LCMS: [M+H]⁺=639.5.

### Step 8:

Compound C136i (18 mg, 0.028 mmol) was dissolved in DCM (5 mL) at room temperature. TFA (5 mL) was added and the reaction solution was stirred at room temperature for 1 h. LCMS showed the complete conversion of the reactants, and the product was detected. The reaction was concentrated in vacuum to give the crude compound C136j (15 mg, yield 98.79%), which was used in the next step without purification. LCMS: [M+H]⁺=539.4.

### Step 9:

Compound C136j (15 mg, 0.028 mmol) was dissolved in DMSO (2 mL) at room temperature. 4-Chloro-3-(2,4-dioxotrihydropyrimidin-1(2H)-yl)benzoic acid (10 mg, 0.037 mmol), HATU (14 mg, 0.037 mmol), and DIEA (20 mg, 0.15 mmol) were added. The reaction solution was stirred at room temperature for 16 h. LCMS showed the complete conversion of the reactants, and the product was detected. The reaction solution was poured into water (50 mL) and then extracted with dichloromethane (20 mL*2). The organic layer was dried over anhydrous sodium sulfate, filtered, and concentrated in vacuum to give a crude product. The crude product was purified by Pre-HPLC to give the compound C136 (4.82 mg, yield: 21.95 %). LCMS: Rt=1.999min; [M+H]⁺=789.5.

### Example 42: Preparation of compound C137

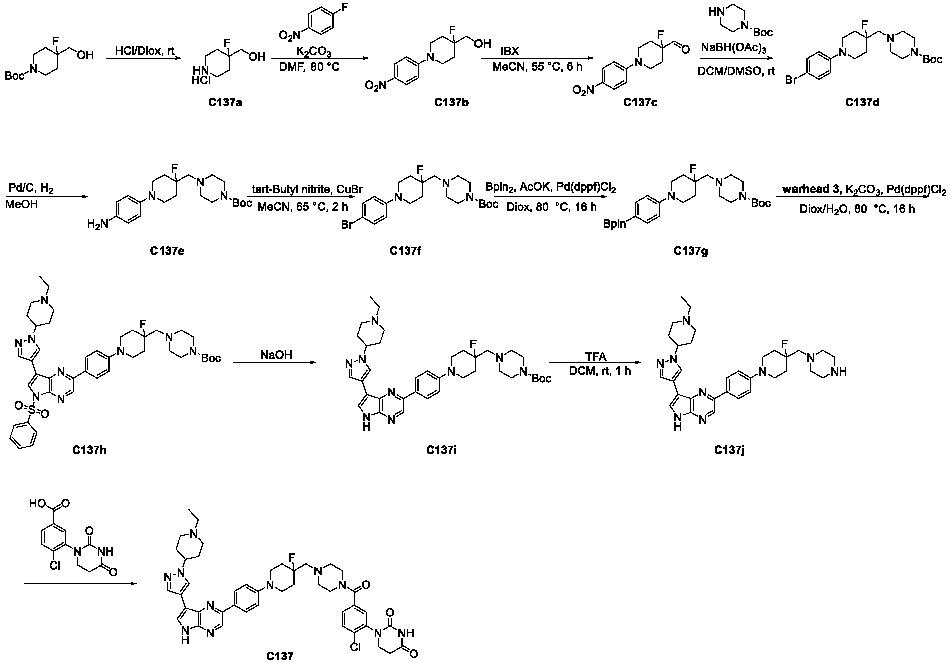

### Step 1:

Compound 1-*tert*-butoxycarbonyl-4-fluoro-4-(hydroxymethyl)piperidine (2.1 g, 9.00 mmol) was dissolved in a hydrochloric acid solution in dioxane (10 mL) (4 M) at room temperature and stirred for 1 h. The reaction solution was concentrated in vacuum to give the crude compound C137a (P0, 1.53 g, yield: 100%), which was used directly for the next step.

### Step 2:

Compound C137a was dissolved in DMF (10 mL) at room temperature, p-fluoronitrobenzene (1.53 g, 10.84 mmol), and potassium carbonate (3.74 g, 27.06 mmol) were added. The mixture was stirred at 80°C for 3 h under a nitrogen atmosphere. TLC showed the complete conversion of the reactants, and a new point was detected. The reaction solution was poured into water (50 mL) and then extracted with ethyl acetate (30 mL *2). The organic layer was dried over anhydrous sodium sulfate, filtered, and concentrated in vacuum to give a crude product. The crude product was purified by silica gel chromatography (petroleum ether: ethyl acetate = 0-47%) to give the compound C137b (1.3 g, yield: 56.74 %).

### Step 3:

Compound C137b (1.2 g, 4.72 mmol) was dissolved in acetonitrile (20 mL) at room temperature. IBX (3.95 g, 14.11 mmol) was added and the reaction solution was stirred at 55°C for 6 h. TLC showed the complete conversion of the reactants, and a new point was detected. The reaction solution was filtered to obtain a filtrate. The filtrate was subjected to rotary evaporation to give a crude compound C137c (1.19 g, yield: 99.96%).

### Step 4:

Compound C137c (1.19 g, 4.72 mmol) was dissolved in dichloromethane (20 mL) and dimethyl sulfoxide (10 mL) at room temperature. Tert-butyl piperazine-1-carboxylate (1.3 g, 6.98 mmol) was added and the reaction solution was stirred at room temperature for 1 h. Then, NaBH(OAc)₃ (3 g, 14.15 mmol) was added and stirred for 16 h. TLC showed the complete conversion of the reactants, and a new point was detected. The reaction solution was poured into water (30 mL) and then extracted with dichloromethane (30 mL*2). The organic layer was dried over anhydrous sodium sulfate, filtered, and concentrated in vacuum to give a crude product. The crude product was purified by silica gel chromatography (petroleum ether: ethyl acetate = 0-23%) to give the compound C137d (870 mg, yield: 43.65 %).

### Step 5:

Compound C137d (870 mg, 2.06 mmol) was dissolved in methanol (10 mL) and dichloromethane (2 mL) at room temperature. Wet palladium on carbon (219.23 mg, 2.06 mmol) (5%) was added and the reaction solution was stirred at room temperature in a hydrogen atmosphere for 16 h. LCMS showed the complete conversion of the reactants, and the product was detected. The reaction solution was filtered and concentrated in vacuum to give a crude product. The crude product was purified by silica gel chromatography (petroleum ether: ethyl acetate = 0-56%) to give the compound C137e (360 mg, yield: 44.54 %). LCMS: [M+H]⁺=393.3.

### Step 6:

Cuprous bromide (330 mg, 2.30 mmol) and tert-butyl nitrite (285 mg, 2.76 mmol) were dissolved in acetonitrile (5 mL) at room temperature and raised to 65°C. Then, compound C137e (360 mg, 0.92 mmol) dissolved in acetonitrile (5 mL) was added and the reaction solution was stirred at 65°C for 2 h under a nitrogen atmosphere. LCMS showed the complete conversion of the reactants, and the product was detected. The reaction solution was filtered. The filtrate was concentrated in vacuum to give a crude product. The crude product was purified by silica gel chromatography (petroleum ether: ethyl acetate = 0-25%) to give the compound C137f (113 mg, yield: 27.00 %). LCMS: [M+H]⁺=456.2.

### Step 7:

Compound C137f (113 mg, 0.25 mmol) was dissolved in dioxane (5 mL) at room temperature. Pd(dppf)Cl₂·DCM (20 mg, 0.024 mmol), bis(pinacolato)diboron (127 mg, 0.50 mmol), and potassium acetate (74 mg, 0.75 mmol) were added. The mixture was stirred at 80°C for 16 h under a nitrogen atmosphere. LCMS showed the complete conversion of the reactants, and the product was detected. The reaction solution was filtered and concentrated in vacuum to give a crude product. The crude product was purified by silica gel chromatography (petroleum ether: ethyl acetate = 0-9%) to give the compound C137g (99 mg, yield: 79.42 %). LCMS: [M+H]⁺=504.4.

### Step 8:

Compound warhead 3 (80 mg, 0.16 mmol) was dissolved in dioxane (5 mL) and water (1 mL) at room temperature. C137g (99 mg, 0.20 mmol), potassium carbonate (67 mg, 0.48 mmol), and tetrakis(triphenylphosphine)palladium (18 mg, 0.016 mmol) were added. The mixture was stirred at 80°C for 3 h under a nitrogen atmosphere. LCMS showed the complete conversion of the reactants, and the product was detected. The reaction solution was poured into water (50 mL) and then extracted with dichloromethane (30 mL*2). The organic layer was dried over anhydrous sodium sulfate, filtered, and concentrated in vacuum to give a crude product. The crude product was purified by silica gel chromatography (methanol: dichloromethane = 0-6%) to give the compound C137h (63 mg, yield: 49.99 %). LCMS: [M+H]⁺=812.5.

### Step 9:

Compound C137h (63 mg, 0.078 mmol) was dissolved in methanol (5 mL) and water (1 mL) at room temperature. Sodium hydroxide (32 mg, 0.80 mmol) was added. The reaction solution was stirred at room temperature for 2 h. LCMS showed the complete conversion of the reactants, and the product was detected. The reaction was poured into water (50 mL) and then extracted with dichloromethane (30 mL*2). The organic layer was dried over anhydrous sodium sulfate, filtered, and concentrated in vacuum to give the crude compound C137i (52 mg, yield: 99.76 %). LCMS: [M+H]⁺=672.5.

### Step 10:

Compound C137i (52 mg, 0.077 mmol) was dissolved in DCM (5 mL) at room temperature. TFA (1 mL) was added and the reaction solution was stirred at room temperature for 2 h. LCMS showed the complete conversion of the reactants, and the product was detected. The reaction solution was concentrated in vacuum to give the crude compound C137j (44 mg, yield: 99.43 %), which was used directly for the next step. LCMS: [M+H]⁺=572.5.

### Step 11:

Compound C137j (44 mg, 0.077 mmol) was dissolved in DMSO (2 mL) at room temperature. 4-Chloro-3-(2,4-dioxotrihydropyrimidin-1(2H)-yl)benzoic acid (23 mg, 0.086 mmol), HATU (38 mg, 0.10 mmol), and DIPEA (50 mg, 0.39 mmol) were added. The reaction solution was stirred at room temperature for 16 h. LCMS showed the complete conversion of the reactants, and the product was detected. The reaction solution was poured into water (50 mL) and then extracted with dichloromethane (30 mL*2). The organic layer was dried over anhydrous sodium sulfate, filtered, and concentrated in vacuum to give a crude product. The crude product was purified by Pre-HPLC to give the compound C137 (10.26 mg, yield: 16.18%). LCMS: [M+H]⁺=822.5.

### Example 43: Preparation of compound C140

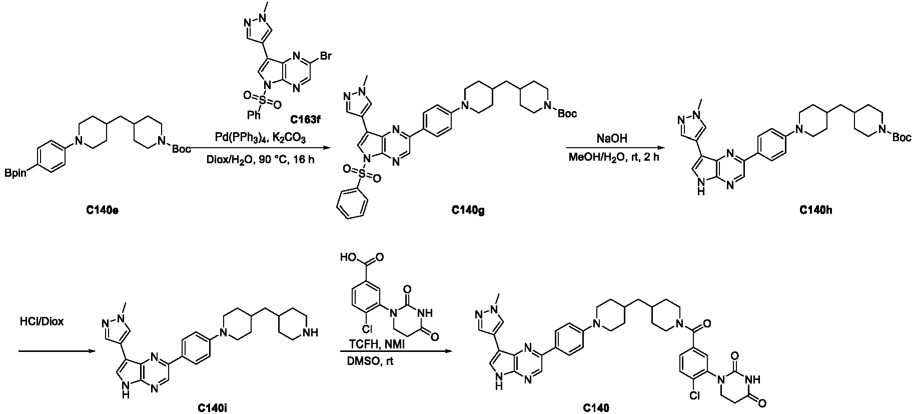

### Step 1:

Compound C140e (50 mg, 0.10 mmol, which can be prepared as described in the first to fourth steps of Example 41) was dissolved in dioxane (5 mL) and water (1 mL) at room temperature. Compound C140f (42 mg, 0.10 mmol), Pd(dppf)Cl₂·DCM (8 mg, 0.010 mmol), and potassium carbonate (42 mg, 0.30 mmol) were added. The reaction solution was stirred at 90°C for 16 h under a nitrogen atmosphere. The reaction solution was filtered and concentrated in vacuum to give a crude product compound C140g (71 mg, yield: 99.95%).

### Step 2:

Compound C140g (71 mg, 0.099 mmol) was dissolved in methanol (5 mL) and water (1 mL) at room temperature. NaOH (40 mg, 0.99 mmol) was added and the reaction solution was stirred at room temperature for 2 h. LCMS showed the complete conversion of the reactants, and the product was detected. The reaction solution was filtered and concentrated in vacuum to give a crude product. The crude product was purified by silica gel chromatography (methanol: dichloromethane = 0-4%) to give the compound C140h (44 mg, yield: 77.11 %). LCMS: [M+H]⁺=574.4.

### Step 3:

Compound C140h (44 mg, 0.077 mmol) was dissolved in a hydrochloric acid solution in dioxane (5 mL, 4 M) at room temperature and stirred for 1 h. LCMS showed the complete conversion of the reactants, and the product was detected. The reaction was concentrated in vacuum to give the crude compound C140i (39 mg, yield: 99.70 %). LCMS: [M+H]⁺=474.3.

### Step 4:

Compound C140i (39 mg, 0.076 mmol) was dissolved in DMSO (3 mL) at room temperature. 4-Chloro-3-(2,4-dioxotrihydropyrimidin-1(2H)-yl)benzoic acid (20 mg, 0.076 mmol), TCFH (28 mg, 0.099 mmol), and NMI (31 mg, 0.38 mmol) were added. The reaction solution was stirred at room temperature for 16 h. LCMS showed the complete conversion of the reactants, and the product was detected. The reaction solution was poured into water (30 mL) and then extracted with dichloromethane (20 mL*2). The organic layer was dried over anhydrous sodium sulfate, filtered, and concentrated in vacuum to give a crude product. The crude product was purified by Pre-HPLC to give the compound C140 (5.71 mg, yield: 10.23 %). LCMS: [M+H]⁺=707.3.

¹H NMR (500 MHz, DMSO-d6) δ 9.01 (d, J = 207.9 Hz, 1H), 8.44 - 8.06 (m, 2H), 7.87 - 7.17 (m, 3H), 6.96 (d, J = 7.5 Hz, 1H), 4.22 - 3.79 (m, 4H), 3.52 (s, 1H), 3.00 (d, J = 11.9 Hz, 2H), 2.75 (s, 1H), 1.87 - 1.69 (m, 3H), 1.64 - 1.23 (m,5H)

### Example 44: Preparation of compound C153

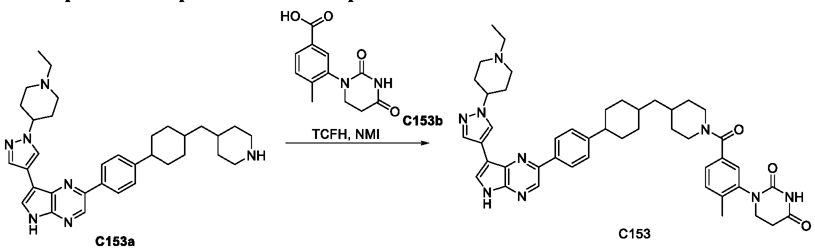

Compound C153a (30 mg, 0.054 mmol) was dissolved in DMSO (3 mL) at room temperature, C153b (14 mg, 0.056 mmol), TCFH (16 mg, 0.057 mmol), and NMI (23 mg, 0.28 mmol) were added. The reaction solution was stirred at room temperature for 16 h. LCMS showed the complete conversion of the reactants, and the product was detected. The reaction solution was poured into water (50 mL) and then extracted with dichloromethane (30 mL*2). The organic layer was dried over anhydrous sodium sulfate, filtered, and concentrated in vacuum to give a crude product. The crude product was purified by Pre-HPLC to give the compound C153 (5.13 mg, yield: 12.06 %). LCMS: [M+H]⁺=783.6.

¹H NMR (500 MHz, DMSO-d6) δ 11.92 (s, 1H), 10.38 (s, 1H), 8.77 (s, 1H), 8.34 (s, 1H), 8.12 - 8.00 (m, 4H), 7.34 (d, J = 7.9 Hz, 1H), 7.31 (s, 1H), 7.25 (d, J = 7.5 Hz, 1H), 7.07 (d, J = 8.8 Hz, 2H), 4.54 - 4.42 (m, 1H), 4.27 - 4.19 (m, 1H), 3.86 - 3.77 (m, 3H), 3.55 - 3.51 (m, 2H), 3.00 (d, J = 11.1 Hz, 2H), 2.82 - 2.65 (m, 5H), 2.39 (q, J = 7.2 Hz, 2H), 2.22 (s, 3H), 2.13 - 1.93 (m, 6H), 1.87 - 1.50 (m, 7H), 1.29 - 1.16 (m, 4H), 1.14 - 1.06 (m, 2H), 1.04 (t, J = 7.1 Hz, 3H).

### Example 45: Preparation of compound C154

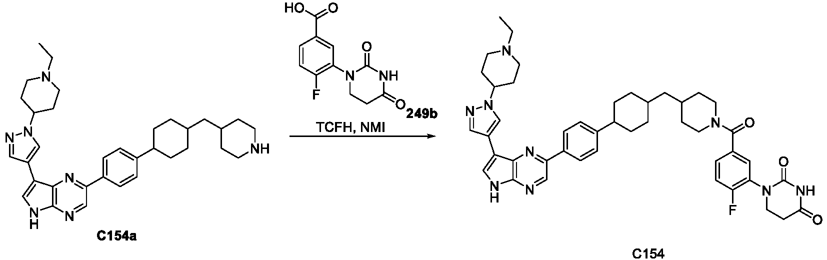

Compound C154a (30 mg, 0.054 mmol) was dissolved in DMSO (3 mL) at room temperature. C154b (14 mg, 0.056 mmol), TCFH (16 mg, 0.057 mmol), and NMI (23 mg, 0.28 mmol) were added. The reaction solution was stirred at room temperature for 16 h. LCMS showed the complete conversion of the reactants, and the product was detected. The reaction solution was poured into water (50 mL) and then extracted with dichloromethane (30 mL*2). The organic layer was dried over anhydrous sodium sulfate, filtered, and concentrated in vacuum to give a crude product. The crude product was purified by Pre-HPLC to give the compound C154 (5.16 mg, yield: 12.06 %). LCMS: [M+H]⁺=787.6.

¹H NMR (500 MHz, DMSO-d6) δ 11.92 (s, 1H), 10.53 (s, 1H), 8.77 (s, 1H), 8.34 (s, 1H), 8.14 - 7.99 (m, 4H), 7.50 (d, J = 6.8 Hz, 1H), 7.38 (d, J = 8.6 Hz, 2H), 7.07 (d, J = 8.5 Hz, 2H), 4.52 - 4.38 (m, 1H), 4.27 - 4.16 (m, 1H), 3.82 (d, J = 12.1 Hz, 2H), 3.76 (t, J = 6.5 Hz, 2H), 3.00 (d, J = 11.0 Hz, 2H), 2.78 - 2.35 (m, 4H), 2.39 (q, J = 7.1 Hz, 2H), 2.10 - 1.95 (m, 6H), 1.85 - 1.43 (m, 7H), 1.29 - 1.16 (m, 4H), 1.15 - 1.07 (m, 2H), 1.04 (t, J = 7.2 Hz, 3H).

### Example 46: Preparation of compound C160

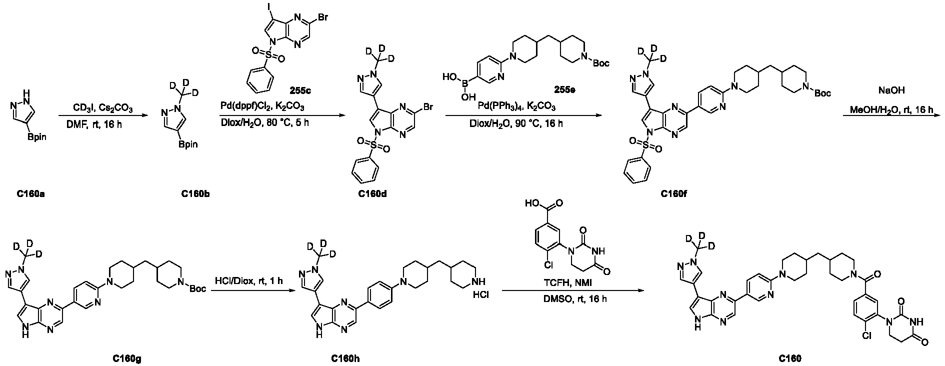

### Step 1:

Compound C160a (6.2 g, 31.95 mmol) was dissolved in DMF (50 mL) at room temperature. Cesium carbonate (12.49 g, 38.34 mmol) and deuterated iodomethane (5.09 g, 35.15 mmol) were added. The mixture was stirred at room temperature for 16 h. LCMS showed the complete conversion of the reactants, and the product was detected. The reaction solution was filtered and the filtrate was concentrated in vacuum to give the crude compound C160b (6.72 g, yield: 99.64%). LCMS: [M+H]⁺=211.1.

### Step 2:

Compound C160b (1 g, 2.15 mmol) was dissolved in dioxane (10 mL) and water (3 mL) at room temperature. Compound C160c (0.54 g, 2.58 mmol), potassium carbonate (0.89 g, 6.45 mmol) and Pd(dppf)Cl₂·DCM (88 mg, 0.11 mmol) were added. The reaction solution was stirred at 80°C for 4 h under a nitrogen atmosphere. LCMS showed the complete conversion of the reactants, and the product was detected. The reaction solution was filtered and concentrated in vacuum to give a crude product. The crude product was purified by silica gel chromatography (petroleum ether: ethyl acetate = 0-30%) to give the compound C160d (322 mg, yield: 35.47 %). LCMS: [M+H]⁺=422.4.

### Step 3:

Compound C160d (60 mg, 0.14 mmol) was dissolved in dioxane (5 mL) and water (1 mL) at room temperature. Compound C160e (59 mg, 0.15 mmol), tetrakis(triphenylphosphine)palladium (8 mg, 0.0071 mmol), and potassium carbonate (60 mg, 0.43 mmol) were added. The reaction solution was stirred at room temperature for 16 h. LCMS showed the complete conversion of the reactants, and the product was detected. The reaction was concentrated in vacuum to give the crude compound C160f (99 mg, yield: 99.32 %). LCMS: [M+H]⁺=699.4.

### Step 4:

Compound C160f (99 mg, 0.14 mmol) was dissolved in methanol (S0, 5 mL) and water (1 mL) at room temperature. Sodium hydroxide (56.00 mg, 1.40 mmol) was added. The reaction solution was stirred at room temperature for 1 h. LCMS showed the complete conversion of the reactants, and the product was detected. The reaction solution was concentrated in vacuum to give a crude product. The crude product was purified by silica gel chromatography (methanol: dichloromethane = 0-3%) to give the compound C160g (70 mg, yield: 88.41 %). LCMS: [M+H]⁺=560.4.

### Step 5:

Compound C160g (70 mg, 0.13 mmol) was dissolved in methanol (5 mL) at room temperature. A hydrochloric acid solution in dioxane (5 mL) was added and the reaction solution was stirred at room temperature for 2 h. LCMS showed the complete conversion of the reactants, and the product was detected. The reaction was concentrated in vacuum to give the crude compound C160h (62 mg, yield: 99.94 %). LCMS: [M+H]⁺=460.5.

### Step 6:

Compound C160h (62 mg, 0.12 mmol) was dissolved in DMSO (3 mL) at room temperature. 4-Chloro-3-(2,4-dioxotrihydropyrimidin-1(2H)-yl)benzoic acid (39 mg, 0.15 mmol), TCFH (44 mg, 0.16 mmol), and NMI (50 mg, 0.61 mmol) were added. The reaction solution was stirred at room temperature for 2 h. LCMS showed the complete conversion of the reactants, and the product was detected. The reaction solution was poured into water (30 mL) and then extracted with dichloromethane (30 mL*2). The organic layer was dried over anhydrous sodium sulfate, filtered, and concentrated in vacuum to give a crude product. The crude product was purified by Pre-HPLC to give the compound C160 (22.68 mg, yield: 25.07 %). LCMS: [M+H]⁺=710.4. ¹H NMR (500 MHz, DMSO-d6) δ 11.98 (s, 1H), 10.54 (s, 1H), 8.97 (s, 1H), 8.80 (s, 1H), 8.34 (d, J = 7.4 Hz, 2H), 8.09 (s, 1H), 8.03 (s, 1H), 7.64 (d, J = 8.2 Hz, 1H), 7.56 (s, 1H), 7.39 (d, J = 8.2 Hz, 1H), 6.97 (d, J = 8.9 Hz, 1H), 4.43 (dd, J = 25.0, 12.6 Hz, 3H), 3.81 - 3.72 (m, 1H), 3.66 - 3.51 (m, 2H), 2.86 (d, J = 12.2 Hz, 2H), 2.81 - 2.67 (m, 3H), 1.70 (dd, J = 49.1, 15.4 Hz, 6H), 1.31 - 0.96 (m, 7H).

### Example 47: Preparation of compound C162

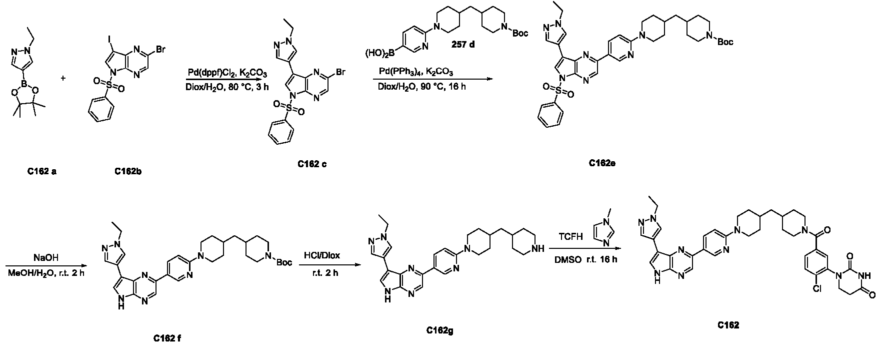

### Step 1:

Compounds C162 a (1.0 g, 4.5 mmol), C162 b (3.7 g, 4.1 mmol), the catalyst (150 mg, 0.2 mmol), potassium carbonate (1.7 g, 12.3 mmol) were added successively to a 250 mL one-necked flask, followed by 30 mL of dioxane and 10 mL of water as solvents. Then, a condenser tube was mounted on the one-necked flask. After replacement with nitrogen using a three-way valve three times, the flask was heated to 80°C for a reaction for 3 h. Then, the reaction was determined to be completed by LCMS. After the reaction solution was filtered, water and dichloromethane were added for extraction three times (10 mL*3). The organic phase was collected, dried over anhydrous sodium sulfate, filtered, concentrated, and purified by silica gel chromatography (EA/PE = 30%). Product C162 c (1.05g, 2.4 mmol) was obtained with a yield of 53.3%.

### Step 2:

Compounds C162 c (95 mg, 0.22 mmol), C162 d (80 mg, 0.20 mmol), the catalyst tetrakis(triphenylphosphine)palladium (23 mg, 0.02 mmol) and potassium carbonate (82 mg, 0.60 mmol) were added successively to a 100 mL one-necked flask, followed by 10 mL of dioxane and 1 mL of water as solvents. Then, a condenser tube was mounted on the one-necked flask. After replacement with nitrogen using a three-way valve three times, the flask was heated to 90°C for a reaction for 16 h. Then, the reaction was determined to be completed by LCMS. The reaction solution was filtered and directly concentrated for the next step.

### Step 3:

The crude product C162 e (crude) from the second step was put into a 100 mL reaction flask. 5 mL of methanol solvent was added, followed by aqueous sodium hydroxide (100 mg) solution. The reaction was continued at normal temperature for about 2 h, and then a small amount of reaction solution was taken. The reaction was completed as determined by LCMS. The reaction solution was filtered and extracted with dichloromethane three times. The organic phase was collected, dried over anhydrous sodium sulfate, filtered, concentrated, and purified by silica gel chromatography (MeOH/CH₂Cl₂ = 4%) to give the product C162 f (80 mg).

### Step 4:

Compound C162f was added to a 100 mL one-necked flask. HCl/dioxane (2 mol/L, 1 mL) was added and partial solid precipitated. 2 mL of methanol was added to aid dissolution. After reacting for 2 h at normal temperature, the reaction was determined to be completed by LCMS. The solvent was directly dried by rotary evaporation and the product was used as the raw material in the next step.

### Step 5:

Compounds C162 g (50 mg, 0.10 mmol), C162 h (35 mg, 0.13 mmol), TCFH (38 mg, 0.14 mmol), and 1-methylimidazole (34 mg, 0.41 mmol) were added successively to a 100 mL one-necked flask. After reaction at normal temperature overnight for 16 h, the reaction was completed as determined by LCMS. The final product C162 (20.78 mg, 0.02 mmol) was obtained by preparative chromatography with a yield of 30%. [M+H]⁺=721.3.

¹H NMR (500 MHz, DMSO-d6) δ 11.96 (s, 1H), 10.51 (s, 1H), 8.96 (d, J = 2.2 Hz, 1H), 8.79 (s, 1H), 8.41 - 8.29 (m, 2H), 8.08 (d, J = 2.6 Hz, 1H), 8.05 (s, 1H), 7.64 (d, J = 8.2 Hz, 1H), 7.55 (d, J = 1.7 Hz, 1H), 7.39 (dd, J = 8.2, 1.7 Hz, 1H), 6.96 (d, J = 9.1 Hz, 1H), 4.51 - 4.37 (m, 3H), 4.23 (q, J = 7.3 Hz, 2H), 3.82 - 3.72 (m, 1H), 3.66 - 3.53 (m, 2H), 3.13 - 3.02 (m, 1H), 2.87 (t, J = 11.9 Hz, 2H), 2.75 (q, J = 7.7, 6.7 Hz, 2H), 1.84 - 1.58 (m, 6H), 1.43 (t, J = 7.3 Hz, 3H), 1.29 - 1.02 (m, 7H).

### Example 48: Preparation of compound C163

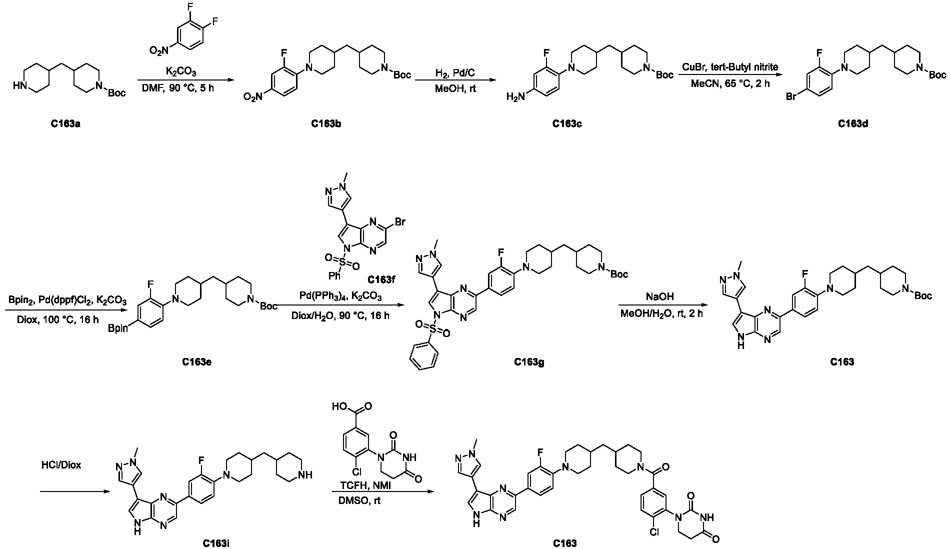

### Step 1:

Compound C163a (1.78 g, 6.29 mmol) was dissolved in DMF (20 mL) at room temperature. 3, 4-Difluoronitrobenzene (1 g, 6.29 mmol) and potassium carbonate (2.61 g, 18.87 mmol) were added. The reaction solution was stirred at 85°C for 3 h under a nitrogen atmosphere. TLC showed the complete conversion of the reactants, and a new point was detected. The reaction solution was filtered and concentrated in vacuum to give a crude product. The crude product was purified by silica gel chromatography (petroleum ether: ethyl acetate = 0-19%) to give the compound C163b (1.5 g, yield: 56.61 %).

### Step 2:

Compound C163b (1.5 g, 3.56 mmol) was dissolved in methanol (30 mL) at room temperature, Pd/C (0.038 g, 0.36 mmol) (5%) was added and the reaction solution was stirred at room temperature under hydrogen atmosphere for 4 h. TLC showed the complete conversion of the reactants, and a new point was detected. The reaction solution was filtered and concentrated in vacuum to give the crude compound C163c (1.39 g, yield: 99.76 %).

### Step 3:

*Tert*-butyl nitrite (1.10 g, 10.65 mmol) and cuprous bromide (R2, 1.53 g, 10.65 mmol) were dissolved in acetonitrile (10 mL) at room temperature, raised to 65°C, then Compound C163c (1.39 g, 3.55 mmol) dissolved in acetonitrile (10 mL) was added. The reaction solution was stirred at 65°C for 2 h under a nitrogen atmosphere. TLC showed the complete conversion of the reactants, and a new point was detected. The reaction solution was filtered and concentrated in vacuum to give a crude product. The crude product was purified by silica gel chromatography (petroleum ether: ethyl acetate = 0-13%) to give the compound C163d (296 mg, yield: 18.31 %).

### Step 4:

Compound C163d (296 mg, 0.65 mmol) was dissolved in dioxane (3 mL) at room temperature. Bis(pinacolato)diboron (0.33 g, 1.3 mmol), Pd(dppf)Cl₂·DCM (53 mg, 0.065 mmol), and potassium acetate (0.19 g, 1.95 mmol) were added. The reaction solution was stirred at 100°C for 16 h under a nitrogen atmosphere. LCMS showed the complete conversion of the reactants, and the product was detected. The reaction solution was filtered and concentrated in vacuum to give a crude product. The crude product was purified by silica gel chromatography (petroleum ether: ethyl acetate = 0-9%) to give the compound C163e (320 mg, yield: 97.98 %). LCMS: [M+H]⁺=503.3.

### Step 5:

Compound C163e (50 mg, 0.10 mmol) was dissolved in dioxane (5 mL) and water (1 mL) at room temperature. Compound C163f (42 mg, 0.10 mmol), Pd(dppf)Cl₂·DCM (8 mg, 0.010 mmol), and potassium carbonate (42 mg, 0.30 mmol) were added. The reaction solution was stirred at 90°C for 16 h under a nitrogen atmosphere. The reaction solution was filtered and concentrated in vacuum to give a crude product compound C163g (71 mg, yield: 99.95%).

### Step 6:

Compound C163g (71 mg, 0.099 mmol) was dissolved in methanol (5 mL) and water (1 mL) at room temperature. NaOH (40 mg, 0.99 mmol) was added and the reaction solution was stirred at room temperature for 2 h. LCMS showed the complete conversion of the reactants, and the product was detected. The reaction solution was filtered and concentrated in vacuum to give a crude product. The crude product was purified by silica gel chromatography (methanol: dichloromethane = 0-4%) to give the compound C163h (44 mg, yield: 77.11 %). LCMS: [M+H]⁺=574.4.

### Step 7:

Compound C163h (44 mg, 0.077 mmol) was dissolved in a hydrochloric acid solution in dioxane (5 mL, 4 M) at room temperature and stirred for 1 h. LCMS showed the complete conversion of the reactants, and the product was detected. The reaction was concentrated in vacuum to give the crude compound C163i (39 mg, yield: 99.70 %). LCMS: [M+H]⁺=474.3.

### Step 8:

Compound C163i (39 mg, 0.076 mmol) was dissolved in DMSO (3 mL) at room temperature. 4-Chloro-3-(2,4-dioxotrihydropyrimidin-1(2H)-yl)benzoic acid (20 mg, 0.076 mmol), TCFH (28 mg, 0.099 mmol), and NMI (31 mg, 0.38 mmol) were added. The reaction solution was stirred at room temperature for 16 h. LCMS showed the complete conversion of the reactants, and the product was detected. The reaction solution was poured into water (30 mL) and then extracted with dichloromethane (20 mL*2). The organic layer was dried over anhydrous sodium sulfate, filtered, and concentrated in vacuum to give a crude product. The crude product was purified by Pre-HPLC to give the compound C163 (5.71 mg, yield: 10.23 %). LCMS: Rt=2.019min; [M+H]⁺=724.4.

### Example 49: Preparation of compound C164

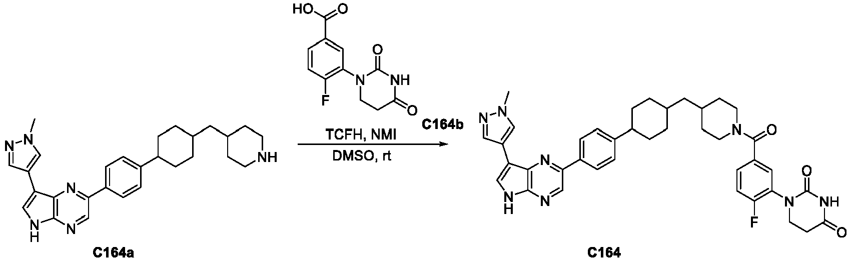

Compound C164a (79 mg, 0.17 mmol) was dissolved in DMSO (3 mL) at room temperature. 3-(2,4-dioxotrihydropyrimidin-1(2H)-yl)-4-fluorobenzoic acid (56.16 mg, 0.22 mmol), HATU (97 mg, 0.26 mmol), and DIPEA (110 mg, 0.85 mmol) were added. The reaction solution was stirred at room temperature for 16 h. LCMS showed the complete conversion of the reactants, and the product was detected. The reaction solution was poured into water (30 mL) and then extracted with dichloromethane (20 mL*2). The organic layer was dried over anhydrous sodium sulfate, filtered, and concentrated in vacuum to give a crude product. The crude product was purified by Pre-HPLC to give the compound C164 (4.33 mg, yield: 3.59 %). LCMS: Rt=2.196min; [M+H]⁺=691.4.

### Example 50: Preparation of compound C165

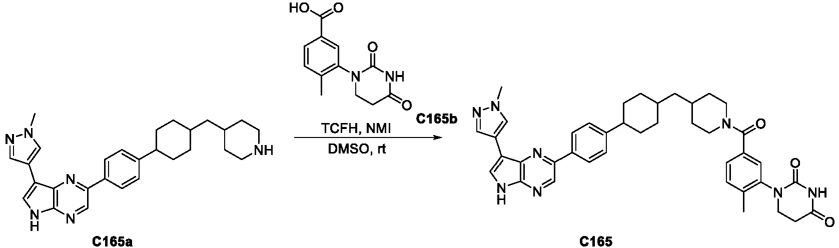

Compound C165a (79 mg, 0.17 mmol) was dissolved in DMSO (3 mL) at room temperature. 3-(2,4-dioxotrihydropyrimidin-1(2H)-yl)-4-methylbenzoic acid (60 mg, 0.22 mmol), HATU (97 mg, 0.26 mmol), and DIPEA (110 mg, 0.85 mmol) were added. The reaction solution was stirred at room temperature for 16 h. LCMS showed the complete conversion of the reactants, and the product was detected. The reaction solution was poured into water (30 mL) and then extracted with dichloromethane (20 mL*2). The organic layer was dried over anhydrous sodium sulfate, filtered, and concentrated in vacuum to give a crude product. The crude product was purified by Pre-HPLC to give the compound C165 (4.42 mg, yield: 3.61%). LCMS: Rt=2.200min; [M+H]⁺=687.5.

### Example 51: Preparation of compound C167

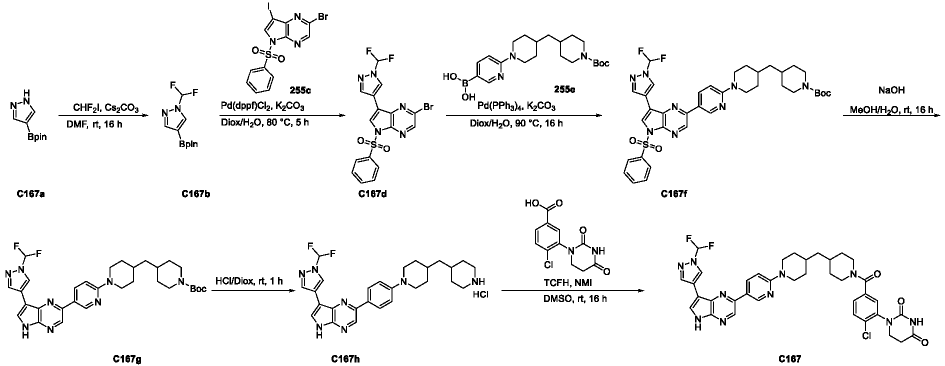

### Step 1:

Compound C167a (6.2 g, 31.95 mmol) was dissolved in DMF (50 mL) at room temperature. Cesium carbonate (12.49 g, 38.34 mmol) and difluoroiodomethane (5.09 g, 35.15 mmol) were added. The mixture was stirred at room temperature for 16 h. LCMS showed the complete conversion of the reactants, and the product was detected. The reaction solution was filtered and the filtrate was concentrated in vacuum to give the crude compound C167b (6.72 g, yield: 99.64%). LCMS: [M+H]⁺=211.1.

### Step 2:

Compound C167b (1 g, 2.15 mmol) was dissolved in dioxane (10 mL) and water (3 mL) at room temperature. Compound C167c (0.54 g, 2.58 mmol), potassium carbonate (0.89 g, 6.45 mmol) and Pd(dppf)Cl₂·DCM (88 mg, 0.11 mmol) were added. The reaction solution was stirred at 80°C for 4 h under a nitrogen atmosphere. LCMS showed the complete conversion of the reactants, and the product was detected. The reaction solution was filtered and concentrated in vacuum to give a crude product. The crude product was purified by silica gel chromatography (petroleum ether: ethyl acetate = 0-30%) to give the compound C167d (322 mg, yield: 35.47 %). LCMS: [M+H]⁺=422.4.

### Step 3:

Compound C167d (60 mg, 0.14 mmol) was dissolved in dioxane (5 mL) and water (1 mL) at room temperature. Compound C167e (59 mg, 0.15 mmol), tetrakis(triphenylphosphine)palladium (8mg, 0.0071 mmol), and potassium carbonate (60 mg, 0.43 mmol) were added. The reaction solution was stirred at room temperature for 16 h. LCMS showed the complete conversion of the reactants, and the product was detected. The reaction was concentrated in vacuum to give the crude compound C167f (99 mg, yield: 99.32 %). LCMS: [M+H]⁺=699.4.

### Step 4:

Compound C167f (99 mg, 0.14 mmol) was dissolved in methanol (S0, 5 mL) and water (1 mL) at room temperature. Sodium hydroxide (56.00 mg, 1.40 mmol) was added. The reaction solution was stirred at room temperature for 1 h. LCMS showed the complete conversion of the reactants, and the product was detected. The reaction solution was concentrated in vacuum to give a crude product. The crude product was purified by silica gel chromatography (methanol: dichloromethane = 0-3%) to give the compound C167g (70 mg, yield: 88.41 %). LCMS: [M+H]⁺=560.4.

### Step 5:

Compound C167g (70 mg, 0.13 mmol) was dissolved in methanol (5 mL) at room temperature. A hydrochloric acid solution in dioxane (5 mL) was added and the reaction solution was stirred at room temperature for 2 h. LCMS showed the complete conversion of the reactants, and the product was detected. The reaction was concentrated in vacuum to give the crude compound C167h (62 mg, yield: 99.94 %). LCMS: [M+H]⁺=460.5.

### Step 6:

Compound C167h (62 mg, 0.12 mmol) was dissolved in DMSO (3 mL) at room temperature. 4-Chloro-3-(2,4-dioxotrihydropyrimidin-1(2H)-yl)benzoic acid (39 mg, 0.15 mmol), TCFH (44 mg, 0.16 mmol), and NMI (50 mg, 0.61 mmol) were added. The reaction solution was stirred at room temperature for 2 h. LCMS showed the complete conversion of the reactants, and the product was detected. The reaction solution was poured into water (30 mL) and then extracted with dichloromethane (30 mL*2). The organic layer was dried over anhydrous sodium sulfate, filtered, and concentrated in vacuum to give a crude product. The crude product was purified by Pre-HPLC to give the compound C167 (22.68 mg, yield: 25.07 %). LCMS: [M+H]⁺=743.2.

¹H NMR (500 MHz, DMSO-d6) δ 9.21 (s, 1H), 9.01 - 8.35 (m, 4H), 8.41 - 7.99 (m, 2H), 7.73 - 6.76 (m, 6H), 4.30 - 3.80 (m, 6H), 3.21 - 2.62 (m, 6H), 1.99 - 1.53 (m, 9H), 1.48 - 1.18 (m, 6H).

### Example 52: Preparation of compound C169

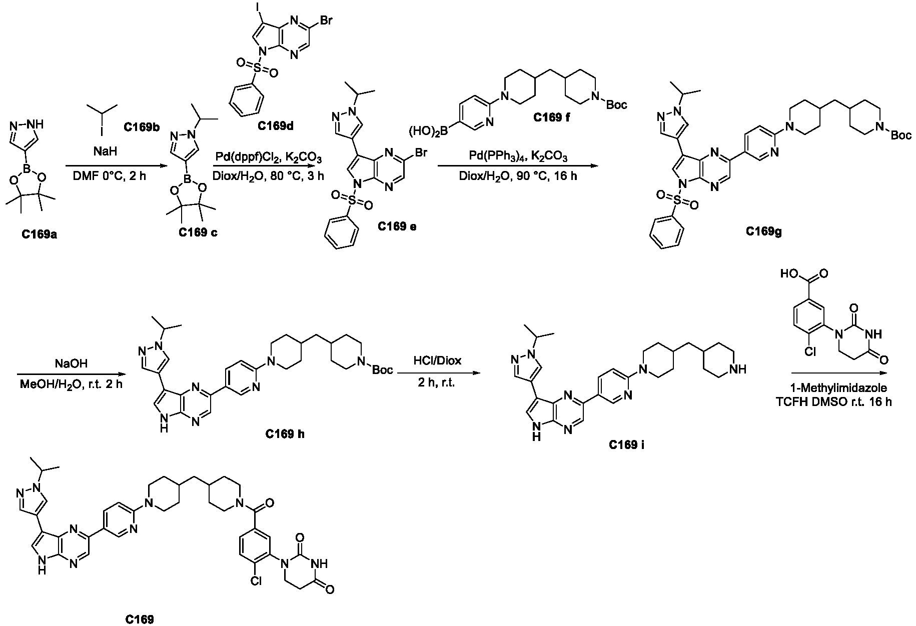

### Step 1:

C169a (2.0 g, 10.3 mmol) was dissolved in 20 mL solvent DMF. Sodium hydride (1.2 g, 30.9 mmol) was added in an ice bath for reaction for 30 min. C169b (3.5 g, 20.6 mmol) was added for reaction for 2 h. LCMS showed the complete conversion of the reactants and the reaction product was detected. The reaction was then completed. Saturated aqueous ammonium chloride was added to quench the reaction. The reaction solution was extracted three times with ethyl acetate (20 mL*3). The organic layer was dried over anhydrous sodium sulfate, filtered, and concentrated in vacuum. After purification by silica gel chromatography (EA/PE = 1: 2), product C169c (2.2 g, 9.3 mmol) with a yield of 90.3% was obtained.

### Step 2:

Compounds C169c (2.1 g, 8.9 mmol), C169d (3.7 g, 8.0 mmol), the catalyst (295 mg, 0.4 mmol), and potassium carbonate (3.35 g, 24.2 mmol) were added successively to a 250 mL one-necked flask, followed by 60 mL of dioxane and 20 mL of water as solvents. Then, a condenser tube was mounted on the one-necked flask. After replacement with nitrogen using a three-way valve three times, the flask was heated to 80°C for reaction for 3 h. Then, the reaction was determined to be completed by LCMS. After the reaction solution was filtered, water and dichloromethane were added for extraction three times (10 mL*3). The organic phase was collected, dried over anhydrous sodium sulfate, filtered, concentrated, and purified by silica gel chromatography (EA/PE = 30%). Product C169 e (1.13 g, 2.5 mmol) was obtained with a 31.3% yield.

### Step 3:

To a 100 mL one-necked flask added were compounds C169 e (97 mg, 0.22 mmol), C169 f (80 mg, 0.20 mmol), tetrakis(triphenylphosphine)palladium (23 mg, 0.02 mmol) as the catalyst, and potassium carbonate (82 mg, 0.60 mmol) successively, followed by 10 mL of dioxane and 1 mL of water as solvents. Then, a condenser tube was mounted on the one-necked flask. After replacement with nitrogen using a three-way valve three times, the flask was heated to 90°C for a reaction for 16 h. Then, the reaction was determined to be completed by LCMS. The reaction solution was filtered and directly concentrated for the next step.

### Step 4:

The crude product C169 g (crude) from the third step was added to a 100 mL reaction flask. 5 mL of methanol solvent was added, followed by aqueous sodium hydroxide (100 mg) solution. The reaction was continued at normal temperature for about 2 h, and then a small amount of reaction solution was taken. The reaction was completed as demonstrated by LCMS. The reaction solution was filtered and extracted with dichloromethane three times. The organic phase was collected, dried over anhydrous sodium sulfate, filtered, concentrated, and purified by silica gel chromatography (MeOH/CH₂Cl₂ = 4%) to give the product C169 h (80 mg).

### Step 5:

Compound C169 h was added to a 100 mL one-necked flask. HCl/dioxane (2 mol/L, 1 mL) was added and partial solid precipitated. 2 mL of methanol was added to aid dissolution. After reacting for 2 h at normal temperature, the reaction was determined to be completed by LCMS. The solvent was directly dried by rotary evaporation and the product was used as the raw material in the next step.

### Step 6:

Compounds C169 i (50 mg, 0.10 mmol), C169 j (35 mg, 0.13 mmol), TCFH (38 mg, 0.14 mmol), and 1-methylimidazole (34 mg, 0.41 mmol) were added successively to a 100 mL one-necked flask. After reaction at normal temperature overnight for 16 h, the reaction was completed as demonstrated by LCMS. The final product C169 (31.2 mg, 0.02 mmol) was isolated by preparative chromatography, with a yield of 42%. [M+H]⁺=735.3.

¹H NMR (500 MHz, DMSO-d6) δ 11.96 (s, 1H), 10.53 (s, 1H), 8.95 (d, J = 2.4 Hz, 1H), 8.79 (s, 1H), 8.35 (s, 1H), 8.33 (dd, J = 9.0, 2.5 Hz, 1H), 8.08 (d, J = 2.6 Hz, 1H), 8.07 (s, 1H), 7.64 (d, J = 8.2 Hz, 1H), 7.56 (d, J = 1.9 Hz, 1H), 7.39 (dd, J = 8.2, 1.9 Hz, 1H), 6.97 (d, J = 9.1 Hz, 1H), 4.64 - 4.58 (m, 1H), 4.47 - 4.39 (m, 3H), 3.79 - 3.74 (m, 1H), 3.64 - 3.60 (m, 2H), 2.87 (t, J = 11.7 Hz, 2H), 2.79 - 2.73 (m, 3H), 1.80 - 1.59 (m, 6H), 1.48 (d, J = 6.7 Hz, 6H), 1.26 - 1.02 (m, 7H).

### Example 53: Preparation of compound C170

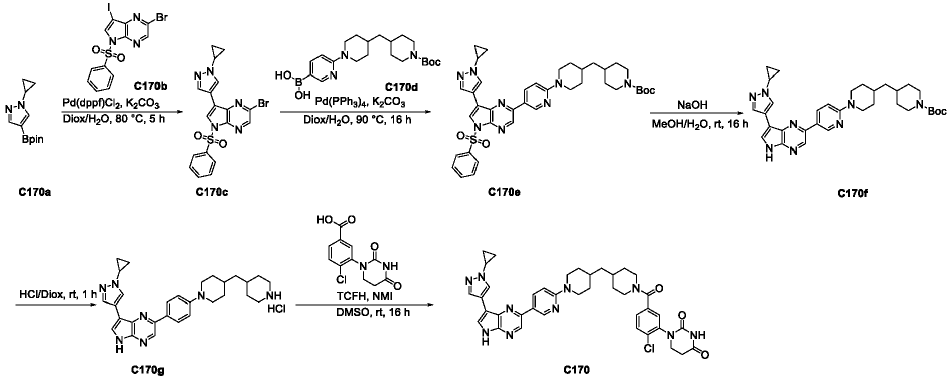

Following the procedures in the second to sixth steps of Example 51, except compound C167b was replaced with compound C170a, compound C170 was obtained (10.52 mg, yield: 7.72%). LCMS: [M+H]⁺=733.4. ¹H NMR (500 MHz, DMSO-d6) δ 12.01 (s, 1H), 10.54 (s, 1H), 8.95 (d, J = 2.4 Hz, 1H), 8.79 (s, 1H), 8.36 (s, 1H), 8.32 (dd, J = 9.0, 2.5 Hz, 1H), 8.09 (s, 1H), 8.05 (s, 1H), 7.64 (d, J = 8.2 Hz, 1H), 7.56 (d, J = 2.0 Hz, 1H), 7.39 (dd, J = 8.1, 2.0 Hz, 1H), 6.98 (d, J = 9.1 Hz, 1H), 4.41 (d, J = 13.1 Hz, 3H), 3.86 - 3.73 (m, 2H), 3.62 (dd, J = 17.1, 10.9 Hz, 2H), 2.87 (t, J = 12.4 Hz, 2H), 2.75 (q, J = 7.6, 6.8 Hz, 3H), 1.81 - 1.58 (m, 6H), 1.27 - 1.16 (m, 4H), 1.10 (tt, J = 7.9, 4.8 Hz, 5H), 1.01 (td, J = 7.3, 4.9 Hz, 2H).

C170c: LCMS: [M+H]⁺=444.0. C170e: LCMS: [M+H]⁺=723.3. C170f: LCMS: [M+H]⁺=583.4. C170g: LCMS: [M+H]⁺=483.3.

### Example 54: Preparation of compound C172

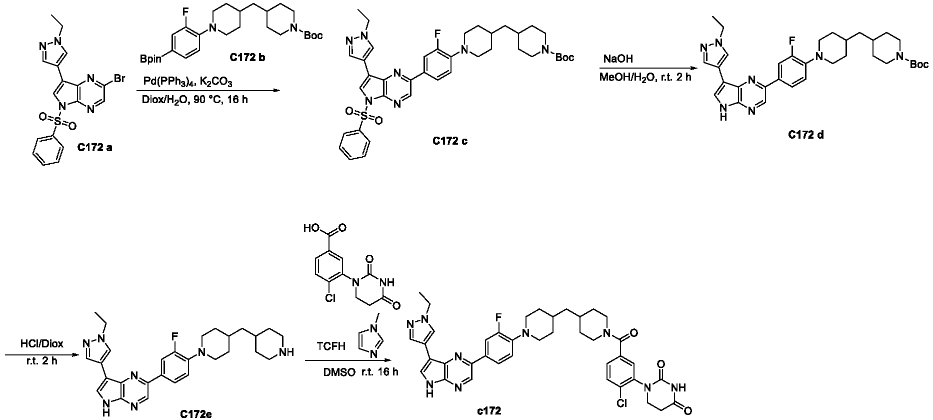

### Step 1:

To a 100 mL one-necked flask added were compounds C172a (95 mg, 0.22 mmol), C172 b (80 mg, 0.20 mmol), the catalyst tetrakis(triphenylphosphine)palladium (23 mg, 0.02 mmol) and potassium carbonate (82 mg, 0.60 mmol) successively, followed by 10 mL of dioxane and 1 mL of water as solvents. Then, a condenser tube was mounted on the one-necked flask. After replacement with nitrogen using a three-way valve three times, the flask was heated to 90°C for a reaction for 16 h. Then, the reaction was determined to be completed by LCMS. The reaction solution was filtered and directly concentrated for the next step.

### Step 2:

The crude product C172 c (crude) from the first step was added to a 100 mL reaction flask. 5 mL of methanol solvent was added, followed by aqueous sodium hydroxide (100 mg) solution. The reaction was continued at normal temperature for about 2 h, and then a small amount of reaction solution was taken. The reaction was completed as demonstrated by LCMS. The reaction solution was filtered and extracted with dichloromethane three times. The organic phase was collected, dried over anhydrous sodium sulfate, filtered, concentrated, and purified by silica gel chromatography (MeOH/CH₂Cl₂ = 4%) to give the product C172 d (60 mg).

### Step 3:

Compound C172 d was added to a 100 mL one-necked flask. HCl/dioxane (2 mol/L, 1 mL) was added and partial solid precipitated. 2 mL of methanol was added to aid dissolution. After reacting for 2 h at normal temperature, the reaction was determined to be completed by LCMS. The solvent was directly dried by rotary evaporation and the product was used as the raw material in the next step.

### Step 4:

To a 100 mL one-necked flask added were C172 e (50 mg, 0.10 mmol), C172 f (35 mg, 0.13 mmol), TCFH (38 mg, 0.14 mmol), and 1-methylimidazole (34 mg, 0.41 mmol) successively. After reaction at normal temperature overnight for 16 h, the reaction was completed as determined by LCMS. The final product C172 (33.2 mg, 0.02 mmol) was isolated by preparative chromatography, with a yield of 45%. [M+H]⁺=738.3.

¹H NMR (500 MHz, DMSO-d6) δ 12.03 (s, 1H), 10.53 (s, 1H), 8.85 (s, 1H), 8.34 (s, 1H), 8.11 (d, J = 2.7 Hz, 1H), 8.07 (s, 1H), 8.02 - 7.95 (m, 2H), 7.65 (d, J = 8.2 Hz, 1H), 7.56 (d, J = 2.0 Hz, 1H), 7.39 (dd, J = 8.2, 1.9 Hz, 1H), 7.16 (t, J = 9.0 Hz, 1H), 4.53 - 4.40 (m, 1H), 4.23 (q, J = 7.3 Hz, 2H), 3.79 - 3.74 (m, 1H), 3.67 - 3.52 (m, 2H), 3.46 (d, J = 11.5 Hz, 2H), 2.84 - 2.66 (m, 5H), 1.85 - 1.77 (m, 3H), 1.73 - 1.47 (m, 4H), 1.43 (t, J = 7.3 Hz, 3H), 1.38 - 1.15 (m, 5H), 1.14 - 1.06 (m, 2H).

### Example 55: Preparation of compound C175

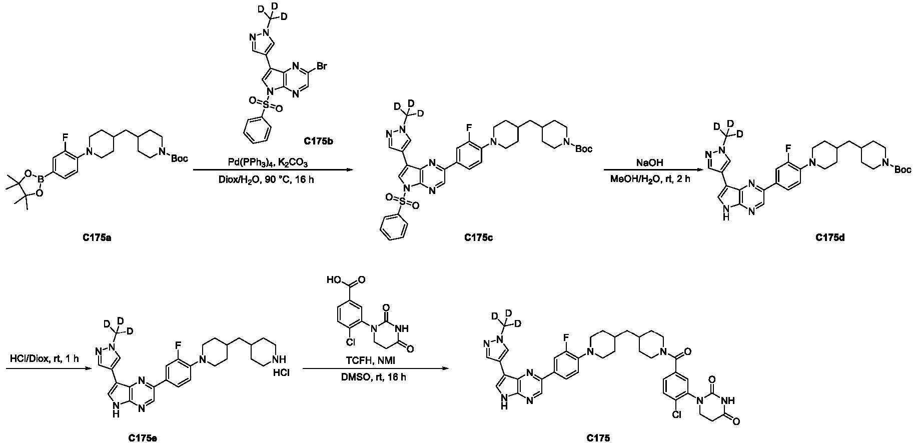

### Step 1:

Compound C175a (45 mg, 0.090 mmol) was dissolved in dioxane (50 mL) and water (5 mL) at room temperature. C175b (45 mg, 0.11 mmol), potassium carbonate (38 mg, 0.27 mmol), and tetrakis(triphenylphosphine)palladium (R3, 10 mg, 0.0087 mmol, purity 100%) were added. The reaction solution was stirred at 90°C for 3 h under a nitrogen atmosphere. The raw material was fully reacted by TLC. The reaction solution was concentrated in vacuum to give a crude product. The crude product was then dissolved in dichloromethane (100 ml). The organic layer was dried over anhydrous sodium sulfate, filtered and concentrated in vacuum to give the crude compound C175c (62 mg, yield: 96.57%), which was used directly for the next step.

### Step 2:

Compound C175c (62 mg, 0.086 mmol) was dissolved in methanol (5 mL) and water (1 mL) at room temperature. Sodium hydroxide (34 mg, 0.86 mmol) was added. The reaction solution was stirred at room temperature for 2 h. LCMS showed the complete conversion of the reactants, and the product was detected. The reaction solution was filtered and concentrated in vacuum to give a crude product. The crude product was purified by silica gel chromatography (methanol: dichloromethane = 0-4%) to give the compound C175d (42 mg, yield: 84.21 %). LCMS: [M+H]⁺=577.5.

### Step 3:

Compound C175d (42 mg, 0.073 mmol) was dissolved in methanol (3 mL) at room temperature. A hydrochloric acid solution in dioxane (3 mL, 4M) was added and the reaction solution was stirred at room temperature for 2 h. LCMS showed the complete conversion of the reactants, and the product was detected. The reaction solution was concentrated in vacuum to give the crude compound C175e (37 mg, yield: 99.03 %), which was used directly for the next step. LCMS: [M+H]⁺=482.3.

### Step 4:

Compound C175e (37 mg, 0.078 mmol) was dissolved in DMSO (3 mL) at room temperature. 4-Chloro-3-(2,4-dioxotrihydropyrimidin-1(2H)-yl)benzoic acid (23 mg, 0.086 mmol), TCFH (R2, 33 mg, 0.12 mmol), and NMI (32 mg, 0.39 mmol) were added. The reaction solution was stirred at room temperature for 16 h. LCMS showed the complete conversion of the reactants and the product was detected. The reaction solution was poured into water (30 mL) and then extracted with dichloromethane (20 mL*2). The organic layer was dried over anhydrous sodium sulfate, filtered, and concentrated in vacuum to give a crude product. The crude product was purified by Pre-HPLC to give the compound C175 (14.01 mg, yield: 23.46 %). LCMS: Rt=2.100 min; [M+H]⁺=727.4.

### Example 56: Preparation of compound C177

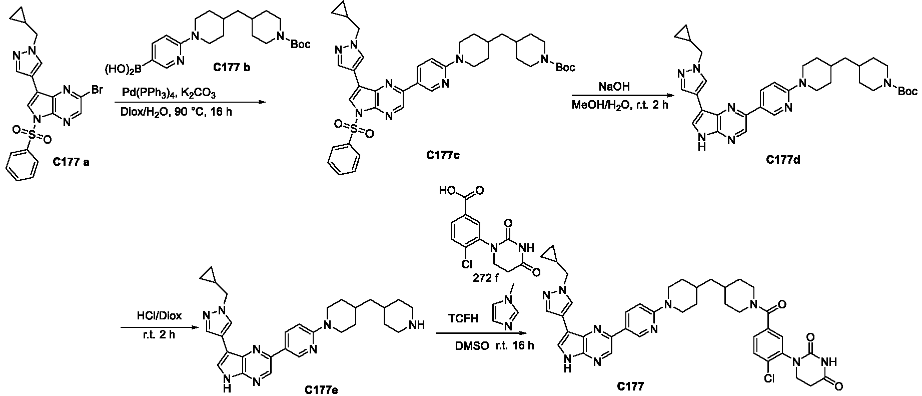

### Step 1:

Compounds C177a (60 mg, 0.13 mmol), C177b (50 mg, 0.12 mmol), the catalyst tetrakis(triphenylphosphine)palladium (14 mg, 0.01 mmol), and potassium carbonate (51 mg, 0.36 mmol) were added successively to a 100 mL one-necked flask, followed by 10 mL of dioxane and 1 mL of water as solvents. Then, a condenser tube was mounted on the one-necked flask. After replacement with nitrogen using a three-way valve three times, the flask was heated to 90°C for a reaction for 16 h. Then, the reaction was determined to be completed by LCMS. The reaction solution was filtered and directly concentrated for the next step.

### Step 2:

The crude product C177c (crude) from the first step was put into a 100 mL reaction flask. 5 mL of methanol solvent was added, followed by aqueous sodium hydroxide (100 mg) solution. The reaction was continued at normal temperature for about 2 h, and then a small amount of reaction solution was taken. The reaction was completed as demonstrated by LCMS. The reaction solution was filtered and extracted with dichloromethane three times. The organic phase was collected, dried over anhydrous sodium sulfate, filtered, concentrated, and purified by silica gel chromatography (MeOH/CH₂Cl₂ = 4%) to give the product C177d (60 mg).

### Step 3:

Compound C177d was added to a 100 mL one-necked flask. HCl/dioxane (2 mol/L, 1 mL) was added and partial solid precipitated. 2 mL of methanol was added to aid dissolution. After 2 h of reaction at normal temperature, the reaction was determined to be completed by LCMS. The solvent was directly dried by rotary evaporation and the product was used as the raw material in the next step.

### Step 4:

Compounds C177 e (50 mg, 0.10 mmol), C177 f (35 mg, 0.13 mmol), TCFH (38 mg, 0.14 mmol) and 1-methylimidazole (34 mg, 0.41 mmol) were added successively to a 100 mL one-necked flask. After reaction at normal temperature overnight for 16 h, the reaction was completed as determined by LCMS. The final product C177 (25.03 mg, 0.033 mmol) was isolated by preparative chromatography, with a yield of 33%. [M+H]⁺= 747.3.

¹H NMR (500 MHz, DMSO-d6) δ 11.98 (s, 1H), 10.53 (s, 1H), 8.95 (s, 1H), 8.81 (s, 1H), 8.41 (s, 1H), 8.36 (d, J = 8.0 Hz, 1H), 8.10 (d, J = 2.7 Hz, 1H), 8.05 (s, 1H), 7.64 (d, J = 8.2 Hz, 1H), 7.56 (d, J = 2.0 Hz, 1H), 7.39 (dd, J = 8.2, 2.0 Hz, 1H), 7.00 (d, J = 6.3 Hz, 1H), 4.51 - 4.34 (m, 3H), 4.06 (d, J = 7.1 Hz, 2H), 3.81 - 3.71 (m, 1H), 3.66 - 3.51 (m, 2H), 2.89 (t, J = 12.0 Hz, 2H), 2.81 - 2.68 (m, 3H), 1.84 - 1.58 (m, 6H), 1.36 - 1.02 (m, 8H), 0.60 - 0.52 (m, 2H), 0.45 - 0.37 (m, 2H).

### Example 57: Preparation of compound C179

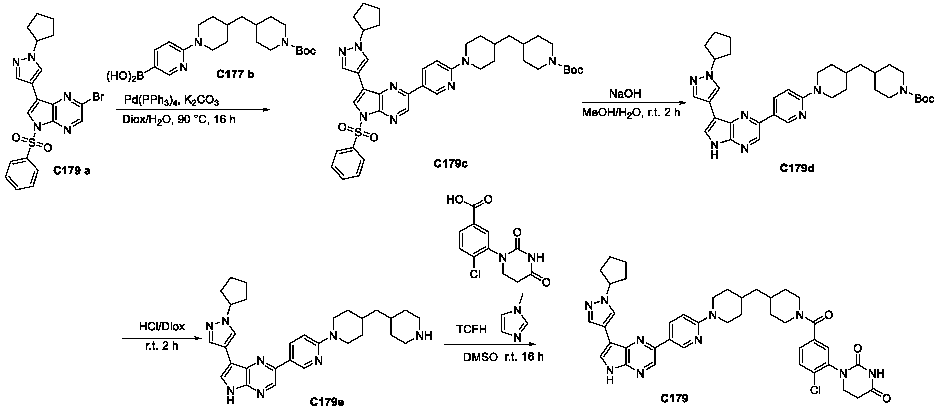

Following the method as described in Example 56, except that compound C177a in the first step was replaced with compound C179a, C179 was prepared (25.03 mg, 0.033 mmol, 33% yield). [M+H]⁺=761.3.

¹H NMR (500 MHz, DMSO-d6) δ 11.96 (s, 1H), 10.52 (s, 1H), 8.95 (s, 1H), 8.79 (s, 1H), 8.42 - 8.27 (m, 2H), 8.08 (s, 2H), 7.64 (d, J = 7.9 Hz, 1H), 7.56 (s, 1H), 7.39 (d, J = 7.6 Hz, 1H), 6.96 (d, J = 8.5 Hz, 1H), 4.87 - 4.74 (m, 1H), 4.54 - 4.28 (m, 3H), 3.83 - 3.52 (m, 4H), 2.92 - 2.71 (m, 5H), 2.21 - 2.06 (m, 2H), 2.04 - 1.91 (m, 2H), 1.88 - 1.62 (m, 9H), 1.26 - 1.01 (m, 7H).

### Example 58: Preparation of compound C181

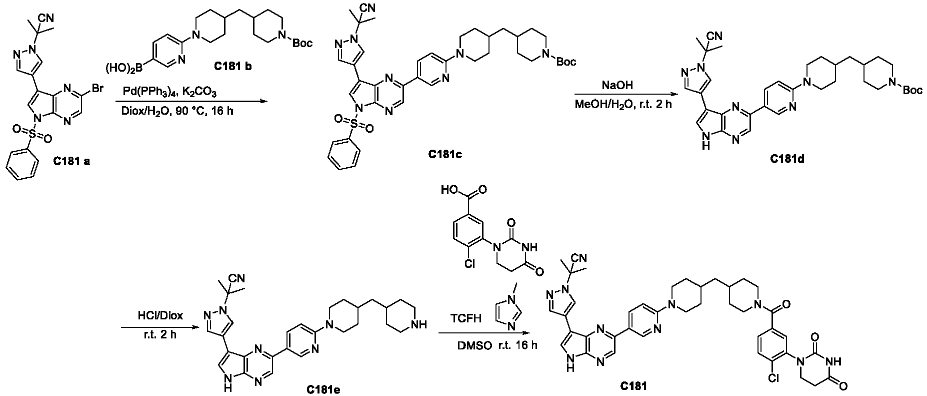

Following the method as described in Example 56, except that compound C177a in the first step was replaced with compound C181a, C181 (25.03 mg, 0.033 mmol, 33% yield) was prepared. [M+H]⁺=761.3.

### Example 59: Preparation of compound C195

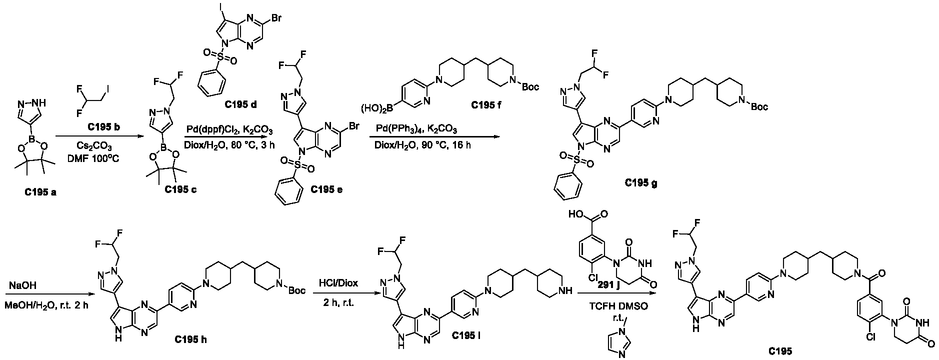

Following the method as described in Example 52, except that compound C169b in the first step was replaced with compound C195b, C195 (29 mg, 0.04 mmol) was prepared with a yield of 67%. [M+H]⁺=758.2.

¹H NMR (500 MHz, DMSO-d6) δ 12.01 (s, 1H), 10.52 (s, 1H), 8.99 (d, J = 2.5 Hz, 1H), 8.81 (s, 1H), 8.48 (s, 1H), 8.33 (dd, J = 9.0, 2.5 Hz, 1H), 8.19 - 8.08 (m, 2H), 7.64 (d, J = 8.2 Hz, 1H), 7.56 (d, J = 2.0 Hz, 1H), 7.39 (dd, J = 8.2, 2.0 Hz, 1H), 6.96 (d, J = 9.1 Hz, 1H), 6.42 (tt, J = 55.1, 3.9 Hz, 1H), 4.74 (td, J = 15.0, 3.7 Hz, 2H), 4.43 (dd, J = 24.0, 9.9 Hz, 3H), 3.82 - 3.71 (m, 1H), 3.68 - 3.50 (m, 2H), 2.87 (t, J = 11.8 Hz, 2H), 2.75 (q, J = 7.5, 6.7 Hz, 3H), 1.85 - 1.54 (m, 6H), 1.29 - 1.03 (m, 7H).

### Example 60: Preparation of compound C196

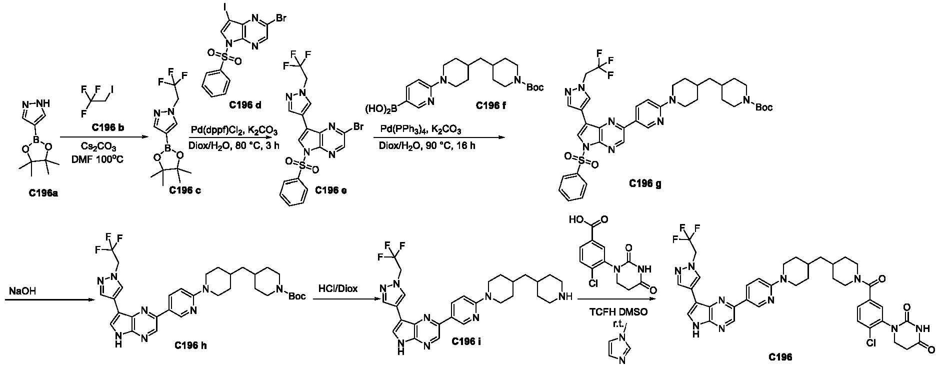

Following the method as described in Example 59, except that compound C195b in the first step was replaced with compound C196b, C 196 was prepared. The final product C 196 was obtained by chromatographic separation with a yield of 67%. [M+H]⁺=776.2.

¹H NMR (500 MHz, DMSO-d6) δ 12.05 (s, 1H), 10.52 (s, 1H), 9.00 (d, J = 2.4 Hz, 1H), 8.81 (s, 1H), 8.55 (s, 1H), 8.33 (dd, J = 9.0, 2.5 Hz, 1H), 8.18 (d, J = 2.5 Hz, 2H), 7.64 (d, J = 8.2 Hz, 1H), 7.56 (d, J = 2.0 Hz, 1H), 7.39 (dd, J = 8.2, 1.9 Hz, 1H), 6.96 (d, J = 9.1 Hz, 1H), 5.26 (q, J = 9.1 Hz, 2H), 4.43 (dd, J = 24.4, 11.2 Hz, 3H), 3.80 - 3.73 (m, 1H), 3.66 - 3.54 (m, 2H), 3.12 - 3.01 (m, 1H), 2.89 (s, 2H), 2.75 (q, J = 7.4, 6.6 Hz, 2H), 1.80 - 1.60 (m, 6H), 1.26 - 1.04 (m, 7H).

### Example 61: Preparation of compound C197

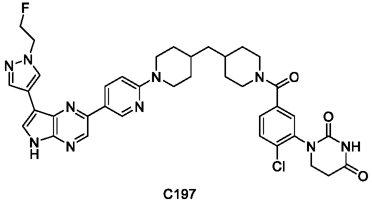

Following the method as described in Example 59, except that compound C195b in the first step was replaced with fluoroethane, C197 was prepared. The final product C197 was obtained by chromatographic separation. [M+H]⁺=740.2.

### Example 62: Preparation of compound C198

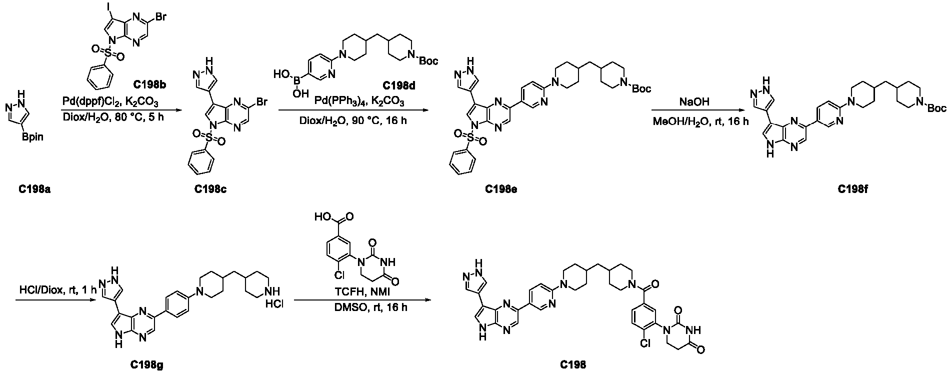

Following the method as described in Example 53, except that compound C170a in the first step was replaced with compound C198a, compound C198 was obtained (10.52 mg, yield: 7.72 %).

### Example 63: Preparation of compound C199

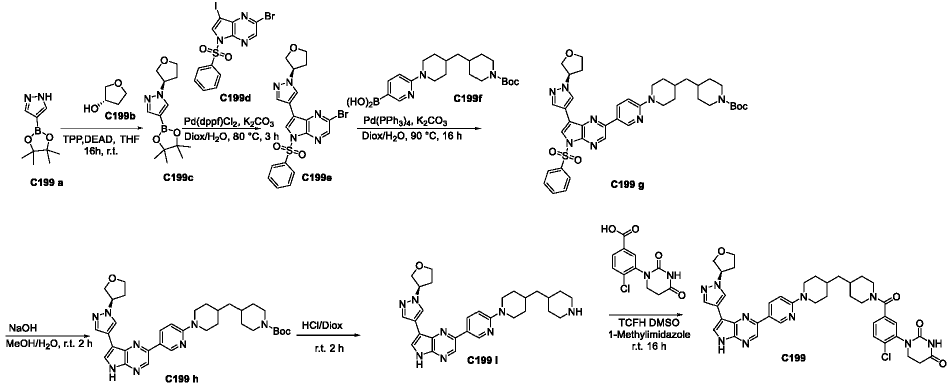

### Step 1:

Compounds C199 a (1.5 g, 7.8 mmol), C199 b (1.3 g, 14.7 mmol), and TPP (2.4 g, 9.2 mmol) were added to a 100 mL three-necked flask, followed by 30 mL of redistilled tetrahydrofuran. Then, the system was replaced with nitrogen three times and stirred at -20°C for about 30 min. 1.8 ML of reagent DEAD (9.2 mmol) was gradually added dropwise. After 2 h of reaction, the system was then transferred to room temperature for reaction overnight. A new product was found to be formed by plate spotting and the reaction was completed. The reaction was quenched by adding saturated aqueous ammonium chloride solution and extracted three times with ethyl acetate (20 mL*3). The organic layer was dried over anhydrous sodium sulfate, filtered, and concentrated in vacuum. By chromatographic column purification (EA/PE = 30%), a new product C199c (850 mg) was obtained.

### Step 2 to Step 6:

Following the procedures in the second step to the sixth step of Example 52, compound C199 was obtained (29 mg, 0.04 mmol). [M+H]⁺=764.3.

¹H NMR (500 MHz, DMSO-d6) δ 11.99 (s, 1H), 10.51 (s, 1H), 8.93 (s, 1H), 8.80 (s, 1H), 8.40 (s, 1H), 8.35 (d, J = 6.3 Hz, 1H), 8.11 (s, 2H), 7.64 (d, J = 8.2 Hz, 1H), 7.58 - 7.53 (m, 1H), 7.42 - 7.35 (m, 1H), 7.01 (d, J = 6.5 Hz, 1H), 5.17 - 5.11 (m, 1H), 4.56 - 4.33 (m, 3H), 4.07 - 4.00 (m, 2H), 3.97 - 3.92 (m, 1H), 3.90 - 3.84 (m, 1H), 3.80 - 3.71 (m, 1H), 3.67 - 3.52 (m, 2H), 2.90 (t, J = 12.2 Hz, 2H), 2.81 - 2.67 (m, 3H), 2.47 - 2.35 (m, 2H), 2.34 - 2.28 (m, 1H), 1.86 - 1.57 (m, 6H), 1.25 - 1.02 (m, 6H).

### Example 64: Preparation of compound C200

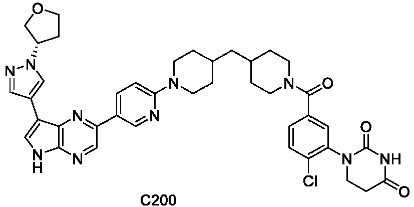

Following the method of Example 63, compound C200 was obtained. The final product C200 was isolated by preparative chromatography. [M+H]⁺= 764.3.

### Example 65: Preparation of compound C201

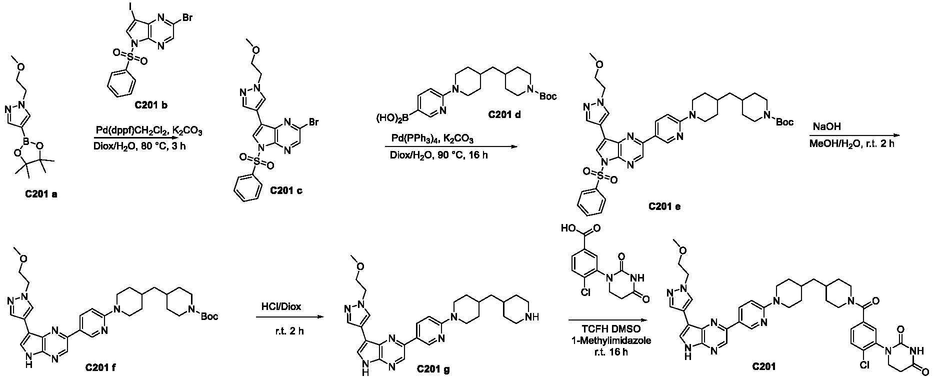

Following the procedures as described in the second step to the sixth step of Example 52, except that compound C169c in the second step was replaced with compound C201a, compound C201 (31 mg, 0.04 mmol) was obtained. [M+H]⁺=752.3.

¹H NMR (500 MHz, DMSO-d6) δ 11.96 (s, 1H), 10.51 (s, 1H), 8.96 (d, J = 2.0 Hz, 1H), 8.79 (s, 1H), 8.36 (s, 1H), 8.33 (dd, J = 9.0, 2.3 Hz, 1H), 8.09 (d, J = 2.6 Hz, 1H), 8.05 (s, 1H), 7.64 (d, J = 8.2 Hz, 1H), 7.55 (d, J = 1.7 Hz, 1H), 7.39 (dd, J = 8.2, 1.7 Hz, 1H), 6.96 (d, J = 9.0 Hz, 1H), 4.55 - 4.30 (m, 3H), 4.35 (t, J = 5.3 Hz, 2H), 3.83 - 3.69 (m, 3H), 3.66 - 3.53 (m, 2H), 3.27 (s, 3H), 2.87 (t, J = 12.1 Hz, 2H), 2.82 - 2.69 (m, 3H), 1.82 - 1.58 (m, 6H), 1.29 - 0.99 (m, 7H).

### Example 66: Preparation of compound C203

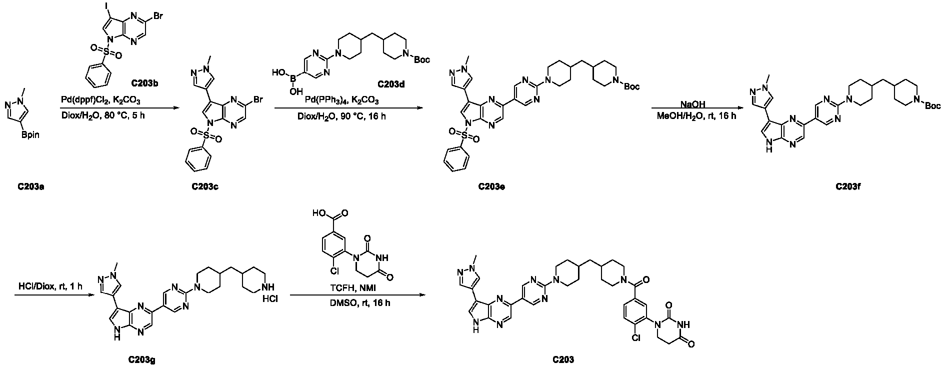

Following the method as described in Example 53, except that compound C170a in the first step was replaced with compound C203a, compound C203 was obtained (10.52 mg, yield: 7.72 %). LCMS: [M+H]⁺=709.2.

¹H NMR (500 MHz, DMSO-d6) δ 9.32 - 8.83 (m, 3H), 8.42 - 8.10 (m, 3H), 7.56 (d, J = 7.5 Hz, 1H), 7.41 - 7.08 (m, 2H), 4.08 (d, J = 34.1 Hz, 2H), 3.96 - 3.81 (m, 7H), 3.19 - 2.69 (m, 5H), 1.90 - 1.69 (m, 5H), 1.64 - 1.37 (m, 5H), 1.23 (d, J = 12.5 Hz, 1H).

### Example 67: Preparation of compound C204

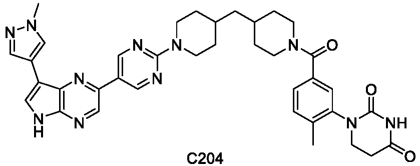

Following the method as described in Example 66, compound C204 was obtained. The final product C204 was isolated by preparative chromatography. [M+H]⁺=688.8.

### Example 68: Preparation of compound C205

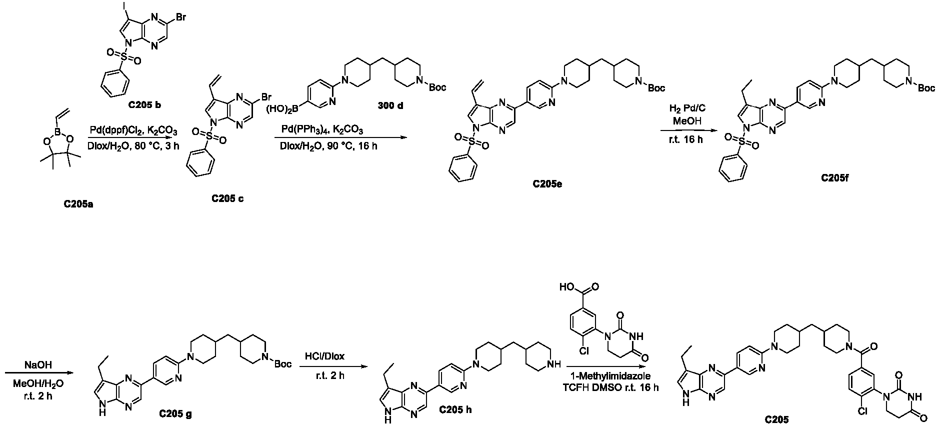

### Step 1:

Compounds C205a (0.2 g, 1.3 mmol), C205 b (0.5 g, 1.1 mmol), the catalyst (40 mg, 0.05mmol), potassium carbonate (0.5 g, 3.6 mmol) were added successively to a 250 mL one-necked flask, followed by 30 mL of dioxane and 10 mL of water as solvents. Then, a condenser tube was mounted on the one-necked flask. After replacement with nitrogen using a three-way valve three times, the flask was heated to 80°C for reaction for 3 h. Then, the reaction was determined to be completed by LCMS. After the reaction solution was filtered, water and dichloromethane were added for extraction three times (10 mL*3). The organic phase was collected, dried over anhydrous sodium sulfate, filtered, concentrated, and purified by silica gel chromatography (EA/PE = 30%). Product C205c (0.1 g, 0.3 mmol) was obtained with a yield of 23%.

### Step 2:

Compounds C205c (100 mg, 0.3 mmol), C205d (70 mg, 0.2 mmol), the catalyst tetrakis(triphenylphosphine)palladium (20 mg, 0.02 mmol) and potassium carbonate (100 mg, 0.54 mmol) were added successively to a 100 mL one-necked flask, followed by 10 mL of dioxane and 1 mL of water as solvents. Then, a condenser tube was mounted on the one-necked flask. After replacement with nitrogen using a three-way valve three times, the flask was heated to 90°C for reaction for 16 h. Then, the reaction was determined to be completed by LCMS. The reaction solution was filtered and directly concentrated for the next step. The reaction was completed as determined by LCMS and purified by silica gel chromatography (MeOH/CH₂Cl₂ = 4%) to give the product C205e (39 mg).

### Step 3:

To a 50 mL one-necked flask added was the product of the second step, followed by 10% Pd/C (30 mg). The system was replaced with hydrogen and started to react at normal temperature for 16 h. The reaction was determined to be completed by LCMS. The reaction was directly filtered through Celite and subjected to rotary evaporation to remove methanol for the next step.

### Step 4:

The crude product C205f (crude) from the third step was put into a 100 mL reaction flask. 5 ML of methanol solvent was added, followed by aqueous sodium hydroxide (100 mg) solution. The reaction was continued at normal temperature for about 2 h, and then a small amount of reaction solution was taken. The reaction was completed as determined by LCMS. The reaction solution was filtered and extracted with dichloromethane three times. The organic phase was collected, dried over anhydrous sodium sulfate, filtered, concentrated, and purified by silica gel chromatography (MeOH/CH₂Cl₂ = 4%) to give the product C205 g (29 mg).

### Step 5:

To a 100 mL one-necked flask added was C205g (29 mg, 0.06 mmol). HCl/dioxane (2 mol/L, 1 mL) was added and partial solid precipitated. 2 mL of methanol was added to aid dissolution. After reacting for 2 h at normal temperature, the reaction was determined to be completed by LCMS. The solvent was directly dried by rotary evaporation and the product was used as the raw material in the next step.

### Step 6:

Compounds C205h (29 mg, 0.07 mmol), C205I (19 mg, 0.07 mmol), TCFH (20 mg, 0.08 mmol), and 1-methylimidazole (20 mg, 0.24 mmol) were added successively to a 100 mL one-necked flask. After reaction at normal temperature overnight for 16 h, the reaction was completed as determined by LCMS. The final product C205 (3.6 mg, 0.005 mmol) was isolated by preparative chromatography, 7.1% yield. [M+H]⁺=655.2.

### Example 69: Preparation of compound C206

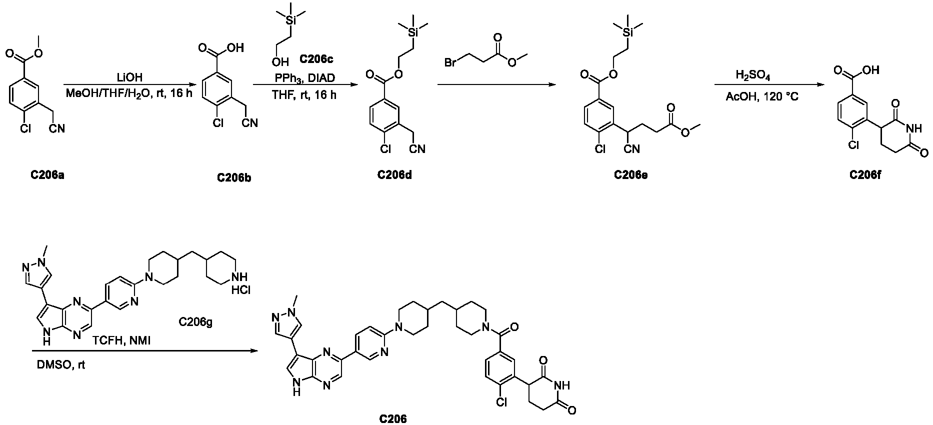

### Step 1:

Compound C206a (4.5 g, 21.47 mmol) was dissolved in THF (10 mL), methanol (10 mL) and water (5 mL) at room temperature. Lithium hydroxide monohydrate (1.35 g, 32.20 mmol) was added and the reaction solution was stirred at room temperature for 16 h. LCMS showed the complete conversion of the reactants, and the product was detected. The reaction solution was concentrated in vacuum to give a crude product. The crude product was then dissolved in water (2 mL). 1 N HCl solution was added to adjust pH to about 3, stirred, and filtered. The filter cake was washed with water (30 mL) and then dried to give the compound C206b (4.2 g, yield: 100.03%). LCMS: [M+H]⁺=196.1.

### Step 2:

Compound C206b (4.2 g, 21.47 mmol) was dissolved in THF (20 mL) in an ice bath. Compound C206c (2.54 g, 21.47 mmol), triphenylphosphine (5.63 g, 21.47 mmol), and DIAD (4.34 g, 21.47 mmol) were added. The reaction solution was stirred at room temperature under a nitrogen atmosphere for 16 h. Most of the reactants were converted as demonstrated by TLC, and a new point was detected. The reaction solution was poured into water (50 mL) and then extracted with ethyl acetate (30 mL *2). The organic layer was dried over anhydrous sodium sulfate, filtered, and concentrated in vacuum to give a crude product. The crude product was purified by silica gel chromatography (petroleum ether: ethyl acetate = 0-4%) to give the compound C206c (2.14 g, yield: 33.69 %).

### Step 3:

Compound C206c (2.14 g, 7.23 mmol) was dissolved in DMF (20 mL) at room temperature. NaH (350 mg, 8.75 mmol) (60%) was added in an ice bath and stirred for 10 minutes. Then, methyl 3-bromopropionate (1.81 g, 10.85 mmol) was added and the reaction solution was stirred at room temperature for 16 h. The reactants were partially converted as demonstrated by TLC, and a new point was detected. The reaction solution was concentrated in vacuum to give a crude product. The crude product was purified by silica gel chromatography (petroleum ether: ethyl acetate = 0-9%) to give the compound C206d (1.745 g, yield: 63.16 %).

### Step 4:

Compound C206d (1.7 g, 4.45 mmol) was dissolved in acetic acid (20 mL) at room temperature. Concentrated sulfuric acid (44 mg, 0.45 mmol) was added and the reaction solution was stirred at 120°C for 16 h. LCMS showed the complete conversion of the reactants, and the product was detected. The reaction solution was concentrated in vacuum to give a crude product. The crude product was purified by silica gel chromatography (methanol: dichloromethane = 0-2%) to give the compound C206e (0.9 g, yield: 75.5 %). LCMS: [M+H]⁺=268.4.

### Step 5:

Compound C206f (100 mg, 0.20 mmol) was dissolved in DMSO (3 mL) at room temperature, C206e (64 mg, 0.24 mmol), TCFH (84.17 mg, 0.30 mmol), and NMI (82.1 mg, 1 mmol) were added. The reaction solution was stirred at room temperature for 16 h. LCMS showed the complete conversion of the reactants, and the product was detected. The reaction solution was poured into water (30 mL) and then extracted with dichloromethane (30 mL*2). The organic layer was dried over anhydrous sodium sulfate, filtered, and concentrated in vacuum to give a crude product. The crude product was purified by pre-HPLC to give the compound C206 (4.4 mg, yield: 2.76 %). LCMS: [M+H]⁺=706.6.

¹H NMR (500 MHz, DMSO-d6) δ 11.97 (s, 1H), 10.93 (s, 1H), 8.97 (d, J = 2.5 Hz, 1H), 8.79 (s, 1H), 8.36 - 8.30 (m, 2H), 8.08 (d, J = 2.3 Hz, 1H), 8.02 (s, 1H), 7.56 - 7.52 (m, 1H), 7.37 (d, J = 2.0 Hz, 1H), 7.32 (dd, J = 8.2, 2.1 Hz, 1H), 6.96 (d, J = 9.0 Hz, 1H), 4.48 - 4.39 (m, 3H), 4.31 - 4.24 (m, 1H), 3.93 (s, 3H), 2.87 (t, J = 12.4 Hz, 2H), 2.80 - 2.71 (m, 2H), 2.38 - 2.30 (m, 1H), 2.03 - 1.96 (m, 1H), 1.82 - 1.59 (m, 7H), 1.22 - 1.03 (m, 7H).

### Example 70: Preparation of compound C209

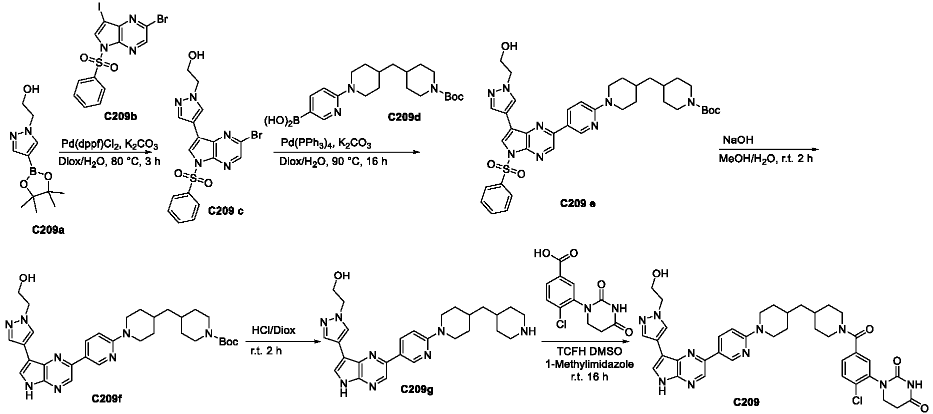

Following the method as described in the second step to the sixth step of Example 52, except that compound C169c in the second step was replaced with C209a, compound C209 (17.8 mg, 0.04 mmol) was obtained, with a yield of 39%. [M+H]⁺=737.3.

The compounds provided herein and their uses are described in detail above.

Specific examples are used herein to explain the principles and implementation modes of the present invention. The description of the above embodiments is only used to help understand the methods and core ideas of the present invention. Of noted is that, for those of ordinary skill in the art, various improvements and modifications can be made to the present invention without departing from the principles of the present invention, and these improvements and modifications also fall within the protection scope of the claims of the present invention.

## Claims

1. A compound represented by general formula (I):
[B-L]ₙ-HPK1 ligand (I),
or a pharmaceutically acceptable salt, or a stereoisomer thereof,
wherein:
the HPK1 ligand is an HPK1 inhibitor; B is a degradation tag, such as an E3 ligase ligand; L is a linking group between the B and the HPK1 ligand; n is the number of the degradation tags attached to the HPK1 ligand and is selected from 1, 2, or, 3, preferably n is 1.

2. The compound of claim 1, wherein the HPK1 ligand is a compound of general formula (H-I): 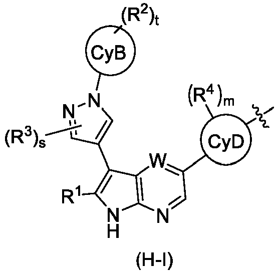 or a pharmaceutically acceptable salt, or a stereoisomer thereof,
wherein:
W is selected from CR¹ or N;
ring CyB is selected from 4-10 membered cycloalkyl, 4-10 membered heterocyclyl, 5-8 membered aryl, or 5-8 membered heteroaryl;
R¹ is independently selected from hydrogen, halogen, -C₁₋₈ alkyl, -C₂₋₈ alkenyl, -C₂₋₈ alkynyl, cycloalkyl, heterocyclyl, aryl, heteroaryl, -CN, -NO₂, or -OR^{1a}, wherein the -C₁₋₈ alkyl, -C₂₋₈ alkenyl, -C₂₋₈ alkynyl, cycloalkyl, heterocyclyl, aryl, or heteroaryl is each optionally substituted with halogen, hydroxyl, -C₁₋₈ alkoxy, cycloalkyl, heterocyclyl, aryl, or heteroaryl;
R² is selected from hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{2a}, -SO₂R^{2a}, -COR^{2a}, -CO₂R^{2a}, -CONR^{2a}R^{2b}, -C(=NR^{2a})NR^{2b}R^{2c}, -NR^{2a}R^{2b}, -NR^{2a}COR²⁶, -NR^{2a}CONR^{2b}R²°, -NR^{2a}CO₂R^{2b}, - NR^{2a}SONR^{2b}R^{2c}, -NR^{2a}SO₂NR^{2b}R^{2c}, or -NR^{2a}SO₂R^{2b}, wherein the -C₁₋₈ alkyl, -C₂₋₈ alkenyl, - C₂₋₈ alkynyl, cycloalkyl, heterocyclyl, aryl, or heteroaryl is each optionally substituted with halo, hydroxyl, -C₁₋₈ alkoxy, cycloalkyl, heterocyclyl, aryl, or heteroaryl;
R^{2a}, R^{2b} and R^{2c} are each independently hydrogen, -C₁₋₈ alkyl, -C₂₋₈ alkenyl, -C₂₋₈ alkynyl, cycloalkyl, heterocyclyl, aryl, or heteroaryl, wherein the -C₁₋₈ alkyl, -C₂₋₈ alkenyl, -C₂₋₈ alkynyl, cycloalkyl, heterocyclyl, aryl, or heteroaryl is each optionally substituted with at least one substituent R^{2d}; or
(R^{2a} and R^{2b}), (R^{2b} and R^{2c}), or (R^{2c} and R^{2a}), together with the one or more atoms to which they are attached, form a 3- to 9-membered ring comprising 0, 1, or 2 heteroatoms independently selected from nitrogen, oxygen, or optionally oxidized sulfur as one or more ring members, wherein the ring is optionally substituted with at least one substituent R^{2e};
wherein R^{2d} and R^{2e} are each independently hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{2f}, -SO₂R^{2f}, - COR^{2f}, -CO₂R^{2f}, -CONR^{2f}R^{2g}, -C(=NR^{2f})NR^{2g}R^{2h}, -NR^{2f}R^{2g}, -NR^{2f}COR^{2g}, -NR^{2f}CONR^{2g}R^{2h}, - NR^{2f}CO₂R^{2g}, -NR^{2f}SONR^{2g}R^{2h}, -NR^{2f}SO₂NR^{2g}R^{2h}, or -NR^{2f}SO₂R^{2g}, wherein the -C₁₋₈ alkyl, - C₂₋₈ alkenyl, -C₂₋₈ alkynyl, cycloalkyl, heterocyclyl, aryl, or heteroaryl is each optionally substituted with at least one substituent selected from halo, -C₁₋₈ alkyl, -OR²ⁱ, -NR²ⁱR^{2j}, cycloalkyl, heterocyclyl, aryl, or heteroaryl;
R^{2f}, R^{2g}, R^{2h}, R²ⁱ, and R^{2j} are each independently hydrogen, -C₁₋₈ alkyl, C₁₋₈ alkoxy-C₁₋₈ alkyl-, -C₂₋₈ alkenyl, -C₂₋₈ alkynyl, cycloalkyl, heterocyclyl, aryl, or heteroaryl;
R³ is selected from hydrogen, halogen, -C₁₋₈ alkyl, -C₂₋₈ alkenyl, -C₂₋₈ alkynyl, cycloalkyl, heterocycloalkyl, aryl, heteroaryl, -CN, or -NO₂;
R⁴ is independently hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{4a}, -SO₂R^{4a}, -SO₂NR^{4a}R⁴⁶, -COR^{4a}, -CO₂R^{4a}, -CONR^{4a}R^{4b}, -C(=NR^{4a})NR^{4b}R^{4c}, -NR^{4a}R^{4b}, -NR^{4a}COR^{4b} -NR^{4a}CONR^{4b}R^{4c}, -NR^{4a}CO₂R^{4b}, - NR^{4a}SONR^{4b}R^{4c}, -NR^{4a}SO₂NR^{4b}R^{4c}, or -NR^{4a}SO₂R^{4b}, wherein the -C₁₋₈ alkyl, -C₂₋₈ alkenyl, - C₂₋₈ alkynyl, cycloalkyl, heterocyclyl, aryl, or heteroaryl is each optionally substituted with at least one substituent R^{4d};
R^{4a}, R^{4b}, and R^{4c} are each independently hydrogen, -C₁₋₈ alkyl, -C₂₋₈ alkenyl, -C₂₋₈ alkynyl, cycloalkyl, heterocyclyl, aryl, or heteroaryl, wherein the -C₁₋₈ alkyl, -C₂₋₈ alkenyl, -C₂₋₈ alkynyl, cycloalkyl, heterocyclyl, aryl, or heteroaryl is each optionally substituted with at least one substituent R^{4e},
R^{4d} and R^{4e} are each independently hydrogen, halogen, -C₁₋₈ alkyl, -C₂₋₈ alkenyl, -C₂₋₈ alkynyl, cycloalkyl, heterocyclyl, aryl, heteroaryl, oxo, -CN, -NO₂, -OR^{4f}, -SO₂R^{4f}, - SO₂NR^{4f}R^{4g}, -COR^{4f}, -CO₂R^{4f}, -CONR^{4f}R^{4g}, -C(=NR^{4f})NR^{4g}R^{4h}, -NR^{4f}R^{4g}, -NR^{4f}COR^{4g}, - NR^{4f}CONR^{4g}R^{4h}, -NR^{4f}CO₂R^{4f}, -NR^{4f}SONR^{4f}R^{4g}, -NR^{4f}SO₂NR^{4g}R^{4h}, or -NR^{4f}SO₂R^{4g}, wherein the -C₁₋₈ alkyl, -C₂₋₈ alkenyl, -C₂₋₈ alkynyl, cycloalkyl, heterocyclyl, aryl, or heteroaryl is each optionally substituted with at least one substituent selected from halogen, -C₁₋₈ alkyl, -OR⁴ⁱ, - NR⁴ⁱR^{4j}, cycloalkyl, heterocyclyl, aryl, or heteroaryl;
R^{4f}, R^{4g}, R^{4h}, R⁴ⁱ, and R^{4j} are each independently hydrogen, -C₁₋₈ alkyl, C₁₋₈ alkoxy-C₁₋₈ alkyl-, -C₂₋₈ alkenyl, -C₂₋₈ alkynyl, cycloalkyl, heterocyclyl, aryl, or heteroaryl;
s is 0, 1, or 2, provided that the valence theory is satisfied;
t is 0, 1, 2, 3, or 4, provided that the valence theory is satisfied;
m is 0, 1, 2, 3, or 4, provided that the valence theory is satisfied;
CyD is selected from cycloalkyl, heterocyclyl, aryl, or heteroaryl.

3. The compound of claim 2, wherein the HPK1 ligand is a compound of general formula (H-II): 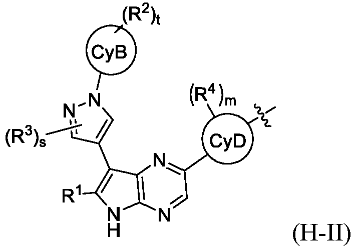
or a pharmaceutically acceptable salt, or a stereoisomer thereof,
wherein R¹, CyB, R², t, R³, s, R⁴, m and CyD are each as defined in claim 2;
preferably, 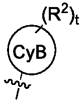 is 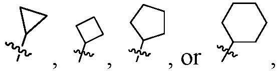 preferably 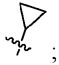 and/or
R¹ and R³ are each hydrogen; and/or
CyD is pyridyl, preferably 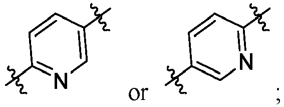 and/or
R⁴ is hydrogen.

4. The compound of claim 1, wherein the HPK1 ligand is a compound of general formula (H-III): 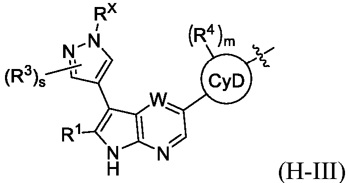 wherein:
the W, R¹, R³, s, R⁴, m, and CyD are each as defined in claim 2;
R^{X} is selected from H and C₁₋₈ alkyl, wherein the C₁₋₈ alkyl is optionally substituted with one or more substituents independently selected from deuterium, tritium, halogen, -OH, -CN, - NR^{Xa}R^{Xb}, -OR^{Xa}, -CO-NHR^{Xb}, -CO-NR^{Xa}R^{Xb}, and 3-10-membered cycloalkyl; and
R^{Xa} and R^{Xb} are each independently selected from C₁₋₈ alkyl,
preferably, R¹ is selected from hydrogen or -C₁₋₃ alkyl, preferably hydrogen; and/or
R³ is selected from hydrogen or -C₁₋₃ alkyl, preferably hydrogen; and/or
s is 0 or 1; and/or
R^{X} is selected from C₁₋₄ alkyl, wherein the C₁₋₄ alkyl is optionally substituted with 1, 2, or 3 substituents independently selected from deuterium, F, Cl, Br, -OH, -CN, -NR^{Xa}R^{Xb} or -OR^{Xa}, wherein the C₁₋₄ alkyl is preferably selected from methyl, ethyl, isopropyl, isobutyl, sec-butyl, and tert-butyl, more preferably methyl or ethyl; and R^{Xa} and R^{Xb} are preferably each independently selected from C₁₋₄ alkyl, more preferably methyl; preferably, R^{X} is selected from methyl or ethyl, wherein the methyl or ethyl is optionally substituted with 1, 2, or 3 substituents independently selected from deuterium, F, Cl, -OH, -CN, and -OCH₃, more preferably from methyl or ethyl, wherein the methyl or ethyl is optionally substituted with 1, 2, or 3 substituents independently selected from deuterium, F, and -OCH₃, more preferably from methyl, -CD₃, ethyl, -CH₂F, -CHF₂, -CF₃, -CH₂CH₂F, -CH₂CHF₂, -CH₂CF₃, or 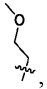 even more preferably selected from methyl, -CD₃, ethyl, -CH₂CH₂F, -CH₂CHF₂, -CH₂CF₃, or 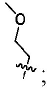 and/or
CyD is selected from 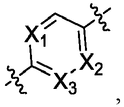 wherein X₁, X₂, and X₃ are each independently selected from CH, C, or N, provided that at least one of X₁, X₂, and X₃ is N; preferably, the CyD is pyridyl, preferably 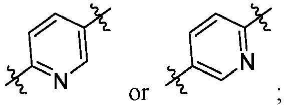 and/or
R⁴ is independently hydrogen or -C₁₋₃ alkyl, preferably hydrogen; and/or
m is 0 or 1.

5. The compound of claim 1, wherein the HPK1 ligand is a compound of general formula (H-III): 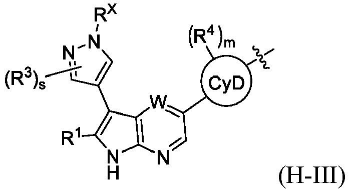 wherein:
W, R¹, R³, s, R⁴, m, and CyD are each as defined in claim 2;
R^{X} is selected from C₁₋₈ alkyl, or C₁₋₈ alkyl substituted with -NR^{Xa}R^{Xb};
R^{Xa} and R^{Xb} are each independently selected from C₁₋₈ alkyl, preferably methyl.

6. The compound of claim 1, wherein the HPK1 ligand is selected from the compounds listed in Table 1-1 of the description.

7. The compound of any one of claims 1-6, wherein the B is a group that binds to an E3 ligase, wherein the E3 ligase is selected from vonHippel-Lindau(VHL), Cereblon, XIAP, E3A, MDM2, Anaphase-promoting complex (APC), UBR5(EDD1), SOCS/BC-box/eloBC/CUL5/RING, LNXp80, CBX4, CBLL1, HACE1, HECTD1, HECTD2, HECTD3, HECW1, HECW2, HERC1, HERC2, HERC3, HERC4, HUWE1, ITCH, NEDD4, NEDD4L, PPIL2, PRPF19, PIAS1, PIAS2, PIAS3, PIAS4, RANBP2, RNF4, RBX1, SMURF1, SMURF2, STUB1, TOPORS, TRIP12, UBE3A, UBE3B, UBE3C, UBE4A, UBE4B, UBOX5, UBR5, WWP1, WWP2, Parkin, A20/TNFAIP3, AMFR/gp78, ARA54, β-TrCP1/BTRC, BRCA1, CBL, CHIP/STUB1, E6, E6AP/UBE3A, F-box protein 15/FBXO15, FBXW7/Cdc4, GRAIL/RNF128, HOIP/RNF31, cIAP-1/HIAP-2, cIAP-2/HIAP-1, cIAP(pan), ITCH/AIP4, KAP1, MARCH8, MindBomb1/MIB1, MindBomb2/MIB2, MuRF1/TRIM63, NDFIP1, NEDD4, NleL, Parkin, RNF2, RNF4, RNF8, RNF168, RNF43, SART1, Skp2, SMURF2, TRAF-1, TRAF-2, TRAF-3, TRAF-4, TRAF-5, TRAF-6, TRIM5, TRIM21, TRIM32, UBR5, or ZNRF3;
further, the B is a group that binds to an E3 ligase selected from VHL, Cereblon, MDM2 or cIAP.

8. The compound of any one of claims 1-7, wherein the B is of a general formula shown below: 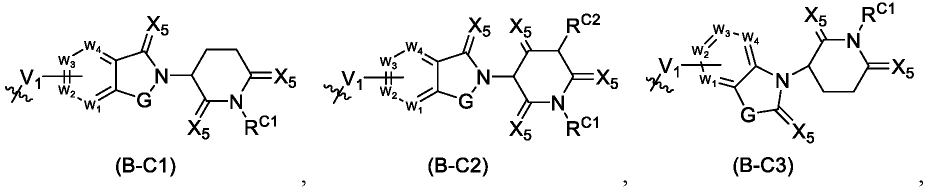 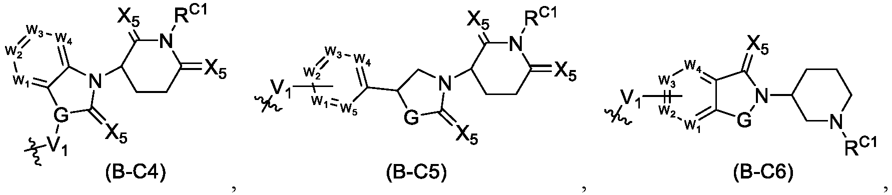 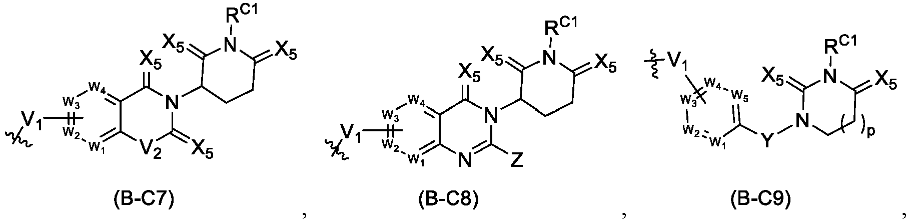 wherein:
G is each independently selected from CR^{C2}R^{C3}, NR^{C2}, CO, or SO₂;
Y is selected from a bond, or NH;
p is selected from 0, 1, or 2;
W₁, W₂, W₃, W₄, and W₅ are each independently selected from N or CR^{C4};
X₅ is each independently selected from O or S;
V₁ is each independently absent or selected from NH, O, S, SO, SO₂, SO₂NR^{C2}, CO, CO₂, C(O)NR^{C2}, C(S)NR^{C2}, NR^{C2}, NR^{C2}CO, NR^{C2}CONR^{C3}, -C₁₋₈ alkyl, -C₂₋₈ alkenyl, -C₂₋₈ alkynyl, cycloalkyl, heterocyclyl, aryl, or heteroaryl, wherein the -C₁₋₈ alkyl, -C₂₋₈ alkenyl, -C₂₋₈ alkynyl, cycloalkyl, heterocyclyl, aryl, or heteroaryl is each optionally substituted with R^{C4}; preferably, V₁ is absent, -O-, -CH₂-, -CH = CH-, or-NH-;
V₂ is each independently selected from CR^{C2}R^{C3}, NR^{C2}, O, or S;
Z is each independently selected from hydrogen, halogen, hydroxyl, amino, -C₁₋₈ alkyl, - C₂₋₈ alkenyl, -C₂₋₈ alkynyl, cycloalkyl, heterocyclyl, aryl, or heteroaryl, wherein the -C₁₋₈ alkyl, -C₂₋₈ alkenyl, -C₂₋₈ alkynyl, cycloalkyl, heterocyclyl, aryl, or heteroaryl is each optionally substituted with R^{C5};
R^{C1}, R^{C2}, R^{C3}, R^{C4}, and R^{C5} are selected from hydrogen, carboxy, cyano, nitro, a halogen atom, -C₁₋₈ alkyl, -C₂₋₈ alkenyl, -C₂₋₈ alkynyl, cycloalkyl, heterocyclyl, aryl, heteroaryl, -OR^{C6}, -SO₂R^{C6}, -SO₂NR^{C6}R^{C7}, -COR^{C6}, -CO₂R^{C6}, -CONR^{C6}R^{C7}, -POR^{C6}R^{C7}, -NR^{C6}R^{C7}, -NR^{C6}COR^{C7}, -NR^{C6}CONR^{C7}R^{C8}, -NR^{C6}CO₂R^{C7}, -NR^{C6}SO₂NR^{C7}R^{C8}, or -NR^{C6}SO₂R^{C7}, wherein the -C₁₋₈ alkyl, -C₂₋₈ alkenyl, -C₂₋₈ alkynyl, cycloalkyl, heterocyclyl, aryl, or heteroaryl is each optionally substituted with at least one substituent R^{C9};
R^{C6}, R^{C7}, R^{C8} and R^{C9} are selected from hydrogen, halogen, hydroxyl, amino, -C₁₋₈ alkyl, -C₂₋₈ alkenyl, -C₂₋₈ alkynyl, cycloalkyl, heterocyclyl, aryl, or heteroaryl;
preferably, the B is selected from: 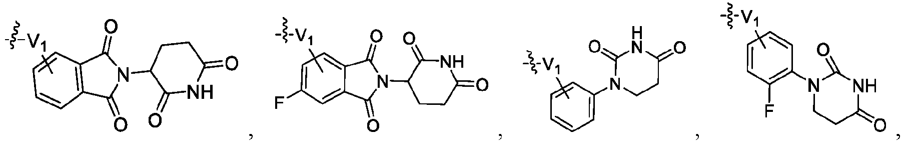 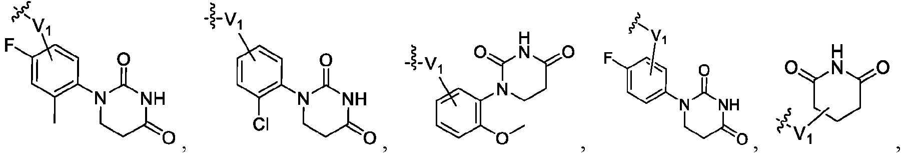 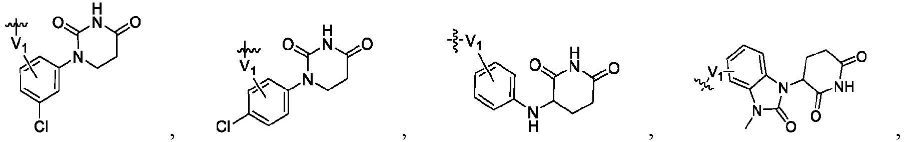 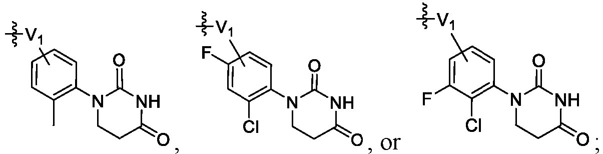 and
V₁ is absent, or is selected from -O-, -CH₂-, -CH=CH- or -NH-;
more preferably, the B is selected from: 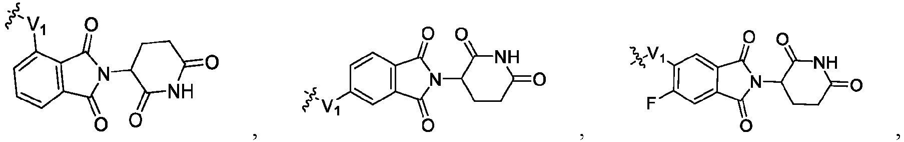 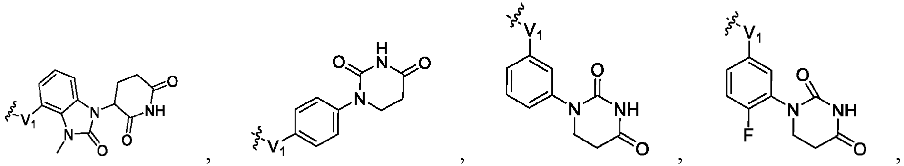 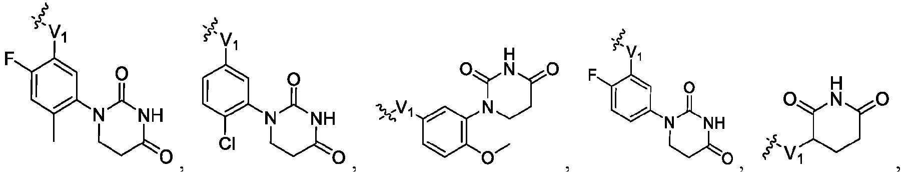 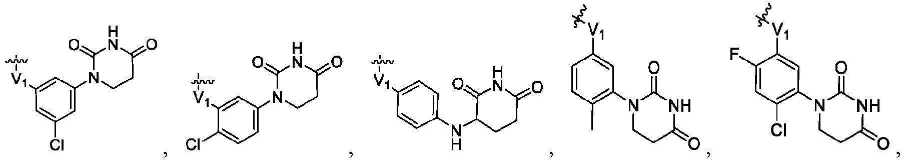 or 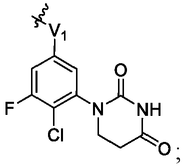 and
V1 is absent or selected from -O-, -CH₂-, or -NH-, preferably absent.

9. The compound of any one of claims 1-8, wherein the B is selected from: 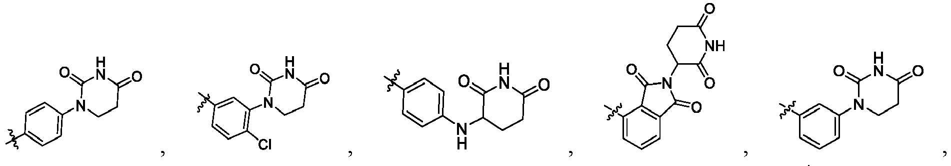 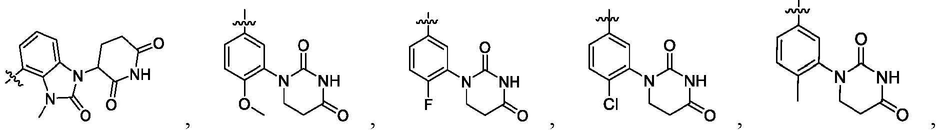 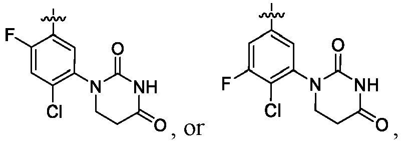 preferably

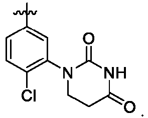

10. The compound of any one of claims 1-9, wherein the L is (LNK)ᵤ,
each LNK is independently absent, or is selected from C₁₋₈ alkylene, C₂₋₈ alkynylene, 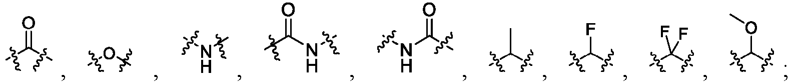 or cycloalkyl, heterocyclyl, a heteroaryl ring, or aryl, which is optionally substituted with one or more halogen or C₁₋₈ alkyl, wherein the cycloalkyl is preferably selected from 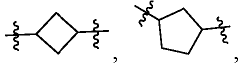 or 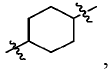 wherein the heterocyclyl is preferably selected from 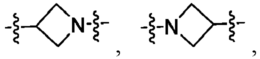 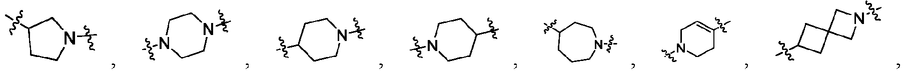 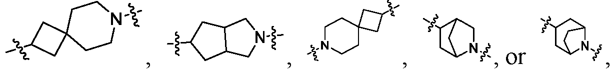 and wherein the aryl is preferably selected from a benzene ring;
m is selected from an integer between 1 and 8; and
u is an integer between 1 and 20.

11. The compound of any one of claims 1-9, wherein the L is (LNK)ᵤ,
each LNK is independently absent, or is selected from C₁₋₈ alkylene, C₂₋₈ alkynylene, 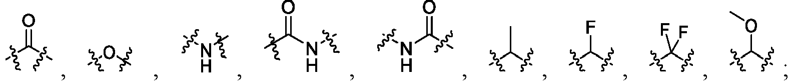 or cycloalkyl, heterocycloalkyl, a heteroaryl ring or an aryl ring, which is optionally substituted with one or more halogen or C₁₋₈ alkyl, wherein the cycloalkyl is selected from 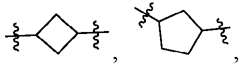 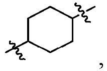 wherein the heterocycloalkyl is selected from 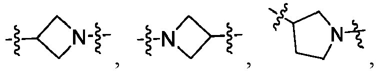 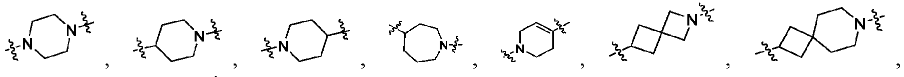 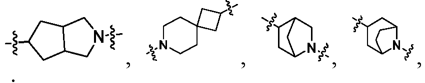 and wherein the aryl ring is selected from a benzene ring;
m is selected from an integer between 1 and 8; and
u is an integer between 1 and 20.

12. The compound of any one of claims 1-11, wherein the L is selected from: 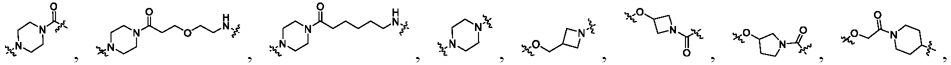 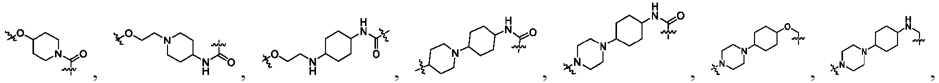 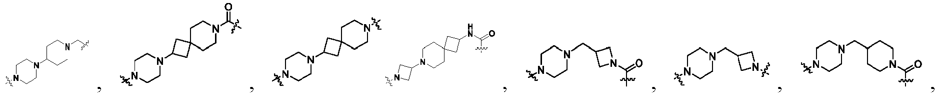 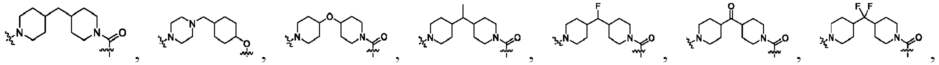 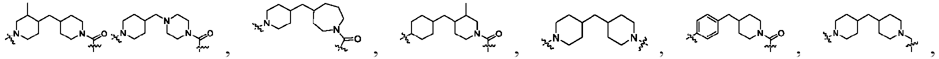 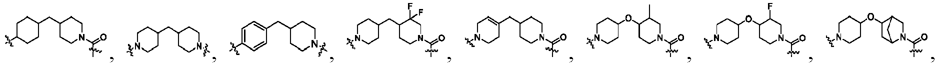 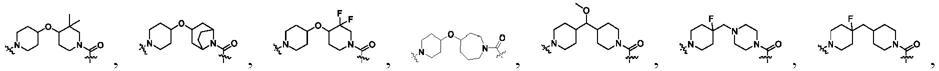 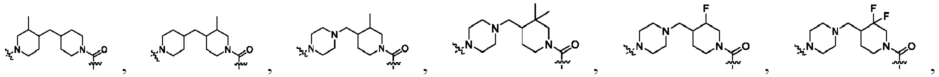 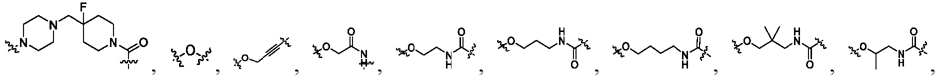 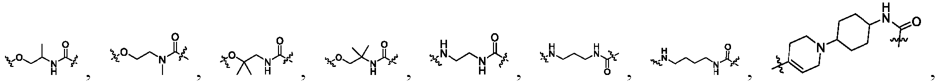 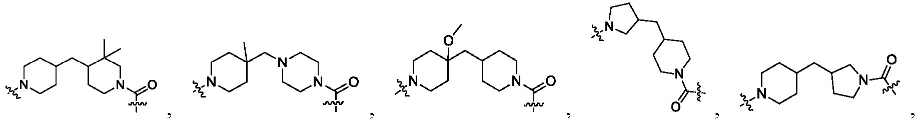 or 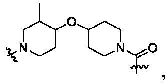 preferably

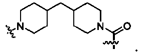

13. The compound of claim 2, wherein:
W is N; 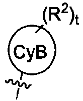 is 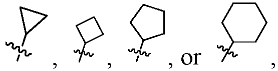 preferably 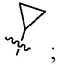
R¹, R³, and R⁴ are each hydrogen;
CyD is pyridyl, preferably 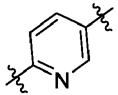 or 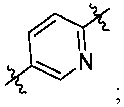
L is 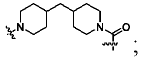
B is 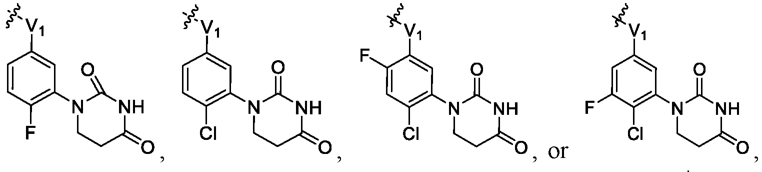 and V₁ is absent, -O-, -CH₂-, or -NH-; preferably 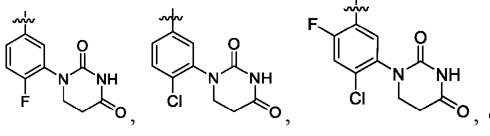 or 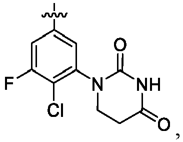 more preferably 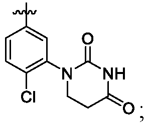 and
n is 1.

14. The compound of claim 2, wherein:
W is N;
R¹ is selected from hydrogen or -C₁₋₃ alkyl, preferably hydrogen;
R³ is selected from hydrogen or -C₁₋₃ alkyl, preferably hydrogen;
s is 0 or 1;
R^{X} is selected from methyl or ethyl, wherein the methyl or ethyl is optionally substituted with 1, 2, or 3 substituents independently selected from deuterium, F, and -OCH₃; preferably selected from methyl, -CD₃, ethyl, -CH₂F, -CHF₂, -CF₃, -CH₂CH₂F, -CH₂CHF₂, -CH₂CF₃, or 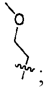 more preferably selected from methyl, -CD₃, ethyl, -CH₂CH₂F, -CH₂CHF₂, -CH₂CF₃, or 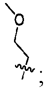
CyD is selected from pyridyl, preferably 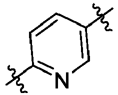 or 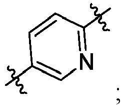
R⁴ is hydrogen or -C₁₋₃ alkyl, preferably hydrogen;
m is 0 or 1;
L is 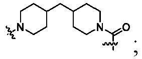
B is 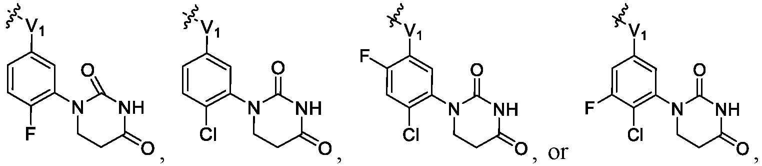 and V₁ is absent, -O-, -CH₂-, or -NH-; preferably 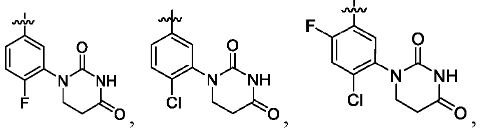 or 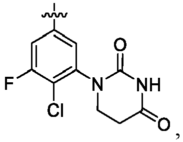 more preferably 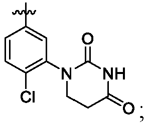 and
n is 1.

15. The compound of claim 1 selected from the compounds listed in Table 2 of the description, or a pharmaceutically acceptable salt, or a stereoisomer thereof.

16. A pharmaceutical composition comprising the compound of any one of claims 1-15, or a pharmaceutically acceptable salt, or a stereoisomer thereof, and a pharmaceutically acceptable excipient.

17. A pharmaceutical composition comprising the compound of any one of claims 1-15, or a pharmaceutically acceptable salt, or a stereoisomer thereof, and one or more therapeutically active ingredients.

18. Use of a pharmaceutical compound of any one of claims 1-15, or a pharmaceutically acceptable salt, or a stereoisomer thereof, or the composition of any one of claims 16-17, in the manufacture of a medicament for the treatment and/or prophylaxis of an HPK1-mediated disease and a related disease, wherein the HPK1-mediated disease and related disease are preferably selected from lung cancer, squamous epithelial cell carcinoma, bladder cancer, stomach cancer, ovarian cancer, peritoneal cancer, breast cancer, breast ductal carcinoma, head and neck cancer, endometrial carcinoma, carcinoma of corpus uteri, rectal cancer, liver cancer, kidney cancer, renal pelvis carcinoma, esophageal carcinoma, esophageal adenocarcinoma, glioma, prostate cancer, thyroid cancer, cancers of the female reproductive system, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin's lymphoma, villous adenoma of the large intestine, melanoma, cytoma and sarcoma, and myelodysplastic syndrome.

19. A method for the prophylaxis and/or treatment of an HPK1-mediated disease and a related disease, comprising administering to a subject a therapeutically effective amount of the compound of any one of claims 1-15, or a pharmaceutically acceptable salt thereof, or a stereoisomer thereof, or the pharmaceutical composition of any one of claims 16-17, wherein the HPK1-mediated disease and related disease are preferably selected from lung cancer, squamous epithelial cell carcinoma, bladder cancer, stomach cancer, ovarian cancer, peritoneal cancer, breast cancer, breast ductal carcinoma, head and neck cancer, endometrial carcinoma, carcinoma of corpus uteri, rectal cancer, liver cancer, kidney cancer, renal pelvis carcinoma, esophageal carcinoma, esophageal adenocarcinoma, glioma, prostate cancer, thyroid cancer, cancers of the female reproductive system, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin's lymphoma, villous adenoma of the large intestine, melanoma, cytoma and sarcoma, and myelodysplastic syndrome.
